# EUROPEAN PATENT APPLICATION

(11) **EP 4 825 382 A1**
(43) Date of publication of application: **21.10.2026**
(21) Application number: 25171360.8
(22) Date of filing: 17.04.2025
(51) Int. Cl.: A61K 31/7088, A61K 38/21, A61K 39/395, A61K 40/31, A61K 40/32, A61N 5/10, A61P 35/00

(54) **A METHOD FOR THE TREATMENT OF A TUMOR**

(71) Applicant: TME Pharma AG, 10589 Berlin (DE)
(72) Inventor: The designation of the inventor has not yet been filed
(74) Representative: Bohmann, Armin K.

(57) **Abstract**

The present invention is related to a CXCL12 antagonist and an interferon for use in a method for treating a tumor in a subject, wherein the method comprises administering to the subject the CXCL12 antagonist and the interferon, wherein the tumor is an immunologically cold tumor or a tumor refractory to an immunotherapy, wherein preferably the tumor refractory to an immunotherapy is refractory to a checkpoint inhibitor.

## Description

The present invention is related to a CXCL12 antagonist for use in a method for treating a tumor in a subject, wherein the tumor is an immunologically cold tumor; an interferon for use in a method for treating a tumor in a subject, wherein the tumor is an immunologically cold tumor; a CXCL12 antagonist for use in a method for treating a tumor in a subject, wherein the tumor is refractory to immunotherapy; an interferon for use in a method for treating a tumor in a subject, wherein the tumor is refractory to immunotherapy; a CXCL12 antagonist for use in a method for treating a tumor in a subject, wherein the tumor is a solid tumor; an interferon for use in a method for treating a tumor in a subject, wherein the tumor is a solid tumor; a CXCL12 antagonist for use in a method for treating a tumor in a subject, wherein the method comprises administering to the subject the CXCL12 antagonist, and an adjuvant therapy, wherein the tumor is an immunologically cold tumor; an interferon for use in a method for treating a tumor in a subject, wherein the method comprises administering to the subject the interferon, and an adjuvant therapy, wherein the tumor is an immunologically cold tumor; a CXCL12 antagonist for use in a method for treating a tumor in a subject, wherein the method comprises administering to the subject the CXCL12 antagonist, and an adjuvant therapy, wherein the tumor is refractory to immunotherapy; an interferon for use in a method for treating a tumor in a subject, wherein the method comprises administering to the subject the interferon, and an adjuvant therapy, wherein the tumor is refractory to immunotherapy; a CXCL12 antagonist for use in a method for treating a tumor in a subject, wherein the method comprises administering to the subject the CXCL12 antagonist, and an adjuvant therapy, wherein the tumor is a solid tumor; an interferon for use in a method for treating a tumor in a subject, wherein the method comprises administering to the subject the interferon, and an adjuvant therapy, wherein the tumor is a solid tumor; a CXCL12 antagonist for use in a method for treating a tumor in a subject, wherein the method comprises administering to the subject a primary therapy and an adjunct therapy, wherein the adjunct therapy comprises administering to the subject the CXCL12 antagonist, wherein the tumor is an immunologically cold tumor; an interferon for use in a method for treating a tumor in a subject, wherein the method comprises administering to the subject a primary therapy and an adjunct therapy, wherein the adjunct therapy comprises administering to the subject the interferon, wherein the tumor is an immunologically cold tumor; a CXCL12 antagonist for use in a method for treating a tumor in a subject, wherein the method comprises administering to the subject a primary therapy and an adjunct therapy, wherein the adjunct therapy comprises administering to the subject the CXCL12 antagonist, wherein the tumor is refractory to immunotherapy; an interferon for use in a method for treating a tumor in a subject, wherein the method comprises administering to the subject a primary therapy and an adjunct therapy, wherein the adjunct therapy comprises administering to the subject the interferon, wherein the tumor is refractory to immunotherapy; a CXCL12 antagonist for use in a method for treating a tumor in a subject, wherein the method comprises administering to the subject a primary therapy and an adjunct therapy, wherein the adjunct therapy comprises administering to the subject the CXCL12 antagonist, wherein the tumor is a solid tumor; an interferon for use in a method for treating a tumor in a subject, wherein the method comprises administering to the subject a primary therapy and an adjunct therapy, wherein the adjunct therapy comprises administering to the subject the interferon, wherein the tumor is a solid tumor; a CXCL12 antagonist for use in a method for converting an immunologically cold tumor in a subject into an immunologically hot tumor; an interferon for use in a method for converting an immunologically cold tumor in a subject into an immunologically hot tumor in the subject; a CXCL12 antagonist for use in a method for converting an immunotherapy refractory tumor in a subject into an immunotherapy non-refractory tumor; an interferon for use in a method for converting an immunotherapy refractory tumor in a subject into an immunotherapy non-refractory tumor; and to such methods.

Cancer morbidity and mortality have continued to rise significantly over the past few years. In 2020, more than 19 million new malignant tumor patients were diagnosed worldwide, with 10 million cancer-related deaths. The cancer incidence rate increased significantly compared with 2018. In many countries, malignant tumors have become the leading cause of death (Sung, Ferlay et al. 2021). With the development of society and technological advancements, precision medicine has gradually replaced conventional radiotherapy (RT) and surgical treatment of malignant tumors (Mimezami, Nicholson et al. 2012, Garraway, Verweij et al. 2013, Collins and Varmus 2015, Rubin 2015, Tsimberidou, Fountzilas et al. 2020). Precision cancer therapy is currently the most promising cancer treatment, including targeted therapy and immunotherapy (Janiaud, Serghiou et al. 2019).

Immunotherapy is a therapeutic modality that aims to regulate and eradicate tumors by reactivating and sustaining the antitumor immune cycle, as well as reinstating the body's innate antitumor immune response. It has become the fifth pillar of cancer treatment and mainly includes immune checkpoint inhibitors (ICIs) (Duan, Cui et al. 2020), adoptive cell transfer therapy (Newick, O'Brien et al. 2017, Ma, Li et al. 2019, Lin, Cheng et al. 2021), tumor-specific vaccines, cytokines (CKs), and small-molecule immunological drugs. Of these, ICIs are currently the most widely used drugs in cancer immunotherapy. Immunotherapy for tumors has been used for over 100 years, but the efficacy of immunotherapy varies even for patients with the same cancer type (Balkwill and Mantovani 2001).

CXCL12 expressed by healthy endothelial cells or stromal fibroblasts has long been understood to be a chemokine that potently attracts CXCR4-expressing lymphocytes in the settings of infection and thymic development (Bleul, Farzan et al. 1996, Janas, Varano et al. 2010, Trampont, Tosello-Trampont et al. 2010). **In** cancer, CXCL12 predominantly expressed by many tumors as well as associated fibroblasts, may play a variety of important roles in cancer progression as well as in the tumor microenvironment (Guo, Wang et al. 2016). Its cognate receptor, CXCR4, is broadly expressed by lymphocytes as well as other immune cells, stromal cells, myeloid cells and even neoplastic cells in some cases (Guyon 2014, Yang, Kumar et al. 2018). **In** some cancers where CXCL12 and CXCR4 are abnormally expressed, they have been seen directly to mediate tumor proliferation and inhibit apoptosis (Machelon, Gaudin et al. 2011). **In** others, CXCL12/CXCR4 signaling has been implicated in metastasis. In models where immune check point inhibitors are applied to activate tumor infiltrating lymphocytes (TIL), high expression of CXCL12 by fibroblasts can thwart these therapies by shielding nests of cancers cells from the TIL without excluding TIL from the TME broadly. Inhibition of CXCL12/CXCR4 signaling prompted deep infiltration of T cells in the TME in these settings, which was interpreted to indicate that the inhibitors were more efficacious against secreted CXCL12 than against CXCL12 on the cell surface (Feig, Jones et al. 2013, Fearon 2014, Zboralski, Hoehlig et al. 2017). CXCR4 has been reported to be expressed in SS cells and examined as a potential target to slow tumor growth (Pazzaglia, Pollino et al. 2019).

A problem underlying the present invention is the provision of means and a method for treating a tumor in a subject, wherein the tumor is an immunologically cold tumor.

A further problem underlying the present invention is the provision of means and a method for treating a tumor in a subject, wherein the tumor is refractory to immunotherapy.

Another problem underlying the present invention is the provision of means and a method for treating a tumor in a subject, wherein the tumor is a solid tumor.

A problem underlying the present invention is the provision of means and a method for treating a tumor in a subject, wherein the method for treating a tumor comprises an adjuvant therapy and the tumor is an immunologically cold tumor.

A further problem underlying the present invention is the provision of means and a method for treating a tumor in a subject, wherein the method for treating a tumor comprises an adjuvant therapy and the tumor is refractory to immunotherapy.

Another problem underlying the present invention is the provision of means and a method for treating a tumor in a subject, wherein the method for treating a tumor comprises an adjuvant therapy and the tumor is a solid tumor.

A problem underlying the present invention is the provision of means and a method for treating a tumor in a subject, wherein the method for treating a tumor comprises administering to the subject a primary therapy and an adjunct therapy, wherein the tumor is an immunologically cold tumor.

A further problem underlying the present invention is the provision of means and a method for treating a tumor in a subject, wherein the method for treating a tumor comprises administering to the subject a primary therapy and an adjunct therapy, wherein the tumor is refractory to immunotherapy.

Another problem underlying the present invention is the provision of means and a method for treating a tumor in a subject, wherein the method for treating a tumor comprises administering to the subject a primary therapy and an adjunct therapy, wherein the tumor is a solid tumor.

A problem underlying the present invention is the provision of means and a method for converting an immunologically cold tumor in a subject into an immunologically hot tumor.

Another problem underlying the present invention is the provision of means and a method for converting an immunotherapy refractory tumor in a subject into an immunotherapy non-refractory tumor.

These and other problems underlying the present invention are solved by the subject matter of the attached independent claims. Preferred embodiments may be taken from the attached dependent claims.

Similarly, these and other problems underlying the present invention are solved by the subject matter of the Embodiments disclosed in the following. Additionally, these and other problems underlying the present invention are solved by the subject matter of the various aspects of the present invention, including any embodiment thereof, as disclosed herein.

Embodiment 1. A CXCL12 antagonist for use in a method for treating a tumor in a subject, wherein the method comprises administering to the subject the CXCL12 antagonist and an interferon, wherein the tumor is an immunologically cold tumor.

Embodiment 2. An interferon for use in a method for treating a tumor in a subject, wherein the method comprises administering to the subject the interferon and a CXCL12 antagonist, wherein the tumor is an immunologically cold tumor.

Embodiment 3. The CXCL12 antagonist for use of Embodiment 1 and the interferon for use of Embodiment 2, wherein the tumor is refractory to an immunotherapy, preferably the immunotherapy is selected from the group comprising a checkpoint inhibitor, an adoptive cell therapy and a tumor vaccine.

Embodiment 4. The CXCL12 antagonist for use of Embodiment 3 and the interferon for use of Embodiment 3, wherein the checkpoint inhibitor is a PD-1 inhibitor, a PD-L1 inhibitor, a CTLA-4 inhibitor, a LAG-3 inhibitor, a TIM-3 inhibitor or a TIGIT inhibitor.

Embodiment 5. The CXCL12 antagonist for use of Embodiment 4 and the interferon for use of Embodiment 4, wherein the PD-1 inhibitor is selected from the group comprising pembrolizumab, nivolumab, cemiplimab, toripalimab, tislelizumab, dostarlimab, retfanlimab, sintilimab, camrelizumab, and balstilimab; wherein the PD-L1 inhibitor is selected from the group comprising atezolizumab, avelumab, durvalumab, cosibelimab, envafolimab, CS1001, PF-06801591; wherein the CTLA-4 inhibitor is selected from the group comprising ipilimumab, tremelimumab, BMS-986218, and zalifrelimab; wherein the LAG-3 inhibitor is selected from the group comprising relatlimab and GSK2831781; wherein the TIGIT inhibitor is selected from the group comprising vibostolimab, domvanalimab, tiragolumab and etigilimab; and wherein the TIM-3 inhibitor is selected from the group comprising sabatolimab, BMS-986258, and cobolimab.

Embodiment 6. The CXCL12 antagonist for use of any one of Embodiments 1 and 3 to 5 and the interferon for use of any one of Embodiments 2 to 5, wherein the tumor has at least one characteristic selected from the group comprising reduced or lacking expression of PD-L1, reduced number of tumor-infiltrating lymphocytes, genomic stability and reduced or lacking preexisting antitumor immune response.

Embodiment 7. The CXCL12 antagonist for use of Embodiment 6 and the interferon for use of Embodiment 6, wherein the tumor-infiltrating lymphocytes are CD3⁺ lymphocytes.

Embodiment 8. The CXCL12 antagonist for use of any one of Embodiments 1 and 3 to 7 and the interferon for use of any one of Embodiments 2 to 7, wherein the tumor has an immune evasion mechanism.

Embodiment 9. The CXCL12 antagonist for use of Embodiment 8 and the interferon for use of Embodiment 8, wherein the tumor is an immune-excluded tumor.

Embodiment 10. The CXCL12 antagonist for use of Embodiment 9 and the interferon for use of Embodiment 9, wherein immune exclusion can be reverted or mitigated by a checkpoint inhibitor.

Embodiment The CXCL12 antagonist for use of Embodiment 10 and the interferon for use of Embodiment 10, wherein the checkpoint inhibitor is a PD-1 inhibitor, a PD-L1 inhibitor, a CTLA-4 inhibitor, a LAG-3 inhibitor, a TIM-3 inhibitor or a TIGIT inhibitor.

Embodiment 12. The CXCL12 antagonist for use of Embodiment 11 and the interferon for use of Embodiment 11, wherein the PD-1 inhibitor is selected from the group comprising pembrolizumab, nivolumab, cemiplimab, toripalimab, tislelizumab, dostarlimab, retfanlimab, sintilimab, camrelizumab, and balstilimab; wherein the PD-L1 inhibitor is selected from the group comprising atezolizumab, avelumab, durvalumab, cosibelimab, envafolimab, CS1001, PF-06801591; wherein the CTLA-4 inhibitor is selected from the group comprising ipilimumab, tremelimumab, BMS-986218, and zalifrelimab; wherein the LAG-3 inhibitor is selected from the group comprising relatlimab and GSK2831781; wherein the TIGIT inhibitor is selected from the group comprising vibostolimab, domvanalimab, tiragolumab and etigilimab; and wherein the TIM-3 inhibitor is selected from the group comprising sabatolimab, BMS-986258, and cobolimab.

Embodiment 13. The CXCL12 antagonist for use of Embodiment 8 and the interferon for use of Embodiment 8, wherein the tumor is an immune-desert tumor.

Embodiment 14. The CXCL12 antagonist for use of any one of Embodiments 8 to 13 and the interferon for use of any one of Embodiments 8 to 13, wherein the immune evasion mechanism is one selected from the group comprising loss of MHC class I expression of malignant cells of the tumor and elevated expression of immune checkpoints by malignant and non-malignant cells of the tumor.

Embodiment 15. The CXCL12 antagonist for use of any one of Embodiments 8 to 14 and the interferon for use of any one of Embodiments 8 to 14, wherein the immune evasion mechanism results from, is caused by and/or is associated with a changed immune cell composition of the tumor microenvironment (TME).

Embodiment 16. The CXCL12 antagonist for use of Embodiment 15 and the interferon for use of Embodiment 15, wherein the tumor is evading from the subject's adaptive immune system.

Embodiment 17. The CXCL12 antagonist for use of Embodiment 16 and the interferon for use of Embodiment 16, wherein the tumor is evading from the subject's adaptive immune system by inhibiting infiltration of cytotoxic lymphocytes.

Embodiment 18. The CXCL12 antagonist for use of any one of Embodiments 16 and 17 and the interferon for use of any one of Embodiments 16 and 17, wherein the tumor is evading from the subject's adaptive immune system by suppressing activity of cytotoxic tumor-infiltrating lymphocytes or inhibiting activation of cytotoxic tumor-infiltrating lymphocytes.

Embodiment 19. The CXCL12 antagonist for use of Embodiment 15 and the interferon for use of Embodiment 15, wherein the tumor is evading from the subject's innate immune system.

Embodiment 20. The CXCL12 antagonist for use of Embodiment 19 and the interferon for use of Embodiment 19, wherein the tumor is characterized by an immunosuppressive tumor microenvironment.

Embodiment 21. The CXCL12 antagonist for use of Embodiment 20 and the interferon for use of Embodiment 20, wherein the immunosuppressive tumor microenvironment results from, is caused by and/or is associated with presence of a cell selected from the group comprising a myeloid-derived suppressor cell (MDSC), a tumor-associated macrophage (TAM) a tumor-associated neutrophil (TAN), and/or a regulatory T cell (Treg).

Embodiment 22. The CXCL12 antagonist for use of Embodiment 21 and the interferon for use of Embodiment 21, wherein the cell is immunomodulatorily active.

Embodiment 23. The CXCL12 antagonist for use of Embodiment 22 and the interferon for use of Embodiment 22, wherein the immunomodulatory active cell is active by means of a secreted immunomodulator, metabolic reprogramming and/or regulatory cell-to-cell contact.

Embodiment 24. The CXCL12 antagonist for use of any one of Embodiments 1 and 3 to 23 and the interferon for use of any one of Embodiments 1 to 23, wherein the tumor is a solid tumor.

Embodiment 25. The CXCL12 antagonist for use of any one of Embodiments 1 and 3 to 24 and the interferon for use of any one of Embodiments 2 to 24, wherein the tumor is selected from the group comprising pancreatic cancer, preferably pancreatic ductal adenocarcinoma (PDAC); triple-negative breast cancer; prostate cancer; ovarian cancer; soft tissue sarcoma and osteosarcoma.

Embodiment 26. The CXCL12 antagonist for use of Embodiment 25 and the interferon for use of Embodiment 25, wherein the tumor is high-grade serous ovarian cancer.

Embodiment 27. The CXCL12 antagonist for use of Embodiment 25 and the interferon for use of Embodiment 25, wherein the tumor is advanced castration-resistant prostate cancer.

Embodiment 28. The CXCL12 antagonist for use of Embodiment 25 and the interferon for use of Embodiment 25, wherein the tumor is synovial sarcoma.

Embodiment 29. A CXCL12 antagonist for use in a method for treating a tumor in a subject, wherein the method comprises administering to the subject the CXCL12 antagonist and an interferon, wherein the **tumor** is refractory to immunotherapy.

Embodiment 30. An interferon for use in a method for treating a tumor in a subject, wherein the method comprises administering to the subject the interferon and a CXCL12 antagonist, wherein the tumor is refractory to immunotherapy.

Embodiment 31. The CXCL12 antagonist for use of Embodiment 29 and the interferon for use of Embodiment 30, wherein the tumor is refractory to an immunotherapy, preferably the immunotherapy is selected from the group comprising a checkpoint inhibitor, an adoptive cell therapy and a tumor vaccine.

Embodiment 32. The CXCL12 antagonist for use of Embodiment 31 and the interferon for use of Embodiment 31, wherein the checkpoint inhibitor is a PD-1 inhibitor, a PD-L1 inhibitor, a CTLA-4 inhibitor, a LAG-3 inhibitor, a TIM-3 inhibitor or a TIGIT inhibitor.

Embodiment 33. The CXCL12 antagonist for use of Embodiment 32 and the interferon for use of Embodiment 32, wherein the PD-1 inhibitor is selected from the group comprising pembrolizumab, nivolumab, cemiplimab, toripalimab, tislelizumab, dostarlimab, retfanlimab, sintilimab, camrelizumab, and balstilimab; wherein the PD-L1 inhibitor is selected from the group comprising atezolizumab, avelumab, durvalumab, cosibelimab, envafolimab, CS1001, PF-06801591; wherein the CTLA-4 inhibitor is selected from the group comprising ipilimumab, tremelimumab, BMS-986218, and zalifrelimab; wherein the LAG-3 inhibitor is selected from the group comprising relatlimab and GSK2831781; wherein the TIGIT inhibitor is selected from the group comprising vibostolimab, domvanalimab, tiragolumab and etigilimab; and wherein the TIM-3 inhibitor is selected from the group comprising sabatolimab, BMS-986258, and cobolimab..

Embodiment 34. The CXCL12 antagonist for use of any one of Embodiments 29 and 31 to 33 and the interferon for use of any one of Embodiments 30 to 33, wherein the tumor has at least one characteristic selected from the group comprising reduced or lacking expression of PD-L1, reduced number of tumor-infiltrating lymphocytes, genomic stability and reduced or lacking preexisting antitumor immune response.

Embodiment 35. The CXCL12 antagonist for use of Embodiment 34 and the interferon for use of Embodiment 34, wherein the tumor-infiltrating lymphocytes are CD3⁺ lymphocytes.

Embodiment 36. The CXCL12 antagonist for use of any one of Embodiments 29 and 31 to 35 and the interferon for use of any one of Embodiments 30 to 35, wherein the tumor has an immune evasion mechanism.

Embodiment 37. The CXCL12 antagonist for use of Embodiment 36 and the interferon for use of Embodiment 35, wherein the tumor is an immune-excluded tumor.

Embodiment 38. The CXCL12 antagonist for use of Embodiment 37 and the interferon for use of Embodiment 37, wherein immune exclusion can be reverted or mitigated by a checkpoint inhibitor.

Embodiment 39. The CXCL12 antagonist for use of Embodiment 38 and the interferon for use of Embodiment 38, wherein the checkpoint inhibitor is a PD-1 inhibitor, a PD-L1 inhibitor, a CTLA-4 inhibitor, a LAG-3 inhibitor, a TIM-3 inhibitor or a TIGIT inhibitor.

Embodiment 40. The CXCL12 antagonist for use of Embodiment 39 and the interferon for use of Embodiment 39, wherein the PD-1 inhibitor is selected from the group comprising pembrolizumab, nivolumab, cemiplimab, toripalimab, tislelizumab, dostarlimab, retfanlimab, sintilimab, camrelizumab, and balstilimab; wherein the PD-L1 inhibitor is selected from the group comprising atezolizumab, avelumab, durvalumab, cosibelimab, envafolimab, CS1001, PF-06801591; wherein the CTLA-4 inhibitor is selected from the group comprising ipilimumab, tremelimumab, BMS-986218, and zalifrelimab; wherein the LAG-3 inhibitor is selected from the group comprising relatlimab and GSK2831781; wherein the TIGIT inhibitor is selected from the group comprising vibostolimab, domvanalimab, tiragolumab and etigilimab; and wherein the TIM-3 inhibitor is selected from the group comprising sabatolimab, BMS-986258, and cobolimab.

Embodiment 41. The CXCL12 antagonist for use of Embodiment 36 and the interferon for use of Embodiment 36, wherein the tumor is an immune-desert tumor.

Embodiment 42. The CXCL12 antagonist for use of any one of Embodiments 36 to 41 and the interferon for use of any one of Embodiments 36 to 41, wherein the immune evasion mechanism is one selected from the group comprising loss of MHC class I expression of malignant cells of the tumor and elevated expression of immune checkpoints by malignant and non-malignant cells of the tumor.

Embodiment 43. The CXCL12 antagonist for use of any one of Embodiments 36 to 42 and the interferon for use of any one of Embodiments 36 to 42, wherein the immune evasion mechanism results from, is caused by and/or is associated with a changed immune cell composition of the tumor microenvironment (TME).

Embodiment 44. The CXCL12 antagonist for use of Embodiment 43 and the interferon for use of Embodiment 43, wherein the tumor evades from the subject's adaptive immune system.

Embodiment 45. The CXCL12 antagonist for use of Embodiment 44 and the interferon for use of Embodiment 44, wherein the tumor evades from the subject's adaptive immune system by inhibiting infiltration of cytotoxic lymphocytes.

Embodiment 46. The CXCL12 antagonist for use of any one of Embodiments 44 and 45 and the interferon for use of any one of Embodiments 44 and 45, wherein the tumor is evading from the subject's adaptive immune system by suppressing activity of cytotoxic tumor-infiltrating lymphocytes or inhibiting activation of cytotoxic tumor-infiltrating lymphocytes.

Embodiment 47. The CXCL12 antagonist for use of Embodiment 43 and the interferon for use of Embodiment 43, wherein the tumor is evading from the subject's innate immune system.

Embodiment 48. The CXCL12 antagonist for use of Embodiment 47 and the interferon for use of Embodiment 47, wherein the tumor is characterized by an immunosuppressive tumor microenvironment.

Embodiment 49. The CXCL12 antagonist for use of Embodiment 48 and the interferon for use of Embodiment 48, wherein the immunosuppressive tumor microenvironment results from, is caused by and/or is associated with presence of a cell selected from the group comprising a myeloid-derived suppressor cell (MDSC), a tumor-associated macrophage (TAM), a tumor-associated neutrophil (TAN) and/or a regulatory T cell (Tregs).

Embodiment 50. The CXCL12 antagonist for use of Embodiment 49 and the interferon for use of Embodiment 49, wherein the cell is immunomodulatorily active.

Embodiment 51. The CXCL12 antagonist for use of Embodiment 50 and the interferon for use of Embodiment 50, wherein the immunomodulatorily active cell is active by means of a secreted immunomodulator, metabolic reprogramming and/or regulatory cell-to-cell contact.

Embodiment 52. The CXCL12 antagonist for use of any one of Embodiments 29 and 31 to 51 and the interferon for use of any one of Embodiments 30 to 51, wherein the tumor is an immunologically cold tumor.

Embodiment 53. The CXCL12 antagonist for use of any one of Embodiments 29 and 31 to 52 and the interferon for use of any one of Embodiments 30 to 52, wherein the tumor is a solid tumor.

Embodiment 54. The CXCL12 antagonist for use of any one of Embodiments 29 and 31 to 53 and the interferon for use of any one of Embodiments 30 to 53, wherein the tumor is selected from the group comprising pancreatic cancer, preferably pancreatic ductal adenocarcinoma (PDAC); triple-negative breast cancer; prostate cancer; ovarian cancer; soft tissue sarcomas and osteosarcoma.

Embodiment 55. The CXCL12 antagonist for use of Embodiment 54 and the interferon for use of Embodiment 54, wherein the tumor is high-grade serous ovarian cancer.

Embodiment 56. The CXCL12 antagonist for use of Embodiment 54 and the interferon for use of Embodiment 54, wherein the tumor is advanced castration-resistant prostate cancer.

Embodiment 57. The CXCL12 antagonist for use of Embodiment 54 and the interferon for use of Embodiment 54, wherein the tumor is synovial sarcoma.

Embodiment 58. A CXCL12 antagonist for use in a method for treating a tumor in a subject, wherein the method comprises administering to the subject the CXCL12 antagonist and an interferon, wherein the tumor is a solid tumor.

Embodiment 59. An interferon for use in a method for treating a tumor in a subject, wherein the method comprises administering to the subject the interferon and a CXCL12 antagonist, wherein the tumor is a solid tumor.

Embodiment 60. The CXCL12 antagonist for use of Embodiment 58 and the interferon for use of Embodiment 59, wherein the tumor is selected from the group comprising pancreatic cancer , preferably pancreatic ductal adenocarcinoma (PDAC); triple-negative breast cancer; prostate cancer; ovarian cancer; soft tissue sarcomas and osteosarcoma.

Embodiment 61. The CXCL12 antagonist for use of Embodiment 60 and the interferon for use of Embodiment 60, wherein the tumor is high-grade serous ovarian cancer.

Embodiment 62. The CXCL12 antagonist for use of Embodiment 60 and the interferon for use of Embodiment 60, wherein the tumor is advanced castration-resistant prostate cancer.

Embodiment 63. The CXCL12 antagonist for use of Embodiment 60 and the interferon for use of Embodiment 60, wherein the tumor is synovial sarcoma.

Embodiment 64. The CXCL12 antagonist for use of Embodiment 58 and the interferon for use of Embodiment 59, wherein the tumor is refractory to an immunotherapy, preferably the immunotherapy is selected from the group comprising a checkpoint inhibitor, an adoptive cell therapy and a tumor vaccine.

Embodiment 65. The CXCL12 antagonist for use of any one of Embodiments 58 and 60 to 64 and the interferon for use of any one of Embodiments 50 to 64, wherein the checkpoint inhibitor is is a PD-1 inhibitor, a PD-L1 inhibitor, a CTLA-4 inhibitor, a LAG-3 inhibitor, a TIM-3 inhibitor or a TIGIT inhibitor.

Embodiment 66. The CXCL12 antagonist for use of Embodiment 65 and the interferon for use of Embodiment 65, wherein the PD-1 inhibitor is selected from the group comprising pembrolizumab, nivolumab, cemiplimab, toripalimab, tislelizumab, dostarlimab, retfanlimab, sintilimab, camrelizumab, and balstilimab; wherein the PD-L1 inhibitor is selected from the group comprising atezolizumab, avelumab, durvalumab, cosibelimab, envafolimab, CS1001, PF-06801591; wherein the CTLA-4 inhibitor is selected from the group comprising ipilimumab, tremelimumab, BMS-986218, and zalifrelimab; wherein the LAG-3 inhibitor is selected from the group comprising relatlimab and GSK2831781; wherein the TIGIT inhibitor is selected from the group comprising vibostolimab, domvanalimab, tiragolumab and etigilimab; and wherein the TIM-3 inhibitor is selected from the group comprising sabatolimab, BMS-986258, and cobolimab.

Embodiment 67. The CXCL12 antagonist for use of any one of Embodiments 58 and 60 to 66 and the interferon for use of any one of Embodiments 59 to 66, wherein the tumor has at least one characteristic selected from the group comprising reduced or lacking expression of PD-L1, reduced number of tumor-infiltrating lymphocytes, genomic stability and reduced or lacking preexisting antitumor immune response.

Embodiment 68. The CXCL12 antagonist for use of Embodiment 67 and the interferon for use of Embodiment 67, wherein the tumor-infiltrating lymphocytes are CD3⁺ lymphocytes.

Embodiment 69. The CXCL12 antagonist for use of any one of Embodiments 58 and 60 to 68 and the interferon for use of any one of Embodiments 59 to 68, wherein the tumor has an immune evasion mechanism.

Embodiment 70. The CXCL12 antagonist for use of Embodiment 69 and the interferon for use of Embodiment 69, wherein the tumor is an immune-excluded tumor.

Embodiment 71. The CXCL12 antagonist for use of Embodiment 70 and the interferon for use of Embodiment 69, wherein immune exclusion can be reverted or mitigated by an immune checkpoint inhibitor.

Embodiment 72. The CXCL12 antagonist for use of Embodiment 71 and the interferon for use of Embodiment 71, wherein the checkpoint inhibitor is a PD-1 inhibitor, a PD-L1 inhibitor, a CTLA-4 inhibitor, a LAG-3 inhibitor, a TIM-3 inhibitor or a TIGIT inhibitor.

Embodiment 73. The CXCL12 antagonist for use of Embodiment 72 and the interferon for use of Embodiment 72, wherein the PD-1 inhibitor is selected from the group comprising pembrolizumab, nivolumab, cemiplimab, toripalimab, tislelizumab, dostarlimab, retfanlimab, sintilimab, camrelizumab, and balstilimab; wherein the PD-L1 inhibitor is selected from the group comprising atezolizumab, avelumab, durvalumab, cosibelimab, envafolimab, CS1001, PF-06801591; wherein the CTLA-4 inhibitor is selected from the group comprising ipilimumab, tremelimumab, BMS-986218, and zalifrelimab; wherein the LAG-3 inhibitor is selected from the group comprising relatlimab and GSK2831781; wherein the TIGIT inhibitor is selected from the group comprising vibostolimab, domvanalimab, tiragolumab and etigilimab; and wherein the TIM-3 inhibitor is selected from the group comprising sabatolimab, BMS-986258, and cobolimab.

Embodiment 74. The CXCL12 antagonist for use of Embodiment 69 and the interferon for use of Embodiment 69, wherein the tumor is an immune-desert tumor.

Embodiment 75. The CXCL12 antagonist for use of any one of Embodiments 69 to 74 and the interferon for use of any one of Embodiments 69 to 74, wherein the immune evasion mechanism is one selected from the group comprising loss of MHC class I expression of malignant cells of the tumor and elevated expression of immune checkpoints by malignant and non-malignant cells of the tumor.

Embodiment 76. The CXCL12 antagonist for use of any one of Embodiments 69 to 75 and the interferon for use of any one of Embodiments 69 to 75, wherein the immune evasion mechanism results from, is caused by and/or is associated with a changed immune cell composition of the tumor microenvironment (TME).

Embodiment 77. The CXCL12 antagonist for use of Embodiment 76 and the interferon for use of Embodiment 76, wherein the tumor is evading from the subject's adaptive immune system.

Embodiment 78. The CXCL12 antagonist for use of Embodiment 77 and the interferon for use of Embodiment 77, wherein the tumor is evading from the subject's adaptive immune system by inhibiting infiltration of cytotoxic lymphocytes.

Embodiment 79. The CXCL12 antagonist for use of any one of Embodiments 77 and 78 and the interferon for use of any one of Embodiments 77 and 78, wherein the tumor is evading from the subject's adaptive immune system by suppressing activity of cytotoxic tumor-infiltrating lymphocytes or inhibiting activation of cytotoxic tumor-infiltrating lymphocytes.

Embodiment 80. The CXCL12 antagonist for use of Embodiment 76 and the interferon for use of Embodiment 76, wherein the tumor is evading from the subject's innate immune system.

Embodiment 81. The CXCL12 antagonist for use of Embodiment 80 and the interferon for use of Embodiment 80, wherein the tumor is characterized by an immunosuppressive tumor microenvironment.

Embodiment 82. The CXCL12 antagonist for use of Embodiment 81 and the interferon for use of Embodiment 81, wherein the immunosuppressive tumor microenvironment results from, is caused by and/or is associated with presence of a cell selected from the group comprising a myeloid-derived suppressor cell (MDSC), a tumor-associated macrophage (TAM), a tumor-associated neutrophil (TAN) and/or a regulatory T cell (Treg).

Embodiment 83. The CXCL12 antagonist for use of Embodiment 82 and the interferon for use of Embodiment 82, wherein the cell is immunomodulatorily active.

Embodiment 84. The CXCL12 antagonist for use of Embodiment 83 and the interferon for use of Embodiment 83, wherein the immunomodulatory active cell is active by means of a secreted immunomodulator, metabolic reprogramming and/or regulatory cell-to-cell contact.

Embodiment 85. A CXCL12 antagonist for use in a method for treating a tumor in a subject, wherein the method comprises administering to the subject the CXCL12 antagonist, an interferon and an adjuvant therapy, wherein the tumor is an immunologically cold tumor.

Embodiment 86. An interferon for use in a method for treating a tumor in a subject, wherein the method comprises administering to the subject the interferon, a CXCL12 antagonist and an adjuvant therapy, wherein the tumor is an immunologically cold tumor.

Embodiment 87. The CXCL12 antagonist for use of Embodiment 85 and the interferon for use of Embodiment 86, wherein the tumor is refractory to an immunotherapy, preferably the immunotherapy is selected from the group comprising a checkpoint inhibitor, an adoptive cell therapy and a tumor vaccine.

Embodiment 88. The CXCL12 antagonist for use of Embodiment 87 and the interferon for use of Embodiment 87, wherein the checkpoint inhibitor is a PD-1 inhibitor, a PD-L1 inhibitor, a CTLA-4 inhibitor, a LAG-3 inhibitor, a TIM-3 inhibitor or a TIGIT inhibitor.

Embodiment 89. The CXCL12 antagonist for use of Embodiment 88 and the interferon for use of Embodiment 88, wherein wherein the PD-1 inhibitor is selected from the group comprising pembrolizumab, nivolumab, cemiplimab, toripalimab, tislelizumab, dostarlimab, retfanlimab, sintilimab, camrelizumab, and balstilimab; wherein the PD-L1 inhibitor is selected from the group comprising atezolizumab, avelumab, durvalumab, cosibelimab, envafolimab, CS1001, PF-06801591; wherein the CTLA-4 inhibitor is selected from the group comprising ipilimumab, tremelimumab, BMS-986218, and zalifrelimab; wherein the LAG-3 inhibitor is selected from the group comprising relatlimab and GSK2831781; wherein the TIGIT inhibitor is selected from the group comprising vibostolimab, domvanalimab, tiragolumab and etigilimab; and wherein the TIM-3 inhibitor is selected from the group comprising sabatolimab, BMS-986258, and cobolimab.

Embodiment 90. The CXCL12 antagonist for use of any one of Embodiments 85 and 87 to 89 and the interferon for use of any one of Embodiments 86 to 89, wherein the tumor has at least one characteristic selected from the group comprising reduced or lacking expression of PD-L1, reduced number of tumor-infiltrating lymphocytes, genomic stability and reduced or lacking preexisting antitumor immune response.

Embodiment 91. The CXCL12 antagonist for use of Embodiment 90 and the interferon for use of Embodiment 90 wherein the tumor-infiltrating lymphocytes are CD3⁺ lymphocytes.

Embodiment 92. The CXCL12 antagonist for use of any one of Embodiments 85 and 87 to 91 and the interferon for use of any one of Embodiments 86 to 91, wherein the tumor has an immune evasion mechanism.

Embodiment 93. The CXCL12 antagonist for use of Embodiment 93 and the interferon for use of Embodiment 92, wherein the tumor is an immune-excluded tumor.

Embodiment 94. The CXCL12 antagonist for use of Embodiment 93 and the interferon for use of Embodiment 913 wherein immune exclusion can be reverted or mitigated by an immune checkpoint inhibitor.

Embodiment 95. The CXCL12 antagonist for use of Embodiment 94 and the interferon for use of Embodiment 94, wherein the checkpoint inhibitor is a PD-1 inhibitor, a PD-L1 inhibitor, a CTLA-4 inhibitor, a LAG-3 inhibitor, a TIM-3 inhibitor or a TIGIT inhibitor.

Embodiment 96. The CXCL12 antagonist for use of Embodiment 95 and the interferon for use of Embodiment 95, wherein the PD-1 inhibitor is selected from the group comprising pembrolizumab, nivolumab, cemiplimab, toripalimab, tislelizumab, dostarlimab, retfanlimab, sintilimab, camrelizumab, and balstilimab; wherein the PD-L1 inhibitor is selected from the group comprising atezolizumab, avelumab, durvalumab, cosibelimab, envafolimab, CS1001, PF-06801591; wherein the CTLA-4 inhibitor is selected from the group comprising ipilimumab, tremelimumab, BMS-986218, and zalifrelimab; wherein the LAG-3 inhibitor is selected from the group comprising relatlimab and GSK2831781; wherein the TIGIT inhibitor is selected from the group comprising vibostolimab, domvanalimab, tiragolumab and etigilimab; and wherein the TIM-3 inhibitor is selected from the group comprising sabatolimab, BMS-986258, and cobolimab.

Embodiment 97. The CXCL12 antagonist for use of Embodiment 92 and the interferon for use of Embodiment 92, wherein the tumor is an immune-desert tumor.

Embodiment 98. The CXCL12 antagonist for use of any one of Embodiments 92 to 97 and the interferon for use of any one of Embodiments 92 to 97, wherein the immune evasion mechanism is one selected from the group comprising loss of MHC class I expression of malignant cells of the tumor and elevated expression of immune checkpoints by malignant and non-malignant cells of the tumor.

Embodiment 99. The CXCL12 antagonist for use of any one of Embodiments 92 to 98 and the interferon for use of any one of Embodiments 92 to 98, wherein the immune evasion mechanism results from, is caused by and/or is associated with a changed immune cell composition of the tumor microenvironment (TME).

Embodiment 100. The CXCL12 antagonist for use of Embodiment 99 and the interferon for use of Embodiment 99, wherein the tumor evades from the subject's adaptive immune system.

Embodiment 101. The CXCL12 antagonist for use of Embodiment 100 and the interferon for use of Embodiment 100, wherein the tumor evades from the subject's adaptive immune system by inhibiting infiltration of cytotoxic lymphocytes.

Embodiment 102. The CXCL12 antagonist for use of any one of Embodiments 100 and 101 and the interferon for use of any one of Embodiments 100 and 101, wherein the tumor evades from the subject's adaptive immune system by suppressing activity of cytotoxic tumor-infiltrating lymphocytes or inhibiting activation of cytotoxic tumor-infiltrating lymphocytes.

Embodiment 103. The CXCL12 antagonist for use of Embodiment 99 and the interferon for use of Embodiment 95, wherein the tumor evades from the subject's innate immune system.

Embodiment 104. The CXCL12 antagonist for use of Embodiment 103 and the interferon for use of Embodiment 103, wherein the tumor is characterized by an immunosuppressive tumor microenvironment.

Embodiment 105. The CXCL12 antagonist for use of Embodiment 104 and the interferon for use of Embodiment 104, wherein the immunosuppressive tumor microenvironment results from, is caused by and/or is associated with presence of a cell selected from the group comprising a myeloid-derived suppressor cell (MDSC), a tumor-associated macrophage (TAM), a tumor-associated neutrophil (TAN) and/or a regulatory T cell (Treg).

Embodiment 106. The CXCL12 antagonist for use of Embodiment 105 and the interferon for use of Embodiment 105, wherein the cell is immunomodulatorily active.

Embodiment 107. The CXCL12 antagonist for use of Embodiment 106 and the interferon for use of Embodiment 106, wherein the immunomodulatorily active cell is active by means of a secreted immunomodulator, metabolic reprogramming and/or regulatory cell-to-cell contact.

Embodiment 108. The CXCL12 antagonist for use of any one of Embodiments 85 and 87 to 107 and the interferon for use of any one of Embodiments 86 to 107, wherein the tumor is a solid tumor.

Embodiment 109. The CXCL12 antagonist for use of any one of Embodiments 85 and 87 to 108 and the interferon for use of any one of Embodiments 86 to 108, wherein the tumor is selected from the group comprising pancreatic cancer preferably pancreatic ductal adenocarcinoma (PDAC); triple-negative breast cancer; prostate cancer; ovarian cancer; soft tissue sarcoma and osteosarcoma.

Embodiment 110. The CXCL12 antagonist for use of Embodiment 109 and the interferon for use of Embodiment 109, wherein the tumor is high-grade serous ovarian cancer.

Embodiment 111. The CXCL12 antagonist for use of Embodiment 110 and the interferon for use of Embodiment 110, wherein the tumor is advanced castration-resistant prostate cancer.

Embodiment 112. The CXCL12 antagonist for use of Embodiment 110 and the interferon for use of Embodiment 110, wherein the tumor is synovial sarcoma.

Embodiment 113. A CXCL12 antagonist for use in a method for treating a tumor in a subject, wherein the method comprises administering to the subject the CXCL12 antagonist, an interferon and adjuvant therapy, wherein the tumor is refractory to immunotherapy.

Embodiment 114. An interferon for use in a method for treating a tumor in a subject, wherein the method comprises administering to the subject the interferon, a CXCL12 antagonist and an adjuvant therapy, wherein the tumor is refractory to immunotherapy.

Embodiment 115. The CXCL12 antagonist for use of Embodiment 113 and the interferon for use of Embodiment 114, wherein the tumor is refractory to an immunotherapy, preferably the immunotherapy is selected from the group comprising a checkpoint inhibitor, an adoptive cell therapy and a tumor vaccine.

Embodiment 116. The CXCL12 antagonist for use of Embodiment 115 and the interferon for use of Embodiment 115, wherein the checkpoint inhibitor is a PD-1 inhibitor, a PD-L1 inhibitor, a CTLA-4 inhibitor, a LAG-3 inhibitor, a TIM-3 inhibitor or a TIGIT inhibitor.

Embodiment 117. The CXCL12 antagonist for use of Embodiment 116 and the interferon for use of Embodiment 116, wherein the PD-1 inhibitor is selected from the group comprising pembrolizumab, nivolumab, cemiplimab, toripalimab, tislelizumab, dostarlimab, retfanlimab, sintilimab, camrelizumab, and balstilimab; wherein the PD-L1 inhibitor is selected from the group comprising atezolizumab, avelumab, durvalumab, cosibelimab, envafolimab, CS1001, PF-06801591; wherein the CTLA-4 inhibitor is selected from the group comprising ipilimumab, tremelimumab, BMS-986218, and zalifrelimab; wherein the LAG-3 inhibitor is selected from the group comprising relatlimab and GSK2831781; wherein the TIGIT inhibitor is selected from the group comprising vibostolimab, domvanalimab, tiragolumab and etigilimab; and wherein the TIM-3 inhibitor is selected from the group comprising sabatolimab, BMS-986258, and cobolimab.

Embodiment 118. The CXCL12 antagonist for use of any one of Embodiments 113 and 115 to 117 and the interferon for use of any one of Embodiments 114 to 117, wherein the tumor has at least one characteristic selected from the group comprising reduced or lacking expression of PD-L1, reduced number of tumor-infiltrating lymphocytes, genomic stability and reduced or lacking preexisting antitumor immune response.

Embodiment 119. The CXCL12 antagonist for use of Embodiment 118 and the interferon for use of Embodiment 118, wherein the tumor-infiltrating lymphocytes are CD3⁺ lymphocytes.

Embodiment 120. The CXCL12 antagonist for use of any one of Embodiments 113 and 115 to 119 and the interferon for use of any one of Embodiments 114 to 119, wherein the tumor has an immune evasion mechanism.

Embodiment 121. The CXCL12 antagonist for use of Embodiment 120 and the interferon for use of Embodiment 120, wherein the tumor is an immune-excluded tumor.

Embodiment 122. The CXCL12 antagonist for use of Embodiment 121 and the interferon for use of Embodiment 121, wherein immune exclusion can be reverted or mitigated by an immune checkpoint inhibitor.

Embodiment 123. The CXCL12 antagonist for use of Embodiment 122 and the interferon for use of Embodiment 122, wherein the checkpoint inhibitor is a PD-1 inhibitor, a PD-L1 inhibitor, a CTLA-4 inhibitor, a LAG-3 inhibitor, a TIM-3 inhibitor or a TIGIT inhibitor.

Embodiment 124. The CXCL12 antagonist for use of Embodiment 123 and the interferon for use of Embodiment 123, wherein the PD-1 inhibitor is selected from the group comprising pembrolizumab, nivolumab, cemiplimab, toripalimab, tislelizumab, dostarlimab, retfanlimab, sintilimab, camrelizumab, and balstilimab; wherein the PD-L1 inhibitor is selected from the group comprising atezolizumab, avelumab, durvalumab, cosibelimab, envafolimab, CS1001, PF-06801591; wherein the CTLA-4 inhibitor is selected from the group comprising ipilimumab, tremelimumab, BMS-986218, and zalifrelimab; wherein the LAG-3 inhibitor is selected from the group comprising relatlimab and GSK2831781; wherein the TIGIT inhibitor is selected from the group comprising vibostolimab, domvanalimab, tiragolumab and etigilimab; and wherein the TIM-3 inhibitor is selected from the group comprising sabatolimab, BMS-986258, and cobolimab.

Embodiment 125. The CXCL12 antagonist for use of Embodiment 120 and the interferon for use of Embodiment 120, wherein the tumor is an immune-desert tumor.

Embodiment 126. The CXCL12 antagonist for use of any one of Embodiments 120 to 125 and the interferon for use of any one of Embodiments 120 to 125, wherein the immune evasion mechanism is one selected from the group comprising loss of MHC class I expression of malignant cells of the tumor and elevated expression of immune checkpoints by malignant and non-malignant cells of the tumor.

Embodiment 127. The CXCL12 antagonist for use of any one of Embodiments 120 to 126 and the interferon for use of any one of Embodiments 120 to 126, wherein the immune evasion mechanism results from, is caused by and/or is associated with a changed immune cell composition of the tumor microenvironment (TME).

Embodiment 128. The CXCL12 antagonist for use of Embodiment 127 and the interferon for use of Embodiment 127, wherein the tumor evades from the subject's adaptive immune system.

Embodiment 129. The CXCL12 antagonist for use of Embodiment 128 and the interferon for use of Embodiment 128, wherein the tumor evades from the subject's adaptive immune system by inhibiting infiltration of cytotoxic lymphocytes.

Embodiment 130. The CXCL12 antagonist for use of any one of Embodiments 128 and 129 and the interferon for use of any one of Embodiments 128 and 129, wherein the tumor is evading from the subject's adaptive immune system by suppressing activity of cytotoxic tumor-infiltrating lymphocytes or inhibiting activation of cytotoxic tumor-infiltrating lymphocytes.

Embodiment 131. The CXCL12 antagonist for use of Embodiment 127 and the interferon for use of Embodiment 127, wherein the tumor is evading from the subject's innate immune system.

Embodiment 132. The CXCL12 antagonist for use of Embodiment 131 and the interferon for use of Embodiment 131, wherein the tumor is characterized by an immunosuppressive tumor microenvironment.

Embodiment 133. The CXCL12 antagonist for use of Embodiment 132 and the interferon for use of Embodiment 132, wherein the immunosuppressive tumor microenvironment results from, is caused by and/or is associated with presence of a cell selected from the group comprising a myeloid-derived suppressor cell (MDSC), a tumor-associated macrophage (TAM), a tumor-associated neutrophil (TAN), and/or a regulatory T cell (Treg).

Embodiment 134. The CXCL12 antagonist for use of Embodiment 133 and the interferon for use of Embodiment 133, wherein the cell is immunomodulatorily active.

Embodiment 135. The CXCL12 antagonist for use of Embodiment 134 and the interferon for use of Embodiment 134, wherein the immunomodulatorily active cell is active by means of a secreted immunomodulator, metabolic reprogramming and/or regulatory cell-to-cell contact.

Embodiment 136. The CXCL12 antagonist for use of any one of Embodiments 113 and 115 to 135 and the interferon for use of any one of Embodiments 114 to 135, wherein the tumor is an immunologically cold tumor.

Embodiment 137. The CXCL12 antagonist for use of any one of Embodiments 113 and 115 to 136 and the interferon for use of any one of Embodiments 114 to 136, wherein the tumor is a solid tumor.

Embodiment 138. The CXCL12 antagonist for use of any one of Embodiments 113 and 115 to 137 and the interferon for use of any one of Embodiments 114 to 137, wherein the tumor is selected from the group comprising pancreatic cancer, preferably pancreatic ductal adenocarcinoma (PDAC); triple-negative breast cancer; prostate cancer; ovarian cancer; soft tissue sarcoma and osteosarcoma.

Embodiment 139. The CXCL12 antagonist for use of Embodiment 138 and the interferon for use of Embodiment 138, wherein the tumor is high-grade serous ovarian cancer.

Embodiment 140. The CXCL12 antagonist for use of Embodiment 138 and the interferon for use of Embodiment 138, wherein the tumor is advanced castration-resistant prostate cancer.

Embodiment 141. The CXCL12 antagonist for use of Embodiment 138 and the interferon for use of Embodiment 138, wherein the tumor is synovial sarcoma.

Embodiment 142. A CXCL12 antagonist for use in a method for treating a tumor in a subject, wherein the method comprises administering to the subject the CXCL12 antagonist, an interferon and an adjuvant therapy, wherein the tumor is a solid tumor.

Embodiment 143. An interferon for use in a method for treating a tumor in a subject, wherein the method comprises administering to the subject the interferon, a CXCL12 antagonist and an adjuvant therapy, wherein the tumor is a solid tumor.

Embodiment 144. The CXCL12 antagonist for use of Embodiment 142 and the interferon for use of Embodiment 143, wherein the tumor is selected from the group comprising pancreatic cancer preferably pancreatic ductal adenocarcinoma (PDAC); triple-negative breast cancer; prostate cancer; ovarian cancer; soft tissue sarcoma and osteosarcoma.

Embodiment 145. The CXCL12 antagonist for use of Embodiment 144 and the interferon for use of Embodiment 144, wherein the tumor is high-grade serous ovarian cancer.

Embodiment 146. The CXCL12 antagonist for use of Embodiment 144 and the interferon for use of Embodiment 144, wherein the tumor is advanced castration-resistant prostate cancer.

Embodiment 147. The CXCL12 antagonist for use of Embodiment 144 and the interferon for use of Embodiment 144, wherein the tumor is synovial sarcoma.

Embodiment 148. The CXCL12 antagonist for use of any one of Embodiments 142 and 144 to 147 and the interferon for use of any one of Embodiments 142 to 147, wherein the tumor is refractory to an immunotherapy, preferably the immunotherapy is selected from the group comprising a checkpoint inhibitor, an adoptive cell therapy and a tumor vaccine.

Embodiment 149. The CXCL12 antagonist for use of any one of Embodiments 142 and 144 to 148 and the interferon for use of any one of Embodiments 143 to 148, wherein the checkpoint inhibitor is a PD-1 inhibitor, a PD-L1 inhibitor, a CTLA-4 inhibitor, a LAG-3 inhibitor, a TIM-3 inhibitor or a TIGIT inhibitor.

Embodiment 150. The CXCL12 antagonist for use of Embodiment 149 and the interferon for use of Embodiment 149, wherein the PD-1 inhibitor is selected from the group comprising pembrolizumab, nivolumab, cemiplimab, toripalimab, tislelizumab, dostarlimab, retfanlimab, sintilimab, camrelizumab, and balstilimab; wherein the PD-L1 inhibitor is selected from the group comprising atezolizumab, avelumab, durvalumab, cosibelimab, envafolimab, CS1001, PF-06801591; wherein the CTLA-4 inhibitor is selected from the group comprising ipilimumab, tremelimumab, BMS-986218, and zalifrelimab; wherein the LAG-3 inhibitor is selected from the group comprising relatlimab and GSK2831781; wherein the TIGIT inhibitor is selected from the group comprising vibostolimab, domvanalimab, tiragolumab and etigilimab; and wherein the TIM-3 inhibitor is selected from the group comprising sabatolimab, BMS-986258, and cobolimab.

Embodiment 151. The CXCL12 antagonist for use of any one of Embodiments 142 and 144 to 150 and the interferon for use of any one of Embodiments 143 to 150, wherein the tumor has at least one characteristic selected from the group comprising reduced or lacking expression of PD-L1, reduced number of tumor-infiltrating lymphocytes, genomic stability and reduced or lacking preexisting antitumor immune response.

Embodiment 152. The CXCL12 antagonist for use of Embodiment 151 and the interferon for use of Embodiment 151, wherein the tumor-infiltrating lymphocytes are CD3⁺ lymphocytes.

Embodiment 153. The CXCL12 antagonist for use of any one of Embodiments 142 and 144 to 152 and the interferon for use of any one of Embodiments 143 to 152, wherein the tumor has an immune evasion mechanism.

Embodiment 154. The CXCL12 antagonist for use of Embodiment 153 and the interferon for use of Embodiment 153, wherein the tumor is an immune-excluded tumor.

Embodiment 155. The CXCL12 antagonist for use of Embodiment 154 and the interferon for use of Embodiment 154, wherein immune exclusion can be reverted or mitigated by an immune checkpoint inhibitor.

Embodiment 156. The CXCL12 antagonist for use of Embodiment 155 and the interferon for use of Embodiment 155, wherein the checkpoint inhibitor is a PD-1 inhibitor, a PD-L1 inhibitor, a CTLA-4 inhibitor, a LAG-3 inhibitor, a TIM-3 inhibitor or a TIGIT inhibitor.

Embodiment 157. The CXCL12 antagonist for use of Embodiment 156 and the interferon for use of Embodiment 156, wherein the PD-1 inhibitor is selected from the group comprising pembrolizumab, nivolumab, cemiplimab, toripalimab, tislelizumab, dostarlimab, retfanlimab, sintilimab, camrelizumab, and balstilimab; wherein the PD-L1 inhibitor is selected from the group comprising atezolizumab, avelumab, durvalumab, cosibelimab, envafolimab, CS1001, PF-06801591; wherein the CTLA-4 inhibitor is selected from the group comprising ipilimumab, tremelimumab, BMS-986218, and zalifrelimab; wherein the LAG-3 inhibitor is selected from the group comprising relatlimab and GSK2831781; wherein the TIGIT inhibitor is selected from the group comprising vibostolimab, domvanalimab, tiragolumab and etigilimab; and wherein the TIM-3 inhibitor is selected from the group comprising sabatolimab, BMS-986258, and cobolimab.

Embodiment 158. The CXCL12 antagonist for use of Embodiment 153 and the interferon for use of Embodiment 153, wherein the tumor is an immune-desert tumor.

Embodiment 159. The CXCL12 antagonist for use of any one of Embodiments 153 to 158 and the interferon for use of any one of Embodiments 153 to 158, wherein the immune evasion mechanism is one selected from the group comprising loss of MHC class I expression of malignant cells of the tumor and elevated expression of immune checkpoints by malignant and non-malignant cells of the tumor.

Embodiment 160. The CXCL12 antagonist for use of any one of Embodiments 153 to 159 and the interferon for use of any one of Embodiments 153 to 159, wherein the immune evasion mechanism results from, is caused by and/or is associated with a changed immune cell composition of the tumor microenvironment (TME).

Embodiment 161. The CXCL12 antagonist for use of Embodiment 160 and the interferon for use of Embodiment 160, wherein the tumor is evading from the subject's adaptive immune system.

Embodiment 162. The CXCL12 antagonist for use of Embodiment 161 and the interferon for use of Embodiment 161, wherein the tumor is evading from the subject's adaptive immune system by inhibiting infiltration of cytotoxic lymphocytes.

Embodiment 163. The CXCL12 antagonist for use of any one of Embodiments 161 and 162 and the interferon for use of any one of Embodiments 161 and 162, wherein the tumor is evading from the subject's adaptive immune system by suppressing activity of cytotoxic tumor-infiltrating lymphocytes or inhibiting activation of cytotoxic tumor-infiltrating lymphocytes.

Embodiment 164. The CXCL12 antagonist for use of Embodiment 160 and the interferon for use of Embodiment 160, wherein the tumor is evading from the subject's innate immune system.

Embodiment 165. The CXCL12 antagonist for use of Embodiment 164 and the interferon for use of Embodiment 164, wherein the tumor is characterized by an immunosuppressive tumor microenvironment.

Embodiment 166. The CXCL12 antagonist for use of Embodiment 165 and the interferon for use of Embodiment 165, wherein the immunosuppressive tumor microenvironment results from, is caused by and/or is associated with presence of a cell selected from the group comprising a myeloid-derived suppressor cell (MDSC), a tumor-associated macrophage (TAM), a tumor-associated neutrophil (TAN), and/or a regulatory T cell (Treg).

Embodiment 167. The CXCL12 antagonist for use of Embodiment 166 and the interferon for use of Embodiment 166, wherein the cell is immunomodulatorily active.

Embodiment 168. The CXCL12 antagonist for use of Embodiment 167 and the interferon for use of Embodiment 167, wherein the immunomodulatory active cell is active by means of a secreted immunomodulator, metabolic reprogramming and/or regulatory cell-to-cell contact.

Embodiment 169. A CXCL12 antagonist for use in a method for treating a tumor in a subject, wherein the method comprises administering to the subject a primary therapy and an adjunct therapy, wherein the adjunct therapy comprises administering to the subject the CXCL12 antagonist and an interferon, wherein the tumor is an immunologically cold tumor.

Embodiment 170. An interferon for use in a method for treating a tumor in a subject, wherein the method comprises administering to the subject a primary therapy and an adjunct therapy, wherein the adjunct therapy comprises administering to the subject the interferon and a CXCL12 antagonist, wherein the tumor is an immunologically cold tumor.

Embodiment 171. The CXCL12 antagonist for use of Embodiment 169 and the interferon for use of Embodiment 170, wherein the tumor is refractory to an immunotherapy, preferably the immunotherapy is selected from the group comprising a checkpoint inhibitor, an adoptive cell therapy and a tumor vaccine.

Embodiment 172. The CXCL12 antagonist for use of Embodiment 171 and the interferon for use of Embodiment 171, wherein the checkpoint inhibitor is a PD-1 inhibitor, a PD-L1 inhibitor, a CTLA-4 inhibitor, a LAG-3 inhibitor, a TIM-3 inhibitor or a TIGIT inhibitor.

Embodiment 173. The CXCL12 antagonist for use of Embodiment 172 and the interferon for use of Embodiment 172, wherein the PD-1 inhibitor is selected from the group comprising pembrolizumab, nivolumab, cemiplimab, toripalimab, tislelizumab, dostarlimab, retfanlimab, sintilimab, camrelizumab, and balstilimab; wherein the PD-L1 inhibitor is selected from the group comprising atezolizumab, avelumab, durvalumab, cosibelimab, envafolimab, CS1001, PF-06801591; wherein the CTLA-4 inhibitor is selected from the group comprising ipilimumab, tremelimumab, BMS-986218, and zalifrelimab; wherein the LAG-3 inhibitor is selected from the group comprising relatlimab and GSK2831781; wherein the TIGIT inhibitor is selected from the group comprising vibostolimab, domvanalimab, tiragolumab and etigilimab; and wherein the TIM-3 inhibitor is selected from the group comprising sabatolimab, BMS-986258, and cobolimab.

Embodiment 174. The CXCL12 antagonist for use of any one of Embodiments 169 and 171 to 173 and the interferon for use of any one of Embodiments 170 to 173, wherein the tumor has at least one characteristic selected from the group comprising reduced or lacking expression of PD-L1, reduced number of tumor-infiltrating lymphocytes, genomic stability and reduced or lacking preexisting antitumor immune response.

Embodiment 175. The CXCL12 antagonist for use of Embodiment 174 and the interferon for use of Embodiment 174, wherein the tumor-infiltrating lymphocytes are CD3⁺ lymphocytes.

Embodiment 176. The CXCL12 antagonist for use of any one of Embodiments 169 and 171 to 175 and the interferon for use of any one of Embodiments 170 to 175, wherein the tumor has an immune evasion mechanism.

Embodiment 177. The CXCL12 antagonist for use of Embodiment 176 and the interferon for use of Embodiment 176, wherein the tumor is an immune-excluded tumor.

Embodiment 178. The CXCL12 antagonist for use of Embodiment 177 and the interferon for use of Embodiment 177, wherein immune exclusion can be reverted or mitigated by an immune checkpoint inhibitor.

Embodiment 179. The CXCL12 antagonist for use of Embodiment 178 and the interferon for use of Embodiment 178, wherein the checkpoint inhibitor is a PD-1 inhibitor, a PD-L1 inhibitor, a CTLA-4 inhibitor, a LAG-3 inhibitor, a TIM-3 inhibitor or a TIGIT inhibitor.

Embodiment 180. The CXCL12 antagonist for use of Embodiment 179 and the interferon for use of Embodiment 179, wherein the PD-1 inhibitor is selected from the group comprising pembrolizumab, nivolumab, cemiplimab, toripalimab, tislelizumab, dostarlimab, retfanlimab, sintilimab, camrelizumab, and balstilimab; wherein the PD-L1 inhibitor is selected from the group comprising atezolizumab, avelumab, durvalumab, cosibelimab, envafolimab, CS1001, PF-06801591; wherein the CTLA-4 inhibitor is selected from the group comprising ipilimumab, tremelimumab, BMS-986218, and zalifrelimab; wherein the LAG-3 inhibitor is selected from the group comprising relatlimab and GSK2831781; wherein the TIGIT inhibitor is selected from the group comprising vibostolimab, domvanalimab, tiragolumab and etigilimab; and wherein the TIM-3 inhibitor is selected from the group comprising sabatolimab, BMS-986258, and cobolimab.

Embodiment 181. The CXCL12 antagonist for use of Embodiment 176 and the interferon for use of Embodiment 176, wherein the tumor is an immune-desert tumor.

Embodiment 182. The CXCL12 antagonist for use of any one of Embodiments 176 to 181 and the interferon for use of any one of Embodiments 176 to 181, wherein the immune evasion mechanism is one selected from the group comprising loss of MHC class I expression of malignant cells of the tumor and elevated expression of immune checkpoints by malignant and non-malignant cells of the tumor.

Embodiment 183. The CXCL12 antagonist for use of any one of Embodiments 176 to 182 and the interferon for use of any one of Embodiments 176 to 182, wherein the immune evasion mechanism results from, is caused by and/or is associated with a changed immune cell composition of the tumor microenvironment (TME).

Embodiment 184. The CXCL12 antagonist for use of Embodiment 184 and the interferon for use of Embodiment 183, wherein the tumor evades from the subject's adaptive immune system.

Embodiment 185. The CXCL12 antagonist for use of Embodiment 184 and the interferon for use of Embodiment 184, wherein the tumor evades from the subject's adaptive immune system by inhibiting infiltration of cytotoxic lymphocytes.

Embodiment 186. The CXCL12 antagonist for use of any one of Embodiments 184 and 185 and the interferon for use of any one of Embodiments 184 and 185, wherein the tumor evades from the subject's adaptive immune system by suppressing activity of cytotoxic tumor-infiltrating lymphocytes or inhibiting activation of cytotoxic tumor-infiltrating lymphocytes.

Embodiment 187. The CXCL12 antagonist for use of Embodiment 183 and the interferon for use of Embodiment 183, wherein the tumor evades from the subject's innate immune system.

Embodiment 188. The CXCL12 antagonist for use of Embodiment 187 and the interferon for use of Embodiment 187, wherein the tumor is characterized by an immunosuppressive tumor microenvironment.

Embodiment 189. The CXCL12 antagonist for use of Embodiment 181 and the interferon for use of Embodiment 188, wherein the immunosuppressive tumor microenvironment results from, is caused by and/or is associated with presence of a cell selected from the group comprising a myeloid-derived suppressor cell (MDSC), a tumor-associated macrophage (TAM), a tumor-associated neutrophil (TAN) and/or a regulatory T cell (T reg).

Embodiment 190. The CXCL12 antagonist for use of Embodiment 189 and the interferon for use of Embodiment 189, wherein the cell is immunomodulatory active.

Embodiment 191. The CXCL12 antagonist for use of Embodiment 189 and the interferon for use of Embodiment 189, wherein the immunomodulatory active cell by means of a secreted immunomodulator, metabolic reprogramming and/or regulatory cell-to-cell contact.

Embodiment 192. The CXCL12 antagonist for use of any one of Embodiments 169 and 171 to 191 and the interferon for use of any one of Embodiments 170 to 191, wherein the tumor is a solid tumor.

Embodiment 193. The CXCL12 antagonist for use of any one of Embodiments 169 and 171 to 192 and the interferon for use of any one of Embodiments 170 to 192, wherein the tumor is selected from the group comprising pancreatic cancer, preferably pancreatic ductal adenocarcinoma (PDAC); triple-negative breast cancer; prostate cancer; ovarian cancer; soft tissue sarcoma and osteosarcoma.

Embodiment 194. The CXCL12 antagonist for use of Embodiment 193 and the interferon for use of Embodiment 193, wherein the tumor is high-grade serous ovarian cancer.

Embodiment 195. The CXCL12 antagonist for use of Embodiment 193 and the interferon for use of Embodiment 193, wherein the tumor is advanced castration-resistant prostate cancer.

Embodiment 196. The CXCL12 antagonist for use of Embodiment 193 and the interferon for use of Embodiment 193, wherein the tumor is synovial sarcoma.

Embodiment 197. A CXCL12 antagonist for use in a method for treating a tumor in a subject, wherein the method comprises administering to the subject a primary therapy and an adjunct therapy, wherein the adjunct therapy comprises administering to the subject the CXCL12 antagonist and an interferon, wherein the tumor is refractory to immunotherapy.

Embodiment 198. An interferon for use in a method for treating a tumor in a subject, wherein the method comprises administering to the subject a primary therapy and an adjunct therapy, wherein the adjunct therapy comprises administering to the subject the interferon and a CXCL12 antagonist, wherein the tumor is refractory to immunotherapy.

Embodiment 199. The CXCL12 antagonist for use of Embodiment 197 and the interferon for use of Embodiment 198, wherein the tumor is refractory to an immunotherapy, preferably the immunotherapy is selected from the group comprising a checkpoint inhibitor, an adoptive cell therapy and a tumor vaccine.

Embodiment 200. The CXCL12 antagonist for use of Embodiment 199 and the interferon for use of Embodiment 199, wherein the checkpoint inhibitor is a PD-1 inhibitor, a PD-L1 inhibitor, a CTLA-4 inhibitor, a LAG-3 inhibitor, a TIM-3 inhibitor or a TIGIT inhibitor.

Embodiment 201. The CXCL12 antagonist for use of Embodiment 200 and the interferon for use of Embodiment 200, wherein the PD-1 inhibitor is selected from the group comprising pembrolizumab, nivolumab, cemiplimab, toripalimab, tislelizumab, dostarlimab, retfanlimab, sintilimab, camrelizumab, and balstilimab; wherein the PD-L1 inhibitor is selected from the group comprising atezolizumab, avelumab, durvalumab, cosibelimab, envafolimab, CS1001, PF-06801591; wherein the CTLA-4 inhibitor is selected from the group comprising ipilimumab, tremelimumab, BMS-986218, and zalifrelimab; wherein the LAG-3 inhibitor is selected from the group comprising relatlimab and GSK2831781; wherein the TIGIT inhibitor is selected from the group comprising vibostolimab, domvanalimab, tiragolumab and etigilimab; and wherein the TIM-3 inhibitor is selected from the group comprising sabatolimab, BMS-986258, and cobolimab.

Embodiment 202. The CXCL12 antagonist for use of any one of Embodiments 197 and 199 to 201 and the interferon for use of any one of Embodiments 198 to 201, wherein the tumor has at least one characteristic selected from the group comprising reduced or lacking expression of PD-L1, reduced number of tumor-infiltrating lymphocytes, genomic stability and reduced or lacking preexisting antitumor immune response.

Embodiment 203. The CXCL12 antagonist for use of Embodiment 202 and the interferon for use of Embodiment 202, wherein the tumor-infiltrating lymphocytes are CD3⁺ lymphocytes.

Embodiment 204. The CXCL12 antagonist for use of any one of Embodiments 197 and 199 to 203 and the interferon for use of any one of Embodiments 198 to 203, wherein the tumor has an immune evasion mechanism.

Embodiment 205. The CXCL12 antagonist for use of Embodiment 204 and the interferon for use of Embodiment 204, wherein the tumor is an immune-excluded tumor.

Embodiment 206. The CXCL12 antagonist for use of Embodiment 205 and the interferon for use of Embodiment 205, wherein immune exclusion can be reverted or mitigated by an immune checkpoint inhibitor.

Embodiment 207. The CXCL12 antagonist for use of Embodiment 206 and the interferon for use of Embodiment 206, wherein the checkpoint inhibitor is a PD-1 inhibitor, a PD-L1 inhibitor, a CTLA-4 inhibitor, a LAG-3 inhibitor, a TIM-3 inhibitor or a TIGIT inhibitor.

Embodiment 208. The CXCL12 antagonist for use of Embodiment 207 and the interferon for use of Embodiment 207, wherein the PD-1 inhibitor is selected from the group comprising pembrolizumab, nivolumab, cemiplimab, toripalimab, tislelizumab, dostarlimab, retfanlimab, sintilimab, camrelizumab, and balstilimab; wherein the PD-L1 inhibitor is selected from the group comprising atezolizumab, avelumab, durvalumab, cosibelimab, envafolimab, CS1001, PF-06801591; wherein the CTLA-4 inhibitor is selected from the group comprising ipilimumab, tremelimumab, BMS-986218, and zalifrelimab; wherein the LAG-3 inhibitor is selected from the group comprising relatlimab and GSK2831781; wherein the TIGIT inhibitor is selected from the group comprising vibostolimab, domvanalimab, tiragolumab and etigilimab; and wherein the TIM-3 inhibitor is selected from the group comprising sabatolimab, BMS-986258, and cobolimab.

Embodiment 209. The CXCL12 antagonist for use of Embodiment 204 and the interferon for use of Embodiment 204, wherein the tumor is an immune-desert tumor.

Embodiment 210. The CXCL12 antagonist for use of any one of Embodiments 204 to 209 and the interferon for use of any one of Embodiments 204 to 209, wherein the immune evasion mechanism is one selected from the group comprising loss of MHC class I expression of malignant cells of the tumor and elevated expression of immune checkpoints by malignant and non-malignant cells of the tumor.

Embodiment 211. The CXCL12 antagonist for use of any one of Embodiments 204 to 210 and the interferon for use of any one of Embodiments 204 to 210, wherein the immune evasion mechanism results from, is caused by and/or is associated with a changed immune cell composition of the tumor microenvironment (TME).

Embodiment 212. The CXCL12 antagonist for use of Embodiment 211 and the interferon for use of Embodiment 211, wherein the tumor evades from the subject's adaptive immune system.

Embodiment 213. The CXCL12 antagonist for use of Embodiment 212 and the interferon for use of Embodiment 212, wherein the tumor evades from the subject's adaptive immune system by inhibiting infiltration of cytotoxic lymphocytes.

Embodiment 214. The CXCL12 antagonist for use of any one of Embodiments 212 and 213 and the interferon for use of any one of Embodiments 212 and 213, wherein the tumor is evading from the subject's adaptive immune system by suppressing activity of cytotoxic tumor-infiltrating lymphocytes or inhibiting activation of cytotoxic tumor-infiltrating lymphocytes.

Embodiment 215. The CXCL12 antagonist for use of Embodiment 211 and the interferon for use of Embodiment 211, wherein the tumor is evading from the subject's innate immune system.

Embodiment 216. The CXCL12 antagonist for use of Embodiment 211 and the interferon for use of Embodiment 211, wherein the tumor is characterized by an immunosuppressive tumor microenvironment.

Embodiment 217. The CXCL12 antagonist for use of Embodiment 216 and the interferon for use of Embodiment 216, wherein the immunosuppressive tumor microenvironment results from, is caused by and/or is associated with presence of a cell selected from the group comprising a myeloid-derived suppressor cell (MDSC), a tumor-associated macrophage (TAM), a tumor-associated neutrophil (TAN) and/or a regulatory T cell (T reg).

Embodiment 218. The CXCL12 antagonist for use of Embodiment 217 and the interferon for use of Embodiment 217, wherein the cell is immunomodulatorily active.

Embodiment 219. The CXCL12 antagonist for use of Embodiment 218 and the interferon for use of Embodiment 218, wherein the immunomodulatorily active cell is active by means of a secreted immunomodulator, metabolic reprogramming and/or regulatory cell-to-cell contact.

Embodiment 220. The CXCL12 antagonist for use of any one of Embodiments 197 and 199 to 219 and the interferon for use of any one of Embodiments 198 to 219, wherein the tumor is an immunologically cold tumor.

Embodiment 221. The CXCL12 antagonist for use of any one of Embodiments 197 and 199 to 220 and the interferon for use of any one of Embodiments 198 to 220, wherein the tumor is a solid tumor.

Embodiment 222. The CXCL12 antagonist for use of any one of Embodiments 197 and 199 to 221 and the interferon for use of any one of Embodiments 198 to 221, wherein the tumor is selected from the group comprising pancreatic cancer, preferably pancreatic ductal adenocarcinoma (PDAC); triple-negative breast cancer; prostate cancer; ovarian cancer; soft tissue sarcoma and osteosarcoma.

Embodiment 223. The CXCL12 antagonist for use of Embodiment 222 and the interferon for use of Embodiment 222, wherein the tumor is high-grade serous ovarian cancer.

Embodiment 224. The CXCL12 antagonist for use of Embodiment 222 and the interferon for use of Embodiment 222, wherein the tumor is advanced castration-resistant prostate cancer.

Embodiment 225. The CXCL12 antagonist for use of Embodiment 222 and the interferon for use of Embodiment 222, wherein the tumor is synovial sarcoma.

Embodiment 226. A CXCL12 antagonist for use in a method for treating a tumor in a subject, wherein the method comprises administering to the subject a primary therapy and an adjunct therapy, wherein the adjunct therapy comprises administering to the subject the CXCL12 antagonist and an interferon, wherein the tumor is a solid tumor.

Embodiment 227. An interferon for use in a method for treating a tumor in a subject, wherein the method comprises administering to the subject a primary therapy and an adjunct therapy, wherein the adjunct therapy comprises administering to the subject the interferon and a CXCL12 antagonist, wherein the tumor is a solid tumor.

Embodiment 228. The CXCL12 antagonist for use of Embodiment 226 and the interferon for use of Embodiment 227, wherein the tumor is selected from the group comprising pancreatic cancer, preferably pancreatic ductal adenocarcinoma (PDAC); triple-negative breast cancer; prostate cancer; ovarian cancer; soft tissue sarcoma and osteosarcoma.

Embodiment 229. The CXCL12 antagonist for use of Embodiment 228 and the interferon for use of Embodiment 228, wherein the tumor is high-grade serous ovarian cancer.

Embodiment 230. The CXCL12 antagonist for use of Embodiment 228 and the interferon for use of Embodiment 228, wherein the tumor is advanced castration-resistant prostate cancer.

Embodiment 231. The CXCL12 antagonist for use of Embodiment 228 and the interferon for use of Embodiment 228, wherein the tumor is synovial sarcoma.

Embodiment 232. The CXCL12 antagonist for use of any one of Embodiments 226 and 228 to 231 and the interferon for use of any one of Embodiments 227 to 231, wherein the tumor is refractory to an immunotherapy, preferably the immunotherapy is selected from the group comprising checkpoint inhibitor, an adoptive cell therapy and a tumor vaccine.

Embodiment 233. The CXCL12 antagonist for use of any one of Embodiments 226 and 228 to 232 and the interferon for use of any one of Embodiments 227 to 232, wherein the checkpoint inhibitor is a PD-1 inhibitor, a PD-L1 inhibitor, a CTLA-4 inhibitor, a LAG-3 inhibitor, a TIM-3 inhibitor or a TIGIT inhibitor.

Embodiment 234. The CXCL12 antagonist for use of Embodiment 233 and the interferon for use of Embodiment 233, wherein the PD-1 inhibitor is selected from the group comprising pembrolizumab, nivolumab, cemiplimab, toripalimab, tislelizumab, dostarlimab, retfanlimab, sintilimab, camrelizumab, and balstilimab; wherein the PD-L1 inhibitor is selected from the group comprising atezolizumab, avelumab, durvalumab, cosibelimab, envafolimab, CS1001, PF-06801591; wherein the CTLA-4 inhibitor is selected from the group comprising ipilimumab, tremelimumab, BMS-986218, and zalifrelimab; wherein the LAG-3 inhibitor is selected from the group comprising relatlimab and GSK2831781; wherein the TIGIT inhibitor is selected from the group comprising vibostolimab, domvanalimab, tiragolumab and etigilimab; and wherein the TIM-3 inhibitor is selected from the group comprising sabatolimab, BMS-986258, and cobolimab.

Embodiment 235. The CXCL12 antagonist for use of any one of Embodiments 226 and 228 to 234 and the interferon for use of any one of Embodiments 227 to 224, wherein the tumor has at least one characteristic selected from the group comprising reduced or lacking expression of PD-L1, reduced number of tumor-infiltrating lymphocytes, genomic stability and reduced or lacking preexisting antitumor immune response.

Embodiment 236. The CXCL12 antagonist for use of Embodiment 235 and the interferon for use of Embodiment 235, wherein the tumor-infiltrating lymphocytes are CD3⁺ lymphocytes.

Embodiment 237. The CXCL12 antagonist for use of any one of Embodiments 226 and 228 to 236 and the interferon for use of any one of Embodiments 227 to 236, wherein the tumor has an immune evasion mechanism.

Embodiment 238. The CXCL12 antagonist for use of Embodiment 237 and the interferon for use of Embodiment 237, wherein the tumor is an immune-excluded tumor.

Embodiment 239. The CXCL12 antagonist for use of Embodiment 238 and the interferon for use of Embodiment 238 wherein immune exclusion can be reverted or mitigated by an immune checkpoint inhibitor.

Embodiment 240. The CXCL12 antagonist for use of Embodiment 237 and the interferon for use of Embodiment 237, wherein the tumor is an immune-desert tumor.

Embodiment 241. The CXCL12 antagonist for use of any one of Embodiments 237 to 240 and the interferon for use of any one of Embodiments 237 to 240, wherein the immune evasion mechanism is one selected from the group comprising loss of MHC class I expression of malignant cells of the tumor and elevated expression of immune checkpoints by malignant and non-malignant cells of the tumor.

Embodiment 242. The CXCL12 antagonist for use of any one of Embodiments 237 to 241 and the interferon for use of any one of Embodiments 237 to 241, wherein the immune evasion mechanism results from, is caused by and/or is associated with a changed immune cell composition of the tumor microenvironment (TME).

Embodiment 243. The CXCL12 antagonist for use of Embodiment 242 and the interferon for use of Embodiment 242, wherein the tumor is evading from the subject's adaptive immune system.

Embodiment 244. The CXCL12 antagonist for use of Embodiment 243 and the interferon for use of Embodiment 243, wherein the tumor is evading from the subject's adaptive immune system by inhibiting infiltration of cytotoxic lymphocytes.

Embodiment 245. The CXCL12 antagonist for use of any one of Embodiments 243 and 244 and the interferon for use of any one of Embodiments 243 and 233, wherein the tumor is evading from the subject's adaptive immune system by suppressing activity of cytotoxic tumor-infiltrating lymphocytes or inhibiting activation of cytotoxic tumor-infiltrating lymphocytes.

Embodiment 246. The CXCL12 antagonist for use of Embodiment 242 and the interferon for use of Embodiment 242, wherein the tumor is evading from the subject's innate immune system.

Embodiment 247. The CXCL12 antagonist for use of Embodiment 246 and the interferon for use of Embodiment 246, wherein the tumor is characterized by an immunosuppressive tumor microenvironment.

Embodiment 248. The CXCL12 antagonist for use of Embodiment 247 and the interferon for use of Embodiment 247, wherein the immunosuppressive tumor microenvironment results from, is caused by and/or is associated with presence of a cell selected from the group comprising a myeloid-derived suppressor cell (MDSC), a tumor-associated macrophage (TAM), a tumor-associated neutrophil (TAN) and/or a regulatory T cell (T reg).

Embodiment 249. The CXCL12 antagonist for use of Embodiment 248 and the interferon for use of Embodiment 248, wherein the cell is immunomodulatorily active.

Embodiment 250. The CXCL12 antagonist for use of Embodiment 249 and the interferon for use of Embodiment 249, wherein the immunomodulatorily active cell is active by means of a secreted immunomodulator, metabolic reprogramming and/or regulatory cell-to-cell contact.

Embodiment 251. A CXCL12 antagonist for use in a method for converting an immunologically cold tumor in a subject into an immunologically hot tumor in the subject, wherein the method comprises administering to the subject the CXCL12 antagonist and an interferon, wherein upon administration of the CXCL12 antagonist and the interferon the immunologically cold tumor is or will be converted into an immunologically hot tumor.

Embodiment 252. An interferon for use in a method for converting an immunologically cold tumor in a subject into an immunologically hot tumor in the subject, wherein the method comprises administering to the subject the interferon and a CXCL12 antagonist, wherein upon administration of the interferon and the CXCL12 antagonist the immunologically cold tumor is or will be converted into an immunologically hot tumor.

Embodiment 253. The CXCL12 antagonist for use of Embodiment 251 and the interferon for use of Embodiment 252, wherein the immunologically cold tumor is refractory to an immunotherapy, preferably the immunotherapy is selected from the group comprising a checkpoint inhibitor, an adoptive cell therapy and a tumor vaccine.

Embodiment 254. The CXCL12 antagonist for use of Embodiment 253 and the interferon for use of Embodiment 253, wherein the checkpoint inhibitor is a PD-1 inhibitor, a PD-L1 inhibitor, a CTLA-4 inhibitor, a LAG-3 inhibitor, a TIM-3 inhibitor or a TIGIT inhibitor.

Embodiment 255. The CXCL12 antagonist for use of Embodiment 254 and the interferon for use of Embodiment 254, wherein the PD-1 inhibitor is selected from the group comprising pembrolizumab, nivolumab, cemiplimab, toripalimab, tislelizumab, dostarlimab, retfanlimab, sintilimab, camrelizumab, and balstilimab; wherein the PD-L1 inhibitor is selected from the group comprising atezolizumab, avelumab, durvalumab, cosibelimab, envafolimab, CS1001, PF-06801591; wherein the CTLA-4 inhibitor is selected from the group comprising ipilimumab, tremelimumab, BMS-986218, and zalifrelimab; wherein the LAG-3 inhibitor is selected from the group comprising relatlimab and GSK2831781; wherein the TIGIT inhibitor is selected from the group comprising vibostolimab, domvanalimab, tiragolumab and etigilimab; and wherein the TIM-3 inhibitor is selected from the group comprising sabatolimab, BMS-986258, and cobolimab.

Embodiment 256. The CXCL12 antagonist for use of any one of Embodiments 251 and 253 to 255 and the interferon for use of any one of Embodiments 252 to 255, wherein the immunologically cold tumor has at least one characteristic selected from the group comprising reduced or lacking expression of PD-L1, reduced number of tumor-infiltrating lymphocytes, genomic stability and reduced or lacking preexisting antitumor immune response.

Embodiment 257. The CXCL12 antagonist for use of Embodiment 256 and the interferon for use of Embodiment 256, wherein the tumor-infiltrating lymphocytes are CD3⁺ lymphocytes.

Embodiment 258. The CXCL12 antagonist for use of any one of Embodiments 251 and 253 to 257 and the interferon for use of any one of Embodiments 252 to 257, wherein the immunologically cold tumor has an immune evasion mechanism.

Embodiment 259. The CXCL12 antagonist for use of Embodiment 258 and the interferon for use of Embodiment 258, wherein the immunologically cold tumor is an immune-excluded tumor.

Embodiment 260. The CXCL12 antagonist for use of Embodiment 259 and the interferon for use of Embodiment 259, wherein immune exclusion can be reverted or mitigated by an immune checkpoint inhibitor.

Embodiment 261. The CXCL12 antagonist for use of Embodiment 260 and the interferon for use of Embodiment 260, wherein the checkpoint inhibitor is a PD-1 inhibitor, a PD-L1 inhibitor, a CTLA-4 inhibitor, a LAG-3 inhibitor, a TIM-3 inhibitor or a TIGIT inhibitor.

Embodiment 262. The CXCL12 antagonist for use of Embodiment 261 and the interferon for use of Embodiment 261, wherein the PD-1 inhibitor is selected from the group comprising pembrolizumab, nivolumab, cemiplimab, toripalimab, tislelizumab, dostarlimab, retfanlimab, sintilimab, camrelizumab, and balstilimab; wherein the PD-L1 inhibitor is selected from the group comprising atezolizumab, avelumab, durvalumab, cosibelimab, envafolimab, CS1001, PF-06801591; wherein the CTLA-4 inhibitor is selected from the group comprising ipilimumab, tremelimumab, BMS-986218, and zalifrelimab; wherein the LAG-3 inhibitor is selected from the group comprising relatlimab and GSK2831781; wherein the TIGIT inhibitor is selected from the group comprising vibostolimab, domvanalimab, tiragolumab and etigilimab; and wherein the TIM-3 inhibitor is selected from the group comprising sabatolimab, BMS-986258, and cobolimab.

Embodiment 263. The CXCL12 antagonist for use of Embodiment 258 and the interferon for use of Embodiment 258, wherein the tumor is an immune-desert tumor.

Embodiment 264. The CXCL12 antagonist for use of any one of Embodiments 258 to 263 and the interferon for use of any one of Embodiments 258 to 263, wherein the immune evasion mechanism is one selected from the group comprising loss of MHC class I expression of malignant cells of the tumor and elevated expression of immune checkpoints by malignant and non-malignant cells of the tumor.

Embodiment 265. The CXCL12 antagonist for use of any one of Embodiments 258 to 264 and the interferon for use of any one of Embodiments 258 to 264, wherein the immune evasion mechanism results from, is caused by and/or is associated with a changed immune cell composition of the tumor microenvironment (TME).

Embodiment 266. The CXCL12 antagonist for use of Embodiment 265 and the interferon for use of Embodiment 265, wherein the immunologically cold tumor evades from the subject's adaptive immune system.

Embodiment 267. The CXCL12 antagonist for use of Embodiment 266 and the interferon for use of Embodiment 266, wherein the immunologically cold tumor is evading from the subject's adaptive immune system by inhibiting infiltration of cytotoxic lymphocytes.

Embodiment 268. The CXCL12 antagonist for use of any one of Embodiments 266 and 267 and the interferon for use of any one of Embodiments 266 and 267, wherein the immunologically cold tumor is evading from the subject's adaptive immune system by suppressing activity of cytotoxic tumor-infiltrating lymphocytes or inhibiting activation of cytotoxic tumor-infiltrating lymphocytes.

Embodiment 269. The CXCL12 antagonist for use of Embodiment 265 and the interferon for use of Embodiment 265, wherein the immunologically cold tumor is evading from the subject's innate immune system.

Embodiment 270. The CXCL12 antagonist for use of Embodiment 269 and the interferon for use of Embodiment 269, wherein the immunologically cold tumor is characterized by an immunosuppressive tumor microenvironment.

Embodiment 271. The CXCL12 antagonist for use of Embodiment 270 and the interferon for use of Embodiment 270, wherein the immunosuppressive tumor microenvironment results from, is caused by and/or is associated with presence of a cell selected from the group comprising a myeloid-derived suppressor cell (MDSC), a tumor-associated macrophage (TAM), a tumor-associated neutrophil (TAN) and/or a regulatory T cell (T reg).

Embodiment 272. The CXCL12 antagonist for use of Embodiment 271 and the interferon for use of Embodiment 271, wherein the cell is immunomodulatory active.

Embodiment 273. The CXCL12 antagonist for use of Embodiment 272 and the interferon for use of Embodiment 272, wherein the immunomodulatory active cell by means of a secreted immunomodulator, metabolic reprogramming and/or regulatory cell-to-cell contact.

Embodiment 274. The CXCL12 antagonist for use of any one of Embodiments 251 and 253 to 273 and the interferon for use of any one of Embodiments 252 to 273, wherein the immunologically hot tumor is non-refractory to an immunotherapy, preferably the immunotherapy is selected from the group comprising a checkpoint inhibitor, an adoptive cell therapy and a tumor vaccine.

Embodiment 275. The CXCL12 antagonist for use of Embodiment 274 and the interferon for use of Embodiment 274, wherein the checkpoint inhibitor is a PD-1 inhibitor, a PD-L1 inhibitor, a CTLA-4 inhibitor, a LAG-3 inhibitor, a TIM-3 inhibitor or a TIGIT inhibitor.

Embodiment 276. The CXCL12 antagonist for use of Embodiment 275 and the interferon for use of Embodiment 275, wherein the PD-1 inhibitor is selected from the group comprising pembrolizumab, nivolumab, cemiplimab, toripalimab, tislelizumab, dostarlimab, retfanlimab, sintilimab, camrelizumab, and balstilimab; wherein the PD-L1 inhibitor is selected from the group comprising atezolizumab, avelumab, durvalumab, cosibelimab, envafolimab, CS1001, PF-06801591; wherein the CTLA-4 inhibitor is selected from the group comprising ipilimumab, tremelimumab, BMS-986218, and zalifrelimab; wherein the LAG-3 inhibitor is selected from the group comprising relatlimab and GSK2831781; wherein the TIGIT inhibitor is selected from the group comprising vibostolimab, domvanalimab, tiragolumab and etigilimab; and wherein the TIM-3 inhibitor is selected from the group comprising sabatolimab, BMS-986258, and cobolimab.

Embodiment 277. The CXCL12 antagonist for use of any one of Embodiments 251 and 253 to 276 and the interferon for use of any one of Embodiments 252 to 276, wherein the immunologically hot tumor has at least one characteristic selected from the group comprising presence of tumor-infiltrating lymphocytes, genomic instability of malignant cells and preexisting antitumor immune response.

Embodiment 278. The CXCL12 antagonist for use of Embodiment 277 and the interferon for use of Embodiment 277, wherein the tumor-infiltrating lymphocytes are CD3⁺ lymphocytes.

Embodiment 279. The CXCL12 antagonist for use of any one of Embodiments 251 and 253 to 278 and the interferon for use of any one of Embodiments 252 to 278, wherein the immunologically hot tumor is subject to the subject's adaptive immune system.

Embodiment 280. The CXCL12 antagonist for use of any one of Embodiments 251 and 253 to 278 and the interferon for use of any one of Embodiments 252 to 278, wherein the immunologically hot tumor is subject to the subject's innate immune system.

Embodiment 281. The CXCL12 antagonist for use of any one of Embodiments 251 and 253 to 280 and the interferon for use of any one of Embodiments 252 to 280, wherein the immunologically hot tumor is characterized by a non-immunosuppressive tumor microenvironment.

Embodiment 282. The CXCL12 antagonist for use of Embodiment 281 and the interferon for use of Embodiment 281, wherein an immunosuppressive tumor microenvironment results from, is caused by and/or is associated with presence of a cell selected from the group comprising a myeloid-derived suppressor cell (MDSC), a tumor-associated macrophage (TAM), a tumor-associated neutrophil (TAN) and/or a regulatory T cell (T reg).

Embodiment 283. The CXCL12 antagonist for use of Embodiment 282 and the interferon for use of Embodiment 282, wherein the cell is immunomodulatory active.

Embodiment 284. The CXCL12 antagonist for use of Embodiment 280 and the interferon for use of Embodiment 280, wherein the immunomodulatory active cell by means of a secreted immunomodulator, metabolic reprogramming and/or regulatory cell-to-cell contact.

Embodiment 285. The CXCL12 antagonist for use of any one of Embodiments 251 and 253 to 284 and the interferon for use of any one of Embodiments 252 to 281, wherein the tumor is a solid tumor.

Embodiment 286. The CXCL12 antagonist for use of any one of Embodiments 251 and 253 to 285 and the interferon for use of any one of Embodiments 252 to 285, wherein the tumor is selected from the group comprising pancreatic cancer, preferably pancreatic ductal adenocarcinoma (PDAC); triple-negative breast cancer; prostate cancer; ovarian cancer; soft tissue sarcoma and osteosarcoma.

Embodiment 287. The CXCL12 antagonist for use of Embodiment 286 and the interferon for use of Embodiment 286, wherein the tumor is high-grade serous ovarian cancer.

Embodiment 288. The CXCL12 antagonist for use of Embodiment 286 and the interferon for use of Embodiment 286, wherein the tumor is advanced castration-resistant prostate cancer.

Embodiment 289. The CXCL12 antagonist for use of Embodiment 286 and the interferon for use of Embodiment 286, wherein the tumor is synovial sarcoma.

Embodiment 290. A CXCL12 antagonist for use in a method for converting an immunotherapy refractory tumor in a subject into an immunotherapy non-refractory tumor in the subject, wherein the method comprises administering to the subject the CXCL12 antagonist and an interferon, wherein upon administration of the CXCL12 antagonist and the interferon the immunotherapy refractory tumor is or will be converted into an immunotherapy non-refractory tumor.

Embodiment 291. An interferon for use in a method for converting an immunotherapy refractory tumor in a subject into an immunotherapy non-refractory tumor in the subject, wherein the method comprises administering to the subject the interferon and a CXCL12 antagonist, wherein upon administration of the interferon and the CXCL12 antagonist the immunotherapy refractory tumor is or will be converted into an immunotherapy non-refractory tumor.

Embodiment 292. The CXCL12 antagonist for use of Embodiment 290 and the interferon for use of Embodiment 291, wherein the immunotherapy refractory tumor is refractory to an immunotherapy, preferably the immunotherapy is selected from the group comprising a checkpoint inhibitor, an adoptive cell therapy and a tumor vaccine.

Embodiment 293. The CXCL12 antagonist for use of Embodiment 292 and the interferon for use of Embodiment 292, wherein the checkpoint inhibitor is a PD-1 inhibitor, a PD-L1 inhibitor, a CTLA-4 inhibitor, a LAG-3 inhibitor, a TIM-3 inhibitor or a TIGIT inhibitor.

Embodiment 294. The CXCL12 antagonist for use of Embodiment 293 and the interferon for use of Embodiment 293, wherein the PD-1 inhibitor is selected from the group comprising pembrolizumab, nivolumab, cemiplimab, toripalimab, tislelizumab, dostarlimab, retfanlimab, sintilimab, camrelizumab, and balstilimab; wherein the PD-L1 inhibitor is selected from the group comprising atezolizumab, avelumab, durvalumab, cosibelimab, envafolimab, CS1001, PF-06801591; wherein the CTLA-4 inhibitor is selected from the group comprising ipilimumab, tremelimumab, BMS-986218, and zalifrelimab; wherein the LAG-3 inhibitor is selected from the group comprising relatlimab and GSK2831781; wherein the TIGIT inhibitor is selected from the group comprising vibostolimab, domvanalimab, tiragolumab and etigilimab; and wherein the TIM-3 inhibitor is selected from the group comprising sabatolimab, BMS-986258, and cobolimab.

Embodiment 295. The CXCL12 antagonist for use of any one of Embodiments 290 and 292 to 294 and the interferon for use of any one of Embodiments 291 to 294, wherein the immunotherapy refractory tumor has at least one characteristic selected from the group comprising reduced or lacking expression of PD-L1, reduced number of tumor-infiltrating lymphocytes, genomic stability and reduced or lacking preexisting antitumor immune response.

Embodiment 296. The CXCL12 antagonist for use of Embodiment 295 and the interferon for use of Embodiment 292, wherein the tumor-infiltrating lymphocytes are CD3⁺ lymphocytes.

Embodiment 297. The CXCL12 antagonist for use of any one of Embodiments 290 and 292 to 296 and the interferon for use of any one of Embodiments 291 to 296, wherein the immunotherapy refractory tumor evades attack of the immune system.

Embodiment 298. The CXCL12 antagonist for use of Embodiment 297 and the interferon for use of Embodiment 297, wherein the immunotherapy refractory tumor is an immune-excluded tumor.

Embodiment 299. The CXCL12 antagonist for use of Embodiment 298 and the interferon for use of Embodiment 298, wherein immune exclusion can be reverted or mitigated by an immune checkpoint inhibitor.

Embodiment 300. The CXCL12 antagonist for use of Embodiment 299 and the interferon for use of Embodiment 299, wherein the checkpoint inhibitor is a PD-1 inhibitor, a PD-L1 inhibitor, a CTLA-4 inhibitor, a LAG-3 inhibitor, a TIM-3 inhibitor or a TIGIT inhibitor.

Embodiment 301. The CXCL12 antagonist for use of Embodiment 300 and the interferon for use of Embodiment 300, wherein the PD-1 inhibitor is selected from the group comprising pembrolizumab, nivolumab, cemiplimab, toripalimab, tislelizumab, dostarlimab, retfanlimab, sintilimab, camrelizumab, and balstilimab; wherein the PD-L1 inhibitor is selected from the group comprising atezolizumab, avelumab, durvalumab, cosibelimab, envafolimab, CS1001, PF-06801591; wherein the CTLA-4 inhibitor is selected from the group comprising ipilimumab, tremelimumab, BMS-986218, and zalifrelimab; wherein the LAG-3 inhibitor is selected from the group comprising relatlimab and GSK2831781; wherein the TIGIT inhibitor is selected from the group comprising vibostolimab, domvanalimab, tiragolumab and etigilimab; and wherein the TIM-3 inhibitor is selected from the group comprising sabatolimab, BMS-986258, and cobolimab.

Embodiment 302. The CXCL12 antagonist for use of Embodiment 297 and the interferon for use of Embodiment 297, wherein the immunotherapy refractory tumor is an immune-desert tumor.

Embodiment 303. The CXCL12 antagonist for use of any one of Embodiments 297 to 302 and the interferon for use of any one of Embodiments 297 to 302, wherein the immune evasion mechanism is one selected from the group comprising loss of MHC class I expression of malignant cells of the tumor and elevated expression of immune checkpoints by malignant and non-malignant cells of the tumor.

Embodiment 304. The CXCL12 antagonist for use of any one of Embodiments 297 to 303 and the interferon for use of any one of Embodiments 297 to 303, wherein the immune evasion mechanism results from, is caused by and/or is associated with a changed immune cell composition of the tumor microenvironment (TME).

Embodiment 305. The CXCL12 antagonist for use of Embodiment 304 and the interferon for use of Embodiment 304, wherein the immunologically cold tumor evades from the subject's adaptive immune system.

Embodiment 306. The CXCL12 antagonist for use of Embodiment 305 and the interferon for use of Embodiment 305, wherein the immunotherapy refractory tumor is evading from the subject's adaptive immune system by inhibiting infiltration of cytotoxic lymphocytes.

Embodiment 307. The CXCL12 antagonist for use of any one of Embodiments 305 and 306 and the interferon for use of any one of Embodiments 305 and 306, wherein the immunotherapy refractory tumor is evading from the subject's adaptive immune system by suppressing activity of cytotoxic tumor-infiltrating lymphocytes or inhibiting activation of cytotoxic tumor-infiltrating lymphocytes.

Embodiment 308. The CXCL12 antagonist for use of Embodiment 304 and the interferon for use of Embodiment 304, wherein the immunotherapy refractory tumor is evading from the subject's innate immune system.

Embodiment 309. The CXCL12 antagonist for use of Embodiment 308 and the interferon for use of Embodiment 308, wherein the immunotherapy refractory tumor is characterized by an immunosuppressive tumor microenvironment.

Embodiment 310. The CXCL12 antagonist for use of Embodiment 309 and the interferon for use of Embodiment 309, wherein the immunosuppressive tumor microenvironment results from, is caused by and/or is associated with presence of a cell selected from the group comprising a myeloid-derived suppressor cell (MDSC), a tumor-associated macrophage (TAM) a tumor-associated neutrophil (TAN) and/or a regulatory T cell (Treg).

Embodiment 311. The CXCL12 antagonist for use of Embodiment 310 and the interferon for use of Embodiment 310, wherein the cell is immunomodulatory active.

Embodiment 312. The CXCL12 antagonist for use of Embodiment 311 and the interferon for use of Embodiment 311, wherein the immunomodulatory active cell by means of a secreted immunomodulator, metabolic reprogramming and/or regulatory cell-to-cell contact.

Embodiment 313. The CXCL12 antagonist for use of any one of Embodiments 290 and 292 to 312 and the interferon for use of any one of Embodiments 291 to 313, wherein the immunotherapy non-refractory tumor is non-refractory to an immunotherapy, preferably the immunotherapy is selected from the group comprising a checkpoint inhibitor, an adoptive cell therapy and a tumor vaccine.

Embodiment 314. The CXCL12 antagonist for use of Embodiment 313 and the interferon for use of Embodiment 313, wherein the checkpoint inhibitor is a PD-1 inhibitor, a PD-L1 inhibitor, a CTLA-4 inhibitor, a LAG-3 inhibitor, a TIM-3 inhibitor or a TIGIT inhibitor.

Embodiment 315. The CXCL12 antagonist for use of Embodiment 314 and the interferon for use of Embodiment 314, wherein the PD-1 inhibitor is selected from the group comprising pembrolizumab, nivolumab, cemiplimab, toripalimab, tislelizumab, dostarlimab, retfanlimab, sintilimab, camrelizumab, and balstilimab; wherein the PD-L1 inhibitor is selected from the group comprising atezolizumab, avelumab, durvalumab, cosibelimab, envafolimab, CS1001, PF-06801591; wherein the CTLA-4 inhibitor is selected from the group comprising ipilimumab, tremelimumab, BMS-986218, and zalifrelimab; wherein the LAG-3 inhibitor is selected from the group comprising relatlimab and GSK2831781; wherein the TIGIT inhibitor is selected from the group comprising vibostolimab, domvanalimab, tiragolumab and etigilimab; and wherein the TIM-3 inhibitor is selected from the group comprising sabatolimab, BMS-986258, and cobolimab.

Embodiment 316. The CXCL12 antagonist for use of any one of Embodiments 290 and 292 to 315 and the interferon for use of any one of Embodiments 291 to 315, wherein the immunotherapy non-refractory tumor has at least one characteristic selected from the group comprising expression of PD-L1, presence of tumor-infiltrating lymphocytes, genomic instability of malignant cells and preexisting antitumor immune response.

Embodiment 317. The CXCL12 antagonist for use of Embodiment 316 and the interferon for use of Embodiment 316, wherein the tumor-infiltrating lymphocytes are CD3⁺ lymphocytes.

Embodiment 318. The CXCL12 antagonist for use of any one of Embodiments 288 and 290 to 317 and the interferon for use of any one of Embodiments 289 to 317, wherein the immunotherapy non-refractory tumor is subject to the subject's adaptive immune system.

Embodiment 319. The CXCL12 antagonist for use of any one of Embodiments 290 and 292 to 318 and the interferon for use of any one of Embodiments 291 to 318, wherein the immunotherapy non-refractory tumor is subject to the subject's innate immune system.

Embodiment 320. The CXCL12 antagonist for use of any one of Embodiments 290 and 292 to 319 and the interferon for use of any one of Embodiments 291 to 319, wherein the immunotherapy non-refractory tumor is characterized by a non-immunosuppressive tumor microenvironment.

Embodiment 321. The CXCL12 antagonist for use of Embodiment 320 and the interferon for use of Embodiment 320, wherein an immunosuppressive tumor microenvironment results from, is caused by and/or is associated with presence of a cell selected from the group comprising a myeloid-derived suppressor cell (MDSC), a tumor-associated macrophage (TAM) a tumor-associated neutrophil (TAN), and/or aregulatory T cell (Treg).

Embodiment 322. The CXCL12 antagonist for use of Embodiment 321 and the interferon for use of Embodiment 321, wherein the cell is immunomodulatorily active.

Embodiment 323. The CXCL12 antagonist for use of Embodiment 322 and the interferon for use of Embodiment 322, wherein the immunomodulatorily active cell is active by means of a secreted immunomodulator, metabolic reprogramming and/or regulatory cell-to-cell contact.

Embodiment 324. The CXCL12 antagonist for use of any one of Embodiments 290 and 292 to 323 and the interferon for use of any one of Embodiments 291 to 323, wherein the tumor is a solid tumor.

Embodiment 325. The CXCL12 antagonist for use of any one of Embodiments 290 and 292 to 324 and the interferon for use of any one of Embodiments 291 to 324, wherein the tumor is selected from the group comprising pancreatic cancer, preferably pancreatic ductal adenocarcinoma (PDAC); triple-negative breast cancer; prostate cancer; ovarian cancer; soft tissue sarcoma and osteosarcoma.

Embodiment 326. The CXCL12 antagonist for use of Embodiment 325 and the interferon for use of Embodiment 325, wherein the tumor is high-grade serous ovarian cancer.

Embodiment 327. The CXCL12 antagonist for use of Embodiment 325 and the interferon for use of Embodiment 325, wherein the tumor is advanced castration-resistant prostate cancer.

Embodiment 328. The CXCL12 antagonist for use of Embodiment 325 and the interferon for use of Embodiment 325, wherein the tumor is synovial sarcoma.

Embodiment 329. The CXCL12 antagonist for use of any one of Embodiments 1 to 328 and the interferon for use of any one of Embodiments 2 to 328, wherein the method comprises administering to the subject an adoptive cell therapy.

Embodiment 330. The CXCL12 antagonist for use of Embodiment 329 and the interferon for use of Embodiment 329, wherein the adoptive cell therapy comprises administering a CAR T cell to the subject.

Embodiment 331. The CXCL12 antagonist for use of Embodiment 329 and the interferon for use of Embodiment 329, wherein the adoptive cell therapy comprises administering a TCR-T cell to the subject.

Embodiment 332. The CXCL12 antagonist for use of any one of Embodiments 1 to 331 and the interferon for use of any one of Embodiments 2 to 331, wherein the CXCL12 antagonist and the interferon are administered concomitantly to the subject.

Embodiment 333. The CXCL12 antagonist for use of any one of Embodiments 1 to 331 and the interferon for use of any one of Embodiments 2 to 331, wherein the CXCL12 antagonist is administered to the subject after the interferon.

Embodiment 334. The CXCL12 antagonist for use of any one of Embodiments 1 to 333 and the interferon for use of any one of Embodiments 2 to 335, wherein the interferon is a type I interferon.

Embodiment 335. The CXCL12 antagonist for use of Embodiment 334 and the interferon for use of Embodiment 334, wherein the type I interferon increases expression of MHC I on tumor cells.

Embodiment 336. The CXCL12 antagonist for use of any one of Embodiments 334 and 335 and the interferon for use of any one of Embodiments 336 and 337, wherein the type I interferon is interferon alpha.

Embodiment 337. The CXCL12 antagonist for use of Embodiment 336 and the interferon for use of Embodiment 336, wherein the type I interferon is interferon alpha comprising an amino acid sequence of SEQ ID NO: 242 or SEQ ID NO: 243

Embodiment 338. The CXCL12 antagonist for use of any one of Embodiments 1 to 333 and the interferon for use of any one of Embodiments 2 to 333, wherein the interferon is a type II interferon.

Embodiment 339. The CXCL12 antagonist for use of Embodiment 338 and the interferon for use of Embodiment 338, wherein the type II interferon increases expression of MHC I on tumor cells.

Embodiment 340. The CXCL12 antagonist for use of any one of Embodiments 338 and 339 and the interferon for use of any one of Embodiments 336 and 337, wherein the type II interferon is interferon gamma.

Embodiment 341. The CXCL12 antagonist for use of Embodiment 340 and the interferon for use of Embodiment 340, wherein the type II interferon is interferon gamma comprising an amino acid sequence of SEQ ID NO: 245.

Embodiment 342. The CXCL12 antagonist for use of any one of Embodiments 1 to 341 and the interferon for use of any one of Embodiments 2 to 341, wherein the CXCL12 antagonist inhibits the binding of CXCL12 to CXCL12 receptor C-X-C chemokine receptor type 4 (CXCR4) and/or to CXCL12 receptor C-X-C chemokine receptor type 7 (CXCR7).

Embodiment 343. The CXCL12 antagonist for use of Embodiment 342 and the interferon for use of Embodiments 342, wherein the CXCL12 antagonist is binding to CXCL12.

Embodiment 344. The CXCL12 antagonist for use of Embodiment 343 and the interferon for use of Embodiments 343, wherein the CXCL12 antagonist is a nucleic acid molecule binding to CXCL12, wherein the nucleic acid molecule is preferably selected from the group comprising an SDF-1 binding nucleic acid molecule of type B, an SDF-1 binding nucleic acid molecule of type C, an SDF-1 binding nucleic acid molecule of type A and an SDF-1 binding nucleic acid molecule of type D.

Embodiment 345. The CXCL12 antagonist for use of Embodiment 343 and the interferon for use of Embodiments 344, wherein the SDF-1 binding nucleic acid molecule of type B comprises a central stretch of nucleotides, whereby the central stretch of nucleotides comprises the following nucleotide sequence:
5' GUGUGAUCUAGAUGUADWGGCUGWUCCUAGUYAGG 3' (SEQ ID NO: 52).

Embodiment 346. The CXCL12 antagonist for use of Embodiment 345 and the interferon for use of Embodiments 345, wherein the central stretch of nucleotides comprises the following nucleotide sequence:
5' GUGUGAUCUAGAUGUADUGGCUGAUCCUAGUCAGG 3' (SEQ ID NO: 53).

Embodiment 347. The CXCL12 antagonist for use of any one of Embodiments 345 and 346 and the interferon for use of any one of Embodiments 345 and 346, wherein the SDF-1 binding nucleic acid molecule of type B comprises in 5'->3' direction a first terminal stretch of nucleotides, the central stretch of nucleotides, and a second terminal stretch of nucleotides.

Embodiment 348. The CXCL12 antagonist for use of any one of Embodiments 345 and 346 and the interferon for use of any one of Embodiments 345 and 346, wherein the SDF-1 binding nucleic acid molecule of type B comprises in 5'->3' direction a second terminal stretch of nucleotides, the central stretch of nucleotides, and a first terminal stretch of nucleotides.

Embodiment 349. The CXCL12 antagonist for use of any one of Embodiments 347 and 348 and the interferon for use of any one of Embodiments 347 and 348, wherein the first terminal stretch of nucleotides comprises a nucleotide sequence of 5' X1X2SVNS 3' and the second terminal stretch of nucleotides comprises a nucleotide sequence of 5' BVBSX3X4 3', whereby
X1 is either absent or is A, X2 is G, X3 is C and X4 is either absent or is U;
   or
X1 is absent, X2 is either absent or is G, X3 is either absent or is C and X4 is absent.

Embodiment 350. The CXCL12 antagonist for use of any one of Embodiments 347 to 349 and the interferon for use of any one of Embodiments 347 to 349, wherein the first terminal stretch of nucleotides comprises a nucleotide sequence of 5' X1X2CRWG 3' and the second terminal stretch of nucleotides comprises a nucleotide sequence of 5' KRYSX3X4 3',
whereby X1 is either absent or A, X2 is G, X3 is C and X4 is either absent or U.

Embodiment 351. The CXCL12 antagonist for use of any one of Embodiments 347 to 350 and the interferon for use of any one of Embodiments 347 to 350, preferably embodiment 349 or 350, wherein the first terminal stretch of nucleotides comprises a nucleotide sequence of 5' X1X2CGUG 3' and the second terminal stretch of nucleotides comprises a nucleotide sequence of 5' UACGX3X4 3',
whereby X1 is either absent or A, X2 is G, X3 is C, and X4 is either absent or U,
preferably the first terminal stretch of nucleotides comprises a nucleotide sequence of 5' AGCGUG 3' and the second terminal stretch of nucleotides comprises a nucleotide sequence of 5' UACGCU 3'.

Embodiment 352. The CXCL12 antagonist for use of any one of Embodiments 347 to 350 and the interferon for use of any one of Embodiments 347 to 350, preferably embodiment 349, wherein the first terminal stretch of nucleotides comprises a nucleotide sequence of 5' X1X2SSBS 3' and the second terminal stretch of nucleotides comprises a nucleotide sequence of 5' BVSSX3X4 3',
whereby X1 is absent, X2 is either absent or G, X3 is either absent or C, and X4 is absent, preferably the first terminal stretch of nucleotides comprises a nucleotide sequence of 5' GCGUG 3' and the second terminal stretch of nucleotides comprises a nucleotide sequence of 5' UACGC 3'.

Embodiment 353. The CXCL12 antagonist for use of any one of Embodiments 344 to 352 and the interferon for use of any one of Embodiments 344 to 352, wherein the SDF-1 binding nucleic acid molecule of type B comprises a nucleotide sequence according to any one of SEQ ID NO: 5 to SEQ ID NO: 20 and SEQ ID NO: 22 to SEQ ID NO: 28 or a nucleotide sequence at least 80 % identical thereto,
preferably any one of SEQ ID NO: 5 to SEQ ID NO: 7, SEQ ID NO: 16, SEQ ID NO: 22 and SEQ ID NO: 28 or a nucleotide sequence at least 80 % identical thereto,
more preferably any one of SEQ ID NO: 22 and SEQ ID NO: 28 or a nucleotide sequence at least 80 % identical thereto.

Embodiment 354. The CXCL12 antagonist for use of Embodiment 344 and the interferon for use of Embodiment 344, wherein the SDF-1 binding nucleic acid molecule of type C comprises a central stretch of nucleotides, whereby the central stretch of nucleotides comprises a nucleotide sequence of GGUYAGGGCUHRX_{A}AGUCGG (SEQ ID NO: 108),
whereby X_{A} is either absent or is A.

Embodiment 355. The CXCL12 antagonist for use of Embodiment 354 and the interferon for use of Embodiment 354, wherein the central stretch of nucleotides comprises a nucleotide sequence of 5' GGUYAGGGCUHRAAGUCGG 3' (SEQ ID NO: 109), 5' GGUYAGGGCUHRAGUCGG 3' (SEQ ID NO: 110) or 5' GGUUAGGGCUHGAAGUCGG 3' (SEQ ID NO: 111), preferably 5' GGUUAGGGCUHGAAGUCGG 3' (SEQ ID NO: 111).

Embodiment 356. The CXCL12 antagonist for use of any one of Embodiments 344 to 352 and the interferon for use of any one of Embodiments 354 to 355, wherein the SDF-1 binding nucleic acid molecule of type C comprises in 5'->3' direction a first terminal stretch of nucleotides, the central stretch of nucleotides, and a second terminal stretch of nucleotides.

Embodiment 357. The CXCL12 antagonist for use of any one of Embodiments 344 to 352 and the interferon for use of any one of Embodiments 354 to 355, wherein the SDF-1 binding nucleic acid molecule of type C comprises in 5'->3' direction a second terminal stretch of nucleotides, the central stretch of nucleotides, and a first terminal stretch of nucleotides.

Embodiment 358. The CXCL12 antagonist for use of any one of Embodiments 356 to 357 and the interferon for use of any one of Embodiments 356 to 357, wherein the first terminal stretch of nucleotides comprises a nucleotide sequence of 5' RKSBUSNVGR 3' (SEQ ID NO: 138) and the second stretch of nucleotides comprises a nucleotide sequence of 5' YYNRCASSMY 3' (SEQ ID NO: 139),
preferably the first terminal stretch of nucleotides comprises a nucleotide sequence of 5' RKSBUGSVGR 3' (SEQ ID NO: 140) and the second terminal stretch of nucleotides comprises a nucleotide sequence of 5' YCNRCASSMY 3' (SEQ ID NO: 141).

Embodiment 359. The CXCL12 antagonist for use of any one of Embodiments 356 to 357 and the interferon for use of any one of Embodiments 356 to 357, wherein the first terminal stretch of nucleotides comprises a nucleotide sequence of 5' XSSSSV 3' and the second terminal stretch of nucleotides comprises a nucleotide sequence of 5' BSSSXS 3', whereby Xs is either absent or is S,
preferably the first terminal stretch of nucleotides comprises a nucleotide sequence of 5' SGGSR 3' and the second terminal stretch of nucleotides comprises a nucleotide sequence of 5' YSCCS 3'.

Embodiment 360. The CXCL12 antagonist for use of any one of Embodiments 356 to 357 and the interferon for use of any one of Embodiments 356 to 357, wherein
a) the first terminal stretch of nucleotides comprises a nucleotide sequence of 5' GCCGG 3' and the second terminal stretch of nucleotides comprises a nucleotide sequence of 5' CCGGC 3'; or
b) the first terminal stretch of nucleotides comprises a nucleotide sequence of 5' CGUGCGCUUGAGAUAGG 3' (SEQ ID NO: 220) and the second terminal stretch of nucleotides comprises a nucleotide sequence of 5' CUGAUUCUCACG 3' (SEQ ID NO: 221); or
c) the first terminal stretch of nucleotides comprises a nucleotide sequence of 5' UGAGAUAGG 3' and the second terminal stretch of nucleotides comprises a nucleotide sequence of 5' CUGAUUCUCA 3' (SEQ ID NO: 222); or
d) the first terminal stretch of nucleotides comprises a nucleotide sequence of 5' GAGAUAGG 3' and the second terminal stretch of nucleotides comprises a nucleotide sequence of 5' CUGAUUCUC 3'.

Embodiment 361. The CXCL12 antagonist for use of any one of Embodiments 354 to 360 and the interferon for use of any one of Embodiments 354 to 360, wherein the type C SDF-1 binding nucleic acid molecule comprises a nucleotide sequence according to any one of SEQ ID NO: 95 to SEQ ID NO: 107, SEQ ID NO: 112 to SEQ ID NO: 137, SEQ ID NO: 223 and SEQ ID NO: 224,
preferably any one of SEQ ID NO: 120, SEQ ID NO: 128, SEQ ID NO: 129, SEQ ID NO:134, SEQ ID NO: 135, SEQ ID NO: 223 and SEQ ID NO: 224.

Embodiment 362. The CXCL12 antagonist for use of Embodiments 344 and the interferon for use of Embodiment 344, wherein the SDF-1 binding nucleic acid molecule of type A comprises a central stretch of nucleotides, whereby the central stretch of nucleotides comprises a nucleotide sequence of 5' AAAGYRACAHGUMAAX_{A}UGAAAGGUARC 3' (SEQ ID NO: 74),
whereby X_{A} is either absent or is A.

Embodiment 363. The CXCL12 antagonist for use of Embodiments 362 and the interferon for use of Embodiment 362, wherein the central stretch of nucleotides comprises a nucleotide sequence of
5'AAAGYRACAHGUMAAUGAAAGGUARC 3' (SEQ ID NO: 75), or
5' AAAGYRACAHGUMAAAUGAAAGGUARC 3' (SEQ ID NO: 76), or
5' AAAGYAACAHGUCAAUGAAAGGUARC 3'(SEQ ID NO: 77), preferably the central stretch of nucleotides comprises a nucleotide sequence of 5' AAAGYAACAHGUCAAUGAAAGGUARC 3' (SEQ ID NO: 77).

Embodiment 364. The CXCL12 antagonist for use of any one of Embodiments 354 to 360 and the interferon for use of any one of Embodiments 354 to 360, wherein the SDF-1 binding nucleic acid molecule of type A comprises in 5'->3' direction a first terminal stretch of nucleotides, the central stretch of nucleotides, and a second terminal stretch of nucleotides.

Embodiment 365. The CXCL12 antagonist for use of any one of Embodiments 362 to 363 and the interferon for use of any one of Embodiments 362 to 363, wherein the SDF-1 binding nucleic acid molecule of type A comprises in 5'->3' direction a second terminal stretch of nucleotides, the central stretch of nucleotides, and a first terminal stretch of nucleotides.

Embodiment 366. The CXCL12 antagonist for use of any one of Embodiments 364 to 365 and the interferon for use of any one of Embodiments 364 to 365, wherein the first terminal stretch of nucleotides comprises a nucleotide sequence of 5' X₁X₂NNBV 3' and the second terminal stretch of nucleotides comprises a nucleotide sequence of 5' BNBNX₃X₄ 3'
whereby X₁ is either absent or R, X₂ is S, X₃ is S and X₄ is either absent or Y;
   or
X₁ is absent, X₂ is either absent or S, X₃ is either absent or S and X₄ is absent.

Embodiment 367. The CXCL12 antagonist for use of any one of Embodiments 364 to 366, preferably Embodiment 366 and the interferon for use of any one of Embodiments 364 to 366, preferably Embodiment 366, wherein the first terminal stretch of nucleotides comprises a nucleotide sequence of 5' RSHRYR 3' and the second terminal stretch of nucleotides comprises a nucleotide sequence of 5' YRYDSY 3', preferably the first terminal stretch of nucleotides comprises a nucleotide sequence of 5' GCUGUG 3' and the second terminal stretch of nucleotides comprises a nucleotide sequence of 5' CGCAGC 3'.

Embodiment 368. The CXCL12 antagonist for use of any one of Embodiments 364 to 366, preferably Embodiment 366, and the interferon for use of any one of Embodiments 364 to 366, preferably Embodiment 366, wherein the first terminal stretch of nucleotides comprises a nucleotide sequence of 5' X₂BBBS 3' and the second terminal stretch of nucleotides comprises a nucleotide sequence of 5' SBBVX₃ 3',
whereby X₂ is either absent or is S and X₃ is either absent or is S;
preferably the first terminal stretch of nucleotides comprises a nucleotide sequence of 5' CUGUG 3' and the second terminal stretch of nucleotides comprises a nucleotide sequence of 5' CGCAG 3';
or the first terminal stretch of nucleotides comprises a nucleotide sequence of 5' GCGUG 3'and the second terminal stretch of nucleotides comprises a nucleotide sequence of 5' CGCGC 3'.

Embodiment 369. The CXCL12 antagonist for use of any one of Embodiments 362 to 368 and the interferon for use of any one of Embodiments 362 to 368, wherein the SDF-1 binding nucleic acid molecule of type A comprises a nucleotide sequence according to any one of SEQ ID NO: 60 to SEQ ID NO: 73, SEQ ID NO: 78 to SEQ ID NO: 82, SEQ ID NO: 84 to SEQ ID NO: 87, SEQ ID NO: 89 to SEQ ID NO: 94, and SEQ ID NO: 145 or a nucleotide sequence at least 80 % identical thereto,
preferably any one of SEQ ID NO: 60, SEQ ID NO: 63, SEQ ID NO: 66, SEQ ID NO: 78, SEQ ID NO: 84, and SEQ ID NO: 146 or a nucleotide sequence at least 80 % identical thereto,
more preferably any one of SEQ ID NO: 84 and SEQ ID NO: 146 or a nucleotide sequence at least 80 % identical thereto.

Embodiment 370. The CXCL12 antagonist for use of Embodiment 344 and the interferon for use of Embodiment 344, wherein the SDF-1 binding nucleic acid molecule of type D comprises a nucleotide sequence according to any one of SEQ ID NO: 142 to SEQ ID NO: 144 or a nucleotide sequence at least 80 % identical thereto.

Embodiment 371. The CXCL12 antagonist for use of any one of Embodiments 344 to 370 and the interferon for use of any one of Embodiments 344 to 370, wherein the nucleic acid molecule comprises a modification, whereby the modification is preferably a high molecular weight moiety and/or whereby the modification preferably allows to modify the characteristics of the nucleic acid molecule in terms of residence time in the animal or human body, preferably the human body.

Embodiment 372. The CXCL12 antagonist for use of Embodiment 371 and the interferon for use of Embodiment 371, wherein the modification is selected from the group comprising a HES moiety, a PEG moiety, biodegradable modifications and combinations thereof.

Embodiment 373. The CXCL12 antagonist for use of Embodiment 372 and the interferon for use of Embodiment 372, wherein the modification is a PEG moiety consisting of a straight PEG or branched PEG, whereby preferably the molecular weight of the straight or branched PEG is from about 20,000 to 120,000 Da, more preferably from about 30,000 to 80,000 Da and most preferably about 40,000 Da.

Embodiment 374. The CXCL12 antagonist for use of Embodiment 372 and the interferon for use of Embodiment 372, wherein the modification is a HES moiety, whereby preferably the molecular weight of the HES moiety is from about 10,000 to 200,000 Da, more preferably from about 30,000 to 170.000 Da and most preferably about 150,000 Da.

Embodiment 375. The CXCL12 antagonist for use of any one of Embodiments 371 to 374 and the interferon for use of any one of Embodiments 371 to 374, wherein the modification is attached to the nucleic acid molecule via a linker, wherein preferably the linker is a biostable or biodegradable linker.

Embodiment 376. The CXCL12 antagonist for use of any one of Embodiments 371 to 375 and the interferon for use of any one of Embodiments 371 to 375, wherein the modification is attached to the nucleic acid molecule at the 5'-terminal nucleotide of the nucleic acid molecule and/or the 3'-terminal nucleotide of the nucleic acid molecule and/or to a nucleotide of the nucleic acid molecule between the 5'-terminal nucleotide of the nucleic acid molecule and the 3'-terminal nucleotide of the nucleic acid molecule.

Embodiment 377. The CXCL12 antagonist for use of any one of Embodiments 344 to 376 and the interferon for use of any one of Embodiments 344 to 376, wherein the nucleotides of the nucleic acid molecule or the nucleotides forming the nucleic acid molecule are L-nucleotides.

Embodiment 378. The CXCL12 antagonist for use of any one of Embodiments 344 to 377 and the interferon for use of any one of Embodiments 344 to 377, wherein the nucleic acid molecule is an **L-**nucleic acid molecule.

Embodiment 379. The CXCL12 antagonist for use of Embodiment 344 and the interferon for use of Embodiment 344, wherein the nucleic acid molecule is an L-ribonucleic acid molecule comprising a nucleotide sequence of
GCGUGGUGUGAUCUAGAUGUAUUGGCUGAUCCUAGUCAGGUACGC (SEQ ID NO: 22).

Embodiment 380. The CXCL12 antagonist for use of Embodiment 379 and the interferon for use of Embodiment 379, wherein the L-ribonucleic acid molecule comprises a 40 kDa polyethylene glycol moiety, wherein the 40 kDa polyethylene glycol moiety is attached to the 5'end of nucleotide sequence.

Embodiment 381. The CXCL12 antagonist for use of Embodiment 380 and the interferon for use of Embodiment 380, wherein N-[ω-methoxypoly(oxy-1,2-ethanediyl], N'-[ω-methoxypoly(oxy-1,2-ethanediyl]-acetyl-9-amino-8-oxo-7-aza-nonyloxy phosphate is interspersed between the 5' terminal nucleotide and the 40 kDa polyethylene glycol moiety.

Embodiment 382. The CXCL12 antagonist for use of Embodiment 381 and the interferon for use of Embodiment 381, wherein the L-ribonucleic acid molecule is present as a sodium salt, preferably the L-nucleic acid molecule is NOX-A12.

Embodiment 383. The CXCL12 antagonist for use of Embodiment 342 and the interferon for use of Embodiment 342, wherein the CXCL12 antagonist is an aptamer binding to CXCL12.

Embodiment 384. The CXCL12 antagonist for use of Embodiment 342 and the interferon for use of Embodiment 342, wherein the CXCL12 antagonist is an antibody binding to CXCL12.

Embodiment 385 The CXCL12 antagonist for use of Embodiment 384 and the interferon for use of Embodiment 384, wherein the antibody binding to CXCL12 is mAB-30D8, 1131-H12 or 1143-H1.

Embodiment 386. The CXCL12 antagonist for use of Embodiment 342 and the interferon for use of Embodiment 342, wherein the CXCL12 antagonist is a CXCL12 binding protein.

Embodiment 387. The CXCL12 antagonist for use of Embodiment 386 and the interferon for use of Embodiment 386, wherein the protein is a TRAP protein or an anticalin.

Embodiment 388. The CXCL12 antagonist for use of Embodiment 342 and the interferon for use of Embodiment 342, wherein the CXCL12 antagonist targets CXCR4.

Embodiment 389. The CXCL12 antagonist for use of Embodiment 388 and the interferon for use of Embodiment 388, wherein the CXCL12 antagonist is binding to CXCR4.

Embodiment 390. The CXCL12 antagonist for use of any one of Embodiments 388 to 389 and the interferon for use of any one of Embodiments 388 to 389, wherein the CXCR4 binding CXCL12 antagonist is selected from the group comprising a small molecule, a peptide, a synthetic peptide, a recombinant protein, a fusion protein and an antibody.

Embodiment 391. The CXCL12 antagonist for use of Embodiment 390 and the interferon for use of Embodiment 390, wherein the CXCR4 binding CXCL12 antagonist is a small molecule.

Embodiment 392. The CXCL12 antagonist for use of Embodiment 391 and the interferon for use of Embodiment 391, wherein the CXCR4 binding CXCL12 antagonist is a small molecule selected from the group comprising plerixafor, mavorixafor, Q-122, HPH-112, BKT-300, X4P-002, X4P-003, X4-136, GP-01CR01, GP-01CR11, GP-01CR21, Pentixather, BKT-170, BMS-585248, CS-3955, CTCE-0012, CTCE-9908, GBV-4086, HPH-112, HPH-211, NB-325, SP-10 and USL-311.

Embodiment 393. The CXCL12 antagonist for use of Embodiment 392 and the interferon for use of Embodiment 392, wherein the small molecule is selected from the group comprising plerixafor, mavorixafor, Q-122, HPH-112, BKT-300, X4P-002, X4P-003, X4-136, GP-01CR01, GP-01CR11, GP-01CR21, and pentixather.

Embodiment 394. The CXCL12 antagonist for use of Embodiment 390 and the interferon for use of Embodiment 390, wherein the CXCR4 binding CXCL12 antagonist is a peptide.

Embodiment 395. The CXCL12 antagonist for use of Embodiment 394 and the interferon for use of Embodiment 394, wherein the CXCR4 binding CXCL12 antagonist is motixafortide.

Embodiment 396. The CXCL12 antagonist for use of Embodiment 390 and the interferon for use of Embodiment 390, wherein the CXCR4 binding CXCL12 antagonist is a synthetic peptide.

Embodiment 397. The CXCL12 antagonist for use of Embodiment 396 and the interferon for use of Embodiment 396, wherein the CXCR4 binding CXCL12 antagonist is a synthetic peptide selected from the group comprising balixafortide, LY-2510924, ALB-408 and POL-5551.

Embodiment 398. The CXCL12 antagonist for use of Embodiment 397 and the interferon for use of Embodiment 397, wherein the CXCR4 binding CXCL12 antagonist is selected from the group comprising balixafortide, and LY-2510924.

Embodiment 399. The CXCL12 antagonist for use of Embodiment 390 and the interferon for use of Embodiment 390, wherein the CXCR4 binding CXCL12 antagonist is a recombinant protein.

Embodiment 400. The CXCL12 antagonist for use of Embodiment 399 and the interferon for use of Embodiment 399, wherein the recombinant protein is selected from the group comprising PTX-9098 and NNL-121.

Embodiment 401. The CXCL12 antagonist for use of Embodiment 390 and the interferon for use of Embodiment 390, wherein the CXCR4 binding CXCL12 antagonist is a fusion protein.

Embodiment 402. The CXCL12 antagonist for use of Embodiment 401 and the interferon for use of Embodiment 401, wherein the fusion protein is selected from the group comprising AD-214 and AM-3114.

Embodiment 403. The CXCL12 antagonist for use of Embodiment 390 and the interferon for use of Embodiment 390, wherein the CXCR4 binding CXCL12 antagonist is an antibody or an antigen-binding fragment of the antibody.

Embodiment 404. The CXCL12 antagonist for use of Embodiment 403 and the interferon for use of Embodiment 403, wherein the antibody is a monoclonal antibody.

Embodiment 405. The CXCL12 antagonist for use of any one of Embodiments 403 and 404 the interferon for use of any one of Embodiments 403 and 404, wherein the CXCR4 binding CXCL12 antagonist is an antibody.

Embodiment 406. The CXCL12 antagonist for use of Embodiment 405 and the interferon for use of Embodiment 405, wherein the antibody is a monoclonal antibody selected from the group comprising ulocuplumab, hz-515H7, KY-1051, PF-06747143, ALX-0651, AT-009, LY-2624587 and STIA-220X.

Embodiment 407. The CXCL12 antagonist for use of Embodiment 406 and the interferon for use of Embodiment 406, wherein the antibody is a monoclonal antibody selected from the group comprising ulocuplumab, hz-515H7 and KY-1051.

Embodiment 408. The CXCL12 antagonist for use of Embodiment 342 and the interferon for use of Embodiment 342, wherein the CXCL12 antagonist is binding to CXCR7.

Embodiment 409. The CXCL12 antagonist for use of Embodiment 408 and the interferon for use of Embodiment 408, wherein the CXCR7 binding CXCL12 antagonist is selected from the group comprising a small molecule, a synthetic peptide and an antibody.

Embodiment 410. The CXCL12 antagonist for use of Embodiment 409 and the interferon for use of Embodiment 409, wherein the CXCR7 binding CXCL12 antagonist is a small molecule.

Embodiment 411. The CXCL12 antagonist for use of Embodiment 410 and the interferon for use of Embodiment 410, wherein the CXCR7 binding CXCL12 antagonist is a small molecule selected from the group comprising ACT-10041239, CCX-650, CCX-662 and CCX-771.

Embodiment 412. The CXCL12 antagonist for use of Embodiment 411 and the interferon for use of Embodiment 411, wherein the CXCR7 binding CXCL12 antagonist is ACT-10041239.

Embodiment 413. The CXCL12 antagonist for use of Embodiment 409 and the interferon for use of Embodiment 409, wherein the CXCR7 binding CXCL12 antagonist is a synthetic peptide.

Embodiment 414. The CXCL12 antagonist for use of Embodiment 413 and the interferon for use of Embodiment 413, wherein the synthetic peptide is selected from the group comprising LIH-383 and POL-6926.

Embodiment 415. The CXCL12 antagonist for use of Embodiment 414 and the interferon for use of Embodiment 414, wherein the synthetic peptide is LIH-383.

Embodiment 416. The CXCL12 antagonist for use of Embodiment 409 and the interferon for use of Embodiment 409, wherein the CXCR7 binding CXCL12 antagonist is an antibody or an antigen-binding fragment of the antibody.

Embodiment 417. The CXCL12 antagonist for use of Embodiment 416 and the interferon for use of Embodiment 416, wherein the antibody is a monoclonal antibody.

Embodiment 418. The CXCL12 antagonist for use of any one of Embodiments 416 and 417 the interferon for use of any one of Embodiments 416 and 417, wherein the antibody is a monoclonal antibody selected from the group comprising JT-07, X-7Ab and STIA-230X.

Embodiment 419. The CXCL12 antagonist for use of Embodiment 418 and the interferon for use of Embodiment 418, wherein the antibody is a monoclonal antibody selected from the group comprising JT-07 and X-7Ab.

The problem underlying the present invention is solved in a first aspect which is also a first embodiment of the first aspect, by a CXCL12 antagonist for use in a method for treating a tumor in a subject, wherein the method comprises administering to the subject the CXCL12 antagonist and an interferon, wherein the tumor is an immunologically cold tumor.

The problem underlying the present invention is solved in a second aspect which is also a first embodiment of the second aspect, by an interferon for use in a method for treating a tumor in a subject, wherein the method comprises administering to the subject the interferon and a CXCL12 antagonist, wherein the tumor is an immunologically cold tumor.

The problem underlying the present invention is solved in a third aspect which is also a first embodiment of the third aspect, by a CXCL12 antagonist for use in a method for treating a tumor in a subject, wherein the method comprises administering to the subject the CXCL12 antagonist and an interferon, wherein the tumor is refractory to immunotherapy.

The problem underlying the present invention is solved in a fourth aspect which is also a first embodiment of the fourth aspect, by an interferon for use in a method for treating a tumor in a subject, wherein the method comprises administering to the subject the interferon and a CXCL12 antagonist, wherein the tumor is refractory to immunotherapy.

The problem underlying the present invention is solved in a fifth aspect which is also a first embodiment of the fifth aspect, by a CXCL12 antagonist for use in a method for treating a tumor in a subject, wherein the method comprises administering to the subject the CXCL12 antagonist and an interferon, wherein the tumor is a solid tumor.

The problem underlying the present invention is solved in a sixth aspect which is also a first embodiment of the sixth aspect, by an interferon for use in a method for treating a tumor in a subject, wherein the method comprises administering to the subject the interferon and a CXCL12 antagonist, wherein the tumor is a solid tumor.

The problem underlying the present invention is solved in a seventh aspect which is also a first embodiment of the seventh aspect, by a CXCL12 antagonist for use in a method for treating a tumor in a subject, wherein the method comprises administering to the subject the CXCL12 antagonist, an interferon and an adjuvant therapy, wherein the tumor is an immunologically cold tumor.

The problem underlying the present invention is solved in an eighth aspect which is also a first embodiment of the eighth aspect, by an interferon for use in a method for treating a tumor in a subject, wherein the method comprises administering to the subject the interferon, a CXCL12 antagonist and an adjuvant therapy, wherein the tumor is an immunologically cold tumor.

The problem underlying the present invention is solved in a ninth aspect which is also a first embodiment of the ninth aspect, by a CXCL12 antagonist for use in a method for treating a tumor in a subject, wherein the method comprises administering to the subject the CXCL12 antagonist, an interferon and adjuvant therapy, wherein the tumor is refractory to immunotherapy.

The problem underlying the present invention is solved in a tenth aspect which is also a first embodiment of the tenth aspect, by an interferon for use in a method for treating a tumor in a subject, wherein the method comprises administering to the subject the interferon, a CXCL12 antagonist and an adjuvant therapy, wherein the tumor is refractory to immunotherapy.

The problem underlying the present invention is solved in an eleventh aspect which is also a first embodiment of the eleventh aspect, by a CXCL12 antagonist for use in a method for treating a tumor in a subject, wherein the method comprises administering to the subject the CXCL12 antagonist, an interferon and an adjuvant therapy, wherein the tumor is a solid tumor.

The problem underlying the present invention is solved in a twelfth aspect which is also a first embodiment of the twelfth aspect, by an interferon for use in a method for treating a tumor in a subject, wherein the method comprises administering to the subject the interferon, a CXCL12 antagonist and an adjuvant therapy, wherein the tumor is a solid tumor.

The problem underlying the present invention is solved in a 13^{th} aspect which is also a first embodiment of the 13^{th} aspect, by a CXCL12 antagonist for use in a method for treating a tumor in a subject, wherein the method comprises administering to the subject a primary therapy and an adjunct therapy, wherein the adjunct therapy comprises administering to the subject the CXCL12 antagonist and an interferon, wherein the tumor is an immunologically cold tumor.

The problem underlying the present invention is solved in a 14^{th} aspect which is also a first embodiment of the 14^{th} aspect, by an interferon for use in a method for treating a tumor in a subject, wherein the method comprises administering to the subject a primary therapy and an adjunct therapy, wherein the adjunct therapy comprises administering to the subject the interferon and a CXCL12 antagonist, wherein the tumor is an immunologically cold tumor.

The problem underlying the present invention is solved in a 15^{th} aspect which is also a first embodiment of the 15^{th} aspect, by a CXCL12 antagonist for use in a method for treating a tumor in a subject, wherein the method comprises administering to the subject a primary therapy and an adjunct therapy, wherein the adjunct therapy comprises administering to the subject the CXCL12 antagonist and an interferon, wherein the tumor is refractory to immunotherapy.

The problem underlying the present invention is solved in a 16^{th} aspect which is also a first embodiment of the 16^{th} aspect, by an interferon for use in a method for treating a tumor in a subject, wherein the method comprises administering to the subject a primary therapy and an adjunct therapy, wherein the adjunct therapy comprises administering to the subject the interferon and a CXCL12 antagonist, wherein the tumor is refractory to immunotherapy.

The problem underlying the present invention is solved in a 17^{th} aspect which is also a first embodiment of the 17^{th} aspect, by a CXCL12 antagonist for use in a method for treating a tumor in a subject, wherein the method comprises administering to the subject a primary therapy and an adjunct therapy, wherein the adjunct therapy comprises administering to the subject the CXCL12 antagonist and an interferon, wherein the tumor is a solid tumor.

The problem underlying the present invention is solved in an 18^{th} aspect which is also a first embodiment of the 18^{th} aspect, by an interferon for use in a method for treating a tumor in a subject, wherein the method comprises administering to the subject a primary therapy and an adjunct therapy, wherein the adjunct therapy comprises administering to the subject the interferon and a CXCL12 antagonist, wherein the tumor is a solid tumor.

The problem underlying the present invention is solved in an 19^{th} aspect which is also a first embodiment of the 19^{th} aspect, by a CXCL12 antagonist for use in a method for converting an immunologically cold tumor in a subject into an immunologically hot tumor in the subject, wherein the method comprises administering to the subject the CXCL12 antagonist and an interferon, wherein upon administration of the CXCL12 antagonist and the interferon the immunologically cold tumor is or will be converted into an immunologically hot tumor.

The problem underlying the present invention is solved in a 20^{th} aspect which is also a first embodiment of the 20^{th} aspect, by an interferon for use in a method for converting an immunologically cold tumor in a subject into an immunologically hot tumor in the subject, wherein the method comprises administering to the subject the interferon and a CXCL12 antagonist, wherein upon administration of the interferon and the CXCL12 antagonist the immunologically cold tumor is or will be converted into an immunologically hot tumor.

The problem underlying the present invention is solved in a 21^{st} aspect which is also a first embodiment of the 21^{st} aspect, by a CXCL12 antagonist for use in a method for converting an immunotherapy refractory tumor in a subject into an immunotherapy non-refractory tumor in the subject, wherein the method comprises administering to the subject the CXCL12 antagonist and an interferon, wherein upon administration of the CXCL12 antagonist and the interferon the immunotherapy refractory tumor is or will be converted into an immunotherapy non-refractory tumor.

The problem underlying the present invention is solved in a 22^{nd} aspect which is also a first embodiment of the 22^{nd} aspect, by an interferon for use in a method for converting an immunotherapy refractory tumor in a subject into an immunotherapy non-refractory tumor in the subject, wherein the method comprises administering to the subject the interferon and a CXCL12 antagonist, wherein upon administration of the interferon and the CXCL12 antagonist the immunotherapy refractory tumor is or will be converted into an immunotherapy non-refractory tumor.

The problem underlying the present invention is solved in a 23^{rd} aspect which is also a first embodiment of the 23^{rd} aspect, by a method for treating a tumor in a subject, wherein the method comprises administering to the subject a CXCL12 antagonist and an interferon, wherein the tumor is an immunologically cold tumor.

The problem underlying the present invention is solved in a 24^{th} aspect which is also a first embodiment of the 24^{th} aspect, by a method for treating a tumor in a subject, wherein the method comprises administering to the subject a CXCL12 antagonist and an interferon, wherein the tumor is refractory to immunotherapy

The problem underlying the present invention is solved in a 25^{th} aspect which is also a first embodiment of the 25^{th} aspect, by a method for treating a tumor in a subject, wherein the method comprises administering to the subject a CXCL12 antagonist and an interferon, wherein the tumor is a solid tumor.

The problem underlying the present invention is solved in a 26^{th} aspect which is also a first embodiment of the 26^{th} aspect, by a method for treating a tumor in a subject, wherein the method comprises administering to the subject the CXCL12 antagonist, an interferon and an adjuvant therapy, wherein the tumor is an immunologically cold tumor.

The problem underlying the present invention is solved in a 27^{th} aspect which is also a first embodiment of the 27^{th} aspect, by a method for treating a tumor in a subject, wherein the method comprises administering to the subject a CXCL12 antagonist, an interferon and adjuvant therapy, wherein the tumor is refractory to immunotherapy.

The problem underlying the present invention is solved in a 28^{th} aspect which is also a first embodiment of the 28^{th} aspect, by a method for treating a tumor in a subject, wherein the method comprises administering to the subject a CXCL12 antagonist, an interferon and an adjuvant therapy, wherein the tumor is a solid tumor.

The problem underlying the present invention is solved in a 29^{th} aspect which is also a first embodiment of the 29^{th} aspect, by a method for treating a tumor in a subject, wherein the method comprises administering to the subject a primary therapy and an adjunct therapy, wherein the adjunct therapy comprises administering to the subject a CXCL12 antagonist and an interferon, wherein the tumor is an immunologically cold tumor.

The problem underlying the present invention is solved in a 30^{th} aspect which is also a first embodiment of the 30^{th} aspect, by a method for treating a tumor in a subject, wherein the method comprises administering to the subject a primary therapy and an adjunct therapy, wherein the adjunct therapy comprises administering to the subject a CXCL12 antagonist and an interferon, wherein the tumor is refractory to immunotherapy.

The problem underlying the present invention is solved in a 31^{st} aspect which is also a first embodiment of the 31^{st} aspect, by a method for treating a tumor in a subject, wherein the method comprises administering to the subject a primary therapy and an adjunct therapy, wherein the adjunct therapy comprises administering to the subject a CXCL12 antagonist and an interferon, wherein the tumor is a solid tumor.

The problem underlying the present invention is solved in a 32^{nd} aspect which is also a first embodiment of the 32^{nd} aspect, by a method for converting an immunologically cold tumor in a subject into an immunologically hot tumor in the subject, wherein the method comprises administering to the subject a CXCL12 antagonist and an interferon, wherein upon administration of the CXCL12 antagonist and the interferon the immunologically cold tumor is or will be converted into an immunologically hot tumor.

The problem underlying the present invention is solved in a 33^{rd} aspect which is also a first embodiment of the 33^{rd} aspect, by a method for converting an immunotherapy refractory tumor in a subject into an immunotherapy non-refractory tumor in the subject, wherein the method comprises administering to the subject a CXCL12 antagonist and an interferon, wherein upon administration of the CXCL12 antagonist and the interferon the immunotherapy refractory tumor is or will be converted into an immunotherapy non-refractory tumor.

It will be appreciated that the first aspect, the second aspect and the 23^{rd} aspect are related and that each and any embodiment of one of these aspects, including any embodiment of these aspects, will also be an embodiment of the other of these aspects, including any embodiment of the other of these aspects.

It will be appreciated that the third aspect, the fourth aspect and the 24^{th} aspect are related and that each and any embodiment of one of these aspects, including any embodiment of these aspects, will also be an embodiment of the other of these aspects, including any embodiment of the other of these aspects.

It will be appreciated that the fifth aspect, the sixth aspect and the 25^{th} aspect are related and that each and any embodiment of one of these aspects, including any embodiment of these aspects, will also be an embodiment of the other of these aspects, including any embodiment of the other of these aspects.

It will be appreciated that the seventh aspect, the eighth aspect and the 26^{th} aspect are related and that each and any embodiment of one of these aspects, including any embodiment of these aspects, will also be an embodiment of the other of these aspects, including any embodiment of the other of these aspects.

It will be appreciated that the ninth aspect, the tenth aspect and the 27^{th} aspect are related and that each and any embodiment of one of these aspects, including any embodiment of these aspects, will also be an embodiment of the other of these aspects, including any embodiment of the other of these aspects.

It will be appreciated that the eleventh aspect, the twelfth aspect and the 28^{th} aspect are related and that each and any embodiment of one of these aspects, including any embodiment of these aspects, will also be an embodiment of the other of these aspects, including any embodiment of the other of these aspects.

It will be appreciated that the 13^{th} aspect, the 14^{th} aspect and the 29^{th} aspect are related and that each and any embodiment of one of these aspects, including any embodiment of these aspects, will also be an embodiment of the other of these aspects, including any embodiment of the other of these aspects.

It will be appreciated that the 15^{th} aspect, the 16^{th} aspect and the 30^{th} aspect are related and that each and any embodiment of one of these aspects, including any embodiment of these aspects, will also be an embodiment of the other of these aspects, including any embodiment of the other of these aspects.

It will be appreciated that the 17^{th} aspect, the 18^{th} aspect and the 31^{st} aspect are related and that each and any embodiment of one of these aspects, including any embodiment of these aspects, will also be an embodiment of the other of these aspects, including any embodiment of the other of these aspects.

It will be appreciated that the 19^{th} aspect, the 20^{th} aspect and the 32^{nd} aspect are related and that each and any embodiment of one of these aspects, including any embodiment of these aspects, will also be an embodiment of the other of these aspects, including any embodiment of the other of these aspects.

It will be appreciated that the 21^{st} aspect, the 22^{nd} aspect and the 33^{rd} aspect are related and that each and any embodiment of one of these aspects, including any embodiment of these aspects, will also be an embodiment of the other of these aspects, including any embodiment of the other of these aspects.

It will be appreciated by a person skilled in the art that each and any embodiment of any aspect disclosed herein is also an embodiment of each and any of the other aspects, including any embodiments thereof, disclosed herein unless explicitly indicated to the contrary.

Any reference to the invention or to the present invention refers in particular to each and any aspect of the invention, including any embodiment of such each and any aspect. Also, the terms aspect and aspect of the invention or aspect of the present invention are used interchangeably and synonymously herein, unless indicated otherwise.

Without wishing to be bound by any theory, the present inventors have surprisingly found that administering both a CXCL12 antagonist and an interferon to a subject suffering from an immunologically cold tumor will transform or convert such immunologically cold tumor into an immunologically hot tumor. Such immunologically hot tumor is susceptible to the subject's own anti-tumor immune response and/or to immunotherapy applied or administered to the subject.

Furthermore and also without wishing to be bound by any theory, the present inventors have surprisingly found that administering both a CXCL12 antagonist and an interferon to a subject suffering from a tumor refractory to immunotherapy will transform or convert such tumor refractory to immunotherapy into a tumor non-refractory to immunotherapy and, respectively, susceptible to immunotherapy. Such tumor non-refractory to immunotherapy is susceptible to immunotherapy applied or administered to the subject and/or to the subject's anti-tumor immune response.

Finally and again without wishing to be bound by any theory, the present inventors have surprisingly found that administering both a CXCL12 antagonist and an interferon to a subject suffering from a solid tumor, preferably a pancreatic cancer, a synovial sarcoma or an osteosarcoma, allows an effective treatment of the solid tumor, in particular if such tumor is an immunologically cold tumor and/or a tumor refractory to immunotherapy.

It will be appreciated by a person skilled in the art that when reference is made to a distinct tumor entity in a subject, this translates in terms of disease from which the subject is suffering into the distinct cancer which is the same as the distinct tumor entity. In other words, if a person is suffering from, e.g., synovial sarcoma, the tumor in the person is also referred to as synovial sarcoma, and vice versa."

In an embodiment of each and any aspect, including any embodiment thereof, the tumor is an immunologically cold tumor. Immunologically cold tumors are cancers that lack an active immune response, making them harder to treat with immunotherapy alone. They often have a hostile tumor microenvironment that suppresses immune activation.

Immune checkpoint inhibitors (ICIs) have been approved to treat various tumors with demonstrated effectiveness (Miller, Nogueira et al. 2019) but many different tumors do not respond to ICI therapy, and even for tumor entities where ICI therapy is part of the standard of care, a large proportion of patients does not respond. A reason for this is the fact that tumors are capable of developing immune evasion mechanisms; therefore, tumors can adapts to acquire 'cold' properties (i.e., lack of effector immune cells in the tumor tissue) (Bai, Chen et al. 2020, Hegde and Chen 2020). Cold tumors are categorized into two types: (i) 'immune-excluded' tumors, where immune effector cells accumulate in the tumor periphery but are unable to infiltrate due to an immunosuppressive tumor microenvironment (TME); (ii) 'immune-desert' tumors, where also the surrounding tissues are devoid of immune effector cells due to low immunogenicity. In these cases, the non-targeted approach of immunostimulants as monotherapy is likely to be therapeutically futile. Combination therapy of immunostimulants together with ICIs could be promising in paving the way to overcome localized immunosuppression entailed by the TME. In particular, immune-excluded tumors could be reverted to 'hot' tumors (i.e., inflamed with a high presence of tumor-infiltrating lymphocytes [TILs]). When administered together with ICIs, these aforementioned immunostimulant drugs have shown a degree of success not only in pre-clinical cancer models (Charych, Hoch et al. 2016, Mariathasan, Turley et al. 2018, Sharma, Khong et al. 2020) but also in clinical trials.

In an embodiment of each and any aspect of the present invention, including any embodiment thereof, the tumor has an immune evasion mechanism. In accordance with this embodiment, the tumor evades the host immune system's surveillance instead of being eliminated. The mechanisms behind this are manifold. The major mechanisms underlying this are (1) camouflage, which hides cancer cells from immune recognition, (2) coercion, which directly or indirectly interferes with immune effector cells, and (3) cytoprotection, which shields malignant cells from immune cytotoxicity (see, e.g., (Galassi, Chan et al. 2024)5).

A key factor that determines whether certain tumors are cold or hot is related to the molecular characteristics of the tumor cells (microsatellite instability/mismatch repair defects, tumor mutational load, neoantigen load, etc.) (Samstein, Lee et al. 2019). "Hot tumors" as subject to various embodiments of the aspects are characterized by a TME rich in TILs, PD-L1 overexpression, genomic instability, and preexisting antitumor immune responses (Galon and Bruni 2019), whereas cold tumors, which usually do not respond to immunotherapy, have opposite characteristics (Sharma and Allison 2015).

In an embodiment of each and any aspect of the present invention, including any embodiment thereof, the tumor has at least one characteristic selected from the group comprising reduced or lacking expression of PD-L1, reduced number of tumor-infiltrating lymphocytes, genomic stability and reduced or lacking preexisting antitumor immune response. It will be appreciated by a person skilled in the art that the reference level for deciding whether there is a reduced or lacking expression of PD-L1, a reduced number of tumor-infiltrating lymphocytes, genomic stability and a reduced or lacking preexisting antitumor immune response, is known to a person skilled in the art and outlined in comprehensive reviews such as in Wang 2023 (Wang, Geng et al. 2023). Similarly, a person skilled in the art is able based on such reference level to determine whether at least one of said characteristics has been reverted which is indicative that the tumor is an immunologically hot tumor.

The immune escape mechanisms that can lead to establishment of cold tumors are signified by cell-autonomous mechanisms, such as loss of MHC class I expression or the elevated expression of inhibitory immune checkpoints (Bai, Chen et al. 2020, Hegde and Chen 2020). Notably, immune escape can be the consequence of changes in the immune cell composition of the TME that affect both the adaptive and the innate immune systems. Escape from the adaptive immune system can be due to the inhibition of cytotoxic tumor-infiltrating lymphocyte (TIL) immunosurveillance and activation, enhanced by the enrichment of regulatory T (Treg) cells in the TME. Treg cells are normally trained to recognize self-antigens and are thus responsible for maintaining self-tolerance. However, since cancer cells commonly express self-antigens beside unique neoantigens, Treg cells could, in principle, counteract the anti-cancer immune response (Ohue and Nishikawa 2019). With respect to the innate immune system, the presence of myeloid-derived suppressor cells (MDSCs), tumor- associated macrophages (TAMs), and tumor-associated neutrophils (TANs) indicates an immunosuppressive TME associated with poor prognosis (Petty, Owen et al. 2021, Ma, Renz et al. 2022). MDSCs and TANs originate from circulating BM-derived myeloid cells (Ma, Renz et al. 2022), while intra-tumoral TAMs either originate from tissue-resident macrophages or differentiate from recruited MDSCs/ monocytes (Petty, Owen et al. 2021). All these cell types show similar immunomodulatory functions, mediated by secreted factors, metabolic reprogramming, or regulatory cell-to-cell contact (Ohue and Nishikawa 2019, Petty, Owen et al. 2021, Ma, Renz et al. 2022). Furthermore, TAMs and MDSCs support tumor vascularization and tumor progression by secreting pro-angiogenic factors and degrading the ECM, respectively (Lugano, Ramachandran et al. 2020, Petty, Owen et al. 2021, Ma, Renz et al. 2022). This highlights the complexity of the crosstalk between TME components that work in concert to maintain tumor viability.

In an embodiment of each and any aspect of the present invention, including any embodiment thereof, the immune evasion mechanism of the tumor is one selected from the group comprising loss of MHC class I expression of malignant cells of the tumor and elevated expression of immune checkpoints by malignant as well as non-malignant cells of the tumor. In connection therewith, a person skilled in the art will acknowledge that many of the checkpoint ligands are not expressed by the malignant cells but by immune-suppressive immune cells in the stroma. These can be: B7 on antigen-presenting cells (ligand of CTLA-4); MHC II on antigen-presenting cells (ligand of LAG-3); galectin (ligand of TIM-3); VISTA on various immune cells, including T cells, and also on some malignant cells; B7-H3 on malignant cells and immune cells in the tumor microenvironment; TIGIT which is expressed on T cells and NK cells and interacts with its ligands, CD155 and CD112, which are expressed on malignant cells; or HLA-G which is a non-classical MHC class I molecule that is expressed by some malignant cells.

In an embodiment of each and any aspect of the present invention, including any embodiment thereof, the tumor is evading from the subject's adaptive immune system by suppressing activity of cytotoxic tumor-infiltrating lymphocytes (TILs) or inhibiting activation of cytotoxic tumor-infiltrating lymphocytes. It will be appreciated by a person skilled in the art that these TILs establish or provide immunosurveillance meaning that TILs can kill malignant cells in the tumor. If a tumor is cold, the TILs may be there but are not activated which is due to suppressive factors in the stroma. Tregs are regulatory T cells, and these cells are generally immunosuppressive. They are thus immunosuppressive lymphocytes and are included along with the immunosuppressive myeloid cells which are MDSCs, TAMs, and TANs.

In an embodiment of each and any aspect of the present invention, including any embodiment thereof, the immunosuppressive tumor microenvironment results from, is caused by and/or is associated with a presence of a cell selected from the group comprising a myeloid-derived suppressor cell (MDSC), a tumor-associated macrophage (TAM) a tumor-associated neutrophils (TAN), and/or a regulatory T cell (Treg). It will be appreciated by a person skilled in the art that such immunosuppressive tumor microenvironment results from, is caused by and/or is associated with a presence of a multitude of said cells.

Typical examples of immunologically cold tumors where targeted immunotherapy has remained to be challenging include pancreatic cancer (pancreatic ductal adenocarcinoma or PDAC) (Hartupee, Nagalo et al. 2024); ovarian cancer, with high-grade serous ovarian cancer being the most common and aggressive form (Peng, He et al. 2022); prostate cancer, particularly in its advanced castration-resistant form (Stultz and Fong 2021); triple-negative breast cancer (Liu, Hu et al. 2023); and also rare cancers such as soft tissue sarcomas (Rytlewski, Milhem et al. 2021) including the subtype of synovial sarcoma (Mitchell, Pollack et al. 2021), and osteosarcoma, the most common form of primary bone sarcoma (Yu and Yao 2024).

In an embodiment of each and any aspect, including any embodiment thereof, the tumor is an immunologically cold tumor is refractory to immunotherapy. In an embodiment of each and any aspect, including any embodiment thereof, the tumor is refractory to immunotherapy. In an embodiment of each and any aspect, including any embodiment thereof, the tumor is non-refractory to or sensitive to immunotherapy. In these embodiments in particular, but also whenever is referred herein to immunotherapy, more preferably immunotherapy includes, but is not limited to a checkpoint inhibitor and, respectively, administering a checkpoint inhibitor to a subject; adoptive cell therapy and, respectively, administering an adoptive cell therapy to a subject, and tumor vaccine and, respectively, administering a tumor vaccine to a subject.

Immune checkpoints are regulators of the immune system. These pathways are crucial for self-tolerance, which prevents the immune system from attacking cells indiscriminately. However, some cancers can protect themselves from attack by stimulating immune checkpoint targets. The success of immune checkpoint blockade in cancer therapy, in which antibodies are used to block signaling pathways that inhibit T cell responses to tumors, illustrates the critical roles of these inhibitory checkpoints in obstructing antitumor immunity.

PD-1 inhibitory receptor PD-L1 regulates T cell activation, effector T cell responses, T cell tolerance, and T cell exhaustion (Francisco, Sage et al. 2010). PD-1 is rapidly induced on naive T cells following TCR engagement, countering T cell activation. When T cells are repetitively stimulated (as in cancer and chronic infection), PD-1 expression is sustained at high levels and T cells enter a dysfunctional state, termed exhaustion (Barber, Wherry et al. 2006). Thus, PD-1 is expressed on both activated and exhausted T cells, and PD-1 expression alone does not signify T cell exhaustion. PD-1 is not only expressed on conventional CD4+ and CD8+ T cells, but also on regulatory T cells, B cells, NK cells, and NKT cells (Schildberg, Klein et al. 2016). PD-1 has two ligands, PD-L1 (also called B7-H1 or CD274) and PD-L2 (also called B7-DC or CD273). PD-L1 is widely expressed on many types of hematopoietic cells (T cells, B cells, macrophages, dendritic cells) and nonhematopoietic cells (epithelial, stromal, and endothelial cells). PD-L2 is expressed mainly on hematopoietic cells (DCs, macrophages, B cells, and Th2 cells). Upregulation of PD-1 ligands by proinflammatory stimuli may serve as a negative feedback mechanism to attenuate effector T cell responses, protecting tumors from immune attack.

CTLA-4 and CD28 are both receptors expressed on the surface of T cells, CD80/86 are receptors on the surface of antigen-presenting cells. The contact of CTLA-4 with CD80/86 receptors results in quiescence of the T cell which remains unactivated. In contrast, the interaction of CD28 on the surface of T cells with CD80/86 on the antigen-presenting cell results in activation of the T cell. Pharmacological blocking of CTLA-4 on the T cell surface increases the likelihood of CD28-CD80/86 binding, resulting in maintenance of T cell activation (Kareva 2017).

LAG-3 is an immune checkpoint receptor that is highly upregulated on exhausted T cells in the tumor microenvironment. LAG3 transmits inhibitory signals to T cells upon binding to MHC class II and other ligands, rendering T cells dysfunctional. Consequently, LAG3 is a major target for cancer immunotherapy with many anti-LAG3 monoclonal antibodies (mAbs) that block LAG3 inhibitory activity in clinical trials (Mariuzza, Shahid et al. 2024).

TIM-3 is an immune checkpoint receptor that suppresses antitumor responses by negatively regulating the activity of CD8 T cells and antigen-presenting cells (Anderson, Joller et al. 2016). It is upregulated on CD8 T cells following anti-PD-1 therapy, suggesting it may be involved in resistance to PD-1 inhibition (Koyama, Akbay et al. 2016). Preclinical studies have found that concurrent inhibition of TIM-3 and PD-1 pathways is more effective in reducing tumor growth and restoring adaptive immunity than blocking TIM-3 or PD-1 alone (Pagliano, Morrison et al. 2022).

TIGIT is an emerging immune checkpoint that inhibits immune cell responses at multiple steps of the cancer-immunity cycle. TIGIT impairs T cell priming by dendritic cells, prevents tumor cell killing by NK cells and cytotoxic T cells, and enhances the immune suppressive activity of regulatory T cells. Consequently, TIGIT constitutes a major target in cancer immunotherapy (Harjunpaa and Guillerey 2020).

Each of the above checkpoint inhibitors constitutes an embodiment of each and any aspect of the invention, including each and any embodiment thereof.

Adoptive cell therapy (ACT) comprises different strategies to enhance the activity of T lymphocytes and other effector cells that orchestrate the antitumor immune response, including chimeric antigen receptor (CAR) T cell therapy, T-cell receptor (TCR) gene-modified T cells, and therapy with tumor-infiltrating lymphocytes (TILs). The outstanding results of CAR-T cells in some hematologic malignancies have launched the investigation of ACT in patients with refractory solid malignancies. However, certain characteristics of solid tumors, such as their antigenic heterogeneity and immunosuppressive microenvironment, hamper the efficacy of antigen-targeted treatments (Albarran, San Roman et al. 2024).

ACT encompasses several techniques that use the T lymphocytes themselves, after a process of artificial modification or genetic engineering, to improve their antitumor activity (Morotti, Albukhari et al. 2021). This implies the extraction of autologous T lymphocytes from the patient, and their manipulation and amplification in vitro. Meanwhile, the patient undergoes treatment with lymphodepleting chemotherapy - usually fludarabine plus cyclophosphamide - to deplete ineffective and immune-suppressing lymphocytes. After this process, the improved cell product is reinfused into the patient.

The difference between different ACT modalities lies in the characteristics of T cell modification in vitro: CAR-T and TCR gene-modified T cells are genetically engineered to incorporate modified membrane receptors with a high affinity for selected tumor antigens. However, in contrast to hematologic malignancies, solid tumors are composed of polyclonal cell populations with huge antigenic heterogeneity, which hampers the efficacy of antigen-targeted therapies. TIL therapy is based on the activation and expansion of infiltrating T cells extracted from the tumor itself, which are intrinsically reactive against tumor antigens, setting an interesting rationale for its use against the changing and heterogeneous cell population of solid cancers.

CAR-T cells are genetically modified lymphocytes that incorporate a chimeric antigen receptor (CAR) composed by three parts, (i) an extracellular domain with a single-chain fragment variable (scFv) that allows antigen recognition, (ii) a transmembrane domain - linked to the extracellular part through a spacer, and (iii) an intracellular domain. This includes a CD3 complex which activates the downstream signaling pathways, and several costimulatory domains (usually CD28 and/or 4-1BB) that intensify the cytoplasmatic activity of T cells unchained by antigenic recognition (Zhang, Liu et al. 2017). In recent years, innovations in the structure and manufacturing of CAR-T cells have led to significant improvements in their clinical efficacy, especially with the development of fourth-generation CAR-T cells (Huang, Li et al. 2020). Fifth generation CARs equipped with three costimulatory domains and able to secrete anti-PDL1 scFv blockade molecules, targeted against B cell maturation antigen (BCMA) have shown heightened antitumor efficacy and decrease of T cell exhaustion in patients with multiple myeloma (Yuti, Sawasdee et al. 2023).

Due to their molecular structure, CAR-T cells only recognize extracellular antigens, and are particularly efficient when their scFv has a high affinity for the targeted protein and it is homogeneously expressed by tumor cells. This explains the efficacy of CAR-T cells in patients with leukemia and lymphoma, which comprise a clonal population of cells that uniformly express certain antigens such as CD19 on their membrane (Shank, Do et al. 2017). Since 2018, tisagenlecleucel and axicabtagene-citoleucel have EMA approval for the treatment of B cell LLA and refractory non-Hodgkin lymphoma, and in 2020, brexucabtagene-autoleucel was approved by the EMA for mantle lymphoma, achieving complete response rates of over 50% in heavily pre-treated patients (Sermer and Brentjens 2019).

The outstanding results of CAR-T cells in hematologic malignancies have led to their investigation in solid tumors, mainly using overexpressed epithelial antigens as targets. EGFR, HER2, CEA, mesothelin and soluble antigen GD2 have been frequent targets of CAR-T therapies, although many other antigens have been the object of preclinical studies (Marofi, Motavalli et al. 2021).

Nearly 500 clinical trials evaluating CAR-T cells in solid tumors have been registered before 2023, with many still ongoing (Wang, Gauthier et al. 2023). Most completed studies are phase 1/2 trials that have reported modest results, with only occasional and generally brief clinical responses; a general breakthrough for CAR T cell therapies of solid cancers is still to come.

The overall process for manufacturing ot CAR-T cells involves isolation of the starting cell population from the leukapheresis product, T-cell activation, genetic modification, ex vivo expansion, final product formulation, and product release testing (Ayala Ceja, Khericha et al. 2024). The manufacturing processes for several FDA-approved CAR-T cell products have been described in literature with broad similarities as well as unique features for tisacel (KYMRIAH), axicel (YESCARTA), brexucel (TECARTUS), lisocel (BREYANZI), idecabtagene vicleucel (ABECMA), and ciltacabtagene autoleucel (CARVYKTI) (Ayala Ceja, Khericha et al. 2024).

T cells expressing an engineered T-cell receptor (TCR-T cells) represent an alternative modality of ACT, with several advantages compared to CAR T therapy (Baulu, Gardet et al. 2023). In contrast to CAR T cells, TCR-T- cells can recognize not only membrane proteins but also intracellular antigens presented by MHC molecules, covering a wider repertoire of tumor neoantigens and therapeutic targets. Intrinsic features of T cells, such as high antigen sensitivity and the use of physiological signaling pathways, improve TCR-T-cells antitumor functions and reduce the risk of on-target off-tumor toxicity.

However, TCR-T-cells also have some disadvantages compared to CAR-T cells, such as their weaker avidity for target antigens and their limitation to a certain human leukocyte antigen (HLA) haplotype, which restricts the number of patients that potentially benefit from each modified TCR. In addition, as antigen-targeted modified T lymphocytes, all the limitations of CAR-T therapy in solid tumors - antigenic heterogeneity, difficulty of T cell trafficking, and detrimental effects of immunosuppressive TME- are also applicable to TCR-T-cells (Foy, Jacoby et al. 2023).

To date, there is only one TCR T cell therapy approved for clinical use, afamicel (TECELRA), to treat metastatic synovial sarcoma (Keam 2024). TECELRA is prepared from the patient's peripheral blood mononuclear cells (PBMCs), which are obtained via a standard leukapheresis procedure. The PBMCs are enriched for T cells and are then transduced with a replication-incompetent lentiviral vector containing the MAGE-A4 TCR transgene. The transduced T cells are expanded, washed, formulated into a suspension, and cryopreserved. The product must pass a sterility test before release and shipping as a frozen suspension in one or more infusion bag(s). The product is thawed prior to infusion back into the patient (FDA 2024).

Both CAR-T and TCR-T cells harbor inherent limitations for the treatment of patients with solid tumors, which may explain the worse clinical outcomes than those obtained with CAR-T therapy in hematologic malignancies.

In contrast to the clonal nature of lymphomas and leukemias, solid tumors - particularly in the context of metastatic disease - are characterized by the progressive acquisition of somatic mutations that lead to polyclonal expansion of different cellular lineages, giving rise to genomic instability and antigenic heterogeneity.

Significant intratumoral heterogeneity in neo-epitope expression and clonal expansion of the adaptive immune system in distant regions of the same disease have also been demonstrated in hepatitis B virus (HBV)-related liver cancer (Losic, Craig et al. 2020). In Her2+ breast cancer, spatial transcriptomic studies have shown intra-patient heterogeneity in the expression of gene signatures that determine cellular interactions with T lymphocytes and other immune cells (Andersson, Larsson et al. 2021). In another study, the expression of several antigens in tumor cells from 7 GBM patients was determined before and after a single infusion of EGFR-targeted CAR-T cells, and in all patients a significant decrease in EGFR expression, but an important increase in several other antigens with known immunosuppressor functions was observed (O'Rourke, Nasrallah et al. 2017). These results shed light on how antigen-specific T cell therapies might quickly promote the selection of resistant cellular subclones with immunosuppressive activity.

T cells with multi-antigenic recognition have been proposed as a potential strategy to overcome this problem. Some preclinical studies have suggested that CAR-T cells with bispecific adapters can facilitate the eradication of antigenically different tumors (Lee, Marks et al. 2019). Moving a step forward, CAR-T cells equipped with synthetic Notch (synNotch) receptors might be able to induce CAR expression only after the recognition of tumor-specific antigens, creating precise prime-and-kill recognition circuits (Yu, Yi et al. 2019) (81). Although these are promising strategies, they have not yet been evaluated in clinical trials, and further research is required to assess their feasibility.

Ensuring contact between CAR-T cells and tumor cells is not a problem in leukemias and lymphomas, since malignant cells concentrate within the blood and lymph nodes; however, it is a significant obstacle in solid metastatic tumors. Several studies have shown that metastatic lesions from solid cancers have significantly lower lymphocytic infiltration than primary tumors, suggesting that the loss of T cell trafficking to the tumor site is a relevant mechanism for immune escape and may facilitate tumor progression (Ogiya, Niikura et al. 2016).

In patients with metastatic melanoma it was shown that the expression of certain chemokines is significantly higher in lesions enriched with tumor-infiltrating lymphocytes than in those with poor lymphocytic infiltration, suggesting that these molecules play an important role in T cell recruitment (Harlin, Meng et al. 2009). For instance, high expression of the chemokine CXCL12 in the tumor microenvironment can shield nests of cancers cells from the immune effector cells in experimental models, an observation which might also be a relevant mechanism that limits efficacy of adoptive cell therapies. Inhibition of CXCL12/CXCR4 signaling prompted deep infiltration of T cells in the tumor microenvironment in these models (Feig, Jones et al. 2013, Fearon 2014, Zboralski, Hoehlig et al. 2017).

Targeting the tumor vasculature and microenvironment to modulate the chemotactic response is an exciting research topic to improve CAR-T cell trafficking to solid tumors (Donnadieu, Dupre et al. 2020).

In addition to hampering T cell trafficking, the tumor microenvironment has inhibitory effects on the lymphocytes that get to infiltrate the tumor site, which is an obstacle for CAR T or TCR T cell function. In addition, sustained exposure to tumor antigens and inflammatory signals is thought to progressively mitigate the function and proliferation of modified T cells, leading to CAR T cell 'exhaustion'. Targeting T cell intrinsic pathways (PD-1/PD-L1 axis, TOX/NR4A, TGF-b, CBL-B), using CRISPR technology to modulate the surface expression of CAR, and uncoupling antigen recognition from CAR activation signaling, are possible approaches to overcome exhaustion and improve adoptive cell therapy clinical outcomes in solid tumors (Gumber and Wang 2022).

Each of the above adoptive cell therapies constitutes an embodiment of each and any aspect of the invention, including each and any embodiment thereof.

In an embodiment of each and any aspect of the invention, including any embodiment thereof, the tumor is a soft tissue sarcoma.

Soft tissue sarcomas represent a heterogeneous group of extraskeletal mesenchymal tumors which are subclassified in approx. 70 subtypes, each characterized by a distinct morphology, that often translates into a specific clinical behaviour as well as into specific therapeutic approaches (Sbaraglia, Bellan et al. 2021). The nomenclature of soft tissue sarcomas is derived from the mature tissue each tumor most closely resembles. The incidence of soft tissue sarcomas among all children age younger than 20 years diagnosed with cancer is approximately 7%-8%, while representing only 1%-2% of all cancer diagnoses in adults (Ferrari, Sultan et al. 2011). Within the adolescent and young adult population, defined as patients between age 15 and 39 years, the incidence of synovial sarcoma is 2%-4% (Lewis, Seibel et al. 2014).

Among the types of soft tissue sarcoma are (American Cancer Society, www.cancer.org/cancer/types/soft-tissue-sarcoma/about/soft-tissue-sarcoma.html, accessed Feb 17, 2025):
- Adult fibrosarcoma, which usually affects fibrous tissue in legs, arms, or trunk; it is most common between the ages of 20 and 60;
- Alveolar soft-part sarcoma, a rare cancer that most commonly start in the legs and mostly affects young adults;
- Angiosarcoma which can start in blood vessels (hemangiosarcomas) or lymph vessels (lymphangiosarcomas), sometimes in a part of the body that has been treated with radiation;
- Clear cell sarcoma, a rare cancer that often starts in tendons of the arms or legs;
- Desmoplastic small round cell tumor, a rare sarcoma of teens and young adults, most frequently found in the abdomen;
- Epithelioid sarcoma, which mostly starts in tissues under the skin of the hands, forearms, feet, or lower legs. Teens and young adults are often affected;
- Fibromyxoid sarcoma, low-grade or Evans' tumor is a slow-growing cancer that most often starts as a painless growth in the trunk or arms and legs It is more common in young to middle aged adults;
- Gastrointestinal stromal tumor (GIST), which starts in the digestive tract. See Gastrointestinal Stromal Tumor (GIST) for more details;
- Kaposi sarcoma, is a type of sarcoma that starts in the cells lining lymph or blood vessels;
- Leiomyosarcoma, a type of cancer that starts in smooth muscle tissue, often in the abdomen;
- Liposarcomas, malignant tumors of fat tissue, which can start anywhere in the body, but most often in the thigh, behind the knee, and inside the back of the abdomen. They occur mostly in adults between 50 and 65 years old;
- Malignant mesenchymoma, a rare type of sarcoma that shows features of fibrosarcoma and features of at least 2 other types of sarcoma;
- Malignant peripheral nerve sheath tumors, which include neurofibrosarcomas, malignant schwannomas, and neurogenic sarcomas. These sarcomas start in the cells that surround a nerve;
- Myxofibrosarcomas, low-grade which are mostly found in the arms and legs of elderly people.
- Rhabdomyosarcoma, which is the most common type of soft tissue sarcoma in children;
- Synovial sarcoma, a malignant tumor of the tissue around joints. The most common locations are the hip, knee, ankle, and shoulder. This tumor is more common in children and young adults, but it can occur in older people;
- Undifferentiated pleomorphic sarcoma (UPS), previously called malignant fibrous histiocytoma (MFH), which is most often found in the arms or legs. Less often, it can start inside at the retroperitoneum. This sarcoma is most common in older adults. It mostly tends to grow into other tissues around the place it started, but it can spread to distant parts of the body.

Each of the above forms of soft tissue sarcoma constitutes an embodiment of each and any aspect of the invention, including each and any embodiment thereof.

In an embodiment of each and any aspect of the invention, including any embodiment thereof, the tumor is a synovial sarcoma.

Synovial sarcoma is a deadly soft tissue malignancy with a predilection for adolescent and young adult victims (Stacchiotti and Van Tine 2018). It is a model immunologically quiet (also called cold) tumor with respect to many of the most important metrics for immunotherapy success including T cell infiltration, MHC expression, and inflammatory gene signatures (Pollack, He et al. 2017) (Blay, von Mehren et al. 2023). It almost never responds to checkpoint inhibitors and in a study of over 50 synovial sarcoma patients treated with PD-L1 inhibition there was not a single responder (Chawla, Van Tine et al. 2022). It has a low tumor mutational burden and frequently is infiltrated with inhibitory macrophages. Systemic administration of IFNγ can increase T cell infiltration and MHC expression in synovial sarcoma patients but this has not led to improved outcomes for synovial sarcoma patients either with IFNγ as a single agent or in combination with PD-1 inhibition (Zhang, Kohli et al. 2019). IFNγ is an FDA approved cytokine therapy, used as a standard of care treatment for osteopetrosis and chronic granulomatous disease. In the 1990s, IFNγ was evaluated as a single agent cancer immunotherapy in several prospective randomized trials (Windbichler, Hausmaninger et al. 2000, Giannopoulos, Constantinides et al. 2003, Alberts, Marth et al. 2008). IFNγ can enhance expression of surface major histocompatibility complex molecules and increase the processing and presentation of tumor-specific antigens (Shankaran, Ikeda et al. 2001), thus facilitating T-cell recognition and cytotoxicity (Gao, Shi et al. 2016, Ayers, Lunceford et al. 2017, Manguso, Pope et al. 2017, Patel, Sanjana et al. 2017).

Synovial sarcoma is among the best disease models for targeted immunotherapy because of its consistently strong and homogenous expression of cancer testis antigens (CTAs). These proteins are normally expressed early in development or within immune-privileged compartments, such as developing germ cells, but are abnormally expressed in some cancers and uniquely so in synovial sarcoma. This type of tumor generally expresses NY-ESO-1, PRAME, and MAGE-A4, among others, elevating synovial sarcoma as a prime target for CTA-specific vaccines or adoptive cellular therapies (ACTs). In fact, synovial sarcoma recently became the first cancer ever to have an engineered T cell receptor-based therapy (TCR-T), afamitresgene autoleucel ("afamicel") targeting MAGE-A4, approved by the FDA. However, despite excellent overall response rates (ORRs) in the 50% range, TCR-T responses are durable in <15% of patients (Tan, Gakhar et al. 2021, Zhang, Liu et al. 2022). Reversing the cold TME is key to successful immunotherapy in synovial sarcoma. Although synovial sarcoma is an uncommon cancer, its immunobiology is broadly important for many cancers.

CXCR4 has been reported to be expressed in synovial sarcoma cells and examined as a potential target to slow tumor growth (Pazzaglia, Pollino et al. 2019).

Synovial sarcoma is a relatively rare malignancy representing a soft tissue sarcoma (STS) of uncertain differentiation (Gazendam, Popovic et al. 2021). It accounts for 5-10% of all soft tissue sarcomas (Toro, Travis et al. 2006, Mastrangelo, Coindre et al. 2012, WHO 2020). The age-adjusted incidence is 0.81/1,000,000 in children and 1.42/1,000,000 in adults with approximately 1000 patients diagnosed with synovial sarcoma in the United States each year(Sultan, Rodriguez-Galindo et al. 2009). Synovial sarcoma is unique from other soft tissue sarcomas as it presents at a younger mean age of onset and commonly occurs in adolescents and young adults (mean age of 39 years at diagnosis) and affects both sexes equally (Aytekin, Ozturk et al. 2020). Synovial sarcoma is the most common non-rhabdomyosarcoma soft tissue sarcoma in children, representing 30% of soft tissue sarcomas diagnosed in childhood (McGrory, Pritchard et al. 2000, Sultan, Rodriguez-Galindo et al. 2009).

The 5-year survival rates range from 59-75% (Lewis, Antonescu et al. 2000, Trassard, Le Doussal et al. 2001, Sultan, Rodriguez-Galindo et al. 2009, Smolle, Parry et al. 2019, Aytekin, Ozturk et al. 2020). There has been a trend for improved survival over time with early cohorts from the 1960s quoting a 25-51% five-year survival rate. Local and metastatic relapse of soft-tissue sarcomas generally occur in the first two years following treatment and thus surveillance and follow-up is most intensive in this period (Wilson, Gazendam et al. 2020). However, synovial sarcoma is unique in this regard in that it tends to recur much later, with occurrence of local recurrence after a mean of 3.6 years and of metastases after a mean of 5.7 years (Krieg, Hefti et al. 2011).

There are several well documented key prognostic factors including tumor size, grade and anatomical location, patient age at diagnosis, negative surgical margins and adjuvant radiotherapy (Lewis, Antonescu et al. 2000, Ferrari, Gronchi et al. 2004, Ferguson, Griffin et al. 2006, Guadagnolo, Zagars et al. 2007, Canter, Qin et al. 2008, Vlenterie, Ho et al. 2015). Osseous or neurovascular invasion, adult age, large tumor size and unplanned excision have been linked to worse prognosis (Singer, Baldini et al. 1996, Lewis, Antonescu et al. 2000, Ferrari, Gronchi et al. 2004, Ferguson, Griffin et al. 2006, Vlenterie, Ho et al. 2015). Similar to other soft tissue sarcomas, tumor size and grade has repeatedly been shown to have prognostic value in patients with synovial sarcoma (Ferrari, Gronchi et al. 2004, Eilber, Brennan et al. 2007, Naing, Monjazeb et al. 2015). Tumor location has also been demonstrated to be of predictive value, with non-extremity based synovial sarcomas tending to have worse overall survival (Ferrari, Bisogno et al. 2008, Aytekin, Ozturk et al. 2020). However, this may be in part due to the lack of early symptoms and later stage of presentation. Although synovial sarcoma has a similar clinical presentation in children and adults, there is a growing evidence that they have different outcomes, with children having significantly better survival rates (Sultan, Rodriguez-Galindo et al. 2009, Vlenterie, Ho et al. 2015, Ferrari, Chi et al. 2017, Smolle, Parry et al. 2019).

Metastasis develops in approximately half of patients and for them, the median overall survival (OS) is 2-years for metastatic SS and the 5 year survival is only 10% (Kampe, Rosen et al. 1993, Ladanyi, Antonescu et al. 2002, Sultan, Rodriguez-Galindo et al. 2009, Su, Cheng et al. 2010, Constantinidou, Pollack et al. 2013, Pollack, Somaiah et al. 2020). Because most patients are young, each death takes an outsized number of years of life (Chen, Borker et al. 2014).

Although many synovial sarcomas originate near articular structures, the name synovial sarcoma is a misnomer in that these lesions do not originate from intra-articular synovium, but from primitive mesenchymal cells (Jo and Fletcher 2014). Synovial sarcomas most commonly present as soft tissue masses but cases of primary synovial sarcoma of bone have been reported (Caracciolo, Henderson-Jackson et al. 2019). These lesions can occur anywhere in the body, with the majority arising in the extremities, particularly in the lower extremity in the anatomic structures adjacent to the knee joint (Sultan, Rodriguez-Galindo et al. 2009, Smolle, Parry et al. 2019). Synovial sarcomas are considered the most common soft tissue sarcoma of the foot (McGrory, Pritchard et al. 2000, Deshmukh, Mankin et al. 2004).

Synovial sarcomas often do not present with the typical soft tissue sarcoma presentation of a large and quickly growing painless mass (Smolle, Parry et al. 2019). Instead, the majority of synovial sarcomas are slow growing and the mean duration of symptoms before diagnosis is approximately 2 years (Chotel, Unnithan et al. 2008). In comparison to other soft tissue sarcomas, there may be longstanding pain or joint contractures at the tumor site that precede swelling (De Silva, Barrett et al. 2003). Only half of patients have clinical findings consistent with soft tissue sarcoma according to the UK National Institute for Health and Care Excellence (NICE) guidelines (De Silva, Barrett et al. 2003, Chotel, Unnithan et al. 2008). Given the insidious onset, younger age at presentation and atypical presenting symptoms, these patients may be initially clinically misdiagnosed with benign processes including myositis, synovitis, bursitis or tendonitis.

Plain radiographs are not required for diagnosis but are typically performed as part of the initial workup and can identify adjacent bony remodeling, bone invasion or calcification of the soft tissue mass (Bixby, Hettmer et al. 2010, Bakri, Shinagare et al. 2012, Kao 2019). Typically, synovial sarcoma presents as a well-defined or lobulated soft tissue mass on plain radiographs. Punctate calcifications, particularly around the periphery of the lesion, are visualized in one third of patients. Occasionally, more extensive calcification can be visualized and can mimic bone forming tumors including osteosarcoma and myositis ossificans (O'Sullivan, Harris et al. 2008).

Similar to other soft tissue sarcomas, MRI with and without contrasting agents is the gold standard for diagnostic imaging for synovial sarcoma (O'Sullivan, Harris et al. 2008). MRI defines the local extent of the soft tissue mass and surrounding edema and provides excellent visualization of the mass with respect to the surrounding anatomy, which is critical for preoperative planning. The utilization of gadolinium contrast can differentiate between hemorrhagic or necrotic areas and areas of solid viable tumor. As with most soft tissue sarcomas, synovial sarcomas are typically heterogenous with low intensity on T1 and high intensity on T2-weighted images with post-gadolinium enhancement (O'Sullivan, Harris et al. 2008).

Although synovial sarcomas generally present as a non-specific heterogenous mass, there are some unique features, which can aid in differentiation from other soft tissue sarcomas. Synovial sarcomas predominately present as well-defined, heterogeneously enhancing solid tumors that are multilobulated in nature. A triple signal intensity demonstrating areas of hyperintensity, isointensity and hypointensity indicating the mix of cystic and hemorrhagic areas, cellular elements and fibrotic areas can be characteristic (O'Sullivan, Harris et al. 2008). Smaller tumors, particularly those smaller than 5 cm in diameter, often show homogeneous enhancement which can be mistaken for a benign process (Liang, Mao et al. 2015). Several findings on MRI have been found to be predictive of high-grade lesions including the absence of calcifications and presence of hemorrhage and the triple signal intensity (Tateishi, Hasegawa et al. 2004).

Computed tomography (CT) with contrasting agents can be utilized when an MRI is contraindicated or unavailable. Synovial sarcoma appears hypointense compared to muscle with heterogeneity in larger lesions (Baheti, Tirumani et al. 2015). CT allows for better visualization of soft-tissue calcifications and local bone reaction (O'Sullivan, Harris et al. 2008).

A biopsy and pathologic assessment are required to differentiate synovial sarcoma from other soft tissue sarcoma subtypes and define the tumor grade. As with all soft tissue sarcomas, a biopsy should be performed prior to definitive surgery to avoid inadequate resection and misdiagnosis (von Mehren, Randall et al. 2016). Options for biopsy include incisional biopsies, core needle biopsies (CNB) and fine needle aspirations (FNA). Historically, open incisional biopsies (IB) have been considered the gold standard for soft tissue lesions as they provide larger volumes of tissue. When compared to CNB and FNA, IB tend to have higher diagnostic accuracy but this comes with a higher rates of complications when compared to percutaneous techniques (Kasraeian, Allison et al. 2010). Core needle biopsies retrieve more tissue than FNA and have higher diagnostic accuracy (Yang and Damron 2004). Fine needle aspirates are rarely used in soft tissue sarcomas due to the small quantity of sample material obtained and a limited ability to assess lesional architecture (Kasraeian, Allison et al. 2010). Advances in diagnostic imaging has allowed for image guided percutaneous biopsies which has improved the diagnostic accuracy of these techniques (Narvani, Tsiridis et al. 2009). Given the lower morbidity and relatively high diagnostic accuracy of CNB, image guided CNB are the preferred method of biopsy, particularly for deeper tumors. When open biopsies are performed, the biopsy principles must be observed to reduce biopsy-related complications (Mankin, Mankin et al. 1996).

Staging investigations are imperative and allow for a better understanding of disease prognosis and risk of recurrence or metastases. The tumor stage also helps to formulate a treatment plan by a multidisciplinary sarcoma team. Staging of synovial sarcoma involves cross-sectional imaging of the affected extremity, systematic staging with a chest CT, and pathological assessment. The two most commonly utilized staging systems are the American Joint Committee on Cancer (AJCC) system and the Enneking staging system (Enneking 1988, Amin 2017). The Enneking staging system relies on tumor grade, local extent of disease and presence of metastases. The AJCC staging system is based on anatomical site of primary tumor and tumor size (pT) tumor grade, nodal involvement (N), and presence of metastases (M).

Synovial sarcomas are malignant and metastasize, most commonly to the lungs with up to 13% of patients having distant metastases at the time of diagnosis (Aytekin, Ozturk et al. 2020). Historically, it was thought that synovial sarcoma had a predilection for metastases to lymph nodes, necessitating the need for further advanced imaging and possible sentinel node biopsy (Maduekwe, Hornicek et al. 2009). However, these findings were based on small case series and more recent SEER database findings suggest that the rates of lymph node metastases are in line with other soft tissue sarcomas and do not require additional workup beyond thorough physical examination (Jacobs, Morris et al. 2018).

The gross pathology appearance of synovial sarcoma is usually tan or grey and may be multinodular or multicystic (Jo and Fletcher 2014). Most synovial sarcomas are 3-10 cm in diameter. Smaller lesions (<1 cm) can occur in hands and feet (Michal, Fanburg-Smith et al. 2006, WHO 2020). Histologically, synovial sarcoma is a monomorphic spindle cell sarcoma with variable epithelial differentiation (WHO 2020). It presents as one of three variants: monophasic, biphasic or poorly differentiated. In the monophasic variant, the tissue is comprised entirely of spindle-cells whereas in biphasic synovial sarcoma, there are epithelial and spindle-cell components present (Jo and Fletcher 2014). In one third of synovial sarcomas, areas of calcifications and/or ossification can be found. In both monophasic and biphasic variants, there may be poorly differentiated areas with increased cellularity, greater nuclear atypia and high mitotic activity (6 mitoses/mm² >10 mitoses per 1.7 mm²) (WHO 2020). Occasionally, the entire tumor shows poorly differentiated morphology. On immunohistochemistry, diffuse expression of bcl-2 is typically seen. In 60% of cases, these tumors stain positive for CD99 (WHO 2020). Immunohistochemistry also demonstrates strong and diffuse nuclear staining for the transcriptional corepressor TLE1 found in the large majority of synovial sarcomas (WHO 2020). NY-ESO-1 is also expressed strongly in most synovial sarcomas and can help differentiate it from other spindle cell neoplasms (Lai, Robbins et al. 2012).

Synovial sarcoma bears one of the simplest genomes across cancers, with low tumor mutational burden (TMB) and even rare copy number variations identified in neoplastic cells (Luk, ME et al. 2024). Synovial sarcoma is defined and driven by the fusion oncoprotein SS18::SSX from the translocation t(X;18) fusing SS18, which codes for a BAF chromatin remodeling complex component, in-frame to one of the SSX genes, SSX1, 2, or 4, which code for putative transcriptional repressors (Fiore, Sambri et al. 2021). This translocation is present in >95% of cases (Stegmaier, Leuschner et al. 2017) and leads to the expression of different SS18:SSX oncogenic fusion proteins, which drive sarcomagenesis. Subtypes include SS18:SSX1 and SS18:SSX2 and less commonly SS18:SSX4 (dos Santos, de Bruijn et al. 2001). Both fluorescence in situ hybridization and reverse transcription polymerase chain reaction testing have been validated in the diagnosis of this translocation (Amary, Berisha et al. 2007). Almost all SS18-SSX2 synovial sarcomas show monophasic morphology and are significantly more common in women. Rare cases are associated with t(X;20) and SS18L1-SSX1 fusion transcript (WHO 2020).

The mainstay of treatment for synovial sarcoma remains surgical excision with negative margins with the addition of radiotherapy and/or chemotherapy based on patient and tumor characteristics (von Mehren, Randall et al. 2016). Historically, patients were often treated with amputation but advances in adjuvant therapy and cross-sectional imaging have allowed the majority of patients to be treated with limb-salvage surgery (Ghert, Abudu et al. 2005). The goal of limb-salvage surgery in synovial sarcoma is to achieve oncologic control of the tumor while providing the patient with a functional limb postoperatively. Negative surgical margins are of upmost importance as they predict both local recurrence and overall survival (Guadagnolo, Zagars et al. 2007, Biau, Ferguson et al. 2012, Bilgeri, Klein et al. 2020). While no specific guidelines exist regarding ideal negative margins in synovial sarcoma, surgical management is similar to that of other soft tissue sarcomas. For superficial tumors or small (<5 cm) deep tumors not intimately associated with critical structures, a wide excision with negative margins (1-2 cm) alone could be considered sufficient (Ferrari, Chi et al. 2017). In tumors that are closely associated with neurovascular structures or bone, the epineurium, adventitia or periosteum is utilized as the margin to allow for a functional limb postoperatively (Kawaguchi, Ahmed et al. 2004, Guadagnolo, Zagars et al. 2007). In these cases, very close or microscopically positive margins may occur and radiotherapy is essential to decrease local recurrence risk (O'Donnell, Griffin et al. 2014, Gundle, Gupta et al. 2017). Carefully planned microscopically positive margins on a fixed structure (bone, nerve, vessel) have been shown to not increase the risk for local recurrence in setting of neoadjuvant radiotherapy (O'Donnell, Griffin et al. 2014). Given the heterogeneity and lack of standardization in the literature, recommendations regarding margin management must be taken into context of the individual patient. Both tumor and anatomic factors should be taken into consideration when determining acceptable surgical margins (Sambri, Caldari et al. 2021).

Due to its atypical presentation (slow-growing, painful mass), synovial sarcomas have a high rate of presentation following an unplanned excision, with up to 50% of patients presenting after unplanned excision (Chandrasekar, Wafa et al. 2008, Chotel, Unnithan et al. 2008). Unplanned excisions of synovial sarcomas result in high rates of residual disease, particularly for larger and deeper tumors and increased risk for local recurrence even after re-excision (Chandrasekar, Wafa et al. 2008, Potter, Adams et al. 2008). In the case of referral following unplanned excision, patients should be re-staged, and the original histology reviewed at a sarcoma referral center. Tumor bed excision should be performed in patients with residual disease with the goal of complete tumor resection and negative margins (Pretell-Mazzini, Barton et al. 2015). Typically, these resections are extensive in nature given that areas of potential contamination must be removed, with may necessitate reconstructive procedures (Potter, Adams et al. 2008). There is a paucity of data to guide the utilization of radiotherapy in this population; however, radiotherapy is recommended as it would be in a primary presentation (Pretell-Mazzini, Barton et al. 2015).

In select cases, limb-salvage techniques are not recommended, and primary amputation may be required. Amputation is considered in patients with tumor location that necessitates excision of vital structures which would result in poor limb function. Patients who present following an unplanned excision may require amputation if there is extensive contamination of vital structures or major joints. Finally, older patients or those with extensive medical comorbidities may not be able to tolerate a major operation and amputation can be considered (Ghert, Abudu et al. 2005).

Neoadjuvant or adjuvant radiation therapy is recommended for larger tumors (>5 cm), or in any case where a close margin may be required to preserve a major neurovascular structure or bone (von Mehren, Randall et al. 2016). In large registry database studies, radiotherapy has been shown to improve local control and may have overall survival benefit in patients with synovial sarcoma (Naing, Monjazeb et al. 2015, Song, Park et al. 2017, Gingrich, Marrufo et al. 2020). Radiotherapy can be administered pre or postoperatively with differing protocols. Preoperative radiation is associated with higher wound complication rates whereas postoperative radiation can cause fibrosis and joint stiffness which may lead to worse long term functional outcomes (Davis, O'Sullivan et al. 2005). Regardless of timing, intensity-modulated radiation therapy (IMRT) is becoming the preferred method of radiation delivery in patients with soft tissue sarcoma (von Mehren, Randall et al. 2016, Wang, Song et al. 2019). Intensity-modulated radiation therapy allows for a higher dose of radiation to more closely contour the tumor, which reduces the volume of radiation to the surrounding normal tissues. The utilization of preoperative IMRT for soft tissue sarcoma has been shown to reduce wound complications and need for reconstructive soft-tissue flaps (O'Sullivan, Griffin et al. 2013). Radiation therapy may be considered in isolation in patients with multiple medical comorbidities or patients with metastatic disease where the risks of surgery outweigh the potential benefits (Ramu, Houdek et al. 2017).

Unlike the majority of soft tissue sarcomas, synovial sarcoma appears to be more chemosensitive, although there is still controversy surrounding which subgroups of patients benefit from systemic therapy (Eilber, Brennan et al. 2007). In general, chemotherapy is reserved for patients with high-risk tumors or advanced disease and is thought to be more effective in younger patients (Canter, Qin et al. 2008, Vlenterie, Ho et al. 2015). In children and adolescents with intermediate or high-risk tumors (i.e., >5 cm, nodal involvement, positive margins), adjuvant or neoadjuvant chemotherapy is generally undertaken with the most common agents being ifosfamide and doxorubicin. In the absence of available data from randomized controlled trials, prospective multicentred cohort studies have demonstrated adequate response to chemotherapy (Ferrari, De Salvo et al. 2015). Recent data has demonstrated that pediatric or adolescent patients with low-risk tumors (Grade 2 or Grade 3 < 5 cm) can be successfully treated with surgical intervention without systemic therapy (Ferrari, Chi et al. 2017). However, the role of chemotherapy in adult patients with synovial sarcoma is less clear (Eilber, Brennan et al. 2007, Canter, Qin et al. 2008, Italiano, Penel et al. 2009).

Chemotherapy is also considered in metastatic or unresectable disease (Spurrell, Fisher et al. 2005, Vlenterie, Ho et al. 2015). In general, anthracycline-based chemotherapy is first line for advanced soft tissue sarcoma and the addition of ifosfamide is dependent on the subtype of the disease. Ifosfamide has well documented efficacy in synovial sarcoma in the palliative setting and should be considered in patients who undergo chemotherapy if the toxicities can be tolerated (Spurrell, Fisher et al. 2005, Sleijfer, Ouali et al. 2010). Front-line treatment is therefore often doxorubicin with ifosfamide, but this combination rarely-if ever-achieves a complete response of disseminated disease (Judson, Verweij et al. 2014).

Trabectedin, a chemotherapy approved for liposarcoma and leiomyosarcoma, has demonstrated activity in translocation-associated sarcomas like synovial sarcoma, but also rarely achieves more than temporarily stable disease (Kawai, Araki et al. 2015). Pazopanib is another option, however it is not specifically labelled for synovial sarcoma, but for advanced non-adipocytic soft tissue sarcomas who have received prior chemotherapy. This is a multi-tyrosine kinase inhibitor whose mechanism of action is related to the selective inhibition of tumor angiogenesis and to the inhibition of key kinases involved in oncogenic pathways (Hurwitz, Dowlati et al. 2009, Gril, Palmieri et al. 2011)

There is currently only one marketed systemic treatment specifically approved for synovial sarcoma, afamitresgene autoleucel. This is an autologous, MAGE-A4 targeted TCR engineered cell therapy, which was launched in 2024 in the US under accelerated approval. The treatment is approved for treatment of adults with unresectable or metastatic synovial sarcoma who have received prior chemotherapy, are HLA-A*02:01P, -A*02:02P, -A*02:03P, or -A*02:06P positive and whose tumor expresses the MAGE-A4 antigen therapy. However, despite excellent overall response rates for synovial sarcoma patients with cancer testis antigen targeted strategies, the responses to such therapies are durable only in <15% of SS patients (Tan, Gakhar et al. 2021, Zhang, Liu et al. 2022). Some lack of durability of response may be related to the TCR-T themselves, and it has been shown that rare persisting cells from the peripheral blood of patients previously treated with TCR-T can be reinvigorated using IL-15, but significant problems exist within the tumor microenvironment (TME) as well (Kohli, Yao et al. 2021).

Systemic treatments in clinical development for synovial sarcoma include (i) catequentinib, a multi-tyrosine kinase inhibitor in clinical Phase 3; (ii) letetresgene autoleucel, a TCR T cell therapy, in clinical Phase 2; (iii) mipetresgene autoleucel, a TCR T cell therapy, in clinical Phase 2; (iv) atezolizumab, an anti-PD-L1 monoclonal antibody, in clinical Phase 2; (v) tazemetostat, a histone methyltransferase inhibitor, in clinical Phase 2; (vi) TC-N201, a TCR T cell therapy, in clinical Phase 1/2; and (vii) DS-2243 with no disclosed mechanism of action, in clinical Phase 1.

Each of the above forms and stages of synovial sarcoma constitutes an embodiment of each and any aspect of the invention, including any embodiment thereof. Also, each of the above methods for treating synovial sarcoma may be applied to the subject in accordance with the present invention as defined in the aspects of the invention, including any embodiment thereof. Finally, each of the above methods for diagnosing synovial sarcoma may be applied to the subject in accordance with the present invention as defined in the aspects of the invention, including any embodiment thereof.

In an embodiment of each and any aspect of the invention, including any embodiment thereof, the tumor is an osteosarcoma.

Osteosarcoma is a common primary malignant tumor of the bone, with a peak incidence in adolescents and adults >60 years of age (Mirabello, Troisi et al. 2009). Although osteosarcoma can present in any bone in the body, the most common sites are around the knee and the proximal humerus (Bielack, Kempf-Bielack et al. 2002). It can also arise in individuals with a history of cancer as secondary osteosarcoma. The diagnosis of osteosarcoma is made following the biopsy of a mass located most commonly at the metaphysis of the long bones based on imaging findings of patients who presented with pain, decreased mobility and, oftentimes, a palpable mass. Histologically, conventional osteosarcomas most commonly appear as spindle cell tumors that produce malignant osteoid and are consequently thought to derive from the malignant transformation of cells of the mesenchymal lineage at an undefined stage of differentiation towards becoming osteoblasts (Klein and Siegal 2006). Microscopically, based on the predominant matrix being produced, the tumors can be subdivided into chondroblastic, fibroblastic, osteoblastic and telangiectatic tumors, suggesting that they maintain some of the pluripotency of their early undifferentiated mesenchymal precursors (Piperdi, Austin-Page et al. 2012). Osteosarcomas can also be divided into three major groups as being low, intermediate and high grade (Bertoni and Bacchini 1998). The grade of the tumor serves as a relative indicator of the risk of developing metastatic disease. Low-grade, or parosteal, osteosarcomas are typically indolent and are treated by surgical removal alone. High-grade tumors have a high risk of developing metastasis in the lungs, lymph nodes, and other bones (Kager, Zoubek et al. 2003, Isakoff, Bielack et al. 2015) and require surgery and adjuvant chemotherapy as treatment. Unfortunately, the outcomes for patients with osteosarcomas have remained relatively stagnant since the advent and remarkable improvement in tumor survival associated with modern chemotherapy in the 1980s (Smith, Seibel et al. 2010).

Although rare, osteosarcoma is the most common primary malignancy of bone, with an incidence in children and adolescents of ~3-4.5 cases per million population per year (Mirabello, Troisi et al. 2009). In the USA, osteosarcoma accounts for <1% of all new cancer diagnoses with ~1,000 new cases diagnosed per year, half of these cases occurring in children and adolescents (Mirabello, Troisi et al. 2009). The global incidence rates in younger age groups (individuals ≤24 years) are relatively consistent across the USA, Europe and Asia. Data regarding differences between ethnic groups are limited, but higher rates of osteosarcoma have been observed in African American children and young adults in the USA than in white individuals (Mirabello, Troisi et al. 2009). Greater geographic variation in osteosarcoma incidence in individuals ≥60 years has been observed, but data are insufficient to determine whether these differences are due to varying criteria for disease classification in registries, differences in environmental exposures, such as prior radiotherapy for other cancer types, or genetic predisposition.

Approximately 80% of patients with osteosarcoma present with radiographically localized disease. Those patients with radiographically confirmed non-metastatic osteosarcoma have a 5-year event-free survival of ~60%. In patients who present with a primary lesion and an isolated pulmonary nodule, 5-year event-free survival is generally <40%. For individuals with a primary lesion and multiple pulmonary nodules or radiographically detectable metastatic disease at other sites, 5-year event-free survival is <20% (Gill and Gorlick 2021).

Osteosarcoma incidence has a bimodal age distribution with a primary peak in adolescents and young adults and a second, smaller peak in the seventh and eighth decade of life (Mirabello, Troisi et al. 2009). It is particularly uncommon in young children <10 years of age in whom the genetic etiology may be different to that in adolescents (Mirabello, Zhu et al. 2020). Osteosarcoma in adults (age >40 years) and elderly populations (age >60 years) tends to occur secondary to other conditions, such as Paget disease of bone, as transformation of other benign bone conditions or as a late effect of therapeutic irradiation.

Osteosarcoma is slightly more common in males, with an average male-to-female ratio of 1.4:1. A Surveillance, Epidemiology, and End Results (SEER) analysis in a US population provides additional insight into demographic differences that relate to age, sex and race/ethnicity of patients with osteosarcoma (Cole, Gianferante et al. 2022). The age-adjusted incidence rate was 1.9 per million for those aged 0-9 years, 6.7 per million for those aged 10-24 years, 1.9 per million for those aged 25-59 years and 2 per million for those aged ≥60 years. In the USA, Hispanic males aged 10-24 had the highest incidence rate compared with any other age group or sex. Data from all age groups combined revealed that the Black population had the highest overall incidence. Notably, the incidence of osteosarcoma in children and adolescents has increased from the 1970s to the 2000s but has declined in adults >60 years of age. Some of the increase in this population may be related to the increased incidence of subsequent osteosarcomas over the past decade, which may be attributable to the increasing number of childhood survivors. In patients >60 years old, the decreased incidence of osteosarcoma may be attributable to a decrease in the rate of Paget-related osteosarcoma. Patient sex does not seem to markedly influence prognosis but reports suggest that males may have a slightly worse outcome than females and older patients have a worse outcome than young patients with osteosarcoma (Cole, Gianferante et al. 2022).

Osteosarcoma is defined histologically as a tumor of osteoid-producing cells, which often exist within an admixture of adipogenic, muscle, spindle, fibroblastic and chondroblastic cells (Klein and Siegal 2006). This microscopic phenotype has long fuelled the assumption that osteosarcoma arises from a multipotent mesenchymal precursor. Epidemiological observations support this interpretation as tumors were found to arise most frequently within the metaphyses of long bones in children, adolescents and young adults during times of peak linear growth, suggesting that the bone-producing and cartilage-producing cells that proliferate rapidly during those growth spurts are those susceptible to the transformation giving rise to osteosarcoma (Jones 2011, Mutsaers and Walkley 2014). Introduction of TP53 mutations into partially differentiated osteogenic stem cells generated osteosarcoma-like cells in vitro (Lin, Jewell et al. 2017). Similarly, genetically engineered mouse models have been most successful when introducing TP53 and other mutations using promoters for genes activated along the path that leads from mesenchymal stem cells to osteoblasts (Jones 2011). Together, these data support the hypothesis that osteosarcomagenesis occurs within a proliferating population of partially differentiated osteoblast precursor cells. While mesenchymal differentiation is not as well characterized as the haematopoietic system, a number of transcription factors have been identified as key regulators of clusters of genes involved in the development of various cell types. Some of these are highly expressed in the context of osteosarcoma and relate to its osteogenic phenotype and include SOX9, RUNX2 and Osterix. Some of these transcription factors themselves are influenced by tumor suppressor genes and oncogenes such as TP53 and MYC.

The genomic landscape of osteosarcoma tumors is usually dominated by widespread structural rearrangements, suggesting that several different mutational mechanisms, including chromothripsis, chromoplexy, kataegis and other structure-altering mechanisms, are involved (Cortes-Ciriano, Lee et al. 2020). These rearrangements give rise to genome-wide copy number alterations, usually dominated by copy number loss, including of PTEN and CDKN2A/B, but with recurrent amplifications of MYC, VEGFA and CCNE1 (Sayles, Breese et al. 2019). Osteosarcoma tumors often show signs of whole-genome duplication, which probably occurs in response to stresses imposed by pervasive copy number losses (Wu, Beird et al. 2020). This genomic complexity has long been interpreted as a sign of chromosomal instability, but emerging data suggest that the mechanisms triggering complexity are active early in the process of malignant transformation (Sayles, Breese et al. 2019).

Apart from these characteristic structural alterations, large-scale sequencing has identified only moderate levels of point mutations with few recurrently mutated genes (Chen, Bahrami et al. 2014, Perry, Kiezun et al. 2014, Behjati, Tarpey et al. 2017, Wu, Beird et al. 2020). The single most frequently altered gene is the tumor suppressor TP53, which is lost in >90% of osteosarcoma. TP53 is an extremely well-known tumor suppressor gene that has been referred to as the 'guardian of the genome'. Its normal function is to induce apoptosis in cells that acquire mutations. Given the chaotic genome typically present in osteosarcoma, abrogating that guardian function is necessary for cancer cell survival. Deletion of RB1 also occurs in up to 30% of osteosarcoma tumors, often through loss of heterozygosity (Chen, Bahrami et al. 2014, Perry, Kiezun et al. 2014). Using these genomic aberrations to infer the evolution of the tumors, loss of TP53 and RB1 likely occurs early in the transformation process (with TP53 inactivation required to propagate abnormal genomes (Overholtzer, Rao et al. 2003), followed by rapid accumulation of driver lesions such as MYC amplification, PTEN loss and deletion of ATRX, which seems to activate alternative lengthening of telomeres and is associated with decreased survival (Lawlor, Veronese et al. 2019).

Many patients later diagnosed with osteosarcoma first seek medical care with concerns for persisting pain in an extremity (Meltzer and Helman 2021). A question frequently asked to assess the severity of pain is whether the pain keeps them up at night; in the context of osteosarcoma, this is typically answered affirmatively. The pain is often accompanied by swelling at the same site and patients might misassociate these symptoms with recent minor injuries. The loss of structural integrity due to tumor-related osteolysis puts patients at risk for pathological fractures, which occur in ~10% of patients and can complicate initial management (Zhou, Lu et al. 2017).

The workup for the presenting symptoms usually includes plain-film radiographs, potentially revealing large lesions that are causing destruction of normal trabecular bone with poorly defined margins (Papagelopoulos, Galanis et al. 2000). Lesions often stimulate periosteal new bone formation, which can give rise to the characteristic Codman triangle. The associated soft tissue mass can exhibit variable patterns of ossification, leading to the characteristic radial sunburst pattern often associated with osteosarcoma. Even if conventional radiographs are highly suggestive of osteosarcoma diagnoses, MRI covering the entire length of the affected bone should still be performed (Strauss, Frezza et al. 2021). MRI can better characterize the associated soft tissue masses and facilitates planning for biopsy and eventual surgical resection. MRI also often reveals skip metastases frequently captured by local site imaging or, if more distant, suggested by bone scan, with implications for management and prognosis.

The diagnosis of osteosarcoma requires the pathological evaluation of a bone tissue biopsy sample, which can be obtained using either a minimally invasive core needle biopsy approach or an open biopsy. For core needle biopsies, adequate sampling of the tissue has to be ensured as osteosarcoma lesions can be quite heterogeneous and diagnostic features (such as malignant osteoid) can vary from sample to sample. For additional downstream molecular diagnostics of fresh or frozen tissue, adequate sampling is even more important. Open biopsies are often preferred as they provide larger amounts of intact tissue. Fine-needle aspirates are usually inadequate for a definitive diagnosis of osteosarcoma due to the lack of sufficient histological context and the resulting difficulty to assess tumor grade and are therefore not recommended.

The histological diagnosis of osteosarcoma depends on the identification of malignant cells producing osteoid and irregular woven bone within fields of malignant tumor cells. The tumor cells usually exhibit marked atypia with a high degree of pleiotropism, and multiple morphologies (spindle, epithelioid, small round and giant cell) may exist within the same tumor. Although SATB2 and osteocalcin immunostaining and negative immunostaining to rule out alternative diagnostic entities can help guide a diagnostic work-up, no immunological or molecular marker has yet been identified that confirms a diagnosis of osteosarcoma (Beird, Bielack et al. 2022).

The post-diagnostic staging work-up aims to identify and characterize established metastatic disease. All patients presenting with newly diagnosed disease should undergo CT imaging of the chest, which has the highest efficiency in identifying lung nodules. Skeletal imaging with PET or technetium bone scans is important to identify covert bony disease. In 2008, guidelines from the Children's Oncology Group were published, and are still widely accepted, advocating PET imaging with accompanying whole-body CT or whole-body MRI, with isotope bone scans if these modalities are not available (Meyer, Nadel et al. 2008). This work-up recommendation reveals lung metastases in 15-20% of patients, and occasionally identifies tumors within other bones or, very rarely, lesions at other sites. Staging is guided primarily by the Musculoskeletal Tumor Society (MSTS) staging system for sarcomas (Wolf and Enneking 1996), which defines tumors as being either low or high grade, confined to an anatomic compartment or violating anatomic barriers, and localized or metastatic. Most patients present with high-grade lesions that have both bony and soft tissue components, making the presence or absence of metastasis the primary risk-stratifying feature at diagnosis. A small number of patients present with localized, lower-grade parosteal and periosteal lesions (Grimer, Bielack et al. 2005).

At baseline, children and adolescents who present with localized osteosarcoma have an overall survival of ~60% (Gorlick, Janeway et al. 2013). Patients who present with lung metastasis have the worst prognosis, with 3-year survival rates of <30% (Aljubran, Griffin et al. 2009). Fractures may also be an indicator of more aggressive disease. Patients experiencing fractures have higher rates of lung metastasis, both at presentation and subsequent to treatment (Zhou, Lu et al. 2017). The response to neoadjuvant chemotherapy, assessed in the definitive resection specimen, has clear prognostic value and has been used in previous clinical trials to stratify patients into good responders and poor responders; however, intensification of treatment did not improve outcomes in the poor responder group (Marina, Smeland et al. 2016).

The complex multi-modality management of osteosarcoma requires an expert multidisciplinary team that includes paediatric, medical and radiation oncologists, surgeons, pathologists, radiologists and specialist nurses (Strauss, Frezza et al. 2021). Combination chemotherapy and complete surgical resection are essential for cure. This applies to both patients with localized disease and those with primary metastatic osteosarcoma, provided complete surgical removal of all known metastatic deposits has been achieved. Current treatment paradigms offer patients with newly diagnosed, resectable osteosarcoma long-term survival rates of 60-70% (Smeland, Bielack et al. 2019). However, outcomes have hardly improved in the past decades, and the intensive chemotherapy regimens used are associated with important acute and long-term toxic effects and considerable impact on quality of life. In addition, patients with unresectable primary or metastatic disease at diagnosis and those with disease relapse have extremely poor outcomes (Bielack, Kempf-Bielack et al. 2002, Kempf-Bielack, Bielack et al. 2005).

Until the 1980s, the extremely high propensity of osteosarcoma to form pulmonary metastases led to an almost universally fatal disease outcome with only local surgical management available. Progress was only made with the introduction of systemic chemotherapy, which was soon administered neoadjuvantly (Jaffe, Puri et al. 2013). Multiple studies using a combined approach of neoadjuvant chemotherapy and surgery showed long-term, disease-free survival rates in the range of 60-70% in young patients with apparently localized disease (Smeland, Bielack et al. 2019). Doxorubicin, high-dose methotrexate with leucovorin rescue, cisplatin, and ifosfamide have since been established as the most active agents in osteosarcoma as both neoadjuvant and adjuvant therapies. The most efficacious regimens employ at least three of these drugs, yet adding a fourth agent may not lead to further benefits (Anninga, Gelderblom et al. 2011).

Although a minority of international investigators apply other, partially divergent protocols, most experts routinely use the neoadjuvant MAP regimen of high-dose methotrexate, doxorubicin and cisplatin as their treatment standard. This choice of regimen is based on the largest osteosarcoma study ever performed, EURAMOS-1 (Marina, Smeland et al. 2016). This prospective, randomized trial, based on the MAP regimen, unequivocally proved that long-term outcomes could not be further improved by postoperative treatment alterations and augmentations for poor responders. In addition, maintenance therapy with IFNα was not of any benefit in those with a good response (Bielack, Smeland et al. 2015).

Attempts to further improve disease outcomes have generally not been met with undisputed progress. Immunotherapy with the macrophage-activator muramyl tripeptide-phosphatidyl ethanolamine encapsulated in liposomes (L-MTP-PE) was investigated in a US population (Meyers, Schwartz et al. 2008). The results were hotly disputed at the time and left many questions open (Bielack, Marina et al. 2008). The use of L-MTP-PE in patients with metastatic osteosarcoma was not found to improve event-free or overall survival and should not be used outside of clinical trials (Chou, Kleinerman et al. 2009). Patients with unresectable or widely metastatic osteosarcoma who are deemed incurable are generally managed with the same systemic therapy options, including MAP chemotherapy and local tumor control, and outcomes are very poor, with <30% of patients surviving long term (Kager, Zoubek et al. 2003). Due to the toxic effects of treatment, quality of life must be balanced against potential treatment benefits for those individuals.

There is no standard-of-care systemic therapy for patients >40 years of age with a poor outcome and few clinical trials to inform practice (Grimer, Cannon et al. 2003). Guidelines suggest that adult patients (defined as older than 40) should be treated similarly to pediatric and young adult patients. However, adult patients may require tailored regimens, especially with regards to high-dose methotrexate. Retrospective analysis of the European Musculoskeletal Oncology Society of patients over 40 years did demonstrate that adult patients may benefit from aggressive treatment with surgery and chemotherapy, with outcomes possibly being related to decreased chemotherapy administered to some of the elderly patients (Grimer, Cannon et al. 2003).

Data on lower-grade lesion management remain sparse; however, most clinicians agree that grade 1 localized parosteal tumors can be treated surgically. The general principle of treating subvariants in osteosarcoma is based on their grade. Low-grade lesions, including low-grade central lesions, are treated by local surgical control only. Intermediate-grade lesions, including periosteal lesions and most osteosarcomas that affect the jaw, similarly need local control, which is also typically surgery. For intermediate-grade osteosarcomas, the role of chemotherapy is controversial and certainly not associated with the same risk-to-benefit relationship as high-grade osteosarcomas. Osteosarcomas in other craniofacial locations can be high grade and are treated with chemotherapy and local control in such case. In craniofacial locations, local control can become challenging and is most often approached by multidisciplinary surgical oncologists and reconstructive teams.

Primary osteosarcoma resection should be conducted by experts in surgical reconstruction to preserve bone function while achieving complete resection. Otherwise, intralesional or marginal resections increase local recurrence rate, which is associated with reduced overall survival (Picci, Sangiorgi et al. 1994, Bielack, Kempf-Bielack et al. 2002). Limb salvage is feasible for most patients with extremity tumors via reconstruction using an endoprosthetic implant, or allogeneic or autologous bone graft. Minimally invasive and non-invasive growing implants enable limb-salvage reconstruction as well as future limb-length equality for patients with skeletal immaturity (Ruggieri, Mavrogenis et al. 2013). Amputation remains optimal for some patients with large tumors when limb preservation is not possible or the expected functional differences between limb-sparing surgery and amputation are small and the risks of limb-sparing surgery high. Technologies such as transosseous suture fixation devices and advances in prosthetics offer the potential for improved functionality for these patients (Hebert, Rehani et al. 2017). Local recurrence rates for extremity osteosarcoma are low and generally less than 5%, suggesting that, in most cases, complete resection is achieved. In selected patients with osteosarcoma, radiation is considered postoperatively, particularly in patients with close surgical margins and a poor grade of necrosis in the resection specimen.

Surgery is also an important local control modality for metastatic sites, with long-term survival improving with the resection of lung metastases. Here, the number of metastases and completeness of excision seem to affect outcomes (Kager, Zoubek et al. 2003).

Although osteosarcoma is regarded as a radio-resistant disease, radiotherapy as local control may be considered if resection of a primary tumor is not possible or anticipated to lead to unacceptable morbidities such as in pelvic, trunk or craniofacial primary sites of disease (Strauss, Frezza et al. 2021). Heavy particles offer a technical advantage to deliver the high doses of 60 Gy or ideally 70 Gy deemed necessary for disease control (Ciernik, Niemierko et al. 2011). Proton beam therapy and carbon ion radiotherapy are, therefore, increasingly used for patients with inoperable disease or disease at challenging primary sites. Five-year local control rates of 62-67% in patients with inoperable pelvic and trunk sarcomas are encouraging (Ciernik, Niemierko et al. 2011, Matsunobu, Imai et al. 2012). The combination of carbon ion radiotherapy and proton beam therapy for inoperable osteosarcoma was found to be feasible (Seidensaal, Mattke et al. 2021). A comprehensive evaluation of particle beam therapy, in this setting, is a priority.

Each of the above forms and stages of osteosarcoma constitutes an embodiment of each and any aspect of the invention, including any embodiment thereof. Also, each of the above methods for treating osteosarcoma may be applied to the subject in accordance with the present invention as defined in the aspects of the invention, including any embodiment thereof. Finally, each of the above methods for diagnosing osteosarcoma may be applied to the subject in accordance with the present invention as defined in the aspects of the invention, including any embodiment thereof.

In an embodiment of each and any aspect of the invention, including any embodiment thereof, the tumor is a pancreatic cancer.

The normal pancreas consists of digestive enzyme-secreting acinar cells, bicarbonate-secreting ductal cells, centro-acinar cells that are the geographical transition between acinar and ductal cells, hormone-secreting endocrine islets and relatively inactive stellate cells. The majority of malignant neoplasms of the pancreas are adenocarcinomas; rare pancreatic neoplasms include neuroendocrine tumors which can secrete hormones such as insulin or glucagon and acinar carcinomas which can release digestive enzymes into the circulation. Specifically, ductal adenocarcinoma is the most common malignancy of the pancreas; this tumor - which is commonly referred to as pancreatic cancer - presents a substantial health problem (Kleeff, Korc et al. 2016). Pancreatic cancer is associated with an extremely poor prognosis for several reasons (Siegel, Miller et al. 2023). It is usually diagnosed at advanced stages, which is often due to nonspecific and - in some cases - no symptoms, a lack of sensitive and specific tumor markers and difficulties in imaging early-stage tumors. Pancreatic cancer is aggressive, with perineural and vascular local growth and early distant metastases that preclude curative surgical resection in most patients. Pancreatic cancer is characterized by a remarkable resistance (or tolerance) to most conventional treatment options, including chemotherapy, radiotherapy, molecularly targeted therapy and immunotherapy. Finally, pancreatic cancer harbors multiple genetic and epigenetic alterations and have complex and dense tumor microenvironments. All of these factors result in an overall 5-year survival rate of <7%, with almost all survivors at this time point being the 10-20% of patients who undergo surgical resection; for these patients, the 5-year survival rate is ~15-25% (He, Ahuja et al. 2014).

In 2015, an estimated 367,000 new cases of pancreatic cancer were diagnosed worldwide, >50% of which occurred in high-income countries (Kleeff, Korc et al. 2016). In the United States, the highest rates were registered among black individuals (12-15 cases per 100,000 men and 8-10 cases per 100,000 women); these rates are ~30-50% higher than in their white counterparts. In Oceania, indigenous populations are most affected, with an >30% excess, although long-term validated statistics are unavailable. The lowest rates, which are probably influenced by under-diagnosis, were recorded in India and in Northern and Central Africa. Mortality rates closely parallel incidence rates. Increases in reported incidence and mortality have taken place since the 1970s, in particular, in high-income countries where these increases can be partially attributed to diagnostic improvements. Although some reports suggest that incidence and mortality have levelled off in Western Europe and in North America, the latest data show the incidence continuing to rise in the United Kingdom and in North America - probably as a result of an ageing population (Kleeff, Korc et al. 2016).

Age is the major determinant of pancreatic cancer. Most patients are diagnosed at >50 years of age, with peak incidence in the seventh and eighth decades of life. In terms of preventable risk factors, tobacco smoking is the most important and most studied. Smokers have a twofold to threefold higher risk of developing pancreatic cancer than non-smokers; a dose-risk relationship has been noted, as has a favorable effect of smoking cessation. The proportion of cases attributable to tobacco smoking has been estimated to be 15-30% in various populations (Bosetti, Lucenteforte et al. 2012). Obesity and low physical activity are also linked to pancreatic cancer (Genkinger, Kitahara et al. 2015). Some nutritional and dietary factors, including high intake of (saturated) fats, low intake of vegetables and fruits and consumption of red and processed meats, are also associated with risk. A positive association with heavy alcohol consumption has been observed, but no such association has been shown with moderate drinking (Tramacere, Scotti et al. 2010). Diabetes mellitus is both a risk factor for disease and a consequence of early-stage pancreatic cancer; longterm diabetes mellitus approximately doubles the risk of pancreatic cancer (Bosetti, Rosato et al. 2014).

Pancreatic cancer most frequently arises from pancreatic intraepithelial neoplasia (PanIN), the classic pre-neoplastic lesions, but can also arise from larger precursor lesions, i.e. intraductal papillary mucinous neoplasms (IPMNs) and mucinous cystic neoplasms (Hezel, Kimmelman et al. 2006). Pancreatic cancer exhibits aberrant autocrine and paracrine signaling cascades that promote pancreatic cancer cell proliferation, migration, invasion and metastasis. For example, many signaling molecules - such as TGFα, IGF1, FGFs and HGF - and their respective tyrosine kinase receptors - such as EGFR, ERBB2 (also known as HER2), HER3, IGF1R, FGFRs and HGFR (also known as MET) - activate multiple pathways that enhance pancreatic cancer cell mitogenic self-sufficiency and promote migration and invasion (Preis and Korc 2011). These signaling cascades are activated in conjunction with the activation of anti-apoptotic and pro-survival pathways, such as signal transducer and activator of transcription 3 (STAT3), nuclear factor-κB (NF-κB) and AKT. Genes that are normally active during development, such as WNT, SHH and NOTCH, are also reactivated in some pancreatic cancers (Pasca di Magliano, Biankin et al. 2007).

The signaling in pancreatic cancer is complex, with multiple nodes and aberrant crosstalk pathways. For example, one of the aberrant signaling nodes in pancreatic cancer is characterized by enhanced activity of HGFR and EGFR, increased expression of neuropilin 1, CD44 expression and β1 integrin, and is aggravated by the ability of HGFR to form heterodimers with EGFR (Tanizaki, Okamoto et al. 2011). These alterations occur in the context of oncogenic KRAS and loss of CDKN2A, which encodes the tumor suppressor p16 (Hezel, Kimmelman et al. 2006). Pancreatic cancer also exhibits metabolic abnormalities and insensitivity to growth inhibitory pathways. Loss of negative growth constraints is best exemplified by aberrant TGFβ signaling, which occurs as a consequence of increased expression of TGFβ isoforms (Gore, Deitz et al. 2014). Although TGFβ is a physiological tumor suppressor, it promotes tumor progression in pancreatic cancer and many other solid tumors by exerting paracrine effects within the tumor microenvironment that lead to enhanced growth and metastasis. TGFβ can also directly induce pancreatic cancer cell proliferation by activating non-canonical signaling through MAPK phosphorylation, SRC and AKT phosphorylation, and by upregulating WNT7B expression through canonical SMAD4-dependent mechanisms (Gore, Deitz et al. 2014).

The molecular pathology of pancreatic cancer is dominated by activating mutations in KRAS, which are present in >90% of tumors. Inactivating mutations of TP53, CDKN2A and SMAD4 occur in 50-80% of pancreatic cancers, whereas other genes, including ARID1A, MLL3 and TGFBR2, are mutated in approx. 10% of tumors. However, few genes stand out among the myriad of infrequently mutated genes, which mostly occur at a prevalence of less than 2% (Chang, Grimmond et al. 2014). Although point mutations of individual genes reveal some aspects of disease pathophysiology, other genomic events contribute to carcinogenesis and can provide insight into molecular mechanisms. For example, CDKN2A is commonly inactivated by methylation, and CDKN2A and SMAD4 are commonly inactivated through homozygous deletion. Copy number alterations, which can be difficult to interpret due to the large number of genes that are amplified or lost in these regional events, also seem to play an important part. Four subtypes of pancreatic cancer have been proposed based on the number and location of structural variants (Waddell, Pajic et al. 2015). The first three subtypes comprise tumors with stable genomes with few (<50) structural variants, those with scattered structural events (50-200) and those with unstable genomes (>200 structural variants). Those tumors with unstable genomes are suggestive of defects in DNA maintenance and are a potential biomarker for platinum and poly (ADP-ribose) polymerase (PARP) inhibitor responsiveness (Waddell, Pajic et al. 2015). The fourth group, termed locally rearranged, is defined by >50 events localized to 1-3 chromosomes. These events are typically amplifications that encompass oncogenes, which can be the targets of existing therapeutics, or genomic catastrophes such as chromothripsis.

Pancreatic cancers characteristically have an abundant and dense collagenous stroma (desmoplasia) (Seymour, Hruban et al. 1994), resulting in a considerable hypoxic environment for cancer cells. This stroma is composed of extracellular matrix (ECM) proteins - collagens, fibronectin and laminin - as well as non-collagenous proteins such as glycoproteins, proteoglycans and glycosaminoglycans. Other factors in the stroma that possibly mediate the interaction of cancer cells with the ECM include growth factors, osteopontin, periostin and serine protein acidic and rich in cysteine. Cellular elements of the stroma include pancreatic stellate cells, which produce the collagenous matrix (activated stellate cells have also been referred to as cancer-associated fibroblasts in the literature), infiltrating immune cells, endothelial cells and neuronal cells. The immune cell complement in pancreatic cancer includes T cells (a majority being CD4+ regulatory T cells), myeloid-derived suppressor cells, macrophages and mast cells (Clark, Hingorani et al. 2007). Overall, immune cell infiltration suggests an immunosuppressive phenotype, even at the earliest stages of pancreatic cancer. A growing body of evidence suggests that CD4+ regulatory T cells in the stroma play a crucial part in warding off the host immune system. This feature is clinically important because factors that mediate the suppression of active antitumor immunity - such as the ligand for programmed cell death protein 1 (PD-1), PD-L1, expressed on cancer cells - form the basis of novel immunotherapeutic approaches study in pancreatic cancer.

The clinical manifestations of pancreatic cancer are nonspecific and include jaundice, unexplained weight loss, epigastric pain that radiates to the back, nausea, new-onset diabetes mellitus and, rarely, migratory thrombophlebitis (Wolfgang, Herman et al. 2013). When a diagnosis of a pancreatic cancer is suspected, imaging must be conducted. Multidetector CT provides excellent resolution of the pancreas and surrounding vasculature and can be used to evaluate other organs for spread of the disease (Wolfgang, Herman et al. 2013). A pancreas-specific protocol with dual-phase or multi-phase dynamic contrast is usually used, including early arterial phase images (to evaluate the involvement of the coeliac trunk, the superior mesenteric artery and other arteries), pancreatic phase images (to evaluate pancreatic lesions) and portal venous phase images (to evaluate the involvement of the portal vein, the superior mesenteric vein and other veins) (Lee and Lee 2014). Most pancreatic cancers form solid hypodense lesions. Cancers of the pancreas head typically obstruct both the pancreatic ducts and the bile ducts, producing upstream dilatation of both ducts (the so-called double duct sign), whereas carcinomas of the corpus and tail of the gland will only obstruct distal parts of the pancreatic duct. Multidetector CT can define lesions in the pancreas, but is also the current gold standard to evaluate the resectability of any lesion. Involvement of the large vessels adjacent to the pancreas (such as the superior mesenteric artery and vein), might render the tumor borderline resectable, or locally advanced distant organs, such as the liver, would indicate advanced stage disease and that the patient is best treated with palliative therapy only (chemotherapy in most cases). MRI can provide excellent resolution of the pancreatic ducts and of any cysts that might be present. MRI might also be more sensitive than MDCT in detecting and evaluating liver metastases (Motosugi, Ichikawa et al. 2011). Given that pathology is required for establishing a diagnosis of pancreatic cancer, most patients will undergo endoscopic ultrasonography with fine-needle aspiration biopsy (Shin, Topazian et al. 2015). Endoscopic ultrasonography, although highly operator dependent, provides excellent resolution of the pancreas and peripancreatic vessels and lymph nodes, and can be used to obtain tissue for definitive diagnosis. PET can supplement these technologies, particularly when evaluating enlarged lymph nodes and larger masses of uncertain clinical significance, such as a persistent mass present after therapy (Parikh, Marcus et al. 2015). CA19-9 biomarker levels, although not useful for screening asymptomatic individuals, can be useful in monitoring patients with an established pancreatic cancer (Poruk, Gay et al. 2013). CA19-9 levels in the thousands of IU per ml are suggestive of metastatic disease, whereas a significant decline in levels after treatment suggests a good response. Other blood tests that are particularly useful in managing patients with pancreatic cancer include serum bilirubin and fasting blood glucose levels.

Biomarkers can be used for detection and response evaluation in palliative and neoadjuvant settings, as well as for post-resection follow-up. Several types of diagnostic biomarkers could be useful in pancreatic cancer. The most widely used biomarker is serum CA19-9. Unfortunately, CA19-9 lacks sufficient sensitivity or specificity to be useful for early pancreatic cancer diagnosis, but is routinely used to monitor disease progression, recurrence and/or therapy response (Poruk, Gay et al. 2013). Similarly, the presence of circulating tumor cells derived from pancreatic cancer could be diagnostic but are present in only some patients with metastatic disease. By contrast, circulating tumor DNA encoding mutant KRAS has been detected at the time of diagnosis in 43% of patients with localized disease, suggesting that circulating tumor DNA consisting of a panel of mutated genes such as mutated KRAS or mutated TP53 could serve as a non-invasive early diagnostic test (Bettegowda, Sausen et al. 2014).

The management of pancreatic cancer requires a comprehensive approach that integrates various treatment modalities, including surgery, radiotherapy, chemotherapy, and emerging therapies such as immunotherapy and targeted therapy. Given the aggressive nature of pancreatic cancer and the complexity of its treatment, a multimodality strategy is essential to address the disease at different stages and to improve patient outcomes.

Surgery remains the only potentially curative option for pancreatic cancer. Major pancreatectomy such as pancreaticoduodenectomy (also called the Whipple procedure) is safe when performed at high-volume centers with reported perioperative 30-day mortality rates of less than 3% (Cameron, Riall et al. 2006) (Kotecha, Tree et al. 2024). Based on the severity of the blood vessel involvement, localized pancreatic cancers are categorized into resectable pancreatic cancer, borderline resectable pancreatic cancer (BRPC), and locally advanced unresectable pancreatic cancer (LAPC) (Tempero, Malafa et al. 2017). At diagnosis, approx. 10-20% of patients present with resectable tumours, 30-40% present with BRPC or locally advanced/unresectable pancreatic cancer LAPC, and 50-60% present with metastatic or systemic disease. Although upfront surgical resection followed by adjuvant chemotherapy is the standard of care for patients with resectable pancreatic cancer, the role of neoadjuvant therapy in resectable pancreatic cancer remains controversial. Given that surgery alone is not sufficient to achieve long-term survival (Traverso 2006), adjuvant chemotherapy is standard following tumor resection (Neoptolemos, Stocken et al. 2010, Oettle, Neuhaus et al. 2013).

Radiotherapy is one of the most commonly used local therapy approaches. Radiotherapy can be used as a neoadjuvant or adjuvant therapy to improve tumor resection rate or to reduce recurrence rate or as a definitive therapy in the localized, unresectable setting to improve local control. Many adjuvant studies have included radiotherapy, usually chemoradiotherapy, in addition to a course of systemic chemotherapy (Regine, Winter et al. 2011). Of note, the exact indications for radiation across disease stages remain controversial. In part, this is reflective of variation in outcomes across historical studies that used techniques which are no longer applicable. However, recent data using modern techniques have shown increasing signals for the benefit that radiation therapy may offer across the neoadjuvant, adjuvant, and definitive settings (Wang, Yang et al. 2024).

For patients with advanced or metastatic disease, chemotherapy is the only systemic therapy that offers a meaningful benefit. Over the past decade, significant advancements have been made in chemotherapy options for pancreatic cancer, including the introduction of FOLFIRINOX (oxaliplatin, irinotecan, leucovorin, and fluorouracil), gemcitabine plus nab-paclitaxel, S-1, liposomal irinotecan (Nal-IRI), gemcitabine plus capecitabine (Gem + Cap),modified FOLFIRINOX (mFOLFIRINOX), and NALIRIFOX (liposomal irinotecan, oxaliplatin, leucovorin, and fluorouracil) (Wang, Yang et al. 2024). Immunotherapy has also made strides, with pembrolizumab, which targets the PD-1/PD-L1 immune checkpoint pathway, showing improved outcomes in MSI-H/dMMR and TMB-H solid tumors. Erlotinib, an EGFR-targeting agent, showed slight improvement when combined with gemcitabine. Recent clinical investigations have highlighted the efficacy of PARP inhibitors such as olaparib in significantly prolonging survival for patients with germline BRCA-mutated pancreatic cancer, leading to FDA approval for maintenance treatment in cases with non-progressing disease following at least 16 weeks of first-line platinum-based chemotherapy. Moreover, targeted therapies such as larotrectinib and entrectinib for NTRK fusion-positive solid tumors, dabrafenib and trametinib for solid tumors with BRAFV600E mutations, and selpercatinib for RET fusion-positive solid tumors have received FDA approval (Wang, Yang et al. 2024).

Each of the above forms and stages of pancreatic cancer constitutes an embodiment of each and any aspect of the invention, including any embodiment thereof. Also, each of the above methods for treating pancreatic cancer may be applied to the subject in accordance with the present invention as defined in the aspects of the invention, including any embodiment thereof. Finally, each of the above methods for diagnosing pancreatic cancer may be applied to the subject in accordance with the present invention as defined in the aspects of the invention, including any embodiment thereof.

In an embodiment of each and any aspect of the invention, including any embodiment thereof, an interferon is administered to the subject.

Interferons (IFNs) comprise an extensive family of cytokines that not only possess antiviral activities, but also play a fundamental role in innate and adaptive immune regulation. There are three major types of IFNs, type I, II and III. Type I IFNs, especially IFNα and IFNβ, can be produced by most cell types in the body in response to a wide range of stimuli and, in simplified terms, act as the initiators of many components of immune activation. In addition, they can kill tumor cells directly, sensitize cancer cells to chemotherapy and inhibit angiogenesis. Type II interferon, namely IFNγ, produced by cytotoxic T cells and T helper cells, plays an essential role their function. Type III interferons are less well studied but play a specific role in antiviral defense (Yusuf, Allie et al. 2023).

An important function of all interferons is the upregulation of major histocompatibility complex (MHC) molecules MHC I and MHC II, and an increase of immunoproteasome activity. All interferons significantly enhance the presentation of MHC I dependent antigens, and IFNγ also significantly stimulates the MHC II-dependent presentation of antigens. Higher MHC I expression increases presentation of viral and abnormal peptides from cancer cells to cytotoxic T cells, while the immunoproteasome processes these peptides for loading onto the MHC I molecule, thereby increasing the recognition and killing of infected or malignant cells. Higher MHC II expression increases presentation of these peptides to helper T cells; these cells release cytokines (such as more interferons and interleukins, among others) that signal to and co-ordinate the activity of other immune cells (Ikeda, Old et al. 2002, Dunn, Bruce et al. 2005, Borden, Sen et al. 2007).

Type I interferons can induce an anti-bacterial state in infected cells, controlling the spread of infectious and inflammatory agents, particularly viral pathogens (Ivashkiv and Donlin 2014, Duncan, Randall et al. 2021, Pasrija and Naime 2021). In addition, they regulate the innate immune response by facilitating antigen presentation and natural killer cell function (Meyer 2009), while mediating inflammatory pathways and cytokines (Lopez de Padilla and Niewold 2016, Mayer-Barber and Yan 2017, Gallucci, Meka et al. 2021). Finally, type I interferons can trigger the adaptive immune system, prompting high-affinity antigen-immune cell responses and the development of immune memory (McNab, Mayer-Barber et al. 2015, Schreiber 2020).

IFNγ, the only type II interferon, can enhance expression of surface major histocompatibility complex (MHC) molecules and increase the processing and presentation of tumor-specific antigens (Shankaran, Ikeda et al. 2001), thus facilitating T-cell recognition and cytotoxicity (Gao, Shi et al. 2016, Ayers, Lunceford et al. 2017, Manguso, Pope et al. 2017, Patel, Sanjana et al. 2017). However, even though IFNγ is one of the best-studied cytokines in animal models and has undergone extensive clinical testing, little is known regarding the impact of IFNγ on the immune cells of the human tumor microenvironment (TME). Most of the clinical studies focus on the efficacy of IFNy, although one study shows increased MHC expression on tumor cells in fine needle aspirates from melanoma patients treated with IFNγ (Propper, Chao et al. 2003).

Clearly, the functions of type I and II IFNs suggest that they would be beneficial if harnessed as anticancer agents and, indeed, the interaction between IFNs, tumor cells and immune cells has been extensively studied as components of cancer immunotherapies.

Preclinical and clinical research have confirmed the essential role of interferons for effective host immunological responses to malignant cells. Type I interferons (IFNα and IFNβ) directly regulate transcription of >100 downstream genes, which results in a myriad of direct (on cancer cells) and indirect (through immune effector cells and vasculature) effects on the tumor (Borden 2019). New insights into endogenous and exogenous activation of type I interferons in the tumor and its microenvironment have given impetus to drug discovery and patient evaluation of interferon-directed strategies.

Interferons are useful for treatment of few cancer types for their ability to stimulate the immune system, directly inhibit tumor growth, and improve the effectiveness of other treatments. However, their use is typically limited to certain types of cancer, and the therapy is often associated with side effects that need to be managed carefully.

Two IFNα forms have been approved for treatment of tumors, INTRON A (Interferon alfa-2b (FDA 2021)) and ROFERON-A (interferon alfa-2b).

IFNα recombinant (INTRON A) is indicated for treatment of few cancer forms,
- Hairy cell leukemia at 2 million IU/m²administered intramuscularly or subcutaneously 3 times a week for up to 6 months;
- Malignant melanoma adjuvant to surgical treatment at 20 million IU/m2 as an intravenous infusion, on 5 consecutive days per week, for 4 weeks during induction and at 10 million IU/m² as subcutaneous injection three times per week for 48 weeks for maintenance;
- Clinically aggressive follicular non-Hodgkin's lymphoma at 5 million IU subcutaneously three times per week for up to 18 months in conjunction with anthracycline-containing chemotherapy regimen and following completion of the chemotherapy regimen;
- AIDS-related Kaposi's sarcoma at 30 million IU/m²/dose administered subcutaneously or intramuscularly three times a week until disease progression or maximal response has been achieved after 16 weeks of treatment.
In addition, INTRON A is approved for the following non-cancer indications
- Condylomata acuminata involving external surfaces of the genital and perianal areas at 1 million IU per lesion in a maximum of 5 lesions in a single course. The lesions are directly injected three times weekly on alternate days for 3 weeks. An additional course may be administered at 12 to 16 weeks;
- Chronic hepatitis C at 3 million IU three times a week (TIW) administered subcutaneously or intramuscularly; in patients tolerating therapy with normalization of ALT at 16 weeks of treatment, the therapy should be extended to 18 to 24 months at 3 million IU three times per week to improve the sustained response rate;
- Chronic hepatitis B at 30 to 35 million IU per week, administered subcutaneously or intramuscularly, either as 5 million IU daily or as 10 million IU three times a week for 16 weeks.

ROFERON-A (interferon alfa-2b) was another approved IFNα preparation but is no longer marketed. It was approved for the treatment of chronic myelogenous leukemia (CML), renal cell carcinoma (RCC), melanoma and hepatitis C.

PEGylated IFNα (PEGASYS (FDA 2023)) is FDA-approved for as antiviral agent, but not for cancer indications. It is indicated for treatment of (i) chronic hepatitis C (CHC) in patients ≥ 5 years of age, and (ii) of adult patients with chronic hepatitis B infection. Adult patients receive a subcutaneous dose of 180 µg per week. Other approved PEGylated IFNα preparations are PEGINTRON and SYLATRON.

The amino acid sequence of INFα 2b (INTRON A, ROFERON-A, KEGG DRUG: Interferon alfa-2b, accessed Mar 19, 2025) is:



 (SEQ ID NO: 242)

The IFNα family has 13 subtypes and 23 different variants. The individual proteins have molecular masses between 19-26 kDa and consist of proteins with lengths of 156-166 and 172 amino acids. All IFNα subtypes possess a common conserved sequence region between amino acid positions 115-151 while the amino-terminal ends are variable. Many IFNα subtypes differ in their sequences at only one or two positions. Naturally occurring variants also include proteins truncated by 10 amino acids at the carboxy-terminal end.

The aa sequence of IFNa 2a (PEGASYS, KEGG DRUG: Peginterferon alfa-2a, accessed Mar 19, 2025) is:

CDLPQTHSLG SRRTLMLLAQ MRKISLFSCL KDRHDFGFPQ EEFGNQFQKA ETIPVLHEMI
QQIFNLFSTK DSSAAWDETL LDKFYTELYQ QLNDLEACVI QGVGVTETPL MKEDSILAVR
KYFQRITLYL KEKKYSPCAW EVVRAEIMRS FSLSTNLQES LRSKE (SEQ ID NO: 243)

No form of IFNβ has been approved for treatment of cancer. EXTAVIA is a branded version of interferon beta-1b, a first-line disease-modifying therapy that has been a standard-of-care for multiple sclerosis for many years. EXTAVIA is indicated for the treatment of MS patients with relapsing forms of the disease and for newly diagnosed patients.

The amino acid sequence of interferon beta-1B (EXTAVIA, https://www.kegg,jp/entrv/D00746 accessed Mar 19, 2025) is:

SYNLLGFLQR SSNFQSQKLL WQLNGRLEYC LKDRMNFDIP EEIKQLQQFQ KEDAALTIYE
MLQNIFAIFR QDSSSTGWNE TIVENLLANV YHQINHLKTV LEEKLEKEDF TRGKLMSSLH
LKRYYGRILH YLKAKEYSHC AWTIVRVEIL RNFYFINRLT GYLRN (SEQ ID NO: 244)

No form of IFNγ has been approved for cancer, but ACTIMMUNE (interferon gamma-1B) is an FDA approved cytokine therapy used as a standard of care treatment for osteopetrosis and chronic granulomatous disease where it is administered subcutaneously three times per week at 50 µg/m² (patients with body surface area > 0.5 m²) or 1.5 µg/kg (patients with body surface area ≤ 0.5 m²). From the 1990s, IFNγ was evaluated as a single agent cancer immunotherapy in several prospective randomized trials (Windbichler, Hausmaninger et al. 2000, Giannopoulos, Constantinides et al. 2003, Alberts, Marth et al. 2008). These trials found no benefit in cancer and thus IFNγ was largely written off as an ineffective treatment.

The amino acid sequence of interferon gamma-1B (ACTIMMUNE, https://www.kegg.jp/entry/D00747 accessed Mar 19, 2025) is:

MQDPYVKEAE NLKKYFNAGH SDVADNGTLF LGILKNWKEE SDRKIMQSQI VSFYFKLFKN
FKDDQSIQKS VETIKEDMNV KFFNSNKKKR DDFEKLTNYS VTDLNVQRKA IHELIQVMAE
LSPAAKTGKR KRSQMLFRGR (SEQ ID NO: 245)

Each of the above interferons constitutes an embodiment of each and any aspect of the invention, including each and any embodiment thereof.

In an embodiment of each and any aspect, including any embodiment thereof, a CXCL12 antagonist is an antagonist targeting the CXCL12/CXCR4/CXCR7 axis and more specifically CXCL12, CXCR4 and/or CXCR7. In an embodiment of each and any one of the aspects of the present invention, including any embodiment thereof, the CXCL12/CXCR4/CXCR7 axis refers to the system of interaction of CXCL12 with its receptor CXCL4 and with its receptor CXCL12. It will be appreciated by a person skilled in the art that interfering with this system of interaction is possible by targeting CXCL12 or any one of its receptors, i.e. CXCR4 and CXCR7.

In a more preferred embodiment, the targeting is such that a targeting agent is therapeutically active by targeting CXCL12, CXCR4 and/or CXCR7, preferably is targeting CXCL12, CXCR4 or CXCR7. In a further preferred embodiment, targeting means direct interaction, preferably physical interaction, between the targeting agent and CXCL12, CXCR4 and/or CXCR7. Due to such targeting a physiological and/or biochemical effect arising from the interaction between CXCL12 and CXCR4 and/or between CXCL12 and CXCR7 is affected and results in a therapeutic effect of the targeting agent. Preferably, the targeting agent is also referred to as CXCL12 antagonist herein. It will therefore be appreciated by a person skilled in the art that a CXCL12 antagonist used in accordance with the present invention may make use of different modes of action. Such modes of action include that the CXCL12 antagonist inhibits signaling of CXCL12 via one or both of CXCL12 receptor C-X-C chemokine receptor type 4 (CXCR4) and CXCL12 receptor C-X-C chemokine receptor type 7 (CXCR7).

It will be appreciated by a person skilled in the art that such inhibition of the signaling of CXCL12 via one or both of CXCL12 receptor CXCR4 and CXCR7 can be achieved by (a) providing a molecule binding to CXCL12 so as to inhibit the binding of CXCL12 to CXCR4 and/or CXCR7, providing a molecule binding to CXCR4 so as to inhibit the binding of CXCL12 to CXCR4, and (c) providing a molecule binding to CXCR7 so as to inhibit the binding of CXCL12 to CXCR7. Such molecule which is binding to CXCL12 is preferably one selected from the group comprising an aptamer, preferably a CXCL12 binding aptamer, a spiegelmer, preferably a CXCL12-binding spiegelmer", an antibody, preferably a CXCL12 binding antibody, a protein binding to CXCL12, a CXCL12 binding anticalin, a CXCL12 binding peptide, a fusion protein comprising a CXCL12 binding moiety and a CXCL12 binding small molecule.

Such molecule which is binding to CXCR4 to inhibit the signaling of CXCL12 is preferably one selected from the group comprising an aptamer, preferably a CXCR4 binding aptamer, a spiegelmer, preferably a CXCR4-binding Spiegelmer, an antibody, preferably a CXCR4 binding antibody, a protein binding to CXCR4, a CXCR4 binding anticalin, a CXCR4 binding peptide, a fusion protein comprising a CXCR4 binding moiety and a CXCR4 binding small molecule.

Such molecule which is binding to CXCR7 is preferably one selected from the group comprising an aptamer, preferably a CXCR7 binding aptamer, a spiegelmer, preferably a CXCR7-binding spiegelmer, an antibody, preferably a CXCR7 binding antibody, a protein binding to CXCR4, a CXCR7 binding anticalin, a CXCR7 binding peptide, a fusion protein comprising a CXCR7 binding moiety and a CXCR7 binding small molecule.

It will also be appreciated by a person skilled in the art that that such inhibition of the signaling of CXCL12 via one or both of CXCL12 receptor CXCR4 and CXCR7 can be achieved by a compound binding CXCR4 and/or CXCR7, whereby such binding of the compound interferes with signaling, preferably with the signal transducing characteristics of CXCR 4 and/or CXCR7 rather than interfering with the binding of CXCL12 to CXCR4 and/or CXCR7.

A molecule which is binding to CXCR4 and because of its binding to CXCR4 is interfering with the signal transduction characteristics of CXCR4, preferably signal transduction triggered by the binding of CXCL12 to CXCR4, is preferably one selected from the group comprising an aptamer, preferably a CXCR4 binding aptamer, a spiegelmer, preferably a CXCR4 binding spiegelmer, a protein binding to CXCR4, an antibody or antigen-binding moiety of the antibody, preferably a CXCR4 binding antibody or a CXCR4 binding fragment of the CXCR4 binding antibody, an anticalin, preferably a CXCR4 binding anticalin, a CXCR4 binding peptide, a fusion protein comprising a CXCR4 binding moiety and a CXCR4 binding small molecule.

The generation and identification, respectively, of such aptamer, spiegelmer, antibody, binding protein, anticalin, peptide, fusion protein and small molecule is known to a person skilled in the art and also disclosed herein.

In a preferred embodiment, CXCL12 is human CXCL12, CXCR4 is human CXCR4, and CXCR7 is human CXCR7.

In connection with administering to the subject suffering from the tumor a CXCL12 antagonist, it is to be acknowledged that animal studies showed that radiation-induced devascularization is efficiently restored by vasculogenesis after recruitment of bone marrow-derived cells which leads to rapid recurrence of the tumor (Kioi, Vogel et al. 2010). It is assumed that CXCL12 plays an important role in neovascularization by recruiting endothelial and other bone marrow-derived pro-angiogenic cells through a CXCR4- and CXCR7-dependent mechanism. Notably, irradiation further increases CXCL12 expression (Kioi, Vogel et al. 2010, Kozin, Kamoun et al. 2010, Liu, Alomran et al. 2014).

In an embodiment of each and any aspect of the present invention, including any embodiment thereof, the CXCL12 antagonist inhibits signaling of CXCL12 via one or both of CXCL12 receptor C-X-C chemokine receptor type 4 (CXCR4) and CXCL12 receptor C-X-C chemokine receptor type 7 (CXCR7). In a preferred embodiment thereof, the CXCL12 antagonist inhibits the binding of CXCL12 to CXCL12 receptor C-X-C chemokine receptor type 4 (CXCR4) and/or to CXCL12 receptor C-X-C chemokine receptor type 7 (CXCR7). The interaction between CXCL12 and its receptor CXCR4 is also referred to as CXCL12 - CXCR4 axis. Such CXCL12 - CXCR4 axis may be targeted by a CXCL12 antagonist. Similarly, interaction between CXCL12 and its receptor CXCR7 is also referred to as CXCL12 - CXCR7 axis. Such CXCL12 - CXCR7 axis may be targeted by a CXCL12 antagonist. In an even more preferred embodiment, such binding of CXCL12 to said receptors is effected by the CXCL12 antagonist binding to CXCL12.

CXCL12 antagonists binding to CXCL12 include, among others, a nucleic acid molecule binding to CXCL12. Such CXCL12 binding nucleic acid molecule can be a CXCL12 binding aptamer or a CXCL12 binding spiegelmer.

Aptamers are nucleic acid molecules made of D-nucleotides as building blocks which specifically bind to a target molecule through a mechanism different from Watson Crick base pairing. Spiegelmers are nucleic acid molecules made of L-nucleotides as building blocks which specifically bind to a target molecule through a mechanism different from Watson Crick base pairing. Both aptamers and spiegelmers can be made of ribonucleotides, of deoxyribonucleotides are a combination of both ribonucleotides and deoxyribonucleotides. Aptamers and spiegelmers as such are known to a person skilled in the art and are, among others, described in 'The Aptamer Handbook' (eds. Klussmann, 2006) and spiegelmers comprising both ribonucleotides and deoxyribonucleotides are disclosed in international patent application WO 2012/095303.

**In** an embodiment of each and any aspect of the present invention, including any embodiment thereof, the CXCL12 antagonist is an L-nucleic acid molecule binding to CXCL12, wherein the L-nucleic acid molecule is preferably selected from the group comprising an CXCL12 binding nucleic acid molecule of type B, an CXCL12 binding nucleic acid molecule of type C, an CXCL12 binding nucleic acid molecule of type A and an CXCL12 binding nucleic acid molecule of type D as defined in more detail herein.

The CXCL12 binding L-nucleic acid molecules can be characterized in terms of stretches of nucleotides which are also referred to herein as Boxes (see, Example 1).

The different types of CXCL12 binding nucleic acid molecules comprise three different stretches of nucleotides: the first terminal stretch of nucleotides, the central stretch of nucleotides and second terminal stretch of nucleotides. In general, CXCL12 binding nucleic acid molecules of the present invention comprise at their 5'-end and the 3'-end the terminal stretches of nucleotides: the first terminal stretch of nucleotides and the second terminal stretch of nucleotides (also referred to as 5'-terminal stretch of nucleotides and 3'-terminal stretch of nucleotides). The first terminal stretch of nucleotides and the second terminal stretch of nucleotides can, in principle due to their base complementarity, hybridize to each other, whereby upon hybridization a double-stranded structure is formed. However, such hybridization is not necessarily realized in the molecule under physiological and/or non-physiological conditions. The three stretches of nucleotides of CXCL12 binding nucleic acid molecules - the first terminal stretch of nucleotides, the central stretch of nucleotides and second terminal stretch of nucleotides - are arranged to each other in 5' -> 3'-direction: the first terminal stretch of nucleotides - the central stretch of nucleotides - the second terminal stretch of nucleotides.

However, alternatively, the second terminal stretch of nucleotides, the central stretch of nucleotides and the terminal first stretch of nucleotides are arranged to each other in 5' -> 3'-direction.

The differences in the sequences of the defined boxes or stretches between the different CXCL12 binding nucleic acid molecules influence the binding affinity to CXCL12. Based on binding analysis of the different CXCL12 binding nucleic acid molecules used in accordance with the present invention the central stretch and the nucleotides forming the same are individually and more preferably in their entirety essential for binding to human CXCL12.

The terms 'stretch' and 'stretch of nucleotide' are used herein in a synonymous manner if not indicated to the contrary.

**In** an embodiment of each and any aspect of the present invention, including any embodiment thereof, the nucleic acid used in accordance the present invention is a single nucleic acid molecule. **In** a further embodiment, the single nucleic acid molecule is present as a multitude of the single nucleic acid molecule or as a multitude of the single nucleic acid molecule species.

It will be acknowledged by the ones skilled in the art that the nucleic acid molecule used in accordance with the present invention as subject to each and any of aspect, including any embodiment thereof, preferably consists of nucleotides which are covalently linked to each other, preferably through phosphodiester links or linkages.

It is within the present invention as subject to each and any of aspect, including any embodiment thereof, that the nucleic acids comprise two or more stretches or part(s) thereof can, in principle, hybridise with each other. Upon such hybridisation a double-stranded structure is formed. It will be acknowledged by the ones skilled in the art that such hybridisation may or may not occur, particularly under in vitro and/or in vivo conditions. Also, in case of such hybridisation, it is not necessarily the case that the hybridisation occurs over the entire length of the two stretches where, at least based on the rules for base pairing, such hybridisation and thus formation of a double-stranded structure may, in principle, occur. As preferably used herein, a double-stranded structure is a part of a nucleic acid molecule or a structure formed by two or more separate strands or two spatially separated stretches of a single strand of a nucleic acid molecule, whereby at least one, preferably two or more base pairs exist which are base pairing preferably in accordance with the Watson-Crick base pairing rules. It will also be acknowledged by the one skilled in the art that other base pairing such as Hoogsten base pairing may exist in or form such double-stranded structure. It is also to be acknowledged that the feature that two stretches hybridize preferably indicates that such hybridization is assumed to happen due to base complementarity of the two stretches.

In a preferred embodiment of each and any aspect of the invention, including any embodiment thereof, the term arrangement as used herein, means the order or sequence of structural or functional features or elements described herein in connection with the nucleic acids disclosed herein.

It will be acknowledged by the person skilled in the art that the nucleic acids used in accordance with the present invention as subject to each and any of aspect, including any embodiment thereof, are capable of binding to CXCL12. Without wishing to be bound by any theory, the CXCL12 binding results from a combination of three-dimensional structural traits or elements of the nucleic acid molecule which are caused by orientation and folding patterns of the primary sequence of nucleotides forming such traits or elements, whereby preferably such traits or elements are the first terminal stretch of nucleotides, the central stretch of nucleotides and the second terminal stretch of nucleotides of CXCL12 binding nucleic acid molecules. It is evident that the individual trait or element may be formed by various different individual sequences the degree of variation of which may vary depending on the three-dimensional structure such element or trait has to form. The overall binding characteristic of the claimed nucleic acid results from the interplay of the various elements and traits, respectively, which ultimately results in the interaction of the claimed nucleic acid with its target, i. e. CXCL12. Again without being wished to be bound by any theory, the central stretch of nucleotides that is characteristic for CXCL12 binding nucleic acids seems to be important for mediating the binding of the claimed nucleic acid molecules with CXCL12. Accordingly, the nucleic acids according to the present invention are suitable for the interaction with CXCL12. Also, it will be acknowledged by the person skilled in the art that the nucleic acids according used in accordance with the present invention are antagonists to CXCL12. Because of this, the nucleic acids according to the present invention are suitable for the treatment and prevention of brain cancer in accordance with the method of the present invention.

The nucleic acid molecules used in accordance with the present invention as subject to each and any of aspect, including any embodiment thereof, shall also comprise nucleic acids which are essentially homologous to the particular sequences disclosed herein. The term substantially homologous shall be understood such as the homology is at least 75%, preferably 85%, more preferably 90% and most preferably more that 95 %, 96 %, 97 %, 98 % or 99%.

The actual percentage of homologous nucleotides present in the nucleic acid used in accordance with the present invention as subject to each and any of aspect, including any embodiment thereof, will depend on the total number of nucleotides present in the nucleic acid. The percent modification can be based upon the total number of nucleotides present in the nucleic acid.

The homology between two nucleic acid molecules can be determined as known to the person skilled in the art. More specifically, a sequence comparison algorithm may be used for calculating the percent sequence homology for the test sequence(s) relative to the reference sequence, based on the designated program parameters. The test sequence is preferably the sequence or nucleic acid molecule which is said to be homologous or to be tested whether it is homologous, and if so, to what extent, to a different nucleic acid molecule, whereby such different nucleic acid molecule is also referred to as the reference sequence. In an embodiment, the reference sequence is a nucleic acid molecule as described herein, preferably a nucleic acid molecule having a sequence according to any one of SEQ ID NO: 5 to SEQ ID NO: 225, more preferably a nucleic acid molecule having a sequence according to any one of SEQ ID NO: 22, SEQ ID NO: 28, SEQ ID NO: 120, SEQ ID NO: 128, SEQ ID NO: 129, SEQ ID NO: 134, SEQ ID NO: 135, SEQ ID NO: 223, SEQ ID NO: 224, SEQ ID NO: 84, SEQ ID NO: 146, SEQ ID NO: 142, SEQ ID NO: 143, and SEQ ID NO: 144. Optimal alignment of sequences for comparison can be conducted, e.g., by the local homology algorithm of Smith & Waterman (Smith & Waterman, 1981) by the homology alignment algorithm of Needleman & Wunsch (Needleman & Wunsch, 1970) by the search for similarity method of Pearson & Lipman (Pearson & Lipman, 1988), by computerized implementations of these algorithms (GAP, BESTFIT, FASTA, and TFASTA in the Wisconsin Genetics Software Package, Genetics Computer Group, 575 Science Dr., Madison, Wis.), or by visual inspection.

One example of an algorithm that is suitable for determining percent sequence identity is the algorithm used in the basic local alignment search tool (hereinafter "BLAST "), see, e.g. Altschul et al (Altschul, Gish et al. 1990, Altschul, Madden et al. 1997). Software for performing BLAST analyses is publicly available through the National Center for Biotechnology Information (hereinafter "NCBI"). The default parameters used in determining sequence identity using the software available from NCBI, e.g., BLASTN (for nucleotide sequences) and BLASTP (for amino acid sequences) are described in McGinnis et al (McGinnis and Madden 2004).

The nucleic acid molecules used in accordance with the present invention as subject to each and any of aspect, including any embodiment thereof, shall also comprise nucleic acid molecules which have a certain degree of identity relative to the nucleic acid molecules disclosed herein and defined by their nucleotide sequence. More preferably, the instant invention also comprises those nucleic acid molecules which have an identity of at least 75%, preferably 85%, more preferably 90% and most preferably more than 95 %, 96 %, 97 %, 98 % or 99% relative to the nucleic acid molecules disclosed herein and defined by their nucleotide sequence or a part thereof.

The terms nucleic acid(s) and nucleic acid molecule(s) are used interchangeably herein. Also, the nucleic acid molecules used in accordance with the present invention shall also comprise those nucleic acid molecules comprising the nucleic acids sequences disclosed herein or part thereof, such as, e.g., a metabolite or derivative of the nucleic acid molecules used in accordance with the present invention, preferably to the extent that the nucleic acid molecules or said parts are involved in the or capable of binding to CXCL12. Such a nucleic acid molecule may be derived from the ones disclosed herein, e.g., by truncation. Truncation may be related to either or both of the ends of the nucleic acid molecules as disclosed herein. Also, truncation may be related to the inner sequence of nucleotides, i.e. it may be related to the nucleotide(s) between the 5' and the 3' terminal nucleotide, respectively. Moreover, truncation shall comprise the deletion of as little as a single nucleotide from the sequence of the nucleic acids disclosed herein. Truncation may also be related to more than one stretch of the nucleic acid molecules used in accordance with the present invention, whereby the stretch can be as little as one nucleotide long. The binding of a nucleic acid molecules used in accordance with the present invention can be determined by the ones skilled in the art using routine experiments or by using or adopting a method as described herein, preferably as described herein in the example part.

In addition, it is within that present invention as subject to each and any of aspect, including any embodiment thereof, that one or several parts of the nucleic acid molecule used in accordance with the present invention are present as D-nucleic acids or at least one or several parts of the nucleic acid molecules are L-nucleic acids. The term "part" of the nucleic acid molecules shall mean as little as one nucleotide. Such nucleic acid molecules are generally referred to herein as D- and L-nucleic acids, respectively. Therefore, in a particularly preferred embodiment, the nucleic acid molecules used in accordance with the present invention consist of L-nucleotides and comprise at least one D-nucleotide. Such D-nucleotide is preferably attached to a part different from the stretches defining the nucleic acid molecules used in accordance with the present invention, preferably those parts thereof, where an interaction with other parts of the nucleic acid molecule is involved. Preferably, such D-nucleotide is attached at a terminus of any of the stretches and of any nucleic acid molecule used in accordance with the present invention, respectively. In a further preferred embodiment, such D-nucleotides may act as a spacer or a linker, preferably attaching modifications such as PEG and HES to the nucleic acids according to the present invention.

It is also within the present invention as subject to each and any of aspect, including any embodiment thereof, that the nucleic acid molecules used in accordance with the present invention as subject to each and any of aspect, including any embodiment thereof, are part of a longer nucleic acid molecule, whereby this longer nucleic acid molecule comprises several parts, whereby at least one such part is a nucleic acid molecule, or a part thereof, used in accordance with the present invention. The other part(s) of these longer nucleic acid molecules can be either one or several D-nucleic acid(s) or L-nucleic acid(s). Any combination may be used in connection with the present invention. These other part(s) of the longer nucleic acid molecule can exhibit a function which is different from binding, preferably from binding to CXCL12. One possible function is to allow interaction with other molecules, whereby such other molecules preferably are different from CXCL12, such as, e.g., for immobilization, cross-linking, detection or amplification. In a further embodiment of the present invention the nucleic acid molecules used in accordance with the invention comprise, as individual or combined moieties, several of the nucleic acid molecules used in accordance with the present invention. Such nucleic acid molecule comprising several of the nucleic acid molecules used in accordance with the present invention as subject to each and any of aspect, including any embodiment thereof, is also encompassed by the term longer nucleic acid.

L-nucleic acid molecules as used herein are nucleic acid consisting of consisting of L-nucleotides, preferably consisting completely of L-nucleotides.

D-nucleic acid molecules as used herein are nucleic acid molecules consisting of D-nucleotides, preferably consisting completely of D-nucleotides.

The terms nucleic acid and nucleic acid molecule are used herein in an interchangeable manner if not explicitly indicated to the contrary.

Also, if not indicated to the contrary, any nucleotide sequence is set forth herein in 5' -> 3' direction.

As preferably used herein, any position of a nucleotide is determined or referred to relative to the 5' end of a sequence, a stretch or a substretch. Accordingly, a second nucleotide is the second nucleotide counted from the 5' end of the sequence, stretch and substretch, respectively. Also, in accordance therewith, a penultimate nucleotide is the second nucleotide counted from the 3' end of a sequence, stretch and substretch, respectively.

It is also within the present invention as subject to each and any of aspect, including any embodiment thereof, that the nucleic acid molecules used in accordance with the present invention as subject to each and any of aspect, including any embodiment thereof, regardless whether they are present as D-nucleic acids, L-nucleic acids or D,L-nucleic acids or whether they are DNA or RNA, may be present as single stranded or double stranded nucleic acids. Typically, the nucleic acid molecules used in accordance with the present invention are single stranded nucleic acids which exhibit defined secondary structures due to the primary sequence and may thus also form tertiary structures. The nucleic acid molecules used in accordance with the present invention, however, may also be double stranded in the meaning that two strands which are complementary or partially complementary to each other, are hybridised to each other or at least may be hybridised to each other in light of their base complementarity.

The nucleic acid molecules used in accordance with the present invention as subject to each and any of aspect, including any embodiment thereof, may be modified. Such modifications may be related to a single nucleotide of the nucleic acid and are well known in the art. Examples for such modification are described by, among others, Venkatesan et al. (Venkatesan, Kim et al. 2003) and Kusser (Kusser 2000). Such modification can be a H atom, an F atom or O-CH₃ group or NH₂-group at the 2' position of the individual nucleotide of which the nucleic acid consists. Also, the nucleic acid molecule used in accordance with the present invention can comprises at least one LNA nucleotide. In an embodiment the nucleic acid according to the present invention consists of LNA nucleotides.

In an embodiment of each and any aspect of the invention, including any embodiment thereof, the nucleic acid molecules used in accordance with the present invention as subject to each and any of aspect, including any embodiment thereof, may be a multipartite nucleic acid molecule. A multipartite nucleic acid molecule as used herein, is a nucleic acid molecule which consists of at least two separate nucleic acid strands. These at least two nucleic acid strands form a functional unit whereby the functional unit is a ligand to a target molecule. The at least two nucleic acid strands may be derived from any of the nucleic acid molecules used in accordance with the present invention by either cleaving the nucleic acid molecule to generate two strands or by synthesising one nucleic acid corresponding to a first part of the overall nucleic acid molecule and another nucleic acid molecule corresponding to the second part of the overall nucleic acid molecule. It is to be acknowledged that both the cleavage and the synthesis may be applied to generate a multipartite nucleic acid molecule where there are more than two strands as exemplified above. In other words, the at least two separate nucleic acid strands are typically different from two strands being complementary and hybridising to each other although a certain extent of complementarity between said at least two separate nucleic acid strands may exist and whereby such complementarity may result in the hybridisation of said separate strands.

Finally, it is also within the present invention as subject to each and any of aspect, including any embodiment thereof, that a fully closed, i.e. circular structure for the nucleic acid molecules used in accordance with the present invention is realized, i.e. that the nucleic acid molecules are closed in an embodiment, preferably through a covalent linkage, whereby more preferably such covalent linkage is made between the 5' end and the 3' end of the nucleic acid sequences as disclosed herein or any derivative thereof.

An option for determining the binding constants of a nucleic acid molecule used in accordance with the present invention is the use of the methods as described in Examples 5 and 6 which confirms the above finding that the nucleic acid molecules used in accordance with the present invention according to the present invention exhibit a favourable K_{D} value range. An appropriate measure in order to express the intensity of the binding between the individual nucleic acid molecule and the target which is in the present case CXCL12, is the so-called K_{D} value which as such as well the method for its determination are known to the one skilled in the art.

Preferably, the K_{D} value shown by the nucleic acid molecules used in accordance with the present invention as subject to each and any of aspect, including any embodiment thereof, is below 1 µM. A K_{D} value of about 1 µM is said to be characteristic for a non-specific binding of a nucleic acid to a target. As will be acknowledged by the ones skilled in the art, the K_{D} value of a group of compounds such as the nucleic acid molecules used in accordance with the present invention as subject to each and any of aspect, including any embodiment thereof, is within a certain range. The above-mentioned K_{D} of about 1 µM is a preferred upper limit for the K_{D} value. The lower limit for the K_{D} of target binding nucleic acids can be as little as about 10 picomolar or can be higher. It is within the present invention that the K_{D} values of individual nucleic acid molecule binding to CXCL12 is preferably within this range. Preferred ranges can be defined by choosing any first number within this range and any second number within this range. Preferred upper K_{D} values are 250 nM and 100 nM, preferred lower K_{D} values are 50 nM, 10 nM, 1 nM, 100 pM and 10 pM. The more preferred upper K_{D} value is 2.5 nM, the more preferred lower K_{D} value is 100 pM.

In addition to the binding properties of the nucleic acid molecules used in accordance with the present invention as subject to each and any of aspect, including any embodiment thereof, the nucleic acid molecules used in accordance with the present invention as subject to each and any of aspect, including any embodiment thereof, inhibit the function of the respective target molecule which is in the present case CXCL12. The inhibition of the function of CXCL12 - for instance the stimulation of the respective receptors as described above - is achieved by binding of a nucleic acid molecule used in accordance with the present invention to CXCL12 and forming a complex of the nucleic acid molecule and CXCL12. Such complex of the nucleic acid molecule and CXCL12 cannot stimulate the receptors that normally are stimulated by CXCL12, such as CXCR4 and CXCR7. Accordingly, the inhibition of receptor function by nucleic acid molecules is independent from the respective receptor that can be stimulated by CXCL12 but results from preventing the stimulation of the receptor by CXCL12 by the nucleic acid molecule.

A possibility to determine the inhibitory constant of the nucleic acid molecule used in accordance with the present invention is the use of the methods as described in Example 7 (for CXCR4 and CXCR7, respectively) which confirms the above finding that the nucleic acid molecules used in accordance with the present invention as subject to each and any of aspect, including any embodiment thereof, exhibit a favourable inhibitory constant which allows the use of said nucleic acid molecules in the claimed method of treatment and any treatment scheme comprising such method of treatment. An appropriate measure in order to express the intensity of the inhibitory effect of the individual nucleic acid molecule on interaction of the target which is in the present case CXCL12 and the respective receptor, is the so-called half maximal inhibitory concentration (abbr. IC₅₀) which as such as well the method for its determination are known to the one skilled in the art.

Preferably, the IC₅₀ value shown by the nucleic acid molecules used in accordance with the present invention as subject to each and any of aspect, including any embodiment thereof, is below 1 µM. An IC₅₀ value of about 1 µM is said to be characteristic for a non-specific inhibition of target functions by a nucleic acid molecule. As will be acknowledged by a person skilled in the art, the IC₅₀ value of a group of compounds such as the nucleic acid molecules used in accordance with the present invention is within a certain range. The above-mentioned IC₅₀ of about 1 µM is a preferred upper limit for the IC₅₀ value. The lower limit for the IC₅₀ of target binding nucleic acid molecules can be as little as about 10 picomolar or can be higher. It is within the present invention as subject to each and any of aspect, including any embodiment thereof, that the IC₅₀ values of individual nucleic acid molecule binding to CXCL12 is preferably within this range. Preferred ranges can be defined by choosing any first number within this range and any second number within this range. Preferred upper IC₅₀ values are 250 nM and 100 nM, preferred lower IC₅₀ values are 50 nM, 10 nM, 1 nM, 100 pM and 10 pM. The more preferred upper IC₅₀ value is 2.5 nM, the more preferred lower IC₅₀ value is 100 pM.

The nucleic acid molecules used in accordance with the present invention as subject to each and any of aspect, including any embodiment thereof, may have any length provided that they are still able to bind to the target molecule. It will be acknowledged in the art that there are preferred lengths of the nucleic acid molecules used in accordance with the present invention. Typically, the length is between 15 and 120 nucleotides. It will be acknowledged by the ones skilled in the art that any integer between 15 and 120 is a possible length for the nucleic acid molecules used in accordance with the present invention. More preferred ranges for the length of the nucleic acid molecules used in accordance with the present invention are lengths of about 20 to 100 nucleotides, about 20 to 80 nucleotides, about 20 to 60 nucleotides, about 20 to 50 nucleotides and about 29 to 45 nucleotides.

It is within the present invention as subject to each and any of aspect, including any embodiment thereof, that the nucleic acids used in accordance with the present invention as subject to each and any of aspect, including any embodiment thereof, comprise a moiety which preferably is a high molecular weight moiety and/or which preferably allows to modify the characteristics of the nucleic acid in terms of, among others, residence time in the animal body, preferably the human body. A particularly preferred embodiment of such modification is PEGylation and HESylation of the nucleic acids used in accordance with the present invention. As used herein PEG stands for poly(ethylene glycole) and HES for hydroxyethly starch. PEGylation as preferably used herein is the modification of a nucleic acid used in accordance with the present invention, whereby such modification consists of a PEG moiety which is attached to a nucleic acid used in accordance with the present invention. HESylation as preferably used herein is the modification of a nucleic acid used in accordance with the present invention, whereby such modification consists of a HES moiety which is attached to a nucleic acid used in accordance with the present invention. These modifications as well as the process of modifying a nucleic acid using such modifications, is described in European patent application EP 1 306 382, the disclosure of which is herewith incorporated in its entirety by reference.

In case of PEG being such high molecular weight moiety, the molecular weight is preferably from about 20,000 to about 120,000 Da, more preferably from about 30,000 to about 80,000 Da and most preferably about 40,000 Da. In case of HES being such high molecular weight moiety, the molecular weight is preferably from about 50 to about 1000 kDa, more preferably from about 100 to about 700 kDa and most preferably from about 200 to about 500 kDa. HES exhibits a molar substitution of 0.1 to 1.5, more preferably of 1 to 1.5 and exhibits a substitution sample expressed as the C2/C6 ratio of approximately 0.1 to 15, preferably of approximately 3 to 10. The process of HES modification is, e.g., described in German patent application DE 1 2004 006 249.8 the disclosure of which is herewith incorporated in its entirety by reference.

The modification can, in principle, be made at any position thereof to the nucleic acid molecules used in accordance with the present invention as subject to each and any of aspect, including any embodiment thereof. Preferably such modification is made either to the 5' -terminal nucleotide, the 3'-terminal nucleotide and/or any nucleotide between the 5' nucleotide and the 3' nucleotide of the nucleic acid molecule.

The modification and preferably the PEG and/or HES moiety can be attached to the nucleic acid molecule used in accordance with the present invention as subject to each and any of aspect, including any embodiment thereof, either directly or indirectly, preferably through a linker. It is also within the present invention that the nucleic acid molecule used in accordance with the present invention comprises one or more modifications, preferably one or more PEG and/or HES moiety. In an embodiment the individual linker molecule attaches more than one PEG moiety or HES moiety to a nucleic acid molecule used in accordance with the present invention. The linker used in connection with the present invention can itself be either linear or branched. This kind of linkers are known to the ones skilled in the art and are further described in patent applications WO2005/074993 and WO2003/035665.

In a preferred embodiment of each and any aspect of the invention, including any embodiment thereof, as subject to each and any of aspect, including any embodiment thereof, the linker is a biodegradable linker. The biodegradable linker allows to modify the characteristics of the nucleic acid used in accordance with the present invention in terms of, among other, residence time in an animal body, preferably in a human body, due to release of the modification from the nucleic acid according to the present invention. Usage of a biodegradable linker may allow a better control of the residence time of the nucleic acid molecule used in accordance with the present invention. A preferred embodiment of such biodegradable linker is a biodegradable linker as described in, but not limited to, international patent applications WO2006/052790, WO2008/034122, WO2004/092191 and WO2005/099768.

It is within the present invention as subject to each and any of aspect, including any embodiment thereof, that the modification or modification group is a biodegradable modification, whereby the biodegradable modification can be attached to the nucleic acid molecule used in accordance with the present invention as subject to each and any of aspect, including any embodiment thereof, either directly or indirectly, preferably through a linker. The biodegradable modification allows to modify the characteristics of the nucleic acid used in accordance with the present invention in terms of, among other, residence time in an animal body, preferably in a human body, due to release or degradation of the modification from the nucleic acid molecule used in accordance with the present invention. Usage of biodegradable modification may allow a better control of the residence time of the nucleic acid according to the present invention. A preferred embodiment of such biodegradable modification is biodegradable as described in, but not restricted to, international patent applications WO2002/065963, WO2003/070823, WO2004/113394 and WO2000/41647, preferably in WO2000/41647, page 18, line 4 to 24.

Beside the modifications as described above, other modifications can be used to modify the characteristics of the nucleic acids used in accordance with the present invention as subject to each and any of aspect, including any embodiment thereof, whereby such other modifications may be selected from the group of proteins, lipids such as cholesterol and sugar chains such as amylase, dextran etc.

Without wishing to be bound by any theory, it seems that by modifying the nucleic acid molecules used in accordance with the present invention as subject to each and any of aspect, including any embodiment thereof with high molecular weight moiety such as a polymer and more particularly one or several of the polymers disclosed herein, which are preferably physiologically acceptable, the excretion kinetic is changed. More particularly, it seems that due to the increased molecular weight of such modified nucleic acids and due to the nucleic acid molecules used in accordance with the present invention not being subject to metabolism particularly when in the L-form, excretion from an animal body, preferably from a mammalian body and more preferably from a human body is decreased. As excretion typically occurs via the kidneys, glomerular filtration rate of the thus modified nucleic acid molecules is significantly reduced compared to nucleic acid molecules not having this kind of high molecular weight modification which results in an increase in the residence time in the animal body. **In** connection therewith it is particularly noteworthy that, despite such high molecular weight modification the specificity of the nucleic acid molecules used in accordance with the present invention is not affected in a detrimental manner. Insofar, the nucleic acid molecules used in accordance with the present invention have among others, the surprising characteristic - which normally cannot be expected from pharmaceutically active compounds - such that a pharmaceutical formulation providing for a sustained release is not necessarily required to provide for a sustained release of the nucleic acid molecules used in accordance with the present invention. Rather, the nucleic acid molecules used in accordance with the present invention in their modified form comprising a high molecular weight moiety, can as such already be used as a sustained release-formulation as they act, due to their modification, already as if they were released from a sustained-release formulation. Insofar, the modification(s) of the nucleic acid molecules used in accordance with the present invention as disclosed herein and the thus modified nucleic acid molecules used in accordance with the present invention and any composition comprising the same may provide for a distinct, preferably controlled pharmacokinetics and biodistribution thereof. This also includes residence time in circulation and distribution to tissues. Such modifications are further described in the patent application WO2003/035665.

However, it is also within the present invention as subject to each and any of aspect, including any embodiment thereof, that the nucleic acid molecules used in accordance with the present invention as subject to each and any of aspect, including any embodiment thereof, do not comprise any modification and particularly no high molecular weight modification such as PEGylation or HESylation. Such embodiment is particularly preferred when the nucleic acid molecule used in accordance with the present invention shows preferential distribution to any target organ or tissue in the body or when a fast clearance of the nucleic acid molecule used in accordance with the present invention from the body after administration is desired. Nucleic acid molecules used in accordance with the present invention with a preferential distribution profile to any target organ or tissue in the body would allow establishment of effective local concentrations in the target tissue while keeping systemic concentration of the nucleic acid molecules low. This would allow the use of low doses which is not only beneficial from an economic point of view, but also reduces unnecessary exposure of other tissues to the nucleic acid agent, thus reducing the potential risk of side effects. Fast clearance of the nucleic acid molecules used in accordance with the present invention from the body after administration might be desired, among others, in case of *in vivo* imaging or specific therapeutic dosing requirements using the nucleic acid molecules used in accordance with the present invention as subject to each and any of aspect, including any embodiment thereof, or medicaments comprising the same.

In an embodiment of each and any aspect of the invention, including any embodiment thereof, a pharmaceutical composition comprising a nucleic acid molecule used in accordance with the present invention comprises at least one of said nucleic acid molecules and a pharmaceutically acceptable excipient. Such pharmaceutical composition may additionally comprise one or more further pharmaceutically active compounds. Such pharmaceutically acceptable excipient may be, e.g., water, buffer, PBS, glucose solution, preferably a 5% glucose salt balanced solution, starch, sugar, gelatine or any other acceptable carrier substance. Such excipients are generally known to the one skilled in the art. It will be acknowledged by the person skilled in the art that any embodiments, use and aspects of or related to the medicament of the present invention as subject to each and any of aspect, including any embodiment thereof, is also applicable to the pharmaceutical composition of the present invention and vice versa.

In a preferred embodiment of the present invention as subject to each and any of aspect, including any embodiment thereof, the malignant tumor results from the involvement, either direct or indirect, of CXCL12 in the pathogenic mechanism underlying said malignant tumor.

In a preferred embodiment of each and any aspect of the invention, including any embodiment thereof, the CXCL12 antagonist is NOX-A12. NOX-A12 (CAS-RN 1322069-19-1) is an (L)-oligonucleotide of 45 (L)-ribonucleotides in length (Mol. Wt. 14.5 kDa) that terminates at the 5'-end in a hexylamino linker, to which a branched 40 kDa monomethoxy polyethylene glycol (PEG) moiety is covalently attached. The PEG moiety increases the plasma half-life of the molecule. NOX-A12 is suitable for parenteral administration and has a good subcutaneous bioavailability. NOX-A12 is a nucleic acid molecule of SEQ ID NO: 28. It will be appreciated by a person skilled in the art that a preferred nucleic acid molecule binding to CXCL12 is an L-nucleic acid molecule having an identity of at least 80 % to an L-nucleic acid molecule of SEQ ID NO: 28 or SEQ ID NO: 22.

The chemical structure of NOX-A12 can also be depicted as L-guanylyl-(3'-5')- L-cytidylyl-(3'-5')-L-guanylyl-(3'→5')- L-uridylyl-(3'→5')- L-guanylyl-(3'-5')- L-guanylyl-(3'-5')- L-uridylyl-(3'→5')-L-guanylyl-(3'-5')- L-uridylyl-(3'→5')- L-guanylyl-(3'→5')- L-adenylyl-(3'-5')- L-unidylyl-(3'→5')-L-cytidylyl-(3'→5')- L-uridylyl-(3'→5')- L-adenylyl-(3'→5')- L-guanylyl-(3'-5')- L-adenylyl-(3'→5')- L-uridylyl-(3'→5')- L-guanylyl-(3'-5')- L-uridylyl-(3'→5')- L-adenylyl-(3'-5')- L-uridylyl-(3'→5')- L-uridylyl-(3'→5')- L-guanylyl-(3'-5')- L-guanylyl-(3'→5')- L-cytidylyl-(3'→5')- L-uridylyl-(3'→5')- L-guanylyl-(3'→5')- L-adenylyl-(3'-5')- L-uridylyl-(3'→5')- L-cytidylyl-(3'-5')-L-cytidylyl-(3'→5')- L-uridylyl-(3'→5')- L-adenylyl-(3'-5')- L-guanylyl-(3'→5')- L-uridylyl-(3'→5')-L-cytidylyl-(3'→5')- L-adenylyl-(3'-5')- L-guanylyl-(3'-5')- L-guanylyl-(3'→5')- L-uridylyl-(3'-5')- L-adenylyl-(3'-5')- L-cytidylyl-(3'→5')- L-guanylyl-(3'-5')- L-cytidylyl-(3'-5'), 5'-ester with N-[ω-methoxypoly(oxy-1,2-ethanediyl], N'-[ω-methoxypoly(oxy-1,2-ethanediyl]-acetyl-9-amino-8-oxo-7-aza-nonyloxy phosphate, sodium salt, whereby it additionally comprises a 40 kDa PEG moiety attached to the 5'-terminal end of the nucleotide sequence by means of the above indicated linker. In case of NOX-A12, the 40 kDa PEG moiety increases plasma half-life in humans from a few minutes (for the non-PEGylated L-nucleic acid molecule) to almost two days.

In a preferred embodiment of each and any aspect of the invention, including any embodiment thereof, the CXCL12 antagonist is NOX-A12.

In an embodiment of each and any aspect of the invention, including any embodiment thereof, the CXCL12 antagonist is Olaptesed pegol. Preferably, the terms NOX-A12 and Olaptesed pegol are used synonymously.

In an embodiment of each and any aspect of the invention, including any embodiment thereof, the CXCL12 antagonist is an CXCL12 binding aptamer provided by Aptitude Medical Systems, originally provided for the treatment of wet AMD.

In another embodiment of each and any aspect of the invention, including any embodiment thereof, the CXCL12 antagonist binding to CXCL12 and inhibiting binding of CXCL12 to CXCL12 receptor CXCR4 and/or CXCR7 is an antibody binding to CXCL12, preferably an antibody binding to CXCL12 and inhibiting binding of CXCL12 to CXCL12 receptor CXCR4 and/or CXCR7. In this embodiment, CXCL12 is the target or antigen of the antibody. In a preferred embodiment, the antibody is a monoclonal antibody.

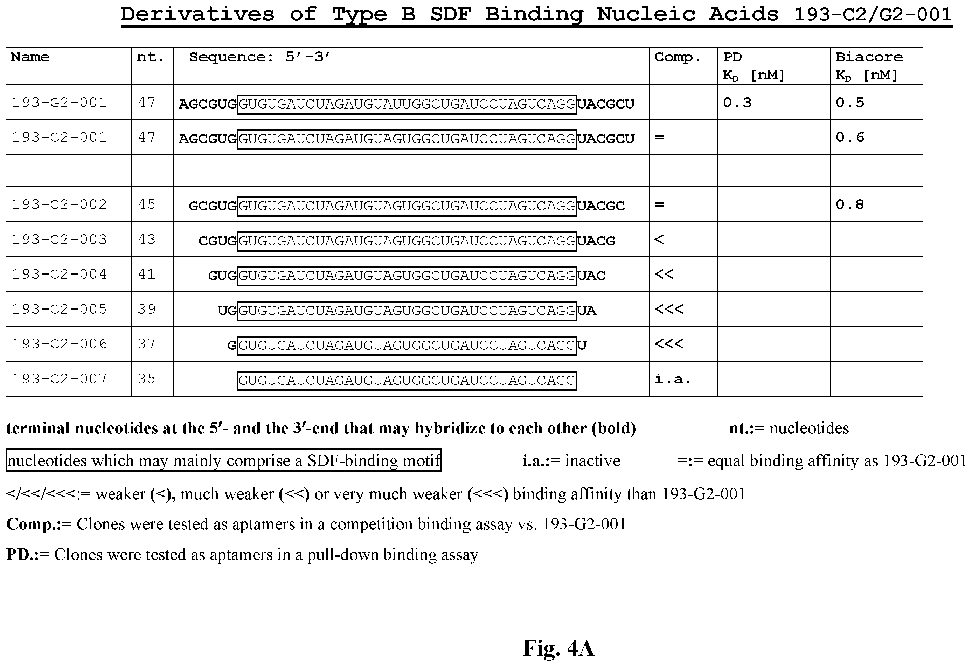

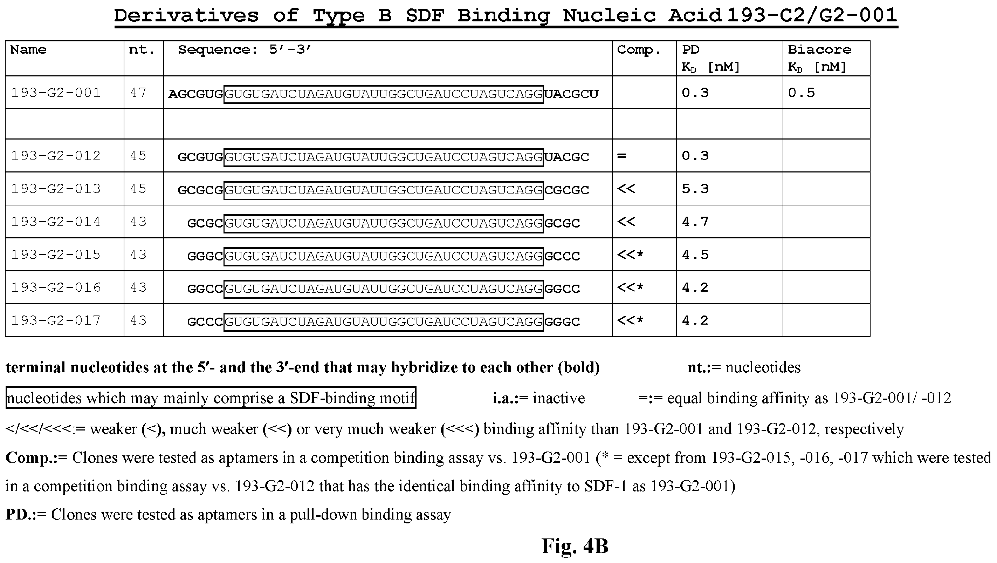

Monoclonal antibodies directed to CXCL12 are known in the art and include, among others, mAB-30D8 (provided by Genentech), 1131-H12 or 1143-H1 (both provided by iOnctura). The antibody sequence of antibody mAB-30D8, including a humanized form thereof, is disclosed in Zhong C et al. (Zhong, Wang et al. 2013); see, suppl. Fig. 4).

In a further embodiment of each and any aspect of the invention, including any embodiment thereof, CXCL12 is bound by a protein, such protein may be a CXCL12 binding TRAP protein (provided by OncoTrap, Inc.) or a CXCL12 binding anticalin. In an embodiment, the TRAP protein is provided through a TRAP protein coding mRNA delivered to the tumor. The expression product of the mRNA is secreted by the transfected cells and diffuses throughout the tumor environment capturing CXCL12 with nanomolar binding affinity, whereupon CXCL12 is trapped and its function in the tumor microenvironment is abolished. In an embodiment and as preferably used herein an anticalin is a target binding polypeptide. This class of target binding polypeptides is, among others, described in German patent application DE 197 42 706.

It will be appreciated by a person skilled in the art that apart from the above classes of compounds, also a small molecule might in principle be used as an antagonist of CXCL12. The latter even more so is due to the either direct or indirect interaction, preferably direct physical interaction of such small molecule the binding of CXCL12 to its receptor CXCR4 and/or CXCR7 is inhibited. As preferably used herein, a small molecule is a molecule the molecule weight of which is 900 Da or less. In an alternative, a small molecule is a molecule which is in compliance with Lipinski's rule of five, i.e. that the compound is no more than one violation of the following criteria: no more than 5 hydrogen bond donors (the total number of nitrogen-hydrogen and oxygen-hydrogen bonds), no more than 10 hydrogen bond acceptors (all nitrogen or oxygen atoms), a molecular mass less than 500 Daltons; and an octanol-water partition coefficient (log P) that does not exceed 5.

In another embodiment of each and any aspect of the invention, including any embodiment thereof, the CXCL12 antagonist is binding directly to CXCL12 and inhibits the binding of CXCL12 to CXCR4. Examples for this kind of CXCL12 antagonists are disclosed in CN 115403510A as CP1 to CP23.

In another embodiment of each and any aspect of the invention, including any embodiment thereof, the CXCL12 antagonist is binding to CXCR4 and inhibits the binding of CXCL12 to CXCR4. In other words, CXCR4 is the target and antigen, respectively, which is bound by this embodiment of a CXCL12 antagonist. As in case of the CXCL12 antagonist binding to CXCL12 and inhibiting the binding of CXCL12 to CXCR4 and/or CXCR7, the CXCL12 antagonist binding to CXCR4 may be a compound selected from the group comprising the following classes of compounds: aptamers, spiegelmers, antibodies, target binding proteins, target binding peptides, synthetic peptides, peptides, anticalins, a small molecules and fusion proteins.

Various small molecules are disclosed in the prior art which may be used as a CXCL12 antagonist due to their binding to CXCR4 in the present invention as subject to each and any of aspect, including any embodiment thereof. Such small molecules include, but are not limited to, plerixafor (Sanofi), mavorixafor (X4 Pharmaceuticals), Q-122 (Que Oncology), HPH-112 (Harmonic Pharma), BKT-300 (Biokine Therapeutics), X4P-002 (X4 Pharmaceuticals), X4P-003 (X4 Pharmaceuticals), X4-136(X4 Pharmaceuticals), GP-01CR01 (GPCR Therapeutics), GP-01CR11 (GPCR Therapeutics), GP-01CR21(GPCR Therapeutics), Pentixather (Pentixapharm), BKT-170 (Biokine Therapeutics), BMS-585248 (Bristol-Myers Squibb), CS-3955 (Kureha), CTCE-0012 (British Canadian Biosciences), CTCE-9908 (British Canadian Biosciences), GBV-4086 (Globavir Biosciences), HPH-112 (Harmonic Pharma), HPH-211 (Harmonic Pharma), NB-325 (Novaflux Biosciences), SP-10 (Samaritan Pharmaceuticals), and USL-311 (Upsher-Smith Laboratories).

Peptides are disclosed in the prior art which may be used as a CXCL12 antagonist due to their binding to CXCR4 in the method of the present invention. Such peptides include, but are not limited to, motixafortide (BioLineRx).

Synthetic peptides are disclosed in the prior art which may be used as a CXCL12 antagonist due to their binding to CXCR4 in the method of the present invention. Such synthetic peptides include, but are not limited to, balixafortide (Spexis), LY-2510924 (Eli Lilly), ALB-408 (Pharis Biotec) and POL-5551 (Spexis).

Recombinant proteins are disclosed in the prior art which may be used as a CXCL12 antagonist due to their binding to CXCR4 in the method of the present invention. Such recombinant proteins include, but are not limited to, PTX-9098 (Pertinax Therapeutics) and NNL-121 (Nanoligent).

Fusion proteins are disclosed in the prior art which may be used as a CXCL12 antagonist due to their binding to CXCR4 in the method of the present invention. Such recombinant fusion proteins include, but are not limited to, AD-214 (Adalta) and AM-3114 (Adalta).

Antibodies and more specifically monoclonal antibodies are disclosed in the prior art which may be used as a CXCL12 antagonist due to their binding to CXCR4 in the present invention as subject to each and any of aspect, including any embodiment thereof. Such antibodies include, but are not limited to, ulocuplumab (Bristol-Myers Squibb), hz-515H7 (Centre d'Immunologie Pierre Fabre), KY-1051 (Kymab), PF-06747143 (Pfizer), ALX-0651 (Ablynx), AT-009 (Affitech), LY-2624587 (Eli Lilly) and STIA-220X Sorrento Therapeutics).

In another embodiment of the present invention as subject to each and any of aspect, including any embodiment thereof, the CXCL12 antagonist is binding to CXCR7 and inhibits the binding of CXCL12 to CXCR7. In other words, CXCR7 is the target and antigen, respectively, which is bound by this embodiment of a CXCL12 antagonist. As in case of the CXCL12 antagonist binding to CXCL12 and inhibiting the binding of CXCL12 to CXCR4 and/or CXCR7, the CXCL12 antagonist binding to CXCR4 may be a compound selected from the group comprising the following classes of compounds: aptamers, spiegelmers, antibodies, target binding proteins, target binding peptides, synthetic peptides, peptides, anticalins, a small molecules and fusion proteins.

Various small molecules are disclosed in the prior art which may be used as a CXCL12 antagonist due to their binding to CXCR7 in the method of the present invention. Such small molecules include, but are not limited to, ACT-10041239 (Idorsia Pharmaceutical), CCX-650 (ChemoCentryx), CCX-662 (ChemoCentryx) and CCX-771 (ChemoCentryx).

Various synthetic peptides are disclosed in the prior art which may be used as a CXCL12 antagonist due to their binding to CXCR7 in the method of the present invention. Such small synthetic peptides include, but are not limited to, LIH-383 (Luxembourg Institute of Health) and POL-6926 (Spexis).

Various antibodies or antibody fragments are disclosed in the prior art which may be used as a CXCL12 antagonist due to their binding to CXCR7 in the method of the present invention. Such antibodies and antibody fragments include, but are not limited to, JT-07 (Jyant Technologies), X-7Ab (The Palo Alto Research Center) and STIA-230X (Serrento Therapeutics).

It will be appreciated by a person skilled in the art that the amount and dosage, respectively, of the CXCL12 antagonist to be administered to the subject will vary depending upon such factors as the patient's age, weight, height, sex, general medical condition and previous medical history.

It will be appreciated by a person skilled in the art that the exact dosage for administration to the subject in the practicing of the method of the invention can be determined by routine measures.

The way (route of administration and/or formulation) CXCL12 antagonist is administered is defined by the chemical nature of said anti-CXCL12 antagonist. For example, if the CXCL12 antagonist is a spiegelmer, aptamer or an antibody, it will be typically administered by intravenous administration.

Each of the above CXCL12 antagonists constitutes an embodiment of each and any aspect of the invention, including any embodiment thereof.

In an embodiment of each and any aspect of the invention, including any embodiment thereof, and as preferably used, an adjuvant therapy is an additional cancer treatment given after the primary treatment to lower the risk that the cancer will come back. In a preferred embodiment, adjuvant therapy may include chemotherapy, radiation therapy, hormone therapy, targeted therapy, immunotherapy or biological therapy.

In an embodiment of each and any aspect of the invention, including any embodiment thereof, and as preferably used, an adjunct therapy is another or additional treatment used together with a primary treatment. The purpose of adjunct therapy is to assist the primary treatment. In a preferred embodiment, adjunct therapy may include chemotherapy, radiation therapy, hormone therapy, targeted therapy, immunotherapy or biological therapy. It is acknowledged that the terms adjunct therapy and adjunctive therapy are used herein interchangeably and synonymously.

It is within the present invention that the CXCL12 antagonist is present in a composition, preferably a pharmaceutical composition, more preferably such pharmaceutical composition comprises a pharmaceutically acceptably excipient. It is also within the present invention that the interferon is present in a composition, preferably a pharmaceutical composition, more preferably such pharmaceutical composition comprises a pharmaceutically acceptably excipient. It will be appreciated that such composition is used in accordance with the present invention as preferably subject to the aspects of the invention, including any embodiment thereof. In an embodiment of each and any aspect of the invention, including any embodiment thereof, the composition comprising the CXCL12 antagonist is used as the CXCXL12 antagonist in the various aspects, including any embodiment thereof. In a further embodiment of each and any aspect of the invention, including any embodiment thereof, the composition comprising the interferon is used as the interferon in the various aspects, including any embodiment thereof.

If reference is made herein to a tumor in general without any further indication whether such tumor is an immunologically cold tumor, an immunologically hot tumor, a tumor refractory to an immunotherapy or a tumor non-refractory to an immunotherapy, such reference is preferably to any tumor including an immunologically cold tumor, an immunologically hot tumor, a tumor refractory to an immunotherapy or a tumor non-refractory to an immunotherapy unless indicated to the contrary.

It will be appreciated by a person skilled in the art that when reference is made to a distinct tumor entity in a subject, this translates in terms of disease from which the subject is suffering into the distinct cancer which is the same as the distinct tumor entity. In other words, if a person is suffering from, e.g., synovial sarcoma, the tumor in the person is also referred to as synovial sarcoma, and vice versa.

In an embodiment of each and any aspect of the invention, including any embodiment thereof, and as preferably used herein, a hot tumor differs from a cold tumor such that at least one of the characteristics of the cold tumor from which the hot tumor has emerged, is at least partially reversed; preferably such reversion results in a tumor which is clinically different from the cold tumor and allows administration of a therapy to which the cold tumor would have been refractory or not responsive.

In an embodiment of each and any aspect, including any embodiment thereof and as preferably used herein, the term treating a tumor in a subject is used interchangeably and synonymously to the term treating a subject suffering from a cancer, more preferably, the tumor and its characteristics defines the cancer and its characteristics. For example, treating a synovial sarcoma in a subject is interchangeably and synonymously to treating a subject suffering from synovial sarcoma; treating a pancreatic cancer in a subject is interchangeably and synonymously to treating a subject suffering from pancreatic cancer; and treating an osteosarcoma in a subject is interchangeably and synonymously to treating a subject suffering from osteosarcoma.

In an embodiment of each and any aspect, including any embodiment thereof and as preferably used herein, the term treating also comprises the term preventing and the term treatment also comprises the term prevention.

The terms "immune checkpoint inhibitor" and "checkpoint inhibitor" are interchangeable and synonymously used herein unless not explicitly indicated to the contrary.

Preferably, the terms immunomodulatorily active and immunomodulatory active are used interchangeably and synonymously herein.

In an embodiment of each and any aspect, including any embodiment thereof and as preferably used herein, the term non-refractory to immunotherapy synonymous to susceptible to immunotherapy.

In an embodiment of each and any aspect of the present invention, including any embodiment thereof, and as preferably used herein, the term immunomodulatory active cell refers to a cell that has, shows or displays an immunomodulatory activity preferably modulates the immune system of a host, preferably the immune system of a subject.

The terms CXCL12 and SDF-1 are interchangeably and synonymously used herein. When reference is made to the invention or the present invention, e.g. "in accordance with the present invention", such reference also and in particular refers to each and any aspect of the invention, including any embodiment thereof.

It is within the present invention that any reference to an aspect of the invention is understood as reference to the aspect including any embodiment thereof. Similarly, any reference to aspects of the invention is understood as reference to aspects of the invention including any embodiment of such aspects of the invention.

If reference is made to a range of figures such as from X to Y, this means that each figure and preferably any integer defined by such range including X and Y is disclosed herein. For example, if the range is from 1 to 4, this means that 1, 2, 3 and 4 are individually disclosed.

If reference is made herein to a range such as in "any of Embodiments X to Y", this means that backreference is made to each and any individual embodiment contained within the range defined by X and Y, including the embodiments numbered as X and Y, respectively. In accordance therewith, all of said embodiments are disclosed individually and back-referenced. For example, if back-reference is to any one of embodiments 1 to 4, such back-reference means any one of embodiment 1, embodiment 2, embodiment 3 and embodiment 4.

The various SEQ.ID. Nos., the chemical nature of the nucleic acid molecules according to the present invention and the target molecules SDF-1 as used herein, the actual sequence thereof and the internal reference number is summarized in the following table.

**TABLE 1 (A)**

| **Seq.-ID** | ***RNA*/*Peptide*** | **Sequence** | ***Internal Reference*** |
|---|---|---|---|
| 1 | L-peptide | KPVSLSYRCPCRFFESHVARANVKHLKILNTPNCALQIVARLKNN NRQVCIDPKLKWIQEYLEKALNK | human/monkey/cat SDF-1α |
| | | | human/monkey/cat SDF-1 |
| 2 | L-peptide | KPVSLSYRCPCRFFESHVARANVKHLKILNTPNCALQIVARLKNN NRQVCIDPKLKWIQEYLEKALNKRFKM | human/monkey/cat SDF-1β |
| 3 | L-peptide | KPVSLSYRCPCRFFESHIARANVKHLKILNTPNCALQIVARLKNN NRQVCIDPKLKWIQEYLEKALNK | murine SDF-1α |
| | | | murine SDF-1 |
| 4 | D-peptide | KPVSLSYRCPCRFFESHVARANVKHLKILNTPNCALQIVARLKNN NRQVCIDPKLKWIQEYLEKALNKRFK-Biotin | biotinylated hu D-SDF-1 |
| 5 | L-RNA (SPIEGELMER) | GCUGUGAAAGCAACAUGUCAAUGAAAGGUAGCCGCAGC | 192-A10-001 |
| 6 | L-RNA (SPIEGELMER) | GCUGUGAAAGUAACAUGUCAAUGAAAGGUAACCACAGC | 192-G10 |
| 7 | L-RNA (SPIEGELMER) | GCUGUGAAAGUAACACGUCAAUGAAAGGUAACCGCAGC | 192-F10 |
| 8 | L-RNA (SPIEGELMER) | GCUGUGAAAGUAACACGUCAAUGAAAGGUAACCACAGC | 192-B11 |
| 9 | L-RNA (SPIEGELMER) | GCUGUAAAAGUAACAUGUCAAUGAAAGGUAACUACAGC | 192-C9 |
| 10 | L-RNA (SPIEGELMER) | GCUGUAAAAGUAACAAGUCAAUGAAAGGUAACUACAGC | 192-E10 |
| 11 | L-RNA (SPIEGELMER) | GCUGUGAAAGUAACAAGUCAAUGAAAGGUAACCACAGC | 192-C10 |
| 12 | L-RNA (SPIEGELMER) | GCAGUGAAAGUAACAUGUCAAUGAAAGGUAACCACAGC | 192-D11 |
| 13 | L-RNA (SPIEGELMER) | GCUGUGAAAGUAACAUGUCAAUGAAAGGUAACCACUGC | 192-G11 |
| 14 | L-RNA (SPIEGELMER) | GCUAUGAAAGUAACAUGUCAAUGAAAGGUAACCAUAGC | 192-H11 |
| 15 | L-RNA (SPIEGELMER) | GCUGCGAAAGCGACAUGUCAAUGAAAGGUAGCCGCAGC | 192-D10 |
| 16 | L-RNA (SPIEGELMER) | GCUGUGAAAGCAACAUGUCAAUGAAAGGUAGCCACAGC | 192-E9 |
| 17 | L-RNA (SPIEGELMER) | GCUGUGAAAGUAACAUGUCAAUGAAAGGUAGCCGCAGC | 192-H9 |

**TABLE 1 (B)**

| **Seq.-ID** | ***RNA*/*Peptide*** | **Sequence** | ***Internal Reference*** |
|---|---|---|---|
| 18 | L-RNA (SPIEGELMER) | AGCGUGAAAGUAACACGUAAAAUGAAAGGUAACCACGCU | 191-A6 |
| 19 | L-RNA (SPIEGELMER) | AAAGYRACAHGUMAAX_{A}UGAAAGGUARC; X_{A} = A or absent | Type A Formula-1 |
| 20 | L-RNA (SPIEGELMER) | AAAGYRACAHGUMAAUGAAAGGUARC | Type A Formula-2 |
| 21 | L-RNA (SPIEGELMER) | AAAGYRACAHGUMAAAUGAAAGGUARC | Type A Formula-3 |
| 22 | L-RNA (SPIEGELMER) | AAAGYAACAHGUCAAUGAAAGGUARC | Type A Formula-4 |
| 23 | L-RNA (SPIEGELMER | RSHRYR | Type A Formula-5-5' |
| 24 | L-RNA (SPIEGELMER | YRYDSY | Type A Formula-5-3' |
| 25 | L-RNA (SPIEGELMER) | CUGUGAAAGCAACAUGUCAAUGAAAGGUAGCCGCAG | 192-A10-002 |
| 26 | L-RNA (SPIEGELMER) | UGUGAAAGCAACAUGUCAAUGAAAGGUAGCCGCA | 192-A10-003 |
| 27 | L-RNA (SPIEGELMER) | GUGAAAGCAACAUGUCAAUGAAAGGUAGCCGC | 192-A10-004 |
| 28 | L-RNA (SPIEGELMER) | UGAAAGCAACAUGUCAAUGAAAGGUAGCCG | 192-A10-005 |
| 29 | L-RNA (SPIEGELMER) | GAAAGCAACAUGUCAAUGAAAGGUAGCC | 192-A10-006 |
| 30 | L-RNA (SPIEGELMER) | AAAGCAACAUGUCAAUGAAAGGUAGC | 192-A10-007 |
| 31 | L-RNA (SPIEGELMER) | GCGUGAAAGCAACAUGUCAAUGAAAGGUAGCCGCGC | 192-A10-008 |
| 32 | L-RNA (SPIEGELMER) | GCGCGAAAGCAACAUGUCAAUGAAAGGUAGCCGCGC | 192-A10-015 |
| 33 | L-RNA (SPIEGELMER) | GCGGAAAGCAACAUGUCAAUGAAAGGUAGCCCGC | 192-A10-014 |
| 34 | L-RNA (SPIEGELMER) | CGUGAAAGCAACAUGUCAAUGAAAGGUAGCCGCG | 192-A10-016 |
| 35 | L-RNA (SPIEGELMER) | GCGCAAAGCAACAUGUCAAUGAAAGGUAGCGUGC | 192-A10-017 |
| 36 | L-RNA (SPIEGELMER) | GUGCAAAGCAACAUGUCAAUGAAAGGUAGCGCGC | 192-A10-018 |

**TABLE 1 (C)**

| **Seq.-ID** | **RNA/Peptide** | **Sequence** | **Internal Reference** |
|---|---|---|---|
| 37 | L-RNA (SPIEGELMER) | CGCGAAAGCAACAUGUCAAUGAAAGGUAGCCGUG | 192-A10-019 |
| 38 | L-RNA (SPIEGELMER) | GGGCAAAGCAACAUGUCAAUGAAAGGUAGCGCCC | 192-A10-020 |
| 39 | L-RNA (SPIEGELMER) | GGCCAAAGCAACAUGUCAAUGAAAGGUAGCGGCC | 192-A10-021 |
| 40 | L-RNA (SPIEGELMER) | GCCCAAAGCAACAUGUCAAUGAAAGGUAGCGGGC | 192-A10-022 |
| 41 | L-RNA (SPIEGELMER) | CCCCAAAGCAACAUGUCAAUGAAAGGUAGCGGGG | 192-A10-023 |
| 42 | L-RNA (SPIEGELMER) | X₂BBBS; X2 = S or absent | Type A Formula-6-5' |
| 43 | L-RNA (SPIEGELMER) | SBBVX₃; X₃ = S or absent | Type A Formula-6-3' |
| 44 | L-RNA (SPIEGELMER) | X₁X₂NNBV; X₁ = R or absent, X₂ = S or absent | Type A Formula-7-5' |
| 45 | L-RNA (SPIEGELMER) | BNBNX₃X₄; X₃ = R or absent, X₄ = Y or absent | Type A Formula-7-3' |
| 46 | L-RNA (SPIEGELMER) | AGCGUGGUGUGAUCUAGAUGUAGUGGCUGAUCCUAGUCAGGUACGCU | 193-C2-001 |
| 47 | L-RNA (SPIEGELMER) | AGCGUGGUGUGAUCUAGAUGUAUUGGCUGAUCCUAGUCAGGUACGCU | 193-G2-001 |
| 48 | L-RNA (SPIEGELMER) | AGCGUGGUGUGAUCUAGAUGUAAUGGCUGAUCCUAGUCAGGUGCGCU | 193-F2-001 |
| 49 | L-RNA (SPIEGELMER) | GCGAGGUGUGAUCUAGAUGUAGUGGCUGAUCCUAGUCAGGUGCGC | 193-G1-002 |
| 50 | L-RNA (SPIEGELMER) | GCGUGGUGUGAUCUAGAUGUAGUGGCUGAUCCUAGUCAGGUGCGC | 193-D2-002 |
| 51 | L-RNA (SPIEGELMER) | GCAUGGUGUGAUCUAGAUGUAGUGGCUGAUCCUAGUCAGGUGCCC | 193-A1-002 |
| 52 | L-RNA (SPIEGELMER) | GCGUGGUGUGAUCUAGAUGUAAUGGCUGAUCCUAGUCAGGGACGC | 193-D3-002 |

**TABLE 1 (D)**

| **Seq.-ID** | **RNA/Peptide** | **Sequence** | **Internal Reference** |
|---|---|---|---|
| 53 | L-RNA (SPIEGELMER) | GCGUGGUGUGAUCUAGAUGUAGAGGCUGAUCCUAGUCAGGUACGC | 193-B3-002 |
| 54 | L-RNA (SPIEGELMER) | GCGUGGUGUGAUCUAGAUGUAAAGGCUGAUCCUAGUCAGGUACGC | 193-H3-002 |
| 55 | L-RNA (SPIEGELMER) | GCGUGGUGUGAUCUAGAUGUAGUGGCUGUUCCUAGUCAGGUAUGC | 193-E3-002 |
| 56 | L-RNA (SPIEGELMER) | GCGUGGUGUGAUCUAGAUGUAGUGGCUGAUCCUAGUUAGGUACGC | 193-D1-002 |
| 57 | L-RNA (SPIEGELMER) | GUGUGAUCUAGAUGUADWGGCUGWUCCUAGUYAGG | Type B Formula-1 |
| 58 | L-RNA (SPIEGELMER) | GUGUGAUCUAGAUGUADUGGCUGAUCCUAGUCAGG | Type B Formula-2 |
| 59 | L-RNA (SPIEGELMER) | X₁GCRWG; X₁ = A or absent | Type B Formula-3-5' |
| 60 | L-RNA (SPIEGELMER) | KRYSCX₄; X₄ = U or absent | Type B Formula-3-3' |
| 61 | L-RNA (SPIEGELMER) | GCGUGGUGUGAUCUAGAUGUAGUGGCUGAUCCUAGUCAGGUACGC | 193-C2-002 |
| 62 | L-RNA (SPIEGELMER) | CGUGGUGUGAUCUAGAUGUAGUGGCUGAUCCUAGUCAGGUACG | 193-C2-003 |
| 63 | L-RNA (SPIEGELMER) | GUGGUGUGAUCUAGAUGUAGUGGCUGAUCCUAGUCAGGUAC | 193-C2-004 |
| 64 | L-RNA (SPIEGELMER) | UGGUGUGAUCUAGAUGUAGUGGCUGAUCCUAGUCAGGUA | 193-C2-005 |
| 65 | L-RNA (SPIEGELMER) | GGUGUGAUCUAGAUGUAGUGGCUGAUCCUAGUCAGGU | 193-C2-006 |
| 66 | L-RNA (SPIEGELMER) | GUGUGAUCUAGAUGUAGUGGCUGAUCCUAGUCAGG | 193-C2-007 |
| 67 | L-RNA (SPIEGELMER) | GCGUGGUGUGAUCUAGAUGUAUUGGCUGAUCCUAGUCAGG UACGC | 193-G2-012 |

**TABLE 1 (E)**

| **Seq.-ID** | **RNA/Peptide** | **Sequence** | **Internal Reference** |
|---|---|---|---|
| 68 | L-RNA (SPIEGELMER) | GCGCGGUGUGAUCUAGAUGUAUUGGCUGAUCCUAGUCAGGCGCGC | 193-G2-013 |
| 69 | L-RNA (SPIEGELMER) | GCGCGUGUGAUCUAGAUGUAUUGGCUGAUCCUAGUCAGGGCGC | 193-G2-014 |
| 70 | L-RNA (SPIEGELMER) | GGGCGUGUGAUCUAGAUGUAUUGGCUGAUCCUAGUCAGGGCCC | 193-G2-015 |
| 71 | L-RNA (SPIEGELMER) | GGCCGUGUGAUCUAGAUGUAUUGGCUGAUCCUAGUCAGGGGCC | 193-G2-016 |
| 72 | L-RNA (SPIEGELMER) | GCCCGUGUGAUCUAGAUGUAUUGGCUGAUCCUAGUCAGGGGGC | 193-G2-017 |
| 73 | L-RNA (SPIEGELMER) | X₂SSBS; X₂ = G or absent | Type B Formula-4-5' |
| 74 | L-RNA (SPIEGELMER) | BVSSX₃; X₃ = C or absent | Type B Formula-4-3' |
| 75 | L-RNA (SPIEGELMER) | X₁GCGUG; X₁ = A or absent | Type B Formula-5-5' |
| 76 | L-RNA (SPIEGELMER) | UACGCX₄; X₄ = U or absent | Type B Formula-5-3' |
| 77 | L-RNA (SPIEGELMER) | X₁X₂SVNS; X₁ = A or absent, X₂ = G or absent | Type B Formula-6-5' |
| 78 | L-RNA (SPIEGELMER) | BVBSX₃X₄; X₃ = C or absent, X₄ = U or absent | Type B Formula-6-3' |
| 79 | L-RNA (SPIEGELMER) | GUGCUGCGGGGGUUAGGGCUAGAAGUCGGCCUGCAGCAC | 197-B2 |
| 80 | L-RNA (SPIEGELMER) | AGCGUGGCGAGGUUAGGGCUAGAAGUCGGUCGACACGCU | 191-D5-001 |
| 81 | L-RNA (SPIEGELMER) | GUGUUGCGGAGGUUAGGGCUAGAAGUCGGUCAGCAGCAC | 197-H1 |
| 82 | L-RNA (SPIEGELMER) | CGUGCGCUUGAGAUAGGGGUUAGGGCUUAAAGUCGGCUGAUUCUCACG | 190-A3-001 |
| 83 | L-RNA (SPIEGELMER) | AGCGUGAAGGGGUUAGGGCUCGAAGUCGGCUGACACGCU | 191-A5 |

**TABLE 1 (F)**

| **Seq.-ID** | ***RNA*/*Peptide*** | **Sequence** | ***Internal Reference*** |
|---|---|---|---|
| 84 | L-RNA (SPIEGELMER) | GUGCUGCGGGGGUUAGGGCUCGAAGUCGGCCCGCAGCAC | 197-H3 |
| 85 | L-RNA (SPIEGELMER) | GUGUUCCCGGGGUUAGGGCUUGAAGUCGGCCGGCAGCAC | 197-B1 |
| 86 | L-RNA (SPIEGELMER) | GUGUUGCAGGGGUUAGGGCUUGAAGUCGGCCUGCAGCAC | 197-E3 |
| 87 | L-RNA (SPIEGELMER) | GUGCUGCGGGGGUUAGGGCUCAAAGUCGGCCUGCAGCAC | 197-H2 |
| 88 | L-RNA (SPIEGELMER) | GUGCUGCCGGGGUUAGGGCUAA-AGUCGGCCGACAGCAC | 197-D1 |
| 89 | L-RNA (SPIEGELMER) | GUGCUGUGGGGGUCAGGGCUAGAAGUCGGCCUGCAGCAC | 197-D2 |
| 90 | L-RNA (SPIEGELMER) | GGUYAGGGCUHRX_{A}AGUCGG; X_{A} = A or absent | Type C Formula-1 |
| 91 | L-RNA (SPIEGELMER) | GGUYAGGGCUHRAAGUCGG | Type C Formula-2 |
| 92 | L-RNA (SPIEGELMER) | GGUYAGGGCUHRAGUCGG | Type C Formula-3 |
| 93 | L-RNA (SPIEGELMER) | GGUUAGGGCUHGAAGUCGG | Type C Formula-4 |
| 94 | L-RNA (SPIEGELMER) | UGAGAUAGGGGUUAGGGCUUAAAGUCGGCUGAUUCUCA | 190-A3-003 |
| 95 | L-RNA (SPIEGELMER) | GAGAUAGGGGUUAGGGCUUAAAGUCGGCUGAUUCUC | 190-A3-004 |
| 96 | L-RNA (SPIEGELMER) | GGGGUUAGGGCUUAAAGUCGGCUGAUUCU | 190-A3-007 |
| 97 | L-RNA (SPIEGELMER) | GCGUGGCGAGGUUAGGGCUAGAAGUCGGUCGACACGC | 191-D5-002 |
| 98 | L-RNA (SPIEGELMER) | CGUGGCGAGGUUAGGGCUAGAAGUCGGUCGACACG | 191-D5-003 |
| 99 | L-RNA (SPIEGELMER) | CGGGCGAGGUUAGGGCUAGAAGUCGGUCGACCG | 191-D5-004 |
| 100 | L-RNA (SPIEGELMER) | CGGGCGAGGUUAGGGCUAGAAGUCGGUCGCCCG | 191-D5-005 |

**TABLE 1 (G)**

| **Seq.-ID** | ***RNA*/*Peptide*** | **Sequence** | ***Internal Reference*** |
|---|---|---|---|
| 101 | L-RNA (SPIEGELMER) | CGGCGAGGUUAGGGCUAGAAGUCGGUCGCCG | 191-D5-006 |
| 102 | L-RNA (SPIEGELMER) | CGGGAGGUUAGGGCUAGAAGUCGGUCCCG | 191-D5-007 |
| 103 | L-RNA (SPIEGELMER) | GGGAGGUUAGGGCUAGAAGUCGGUCCC | 191-D5-010 |
| 104 | L-RNA (SPIEGELMER) | CCGCGGUUAGGGCUAGAAGUCGGGCGG | 191-D5-017 |
| 105 | L-RNA (SPIEGELMER) | CCCGGGUUAGGGCUAGAAGUCGGCGGG | 191-D5-029 |
| 106 | L-RNA (SPIEGELMER) | GGCGGGUUAGGGCUAGAAGUCGGCGCC | 191-D5-024 |
| 107 | L-RNA (SPIEGELMER) | CCCGCGGUUAGGGCUAGAAGUCGGGCGGG | 191-D5-017-29a |
| 108 | L-RNA (SPIEGELMER) | GCCGCGGUUAGGGCUAGAAGUCGGGCGGC | 191-D5-017-29b |
| 109 | L-RNA (SPIEGELMER) | CCCCGGGUUAGGGCUAGAAGUCGGCGGGG | 191-D5-019-29a |
| 110 | L-RNA (SPIEGELMER) | CGGCGGGUUAGGGCUAGAAGUCGGCGCCG | 191-D5-024-29a |
| 111 | L-RNA (SPIEGELMER) | GGGCGGGUUAGGGCUAGAAGUCGGCGCCC | 191-D5-024-29b |
| 112 | L-RNA (SPIEGELMER) | UGCUGCGGGGGUUAGGGCUAGAAGUCGGCCUGCAGCA | 197-B2-001 |
| 113 | L-RNA (SPIEGELMER) | GCUGCGGGGGUUAGGGCUAGAAGUCGGCCUGCAGC | 197-B2-002 |
| 114 | L-RNA (SPIEGELMER) | CUGCGGGGGUUAGGGCUAGAAGUCGGCCUGCAG | 197-B2-003 |
| 115 | L-RNA (SPIEGELMER) | UGCGGGGGUUAGGGCUAGAAGUCGGCCUGCA | 197-B2-004 |
| 116 | L-RNA (SPIEGELMER) | GCGGGGGUUAGGGCUAGAAGUCGGCCUGC | 197-B2-005 |

**TABLE 1 (H)**

| **Seq.-ID** | ***RNA*/*Peptide*** | **Sequence** | ***Internal Reference*** |
|---|---|---|---|
| 117 | L-RNA (SPIEGELMER) | GCCGGGGUUAGGGCUAGAAGUCGGCCGGC | 197-B2-006 |
| 118 | L-RNA (SPIEGELMER) | GGCCGGGGUUAGGGCUAGAAGUCGGCCGGCC | 197-B2-006-31a |
| 119 | L-RNA (SPIEGELMER) | CGCCGGGGUUAGGGCUAGAAGUCGGCCGGCG | 197-B2-006-31b |
| 120 | L-RNA (SPIEGELMER) | RKSBUSNVGR | Type C Formula-5-5' |
| 121 | L-RNA (SPIEGELMER) | YYNRCASSMY | Type C Formula-5-3' |
| 122 | L-RNA (SPIEGELMER) | RKSBUGSVGR | Type C Formula-6-5' |
| 123 | L-RNA (SPIEGELMER) | YCNRCASSMY | Type C Formula-6-3' |
| 124 | L-RNA (SPIEGELMER) | X_{S}SSSV; Xₛ = S or absent | Type C Formula-7-5' |
| 125 | L-RNA (SPIEGELMER) | BSSSX_{S}; Xₛ = S or absent | Type C Formula-7-3' |
| 126 | L-RNA (SPIEGELMER) | SGGSV | Type C Formula-8-5' |
| 127 | L-RNA (SPIEGELMER) | YSCCS | Type C Formula-8-3' |
| 128 | L-RNA (SPIEGELMER) | GCSGG | Type C Formula-9-5' |
| 129 | L-RNA (SPIEGELMER) | CCKGC | Type C Formula-9-3' |
| 130 | L-RNA (SPIEGELMER) | SSSSR | Type C Formula-10-5' |
| 131 | L-RNA (SPIEGELMER) | YSBSS | Type C Formula-10-3' |

**TABLE 1 (I)**

| **Seq.-ID** | ***RNA*/*Peptide*** | **Sequence** | ***Internal Reference*** |
|---|---|---|---|
| 132 | L-RNA (SPIEGELMER) | 5′-40 kDa-PEG- GCGUGGUGUGAUCUAGAUGUAUUGGCUGAUCCUAGUCAGGUACGC | 193-G2-012-5'-PEG (NOX-A12) |
| 133 | L-RNA (SPIEGELMER) | 5'-40 kDa-PEG-GCGUGAAAGCAACAUGUCAAUGAAAGGUAGCCGCGC | 192-A10-008-5'-PEG |
| 134 | L-RNA (SPIEGELMER) | 5'-40 kDa-PEG-CGGGAGGUUAGGGCUAGAAGUCGGUCCCG | 191-D5-007-5'-PEG |
| 135 | L-RNA (SPIEGELMER) | 5'-40 kDa-PEG-GCCGGGGUUAGGGCUAGAAGUCGGCCGGC | 197-B2-006-5'-PEG |
| 136 | L-RNA (SPIEGELMER) | 5'-40 kDa-PEG-CGCCGGGGUUAGGGCUAGAAGUCGGCCGGCG | 197-B2-006-31b-5'PEG |
| 137 | L-RNA (SPIEGELMER) | 5′-40 kDa-PEG- GCUGUGAAAGCAACAUGUCAAUGAAAGGUAGCCGCAGC | 192-A10-001-5'-PEG 192-A10-001-5'-PEG40 |
| 138 | L-RNA (SPIEGELMER) | UAAGGAAACUCGGUCUGAUGCGGUAGCGCUGUGCAGAGCU | Control Spiegelmer |
| 139 | L-RNA (SPIEGELMER) | 5′-30 kDa-PEG- GCUGUGAAAGCAACAUGUCAAUGAAAGGUAGCCGCAGC | 192-A10-001-5'-PEG30 |
| 140 | L-RNA (SPIEGELMER) | 5′-100 kDa-HES- GCUGUGAAAGCAACAUGUCAAUGAAAGGUAGCCGCAGC | 192-A10-001-5'-HES100 |
| 141 | L-RNA (SPIEGELMER) | 5′-130 kDa-HES- GCUGUGAAAGCAACAUGUCAAUGAAAGGUAGCCGCAGC | 192-A10-001-5'-HES130 |
| 142 | L-RNA (SPIEGELMER) | CGUGGUCCGUUGUGUCAGGUCUAUUCGCCCCGGUGCAGGGCAUCCGCG | 194-A2-001 |
| 143 | L-RNA (SPIEGELMER) | GCAGUGUGACGCGGACGUGAUAGGACAGAGCUGAUCCCGCUCAGGUGAG | 196-B12-003 |
| 144 | L-RNA (SPIEGELMER) | CAACAGCAGUGUGACGCGGACGUGAUAGGACAGAGCUGAUCCCGCUCAG | 196-B12-004 |

**TABLE 1 (J)**

| **Seq.-ID** | ***RNA*/*Peptide*** | **Sequence** | ***Internal Reference*** |
|---|---|---|---|
| 145 | ***D-RNA (APTAMER)*** | GCUGUGAAAGCAACAUGUCAAUGAAAGGUAGCCGCAGC | ***192-A10-001*** |
| 146 | D-RNA (APTAMER) | GCUGUGAAAGUAACAUGUCAAUGAAAGGUAACCACAGC | 192-G10 |
| 147 | D-RNA (APTAMER) | GCUGUGAAAGUAACACGUCAAUGAAAGGUAACCGCAGC | 192-F10 |
| 148 | D-RNA (APTAMER) | GCUGUGAAAGUAACACGUCAAUGAAAGGUAACCACAGC | 192-B11 |
| 149 | D-RNA (APTAMER) | GCUGUAAAAGUAACAUGUCAAUGAAAGGUAACUACAGC | 192-C9 |
| 150 | D-RNA (APTAMER) | GCUGUAAAAGUAACAAGUCAAUGAAAGGUAACUACAGC | 192-E10 |
| 151 | D-RNA (APTAMER) | GCUGUGAAAGUAACAAGUCAAUGAAAGGUAACCACAGC | 192-C10 |
| 152 | D-RNA (APTAMER) | GCAGUGAAAGUAACAUGUCAAUGAAAGGUAACCACAGC | 192-D11 |
| 153 | D-RNA (APTAMER) | GCUGUGAAAGUAACAUGUCAAUGAAAGGUAACCACUGC | 192-G11 |
| 154 | D-RNA (APTAMER) | GCUAUGAAAGUAACAUGUCAAUGAAAGGUAACCAUAGC | 192-H11 |
| 155 | D-RNA (APTAMER) | GCUGCGAAAGCGACAUGUCAAUGAAAGGUAGCCGCAGC | 192-D10 |
| 156 | D-RNA (APTAMER) | GCUGUGAAAGCAACAUGUCAAUGAAAGGUAGCCACAGC | 192-E9 |
| 157 | D-RNA (APTAMER) | GCUGUGAAAGUAACAUGUCAAUGAAAGGUAGCCGCAGC | 192-H9 |
| 158 | D-RNA (APTAMER) | AGCGUGAAAGUAACACGUAAAAUGAAAGGUAACCACGCU | 191-A6 |
| 159 | D-RNA (APTAMER) | CUGUGAAAGCAACAUGUCAAUGAAAGGUAGCCGCAG | 192-A10-002 |
| 160 | D-RNA (APTAMER) | UGUGAAAGCAACAUGUCAAUGAAAGGUAGCCGCA | 192-A10-003 |

**TABLE 1 (K)**

| **Seq.-ID** | ***RNA*/*Peptide*** | **Sequence** | ***Internal Reference*** |
|---|---|---|---|
| 161 | D-RNA (APTAMER) | GUGAAAGCAACAUGUCAAUGAAAGGUAGCCGC | 192-A10-004 |
| 162 | D-RNA (APTAMER) | UGAAAGCAACAUGUCAAUGAAAGGUAGCCG | 192-A10-005 |
| 163 | D-RNA (APTAMER) | GAAAGCAACAUGUCAAUGAAAGGUAGCC | 192-A10-006 |
| 164 | D-RNA (APTAMER) | AAAGCAACAUGUCAAUGAAAGGUAGC | 192-A10-007 |
| 165 | D-RNA (APTAMER) | GCGUGAAAGCAACAUGUCAAUGAAAGGUAGCCGCGC | 192-A10-008 |
| 166 | D-RNA (APTAMER) | GCGCGAAAGCAACAUGUCAAUGAAAGGUAGCCGCGC | 192-A10-015 |
| 167 | D-RNA (APTAMER) | GCGGAAAGCAACAUGUCAAUGAAAGGUAGCCCGC | 192-A10-014 |
| 168 | ***D-RNA (APTAMER)*** | CGUGAAAGCAACAUGUCAAUGAAAGGUAGCCGCG | 192-A10-016 |
| 169 | D-RNA (APTAMER) | GCGCAAAGCAACAUGUCAAUGAAAGGUAGCGUGC | 192-A10-017 |
| 170 | D-RNA (APTAMER) | GUGCAAAGCAACAUGUCAAUGAAAGGUAGCGCGC | 192-A10-018 |
| 171 | D-RNA (APTAMER) | CGCGAAAGCAACAUGUCAAUGAAAGGUAGCCGUG | 192-A10-019 |
| 172 | D-RNA (APTAMER) | GGGCAAAGCAACAUGUCAAUGAAAGGUAGCGCCC | 192-A10-020 |
| 173 | D-RNA (APTAMER) | GGCCAAAGCAACAUGUCAAUGAAAGGUAGCGGCC | 192-A10-021 |
| 174 | D-RNA (APTAMER) | GCCCAAAGCAACAUGUCAAUGAAAGGUAGCGGGC | 192-A10-022 |
| 175 | D-RNA (APTAMER) | CCCCAAAGCAACAUGUCAAUGAAAGGUAGCGGGG | 192-A10-023 |
| 176 | D-RNA (APTAMER) | AGCGUGGUGUGAUCUAGAUGUAGUGGCUGAUCCUAGUCAGGUACGCU | 193-C2-001 |

**TABLE 1 (L)**

| **Seq.-ID** | ***RNA*/*Peptide*** | **Sequence** | ***Internal Reference*** |
|---|---|---|---|
| 177 | D-RNA (APTAMER) | AGCGUGGUGUGAUCUAGAUGUAUUGGCUGAUCCUAGUCAGGUACGCU | 193-G2-001 |
| 178 | D-RNA (APTAMER) | AGCGUGGUGUGAUCUAGAUGUAAUGGCUGAUCCUAGUCAGGUGCGCU | 193-F2-001 |
| 179 | D-RNA (APTAMER) | GCGAGGUGUGAUCUAGAUGUAGUGGCUGAUCCUAGUCAGGUGCGC | 193-G1-002 |
| 180 | D-RNA (APTAMER) | GCGUGGUGUGAUCUAGAUGUAGUGGCUGAUCCUAGUCAGGUGCGC | 193-D2-002 |
| 181 | D-RNA (APTAMER) | GCAUGGUGUGAUCUAGAUGUAGUGGCUGAUCCUAGUCAGGUGCCC | 193-A1-002 |
| 182 | D-RNA (APTAMER) | GCGUGGUGUGAUCUAGAUGUAAUGGCUGAUCCUAGUCAGGGACGC | 193-D3-002 |
| 183 | D-RNA (APTAMER) | GCGUGGUGUGAUCUAGAUGUAGAGGCUGAUCCUAGUCAGGUACGC | 193-B3-002 |
| 184 | D-RNA (APTAMER) | GCGUGGUGUGAUCUAGAUGUAAAGGCUGAUCCUAGUCAGGUACGC | 193-H3-002 |
| 185 | D-RNA (APTAMER) | GCGUGGUGUGAUCUAGAUGUAGUGGCUGUUCCUAGUCAGGUAUGC | 193-E3-002 |
| 186 | D-RNA (APTAMER) | GCGUGGUGUGAUCUAGAUGUAGUGGCUGAUCCUAGUUAGGUACGC | 193-D1-002 |
| 187 | D-RNA (APTAMER) | GCGUGGUGUGAUCUAGAUGUAGUGGCUGAUCCUAGUCAGGUACGC | 193-C2-002 |
| 188 | D-RNA (APTAMER) | CGUGGUGUGAUCUAGAUGUAGUGGCUGAUCCUAGUCAGGUACG | 193-C2-003 |
| 189 | D-RNA (APTAMER) | GUGGUGUGAUCUAGAUGUAGUGGCUGAUCCUAGUCAGGUAC | 193-C2-004 |
| 190 | D-RNA (APTAMER) | UGGUGUGAUCUAGAUGUAGUGGCUGAUCCUAGUCAGGUA | 193-C2-005 |
| 191 | D-RNA (APTAMER) | GGUGUGAUCUAGAUGUAGUGGCUGAUCCUAGUCAGGU | 193-C2-006 |

**TABLE 1 (M)**

| **Seq.-ID** | ***RNA*/*Peptide*** | **Sequence** | ***Internal Reference*** |
|---|---|---|---|
| 192 | D-RNA (APTAMER) | GUGUGAUCUAGAUGUAGUGGCUGAUCCUAGUCAGG | 193-C2-007 |
| 193 | ***D-RNA (APTAMER)*** | GCGUGGUGUGAUCUAGAUGUAUUGGCUGAUCCUAGUCAGGUACGC | 193-G2-012 |
| 194 | D-RNA (APTAMER) | GCGCGGUGUGAUCUAGAUGUAUUGGCUGAUCCUAGUCAGGCGCGC | 193-G2-013 |
| 195 | D-RNA (APTAMER) | GCGCGUGUGAUCUAGAUGUAUUGGCUGAUCCUAGUCAGGGCGC | 193-G2-014 |
| 196 | D-RNA (APTAMER) | GGGCGUGUGAUCUAGAUGUAUUGGCUGAUCCUAGUCAGGGCCC | 193-G2-015 |
| 197 | D-RNA (APTAMER) | GGCCGUGUGAUCUAGAUGUAUUGGCUGAUCCUAGUCAGGGGCC | 193-G2-016 |
| 198 | D-RNA (APTAMER) | GCCCGUGUGAUCUAGAUGUAUUGGCUGAUCCUAGUCAGGGGGC | 193-G2-017 |
| 199 | D-RNA (APTAMER) | GUGCUGCGGGGGUUAGGGCUAGAAGUCGGCCUGCAGCAC | 197-B2 |
| 200 | D-RNA (APTAMER) | AGCGUGGCGAGGUUAGGGCUAGAAGUCGGUCGACACGCU | 191-D5-001 |
| 201 | D-RNA (APTAMER) | GUGUUGCGGAGGUUAGGGCUAGAAGUCGGUCAGCAGCAC | 197-H1 |
| 202 | D-RNA (APTAMER) | CGUGCGCUUGAGAUAGGGGUUAGGGCUUAAAGUCGGCUGAUUCUCACG | 190-A3-001 |
| 203 | D-RNA (APTAMER) | AGCGUGAAGGGGUUAGGGCUCGAAGUCGGCUGACACGCU | 191-A5 |
| 204 | D-RNA (APTAMER) | GUGCUGCGGGGGUUAGGGCUCGAAGUCGGCCCGCAGCAC | 197-H3 |
| 205 | D-RNA (APTAMER) | GUGUUCCCGGGGUUAGGGCUUGAAGUCGGCCGGCAGCAC | 197-B1 |

**TABLE 1 (N)**

| **Seq.-ID** | ***RNA*/*Peptide*** | **Sequence** | ***Internal Reference*** |
|---|---|---|---|
| 206 | D-RNA (APTAMER) | GUGUUGCAGGGGUUAGGGCUUGAAGUCGGCCUGCAGCAC | 197-E3 |
| 207 | D-RNA (APTAMER) | GUGCUGCGGGGGUUAGGGCUCAAAGUCGGCCUGCAGCAC | 197-H2 |
| 208 | D-RNA (APTAMER) | GUGCUGCCGGGGUUAGGGCUAA-AGUCGGCCGACAGCAC | 197-D1 |
| 209 | D-RNA (APTAMER) | GUGCUGUGGGGGUCAGGGCUAGAAGUCGGCCUGCAGCAC | 197-D2 |
| 210 | D-RNA (APTAMER) | UGAGAUAGGGGUUAGGGCUUAAAGUCGGCUGAUUCUCA | 190-A3-003 |
| 211 | D-RNA (APTAMER) | GAGAUAGGGGUUAGGGCUUAAAGUCGGCUGAUUCUC | 190-A3-004 |
| 212 | D-RNA (APTAMER) | GGGGUUAGGGCUUAAAGUCGGCUGAUUCU | 190-A3-007 |
| 213 | D-RNA (APTAMER) | GCGUGGCGAGGUUAGGGCUAGAAGUCGGUCGACACGC | 191-D5-002 |
| 214 | D-RNA (APTAMER) | CGUGGCGAGGUUAGGGCUAGAAGUCGGUCGACACG | 191-D5-003 |
| 215 | D-RNA (APTAMER) | CGGGCGAGGUUAGGGCUAGAAGUCGGUCGACCG | 191-D5-004 |
| 216 | D-RNA (APTAMER) | CGGGCGAGGUUAGGGCUAGAAGUCGGUCGCCCG | 191-D5-005 |
| 217 | D-RNA (APTAMER) | CGGCGAGGUUAGGGCUAGAAGUCGGUCGCCG | 191-D5-006 |
| 218 | D-RNA (APTAMER) | CGGGAGGUUAGGGCUAGAAGUCGGUCCCG | 191-D5-007 |
| 219 | D-RNA (APTAMER) | GGGAGGUUAGGGCUAGAAGUCGGUCCC | 191-D5-010 |
| 220 | D-RNA (APTAMER) | CCGCGGUUAGGGCUAGAAGUCGGGCGG | 191-D5-017 |
| 221 | D-RNA (APTAMER) | CCCGGGUUAGGGCUAGAAGUCGGCGGG | 191-D5-029 |
| 222 | D-RNA (APTAMER) | GGCGGGUUAGGGCUAGAAGUCGGCGCC | 191-D5-024 |
| 223 | D-RNA (APTAMER) | CCCGCGGUUAGGGCUAGAAGUCGGGCGGG | 191-D5-017-29a |

**TABLE 1 (O)**

| **Seq.-ID** | ***RNA*/*Peptide*** | **Sequence** | ***Internal Reference*** |
|---|---|---|---|
| 224 | D-RNA (APTAMER) | GCCGCGGUUAGGGCUAGAAGUCGGGCGGC | 191-D5-017-29b |
| 225 | D-RNA (APTAMER) | CCCCGGGUUAGGGCUAGAAGUCGGCGGGG | 191-D5-019-29a |
| 226 | D-RNA (APTAMER) | CGGCGGGUUAGGGCUAGAAGUCGGCGCCG | 191-D5-024-29a |
| 227 | D-RNA (APTAMER) | GGGCGGGUUAGGGCUAGAAGUCGGCGCCC | 191-D5-024-29b |
| 228 | D-RNA (APTAMER) | UGCUGCGGGGGUUAGGGCUAGAAGUCGGCCUGCAGCA | 197-B2-001 |
| 229 | D-RNA (APTAMER) | GCUGCGGGGGUUAGGGCUAGAAGUCGGCCUGCAGC | 197-B2-002 |
| 230 | D-RNA (APTAMER) | CUGCGGGGGUUAGGGCUAGAAGUCGGCCUGCAG | 197-B2-003 |
| 231 | D-RNA (APTAMER) | UGCGGGGGUUAGGGCUAGAAGUCGGCCUGCA | 197-B2-004 |
| 232 | D-RNA (APTAMER) | GCGGGGGUUAGGGCUAGAAGUCGGCCUGC | 197-B2-005 |
| 233 | D-RNA (APTAMER) | GCCGGGGUUAGGGCUAGAAGUCGGCCGGC | 197-B2-006 |
| 234 | D-RNA (APTAMER) | GGCCGGGGUUAGGGCUAGAAGUCGGCCGGCC | 197-B2-006-31a |
| 235 | D-RNA (APTAMER) | CGCCGGGGUUAGGGCUAGAAGUCGGCCGGCG | 197-B2-006-31b |
| 236 | D-RNA (APTAMER) | CGUGGUCCGUUGUGUCAGGUCUAUUCGCCCCGGUGCAGGGCAUCCGCG | 194-A2-001 |
| 236 | D-RNA (APTAMER) | GCAGUGUGACGCGGACGUGAUAGGACAGAGCUGAUCCCGCUCAGGUGAG | 196-B12-003 |
| 238 | D-RNA (APTAMER) | CAACAGCAGUGUGACGCGGACGUGAUAGGACAGAGCUGAUCCCGCUCAG | 196-B12-004 |

**TABLE 1 (P)**

| **Seq.-ID** | ***RNA*/*Peptide*** | **Sequence** | ***Internal Reference*** |
|---|---|---|---|
| 239 | L-RNA (Spiegelmer) | 5' -PEG-UAAGGAAACUCGGUCUGAUGCGGUAGCGCUGUGCAGAGCU | PEGylated Control Spiegelmer |
| 240 | L-RNA (Spiegelmer) | GATCACACCACGC-(C18-PEG-spacer)-(C18-PEG-spacer)-1- NH2-3′ | 193-G2-012-5'-PEG capture probe |
| 241 | L-RNA (Spiegelmer) | 5′-NH₂-(C18-PEG-spacer)-(C18-PEG-spacer)- GCGUACCUGAC | 193-G2-012-5'-PEG detect probe |
| 242 | IFNα 2b: | | |
| 243 | IFNα 2a: | CDLPQTHSLG SRRTLMLLAQ MRKISLFSCL KDRHDFGFPQ EEFGNQFQKA ETIPVLHEMI QQIFNLFSTK DSSAAWDETL LDKFYTELYQ QLNDLEACVI QGVGVTETPL MKEDSILAVR KYFQRITLYL KEKKYSPCAW EVVRAEIMRS FSLSTNLQES LRSKE | |
| 244 | interferon beta-1B | SYNLLGFLQR SSNFQSQKLL WQLNGRLEYC LKDRMNFDIP EEIKQLQQFQ KEDAALTIYE MLQNIFAIFR QDSSSTGWNE TIVENLLANV YHQINHLKTV | |
| | | LEEKLEKEDF TRGKLMSSLH LKRYYGRILH YLKAKEYSHC AWTIVRVEIL RNFYFINRLT GYLRN | |
| 245 | interferon gamma-1B | MQDPYVKEAE NLKKYFNAGH SDVADNGTLF LGILKNWKEE SDRKIMQSQI VSFYFKLFKN FKDDQSIQKS VETIKEDMNV KFFNSNKKKR DDFEKLTNYS VTDLNVQRKA IHELIQVMAE LSPAAKTGKR KRSQMLFRGR | |
| 246 | IL-2 | MAPTSSSTKK TQLQLEHLLL DLQMILNGIN NYKNPKLTRM LTFKFYMPKK ATELKHLQCL EEELKPLEEV LNLAQSKNFH LRPRDLISNI NVIVLELKGS ETTFMCEYAD ETATIVEFLN RWITFAQSII STLT | |
| 247 | NY-ESO-1₁₅₇₋₁₆₅ | SLLMWITQV | |

The present invention is further illustrated by the figures, examples and the sequence listing from which further features, embodiments and advantages may be taken, wherein
- Fig. 1: shows an alignment of sequences of related RNA ligands binding to human SDF-1 indicating the sequence motif ("Type A") that is in a preferred embodiment in its entirety essential for binding to human SDF-1;
- Fig. 2A: shows derivatives of RNA ligand 192-A10-001 (human SDF-1 RNA ligand of sequence motif "Type A");
- Fig. 2B: shows derivatives of RNA ligand 192-A10-001 (human SDF-1 RNA ligand of sequence motif "Type A");
- Fig. 3: shows an alignment of sequences of related RNA ligands binding to human SDF-1 indicating the sequence motif ("Type B") that is in a preferred embodiment in its entirety essential for binding to human SDF-1;
- Fig. 4A: shows derivatives of RNA ligands 193-C2-001 and 193-G2-001 (human SDF-1 RNA ligands of sequence motif "Type B");
- Fig. 4B: shows derivatives of RNA ligands 193-C2-001 and 193-G2-001 (human SDF-1 RNA ligands of sequence motif "Type B");
- Fig. 5: shows an alignment of sequences of related RNA ligands binding to human SDF-1 indicating the sequence motif ("Type C") that is in a preferred embodiment in its entirety essential for binding to human SDF-1;
- Fig. 6: shows derivatives of RNA ligand 190-A3-001 (human SDF-1 RNA ligand of sequence motif "Type C");
- Fig. 7A: shows derivatives of RNA ligand 190-D5-001 (human SDF-1 RNA ligand of sequence motif "Type C");
- Fig. 7B: shows derivatives of RNA ligand 190-D5-001 (human SDF-1 RNA ligand of sequence motif "Type C");
- Fig. 8: shows derivatives of RNA ligand 197-B2 (human SDF-1 RNA ligand of sequence motif "Type C");
- Fig. 9: shows further RNA ligands binding to human SDF-1;

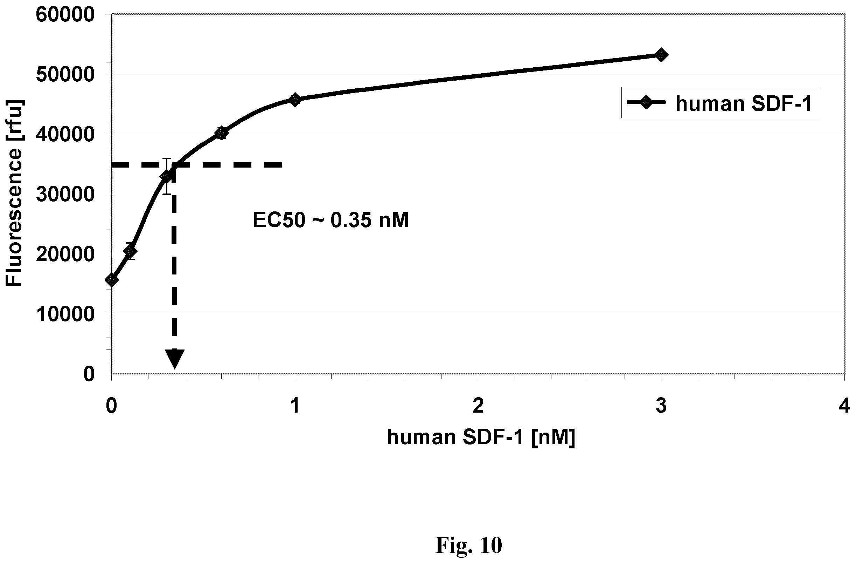

- Fig. 10: shows the human SDF-1-induced chemotaxis of Jurkat human T cell leukemia cells whereas after 3 hours migration of Jurkat human T cell leukemia cells towards various human SDF-1 concentrations a dose-response curve for human SDF-1 was obtained, represented as fluorescence signal over concentration of human SDF-1;
- Fig. 11: shows the result of a binding analysis of the human SDF-1 binding aptamer 192-A10-001 to biotinylated human D-SDF-1 37°C, represented as binding of the aptamer over concentration of biotinylated human D-SDF-1;
- Fig. 12: shows the efficacy of human SDF-1 binding Spiegelmer 192-A10-001 in a chemotaxis assay; cells were allowed to migrate towards human 0.3 nM SDF-1 preincubated at 37°C with various amounts of Spiegelmer 192-A10-001, represented as percentage of control over concentration of Spiegelmer 192-A10-001;
- Fig. 13: shows the result of a competitive binding analysis of the human SDF-1 binding aptamers 192-A10-001, 192-F10-001, 192-C9-001, 192-E10-001, 192-C10-001, 192-D11-001, 192-G11-001, 192-H11-001, 192-D10-001, 192-E9-001 and 192-H9-001 to biotinylated human D-SDF-1 at 37°C, represented as binding of the labeled aptamer 192-A10-001 (used as reference that is displaced by the non-labeled aptamers) at 1 nM and 5 nM non-labeled aptamers 192-A10-001, 192-F10-001, 192-C9-001, 192-E10-001, 192-C10-001, 192-D11-001, 192-G11-001, 192-H11-001, 192-D10-001, 192-E9-001 and 192-H9-001;

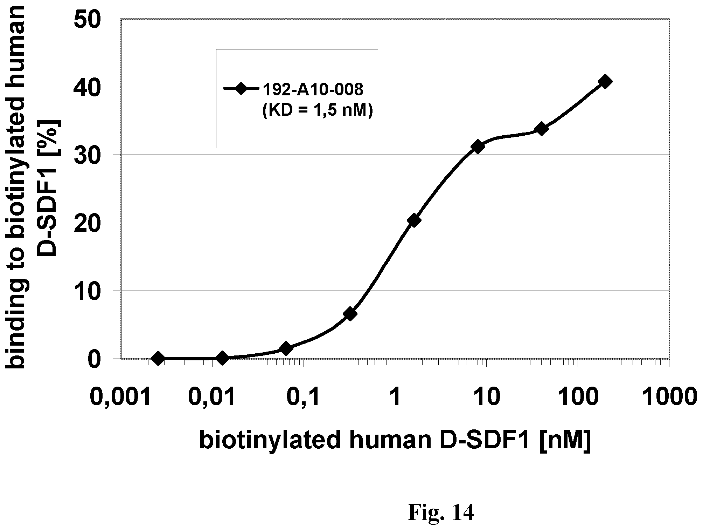

- Fig. 14: shows the result of a binding analysis of the human SDF-1 binding aptamer 192-A10-008 to biotinylated human D-SDF-1 at 37°C, represented as binding of the aptamer over concentration of biotinylated human D-SDF-1;
- Fig. 15: shows a Biacore 2000 sensorgram indicating the K_{D} value of the human SDF-1 binding Spiegelmer 192-A10-008 binding to human SDF-1 which was immobilized on a PioneerF1 sensor chip by amine coupling procedure, represented as response (RU) over time, additionally the on- and off-rates and the K_{D} values of Spiegelmers 192-A10-008 and 192-A10-001 are listed;
- Fig. 16: shows the efficacy of SDF-1 binding Spiegelmer 192-A10-008 in a chemotaxis assay; cells were allowed to migrate towards 0.3 nM human SDF-1 preincubated at 37°C with various amounts of Spiegelmer 192-A10-008, represented as percentage of control over concentration of Spiegelmer 192-A10-008;
- Fig. 17: shows a Biacore 2000 sensorgram indicating the K_{D} value of Spiegelmer 193-G2-01 binding to human SDF-1 which was immobilized on a PioneerF1 sensor chip by amine coupling procedure, represented as response (RU) over time, additionally the on- and off-rates and the K_{D} values of Spiegelmers 193-G2-001 and 193-C2-001 are listed;
- Fig. 18: shows the result of a binding analysis of the human anti-SDF-1 aptamer 193-G2-012 to biotinylated human D-SDF-1 at 37°C, represented as binding of the aptamer over concentration of biotinylated human D-SDF-1;
- Fig. 19: shows the result of a competitive binding analysis of the human SDF-1 binding aptamers 190-A3-001, 190-A3-003, 190-A3-004, 190-A3-007, 191-D5-001, 191-D5-002, 191-D5- 003, 191-D5-004, 191-D5-005, 191-D5-006 and 191-D5-007 to biotinylated human D-SDF-1 at 37°C, represented as binding of the labeled aptamer 190-A3-001 or 191-D5-001 (used as reference that is displaced by the non-labeled aptamers) at 500 nM, 50 nM and 10 nM non-labeled aptamers 190-A3-001, 190-A3-003, 190-A3-004, 190-A3-007, 191-D5-001, 191-D5-002, 191-D5-003, 191-D5-004, 191-D5-005, 191-D5-006 and 191-D5-007;
- Fig. 20: shows the result of a binding analysis of the human SDF-1 binding aptamers 190-A3-004 and 191-D5-007 to biotinylated human D-SDF-1 37°C, represented as binding of the aptamer over concentration of biotinylated human D-SDF-1;
- Fig. 21: shows a Biacore 2000 sensorgram indicating the K_{D} value of Spiegelmer 191-D5-007 binding to human SDF-1 which was immobilized on a PioneerF1 sensor chip by amine coupling procedure, represented as response (RU) over time, additionally the on- and off-rates and the K_{D} values of Spiegelmers 191-D5-001, 191-D5-007, 190-A3-003 and 197-B2 are listed;
- Fig. 22: shows the efficacy of SDF-1 binding Spiegelmer 190-A3-004 in a chemotaxis assay; cells were allowed to migrate towards 0.3 nM human SDF-1 preincubated at 37°C with various amounts of Spiegelmer 190-A3-004, represented as percentage of control over concentration of Spiegelmer 190-A3-004;
- Fig. 23A: shows the efficacy of SDF-1 binding Spiegelmers 193-G2-012-5'-PEG, 197-B2-006-5'-PEG, 191-D5-007-5'-PEG and 191-A10-008-5'-PEG in a chemotaxis assay; cells were allowed to migrate towards 0.3 nM human SDF-1 preincubated at 37°C with various amounts of Spiegelmers 193-G2-012-5'-PEG, 197-B2-006-5'-PEG, 191-D5-007-5'-PEG and 191-A10-008-5'-PEG, represented as percentage of control over concentration of Spiegelmers 193-G2-012-5'-PEG, 197-B2-006-5'-PEG, 191-D5-007-5'-PEG and 191-A10-008-5'-PEG;
- Fig. 23B: shows the efficacy of SDF-1 binding Spiegelmers 197-B2-006-5'PEG and 197-B2-006-31b-5'-PEG in a chemotaxis assay; cells were allowed to migrate towards 0.3 nM human SDF-1 preincubated at 37°C with various amounts of Spiegelmers 197-B2-006-5'PEG and 197-B2-006-31b-5'-PEG, represented as percentage of control over concentration of Spiegelmers 197-B2-006-5'PEG and 197-B2-006-31b-5'-PEG;
- Fig. 24A: shows a Biacore 2000 sensorgram indicating the K_{D} values of Spiegelmers 193-G2-012-5'-PEG, 191-A10-008-5'-PEG and 191-A10-001-5'-PEG binding to human SDF-1 which was immobilized on a PioneerF1 sensor chip by amine coupling procedure, represented as response (RU) over time;
- Fig. 24B: shows a Biacore 2000 sensorgram indicating the K_{D} values of Spiegelmers 197-B2-006-5'PEG, 197-B2-006-31b-5'-PEG and 191-D5-007-5'-PEG binding to human SDF-1 which was immobilized on a PioncerF1 sensor chip by amine coupling procedure, represented as response (RU) over time;
- Fig. 25A: shows the efficacy of SDF-1 binding Spiegelmers 192-A10-001, 192-A10-001-5'-HES130 and 192-A10-001-5'-HES100 in a chemotaxis assay; cells were allowed to migrate towards 0.3 nM human SDF-1 preincubated at 37°C with various amounts of Spiegelmers 192-A10-001, 192-A10-001-5'-HES130 and 192-A10-001-5'-HES100, represented as percentage of control over concentration of Spiegelmers 192-A10-001, 192-A10-001-5'-HES130 and 192-A10-001-5'-HES100;
- Fig. 25B: shows the efficacy of SDF-1 binding Spiegelmers 192-A10-001, 192-A10-001-5'-PEG30 and 192-A10-001-5'-PEG40 in a chemotaxis assay; cells were allowed to migrate towards 0.3 nM human SDF-1 preincubated at 37°C with various amounts of Spiegelmers 192-A10-001, 192-A10-001-5'-PEG30 and 192-A10-001-5'-PEG40, represented as percentage of control over concentration of Spiegelmers 192-A10-001, 192-A10-001-5'-PEG30 and 192-A10-001-5'-PEG40;
- Fig. 26: shows the inefficacy of a control-Spiegelmer in a chemotaxis assay; cells were allowed to migrate towards 0.3 nM human or murine SDF-1 preincubated at 37°C with various amounts of control-Spiegelmer, represented as percentage of control over concentration of control Spiegelmer;
- Fig. 27: shows the murine SDF-1-induced chemotaxis of Jurkat human T cell leukemia cells whereas after 3 hours migration of Jurkat human T cell leukemia cells towards various SDF-1 concentrations a dose-response curve for SDF-1 was obtained, represented as fluorescence signal;
- Fig. 28: shows the efficacy of SDF-1 binding Spiegelmers 192-A10-001 and 191-D5-007-5'PEG in a chemotaxis assay; cells were allowed to migrate towards 0.3 nM murine SDF-1 preincubated at 37°C with various amounts of Spiegelmers 192-A10-001 and 191-D5-007-5'PEG represented as percentage of control over concentration of Spiegelmers 192-A10-001 and 191-D5-007-5'PEG;
- Fig. 29: shows the efficacy of SDF-1 binding Spiegelmer 192-A10-001 in a CXCR4-receptor binding assay using human [¹²⁵J]-SDF-1α that was preincubated at 37°C with various amounts of Spiegelmers 192-A10-001, specifically bound [¹²⁵J]-SDF-1α was plotted over concentration of Spiegelmer 192-A10-001;
- Fig. 30: shows the inhibition of MAP-kinase stimulation of CXCR4-expressing cells with 1 nM human SDF-1α by human SDF-1 binding Spiegelmer 192-A10-001;
- Fig. 31: (A) is a diagram indicating tumor volume (mm³) over time for various treatment schemes determined by caliper measurement showing efficacy of NOX-A12 combined with IFNγ and TCR-T cells targeting the NY-ESO-1₁₅₇₋₁₆₅ epitope in an SYO-1A2-Luc+ xenograft synovial sarcoma model, (B) is a diagram indicating luciferase intensity on Day 21 as a measure of tumor growth for various treatment schemes showing greatest reduction of luciferase intensity for the combination of TCR-T cells combined with NOX-A12 and IFNγ in an SYO1-A2-Luc+ xenograft synovial sarcoma model;
- Fig. 32: (A) and (B) are dot plots indicating TCR-T cell numbers in tumors and spleens of mice treated with TCR-T cells, combination of TCR-T cells with NOX-A12, AMD3100 or IFNγ, combination of TCR-T cells with NOX-A12 and IFNγ, and combination of TCR-T cells with AMD3100 and IFNγ in an SYO1-A2-Luc+ xenograft synovial sarcoma model. (A) shows infiltration of TCR-T cells into tumors by immunohistochemistry as numbers of CD3+ cells (TCR-T cells) per mm² tumor area and (B) expansion of TCR-T cells in spleens by flow cytometry as numbers of hCD45+CD3+ cells (TCR-T cells);
- Fig. 33: (A) is a diagram indicating tumor volume (mm³) over time for various treatment schemes determined by caliper measurement showing efficacy of NOX-A12 combined with IFNγ and TCR-T cells targeting the NY-ESO-1₁₅₇₋₁₆₅ epitope in a Yamoto-Luc+ xenograft synovial sarcoma model and (B) is a diagram indicating luciferase intensity on Day 21 as a measure of tumor growth for various treatment schemes showing greatest reduction of luciferase intensity for the combination of TCR-T cells combined with NOX-A12 and IFNγ in a Yamoto-Luc+ xenograft synovial sarcoma model;
- Fig. 34: is a diagram indicating tumor volume (mm³) over time for various treatment schemes determined by caliper measurement showing efficacy of NOX-A12 combined with IFNα and TCR-T cells targeting the NY-ESO-1₁₅₇₋₁₆₅ epitope in a Yamoto-Luc+ xenograft synovial sarcoma model;
- Fig. 35: is a diagram indicating fractional growth volume (mean +/- STD) over time for various treatment schemes determined by caliper measurement showing efficacy of NOX-A12 combined with IFNγ in the immunocompetent conditional SS18::SSX2 synovial sarcoma model;
- Fig. 36: (A) is a diagram indicating tumor volume (mm³) over time for various treatment schemes determined by caliper measurement showing efficacy of NOX-A12 combined with IFNγ and CAR-T cells targeting the B7-H3 epitope in the xenograft MG-63-Luc+ osteosarcoma model based on caliper measurement; (B) and (C) are diagrams indicating luciferase intensity on Day 21 as a measure of tumor growth for various treatment schemes showing greatest reduction of luciferase intensity for the combination of CAR-T cells combined with NOX-A12 and IFNγ; and
- Fig. 37: (A) is a diagram indicating tumor volume (mm³) over time for various treatment schemes determined by caliper measurement showing efficacy of NOX-A12 combined with IFNγ and CAR-T cells targeting the B7-H3 epitope in the xenograft PANC-1-Luc+ pancreatic cancer model based on caliper measurement; (B) and (C) are diagrams indicating luciferase intensity on Day 21 as a measure of tumor growth for various treatment schemes showing greatest reduction of luciferase intensity for the combination of CAR-T cells combined with NOX-A12 and IFNγ.

### Example 1: Nucleic acids that bind human SDF-1

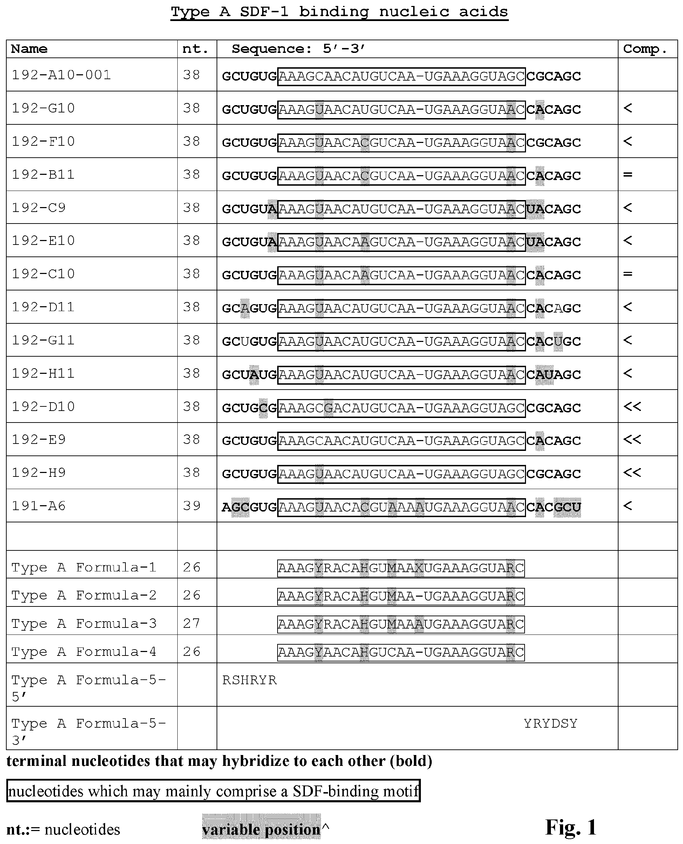

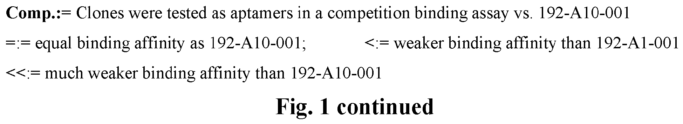

Using biotinylated human D-SDF-1 as a target, several nucleic acids that bind to human SDF-1 could be generated the nucleotide sequences of which are depicted in Figures 1 through 9. The nucleic acids were characterized on the aptamer, i. e. D-nucleic acid level with biotinylated human D-SDF-1 or on the Spiegelmer level, i. e. L-nucleic acid with the natural configuration of SDF-1 (L- SDF-1).

Aptamers were analyzed with biotinylated human D- SDF-1 using competitive or direct pull-down binding assays with biotinylated human D-SDF-1 (Example 4). Spiegelmers were tested with the natural configuration of SDF-1 (L-SDF-1) by surface plasmon resonance measurement using a Biacore 2000 instrument (Example 6) and a cell culture *in vitro* chemotaxis assay (Example 5).

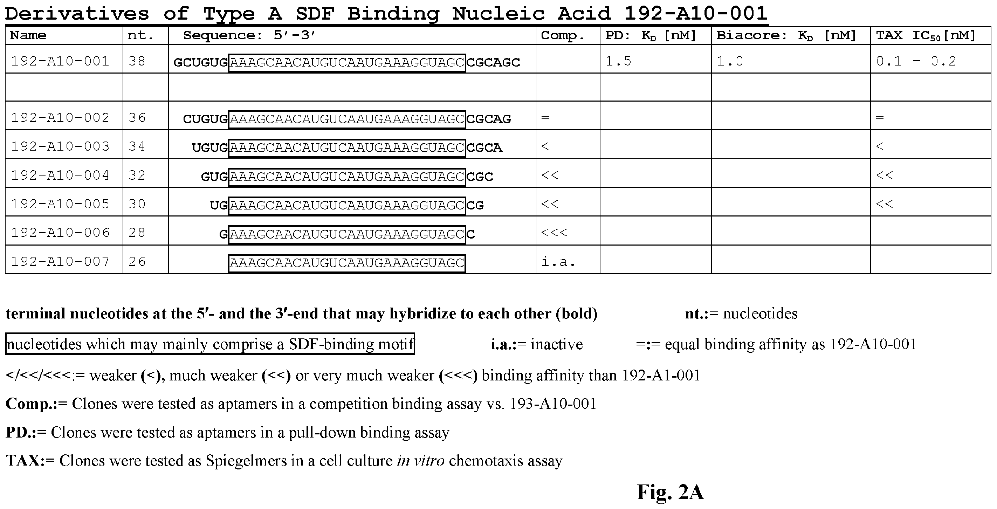

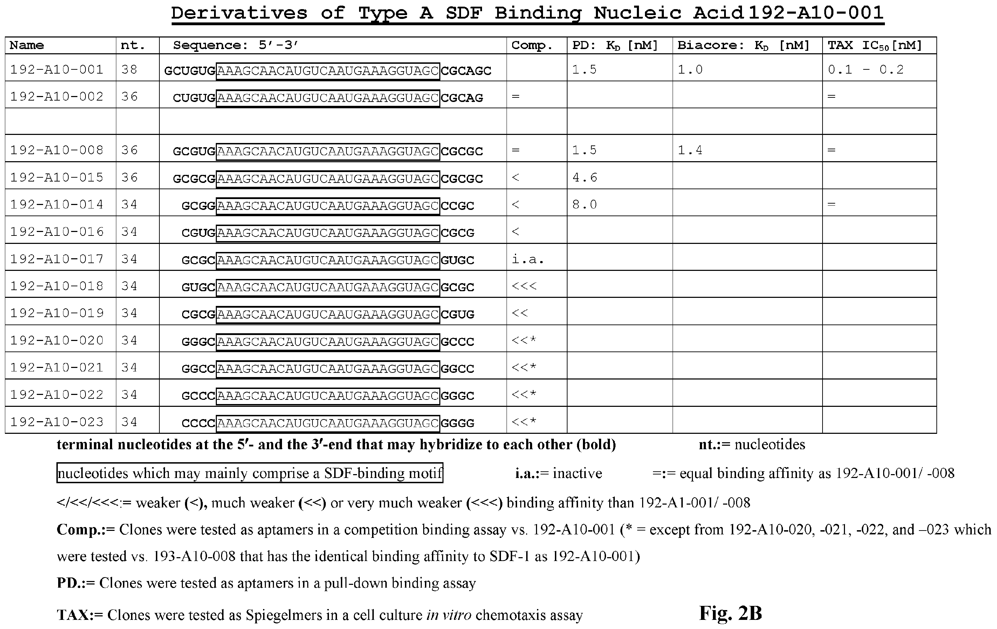

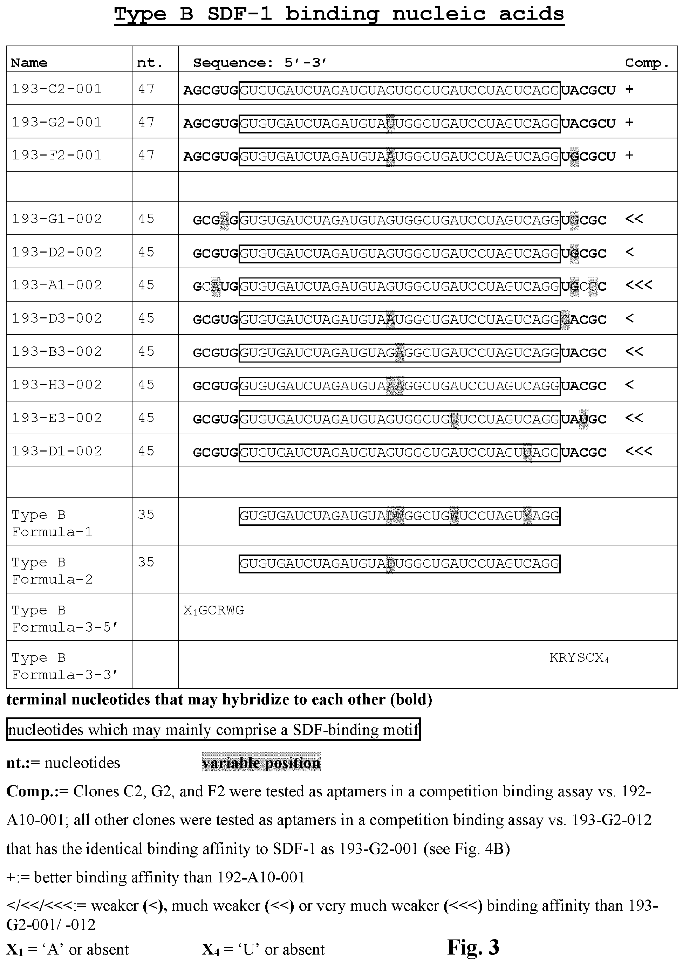

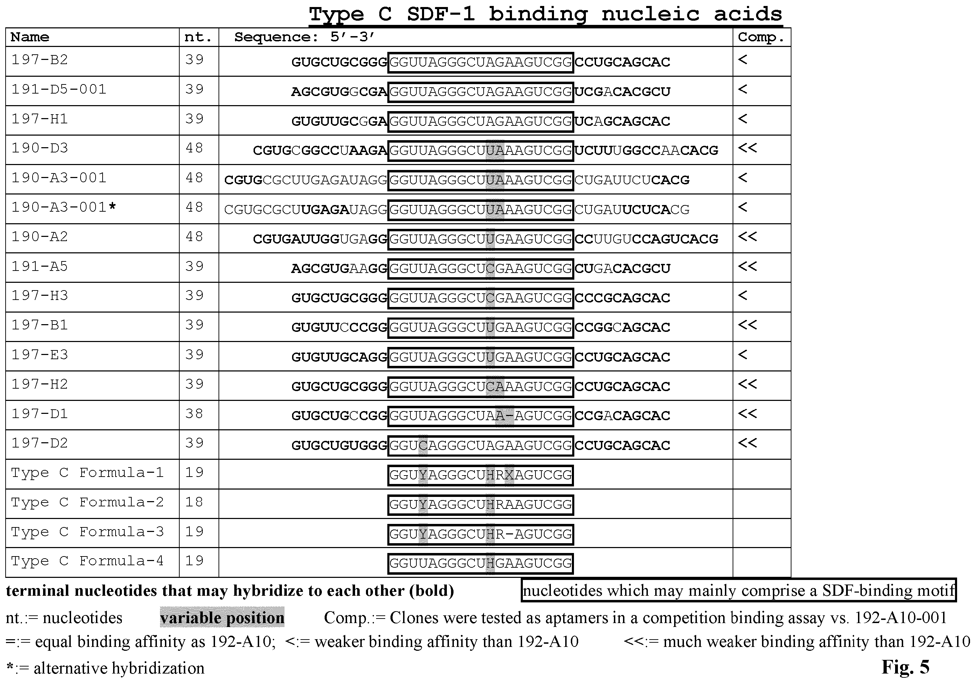

The nucleic acid molecules thus generated exhibit different sequence motifs, three main types are defined in Figs. 1, 2A and 2B (Type A), Figs. 3, 4A and 4B (Type B), Figs. 5, 6, 7A, 7B and 8 (Type C). For definition of nucleotide sequence motifs, the IUPAC abbreviations for ambiguous nucleotides is used:

| | | |
|---|---|---|
| S | strong | G or C; |
| W | weak | A or U; |
| R | purine | G or A; |
| Y | pyrimidine | C or U; |
| K | keto | G or U; |
| M | imino | A or C; |
| B | not A | C or U or G; |
| D | not C | A or G or U; |
| H | not G | A or C or U; |
| V | not U | A or C or G; |
| N | all | A or G or C or U |

If not indicated to the contrary, any nucleic acid sequence or sequence of stretches and boxes, respectively, is indicated in the 5' → 3' direction.

### 1.1 Type A SDF-1 binding nucleic acids

As depicted in Fig. 1 all sequences of SDF-1 binding nucleic acids of Type A comprise one core nucleotide sequence which is flanked by 5'- and 3'-terminal stretches that can hybridize to each other. However, such hybridization is not necessarily given in the molecule.

The nucleic acids were characterized on the aptamer level using direct and competitive pull-down binding assays with biotinylated human D-SDF-1 in order to rank them with respect to their binding behaviour (Example 4). Selected sequences were synthesized as Spiegelmers (Example 3) and were tested using the natural configuration of SDF-1 (L-SDF) in a cell culture *in vitro* chemotaxis assay (Example 5) and by surface plasmon resonance measurement using a Biacore 2000 instrument (Example 6).

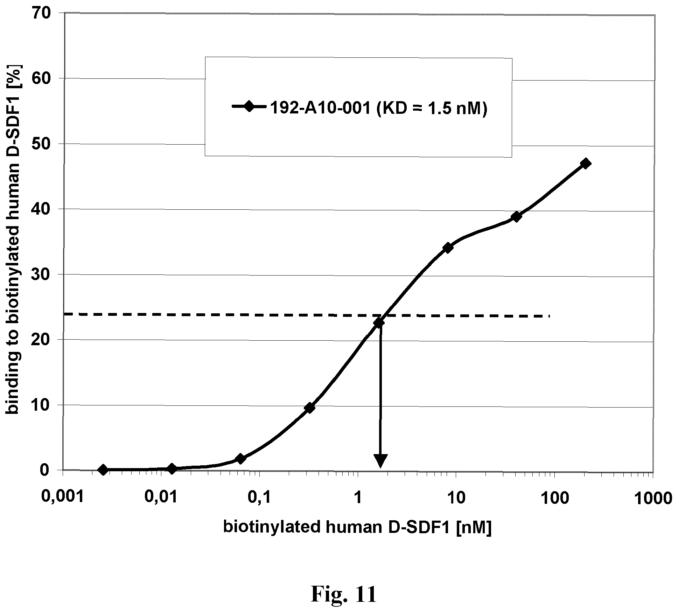

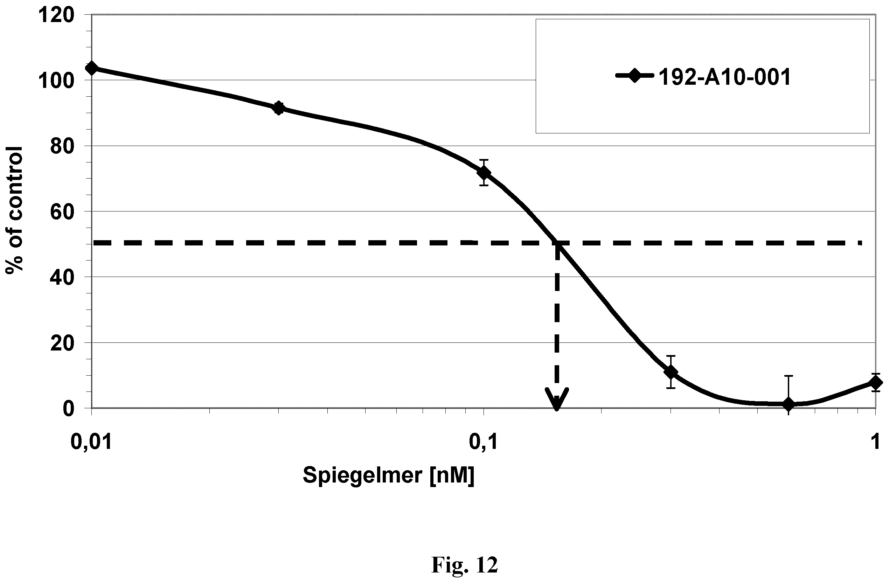

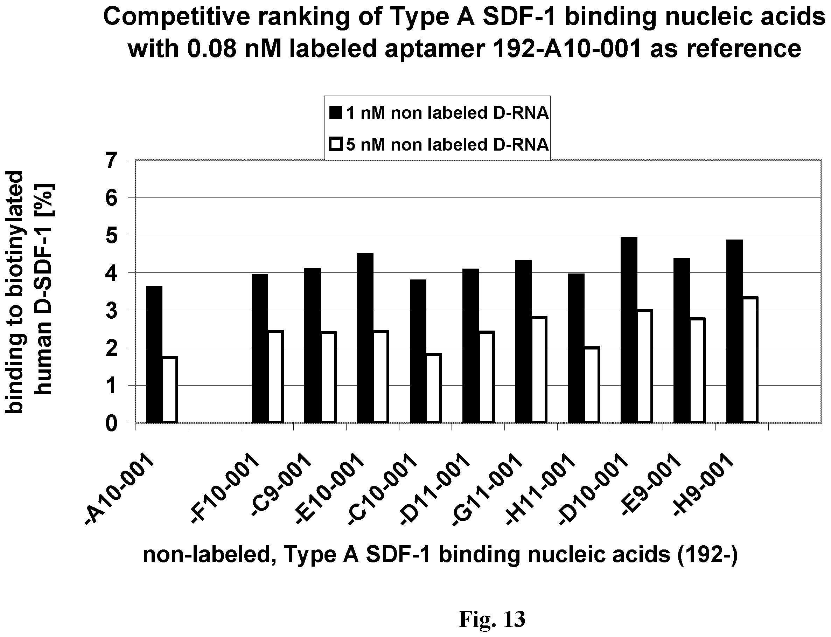

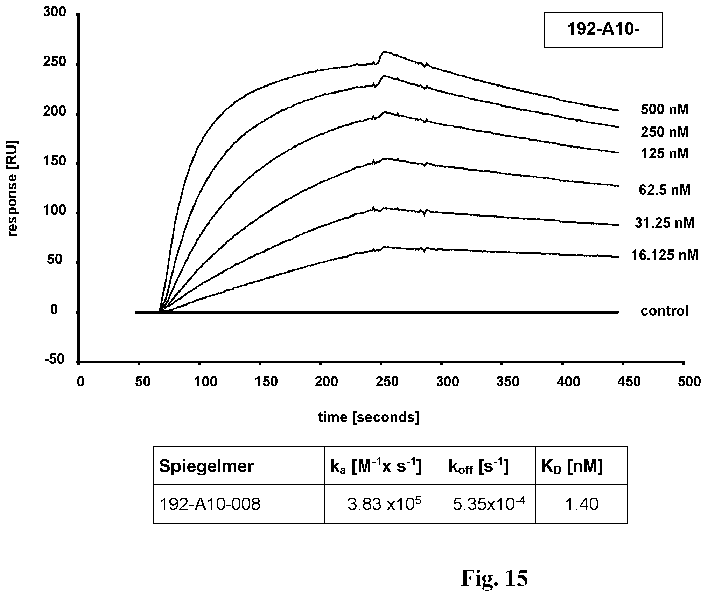

The sequences of the defined boxes or stretches may be different between the SDF-1 binding nucleic acids of Type A which influences the binding affinity to SDF-1. Based on binding analysis of the different SDF-1 binding nucleic acids summarized as Type A SDF-1 binding nucleic acids, the core nucleotide sequence and its nucleotide sequences as described in the following are individually and more preferably in their entirety essential for binding to SDF-1:
The core nucleotide sequence of all identified sequences of Type A SDF-1 binding nucleic acids share the sequence AAAGYRACAHGUMAAX_{A}UGAAAGGUARC (Type A Formula-1), whereby X_{A} is either absent or is 'A'. If 'A' is absent, the sequence of the core nucleotide sequence can be summarized as Type A Formula-2 (AAAGYRACAHGUMAA-UGAAAGGUARC). Type A SDF-1 binding nucleic acid 191-A6 (core nucleotide sequence: AAAGUAACACGUAAAAUGAAAGGUAAC) carrying the additional nucleotide 'A' within the core nucleotide sequence and still binding to SDF-1 let conclude an alternative core nucleotide sequence (AAAGYRACAHGUMAAAUGAAAGGUARC, Type A Formula-3). Exemplarily for all the other nucleic acids of Type A SDF-1 binding nucleic acids, the Type A SDF-1 binding nucleic acid 192-A10-001 was characterized for its binding affinity to human SDF-1. The equilibrium binding constant K_{D} was determined using the pull-down binding assay (K_{D} = 1.5 nM, Fig. 11) and by surface plasmon resonance measurement (K_{D} = 1.0 nM, Fig. 15). The IC₅₀ (inhibitory concentration 50%) of 0.12 nM for 192-A10-001 was measured using a cell culture *in vitro* chemotaxis assay (Fig. 12). Consequently, all Type A SDF-1 binding nucleic acids as depicted in Fig. 1 were analyzed in a competitive pull-down binding assay vs. 192-A10-001 (Fig. 13; not all of Type A SDF-1 binding nucleic acids tested are shown in Fig. 13). The Type A SDF-1 binding nucleic acids 192-B11 and 192-C10 showed equal binding affinities as 192-A10-001 in these competition experiments. Weaker binding affinity was determined for Type A SDF-1 binding nucleic acids 192-G10, 192-F10, 192-C9, 192-E10, 192-D11, 192-G11, 192-H11 and 191-A6. The Type A SDF-1 binding nucleic acids 192-D10, 192-E9 and 192-H9 have much weaker binding affinity than 192-A10-001 (Fig. 13).

As mentioned above, the Type A SDF-1 binding nucleic acid 192-B11 and 192-C10 exhibit equal binding affinity to SDF-1 as 192-A10-001. However, they show slight differences in the nucleotide sequence of the core nucleotide sequence. Therefore, the consensus sequence of the three molecules binding to SDF-1 with almost the same high affinity can be summarized by the nucleotide sequence AAAGYAACAHGUCAAUGAAAGGUARC (Type A Formula-4) whereby the nucleotide sequence of the core nucleotide sequence of 192-A10-001 (nucleotide sequence: AAGCAACAUGUCAAUGAAAGGUAGC) represents the nucleotide sequence with the best binding affinity of Type A SDF-1 binding nucleic acids.

Five or six out of the six nucleotides of the 5'-terminal stretch of Type A SDF-1 binding nucleic acids may hybridize to the respective five or six nucleotides out of the six nucleotides of the 3'-terminal stretch Type A SDF-1 binding nucleic acids to form a terminal helix. Although these nucleotides are variable at several positions, the different nucleotides allow for hybridization of five or six out of the six nucleotides of the 5'- and 3'-terminal stretches each. The 5'-terminal and 3'-terminal stretches of Type A SDF-1 binding nucleic acids as shown in Fig. 1 can be summarized in a generic formula for the 5'-terminal stretch ('RSHRYR', Type A Formula-5-5') and for the 3'-terminal stretch ('YRYDSY', Type A Formula-5-3'). Truncated derivatives of Type A SDF-1 binding nucleic acid 192-A10-001 were analyzed in a competitive pull-down binding assay vs. the original molecule 192-A10-001 and 192-A10-008 (Fig. 2A and 2B). These experiments showed that a reduction of the six terminal nucleotides (5'end: **GCUGUG**; 3'end: **CGCAGC**) of 192-A10-001 to five nucleotides (5'end: **CUGUG**; 3'end: **CGCAG**) of the derivative 192-A10-002 could be done without reduction of binding affinity. However, the truncation to four terminal nucleotides (5'end: **UGUG**; 3'end: **CGCA**; 192-A10-003) or less (192-A10-004/ -005/ -006/ -007) led to reduced binding affinity to SDF-1 (Fig. 2A). The determined 5'-terminal and 3'-terminal stretches with a length of five and four nucleotides of the derivatives of Type A SDF-1 binding nucleic acid 192-A10-001 as shown in Figs. 2A and B can be described in a generic formula for the 5'-terminal stretch ('X₂BBBS', Type A Formula-6-5') and of the 3'-terminal stretch ('SBBVX₃'; Type A Formula-6-3'), whereby X₂ is either absent or is 'S' and X₃ is either absent or is 'S'.

The nucleotide sequence of the 5'- and 3'-terminal stretches has an influence on the binding affinity of Type A SDF-1 binding nucleic acids. This is not only shown by the nucleic acids 192-F10 and 192-E10, but also by derivatives of 192-A10-001 (Fig. 2B;). The core nucleotide sequences of 192-F10 and 192-E10 are identical to 192-B11 and 192-C10, but comprise slight differences at the 3'-end of 5'-terminal stretch and at the 5'-end of 3'-terminal stretch resulting in reduced binding affinity.

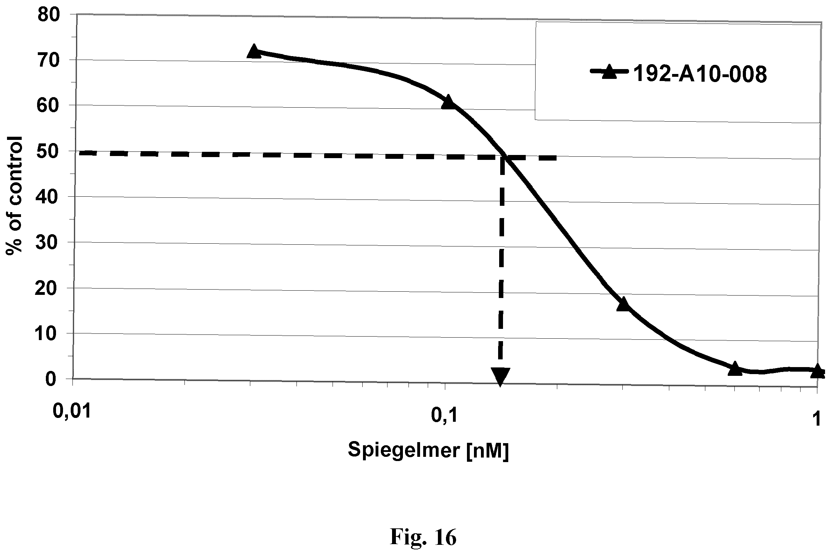

The substitution of 5'- and 3'-terminal nucleotides **'CUGUG'** and **'CGCAG'** of Type A SDF-1 binding nucleic acid 192-A10-002 by **'GCGCG'** and **'CGCGC'** (192-A10-015) resulted in a reduced binding affinity whereas substitutions by **'GCGUG'** and **'CGCGC'** (192-A10-008) resulted in same binding affinity as shown for 192-A10-002 (Fig. 2B, Fig. 15, Fig. 12, Fig. 16). Additionally, nine derivatives of Type A SDF-1 binding nucleic acid 192-A10-001 (192-A10-014/ -015/ -016/ -017/ - 018/ -019/ -020/ -021/ -022/ -023) bearing four 5'- and 3'-terminal nucleotides respectively were tested as aptamers for their binding affinity vs. 192-A10-001 or its derivative 192-A10-008 (both have the identical binding affinity to SDF-1). All clones showed weaker, much weaker or very much weaker binding affinity to SDF-1 as 192-A10-001 (six nucleotides forming a terminal helix) or as 192-A10-008 with five terminal nucleotides, respectively (Fig. 2B). Consequently, the sequence and the number of nucleotides of the 5'- and 3'-terminal stretches are essential for an effective binding to SDF-1. As shown for Type A SDF-1 binding nucleic acids 192-A10-002 and 192-A10-08 the preferred combination of 5'- and 3'-terminal stretches are 'CUGUG' and 'CGCAG' (5'- and 3'-terminal stretches of Type A SDF-1 binding nucleic acid 192-A10-002) and 'GCGUG' and 'CGCGC' (5'- and 3'-terminal stretches of Type A SDF-1 binding nucleic acid 192-A10-008).

However, combining the 5'-and 3'-terminal stretches of all tested Type A SDF-1 binding nucleic acids the generic formula for the 5'-terminal stretch of Type A SDF-1 binding nucleic acids is 'X₁X₂NNBV' (Type A Formula-7-5') and the generic formula for the 3'-terminal stretch of Type A SDF-1 binding nucleic acids is 'BNBNX₃X₄' (Type A Formula-7-3'), whereas
X₁ is 'R' or absent , X₂ is 'S', X₃ is 'S' and X₄ is 'Y' or absent;
   or
X₁ is absent, X₂ is 'S' or absent, X₃ is 'S' or absent and X₄ is absent.

### 1.2 Type B SDF-1 binding nucleic acids

As depicted in Fig. 3 all sequences of SDF-1 binding nucleic acids of Type B comprise one core nucleotide sequence which is flanked by 5'- and 3'-terminal stretches that can hybridize to each other. However, such hybridization is not necessarily given in the molecule.

The nucleic acids were characterized on the aptamer level using direct and competitive pull-down binding assays with biotinylated human D-SDF-1 in order to rank them with respect to their binding behaviour (Example 4). Selected sequences were synthesized as Spiegelmers (Example 3) and were tested using the natural configuration of SDF-1 (L-SDF) in a cell culture *in vitro* chemotaxis assay (Example 5) and by surface plasmon resonance measurement using a Biacore 2000 instrument (Example 6).

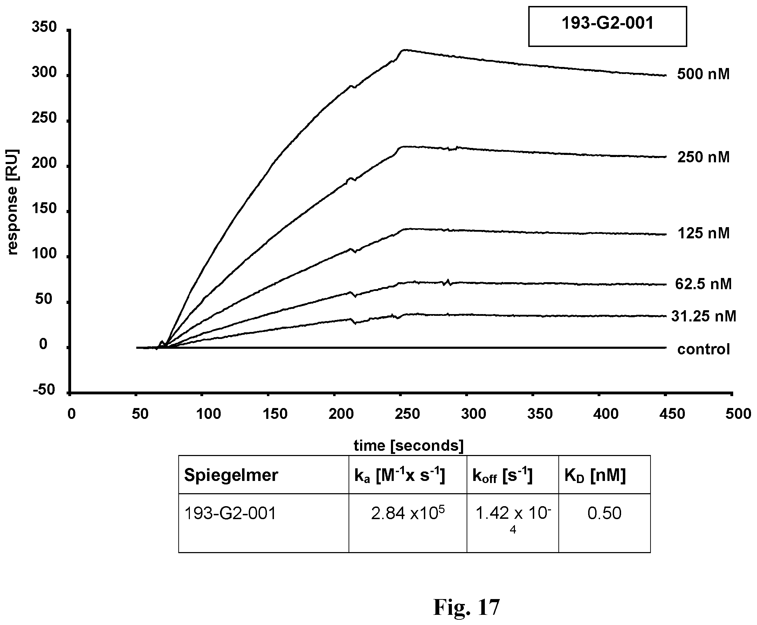

The sequences of the defined boxes or stretches may be different between the SDF-1 binding nucleic acids of Type B which influences the binding affinity to SDF-1. Based on binding analysis of the different SDF-1 binding nucleic acids summarized as Type B SDF-1 binding nucleic acids, the core nucleotide sequence and its nucleotide sequences as described in the following are individually and more preferably in their entirety essential for binding to SDF-1:
The core nucleotide sequence of all identified sequences of Type B SDF-1 binding nucleic acids share the sequence GUGUGAUCUAGAUGUADWGGCUGWUCCUAGUYAGG (Type B Formula-1). The Type B SDF-1 binding nucleic acids 193-G2-001, 193-C2-001 and 193-F2-001 that differ in one position of the core nucleotide sequence were analyzed in a competitive pull-down binding assay vs. the Type A SDF-1 binding nucleic acid 192-A10-001 (K_{D} of 1.5 nM determined in a pull-down binding assay [Fig. 11], K_{D} of 1.0 nM determined by surface plasmon resonance measurement [Fig. 15], IC₅₀ of 0.12 nM; [Fig. 12]). Each of the three tested Type B SDF-1 binding nucleic acids showed superior binding to human SDF-1 in comparison to Type A SDF-1 binding nucleic acid 192-A10-001 whereby the binding affinity of 193-G2-001 is as good as 193-C2-001 and 193-F2-001 (Fig. 3). The data suggests that the difference in the nucleotide sequence of the core nucleotide sequence of Type B SDF-1 binding nucleic acids 193-G2-001, 193-C2-001 and 193-F2-001 has no influence on the binding affinity to SDF-1. Exemplarily the Type B SDF-1 binding nucleic acid 193-G2-001 was characterized for its binding affinity to human SDF-1. The equilibrium binding constant K_{D} was determined using the pull-down binding assay (K_{D} = 0.3 nM) and by surface plasmon resonance measurement (K_{D} = 0.5 nM, Fig. 17). The IC₅₀ (inhibitory concentration 50%) of 0.08 nM for 193-G2-001 was measured using a cell culture *in vitro* chemotaxis assay. In contrast, the Type B SDF-1 binding nucleic acids 193-B3-002, 193-H3-002, 193-E3-002 and 193-D1-002 that differ in the sequence of the core nucleotide sequence have worse binding properties (Fig. 3). As result Type B SDF-1 binding nucleic acids with improved binding affinity to SDF-1 share a core nucleotide sequence with the sequence GUGUGAUCUAGAUGUADUGGCUGAUCCUAGUCAGG (Type B Formula-2).

Four, five or six nucleotides out of the six nucleotides of the 5'-terminal stretch of Type B SDF-1 binding nucleic acids may hybridize to the respective four, five or six out of the six nucleotides of the 3'-terminal stretch of Type B SDF-1 binding nucleic acids to form a terminal helix. Although the nucleotides are variable at several positions, the different nucleotides allow the hybridization for four, five or six nucleotides out of the six nucleotides of the 5'- and 3'-terminal stretches each. The 5'-terminal and 3'-terminal stretches of Type B SDF-1 binding nucleic acids as shown in Fig. 3 can be summarized in a generic formula for the 5'-terminal stretch (Type B Formula-3-5'; 'X₁GCRWG' whereas X₁ is 'A' or absent) and of the 3'-terminal stretch (Type B Formula-3-3'; 'KRYSCX₄' whereas X₄ is 'U' or absent). Type B SDF-1 binding nucleic acids 193-G1-002, 193-D2-002, 193-A1-002 and 193-D3-002 have weaker binding affinities to SDF-1 although they share the identical core nucleotide sequence (Type B Formula-2) with 193-C2-001, 193-G2-001 and 193-F2-001 (Fig. 3). The-unfavorable binding properties of Type B SDF-1 binding nucleic acids 193-G1-002, 193-D2-002, 193-A1-002 and 193-D3-002 may be due to the number of nucleotides and sequence of the 5'- and 3'-terminal stretches.

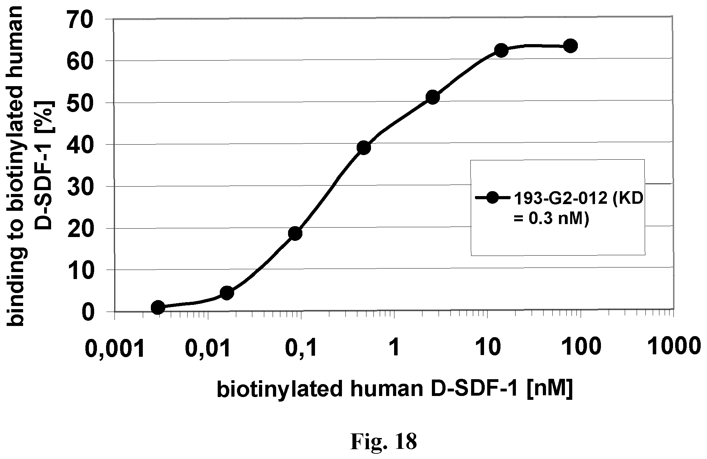

Truncated derivatives of the Type B SDF-1 binding nucleic acids 193-G2-001 and 193-C2-001 were analyzed in a competitive pull-down binding assay vs. 193-G2-001 and 193-G2-012, respectively (Fig. 4A and 4B). These experiments showed that a reduction of the six terminal nucleotides (5'end: **AGCGUG;** 3'end: **UACGCU**) of Type B SDF-1 binding nucleic acids 193-G2-001 and 193-C2-001 to five nucleotides (5'end: **GCGUG;** 3'end: **UACGC**) lead to molecules with similar binding affinity (193-C2-002 and 193-G2-012). The equilibrium dissociation constant K_{D} was determined using the pull-down binding assay (K_{D} = 0.3 nM, Fig. 18). A truncation to four (5'end: **CGUG;** 3'end: **UACG; 193-C2-003**) or less nucleotides (193-C2-004, 193-C2-005, 193-C2-006, 193-C2-007) resulted in a reduced binding affinity to SDF-1 which was measured by using the competition pull-down binding assay (Fig. 4A). The nucleotide sequence of the five terminal nucleotides at the 5'- and 3'-end, respectively, has an influence on the binding affinity of Type B SDF-1 binding nucleic acids. The substitution of 5'- and 3'-terminal nucleotides '**GCGUG**' and '**UACGC**' (193-C2-002, 193-G2-12) by '**GCGCG**' and '**CGCGC**' (193-G2-013) resulted in a reduced binding affinity. Additionally, the four different derivatives of Type B SDF-1 binding nucleic acid 193-G2-001 with a terminal helix with a length of four base-pairing nucleotides (193-G2-014/ -0151 -016/ -017) were tested. All of them showed reduced binding affinity to SDF-1 (Fig. 4B). Therefore, the sequence and the length of the 5'-and 3'-terminal nucleotides are essential for an effective binding to SDF-1. The 5'-terminal and 3'-terminal stretches with a length of five and four nucleotides of the derivatives of Type B SDF-1 binding nucleic acids 193-C2-003 and 193-G2-012 as shown in Figs. 4A and 4B can be described in a generic formula for the 5'-terminal stretch ('X₂SSBS', Type B Formula-4-5'), whereby X₂ is either absent or is 'G', and of the 3'-terminal stretch ('BVSSX₃', Type B Formula-4-3'), and whereby X₃ is either absent or is 'C'. As shown for Type B SDF-1 binding nucleic acids 193-G2-001 and 193-C2-01 and their derivatives 193-G2-012 and 193-C2-002 the preferred combination of 5'- and 3'-terminal stretches are 'X₁GCGUG' (5'-terminal stretch; Type B Formula 5-5') and 'UACGCX₄' (3'-terminal stretch; Type B Formula 5-3'), whereas X₁ is either 'A' or absent and X₄ is 'U' or absent.

However, combining the 5'-and 3'-terminal stretches of all tested Type B SDF-1 binding nucleic acids the generic formula for the 5'-terminal stretch of Type B SDF-1 binding nucleic acids is 'X₁X₂SVNS' (Type B Formula-6-5') and the generic formula for the 3'-terminal stretch Type B SDF-1 binding nucleic acids is 'BVBSX₃X₄' (Type B Formula-6-3'), whereas
X₁ is 'A' or absent, X₂ is 'G', X₃ is 'C' and X₄ is 'U' or absent;
or X₁ is absent, X₂ is 'G' or absent, X₃ is 'C' or absent and X₄ is absent;

### 1.3 Type C SDF-1 binding nucleic acids

As depicted in Fig. 5 all sequences of SDF-1 binding nucleic acids of Type C comprise one core nucleotide sequence which is flanked by 5'- and 3'-terminal stretches that can hybridize to each other. However, such hybridization is not necessarily given in the molecule.

The nucleic acids were characterized on the aptamer level using direct and competitive pull-down binding assays with biotinylated human D-SDF-1 in order to rank them with respect to their binding behaviour (Example 4). Selected sequences were synthesized as Spiegelmers (Example 3) and were tested using the natural configuration of SDF-1 (L-SDF) in a cell culture *in vitro* chemotaxis assay (Example 5) and by surface plasmon resonance measurement using a Biacore 2000 instrument (Example 6).

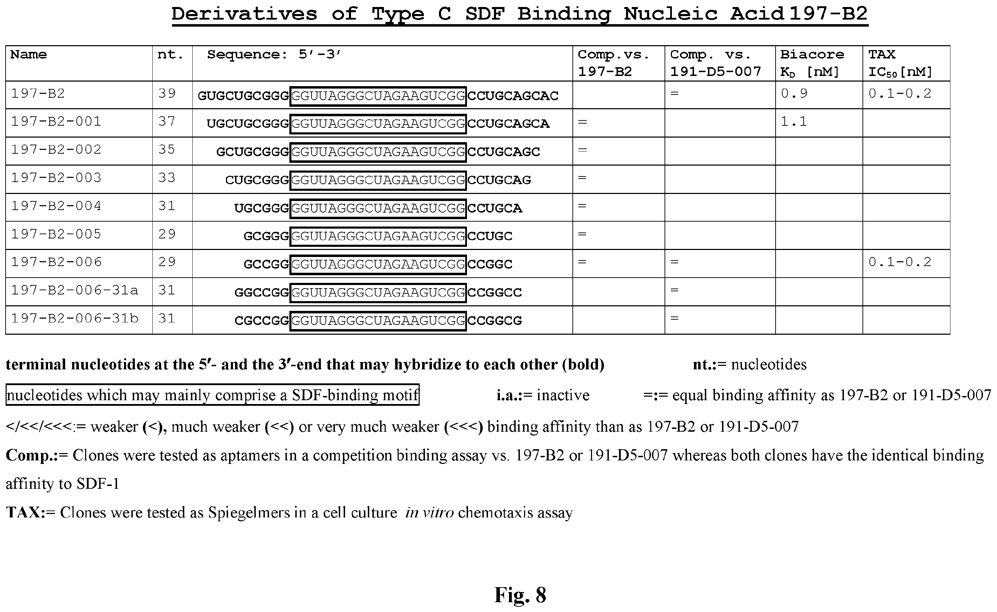

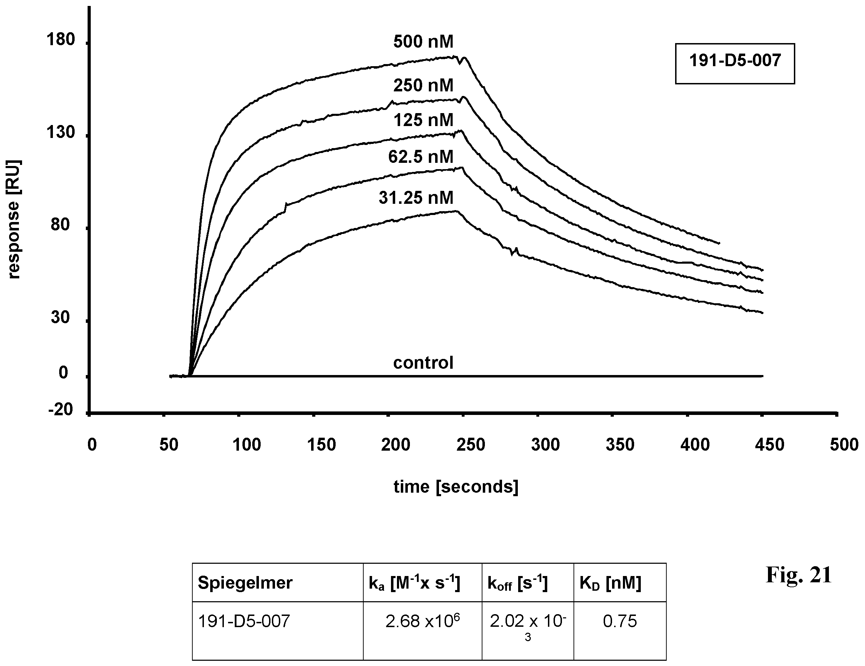

The sequences of the defined boxes or stretches may be different between the SDF-1 binding nucleic acids of Type C which influences the binding affinity to SDF-1. Based on binding analysis of the different SDF-1 binding nucleic acids summarized as Type C SDF-1 binding nucleic acids, the core nucleotide sequence and its nucleotide sequence as described in the following are individually and more preferably in their entirety essential for binding to SDF-1:
The core nucleotide sequence of all identified sequences of Type C SDF-1 binding nucleic acids share the sequence GGYAGGGCUHRX_{A}AGUCGG (Type C Formula-1), whereby X_{A} is either absent or is 'A'. With the exception of Type C SDF-1 binding nucleic acid 197-D1 the core nucleotide sequence of all identified sequences of Type C SDF-1 binding nucleic acids share the nucleotide sequence GGUYAGGGCUHRAAGUCGG (Type C Formula-2). Type C SDF-1 binding nucleic acid 197-D1 (core nucleotide sequence: GGUUAGGGCUAA-AGUCGG) missing one nucleotide 'A' within the core nucleotide sequence and still binding to SDF-1 let conclude an alternative core nucleotide sequence (GGYAGGGCUHR-AGUCGG, Type C Formula-3). Initially, all Type C SDF-1 binding nucleic acids as depicted in Fig. 5 were analyzed in a competitive pull-down binding assay vs. Type A SDF-1 binding nucleic acid 192-A10-001 (K_{D} = 1.5 nM determined by pull-down assay and by surface plasmon resonance measurements; IC₅₀ = 0.12 nM). The Type C SDF-1 binding nucleic acids 191-D5-001, 197-B2, 190-A3-001, 197-H1, 197-H3 and 197-E3 showed weaker binding affinities than 192-A10-001 in competition experiments. Much weaker binding affinity was determined for 191-A5, 197-B1, 197-D1, 197-H2 and 197-D2 (Fig. 5). The molecules or derivatives thereof were further characterized by further competitive pull-down binding assays, plasmon resonance measurements and an *in vitro* chemotaxis assay. The Type C SDF-1 binding nucleic acid 191-D5-001 was characterized for its binding affinity to human SDF-1 whereas the equilibrium binding constant K_{D} was determined by surface plasmon resonance measurement (K_{D} = 0.8 nM, Fig. 21). The IC₅₀ (inhibitory concentration 50%) of 0.2 nM for 191-D5-001 was measured using a cell-culture *in vitro* chemotaxis assay. The binding affinity of Type C SDF-1 binding nucleic acid 197-B2 for human SDF-1 was determined by surface plasmon resonance measurement (K_{D} = 0.9 nM), its IC₅₀ (inhibitory concentration 50%) of 0.2 nM was analyzed in a cell-culture *in vitro* chemotaxis assay. These data indicates that Type C SDF-1 binding nucleic acids 191-D5-001 and 197-B2 have similar binding affinity to SDF-1 (Fig. 5 and 8).

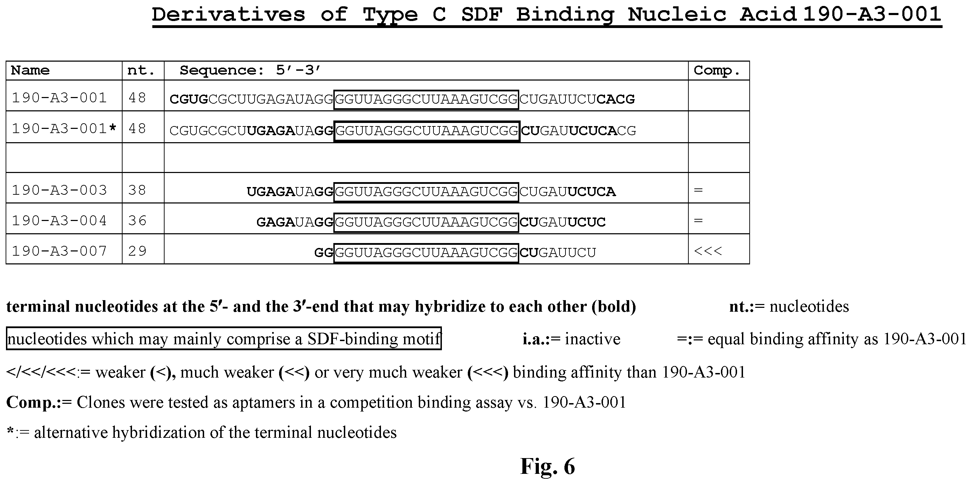

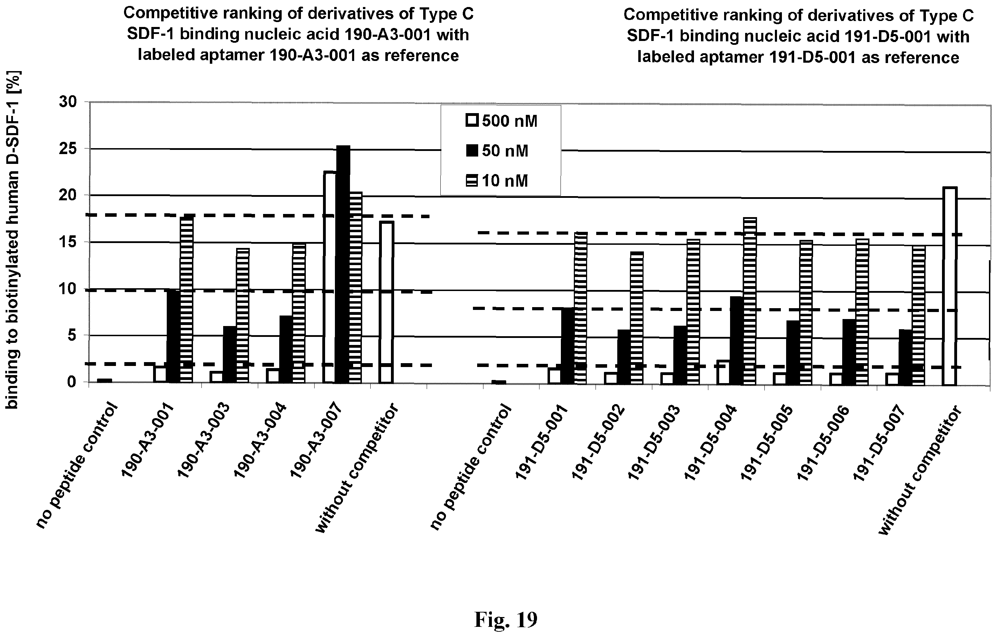

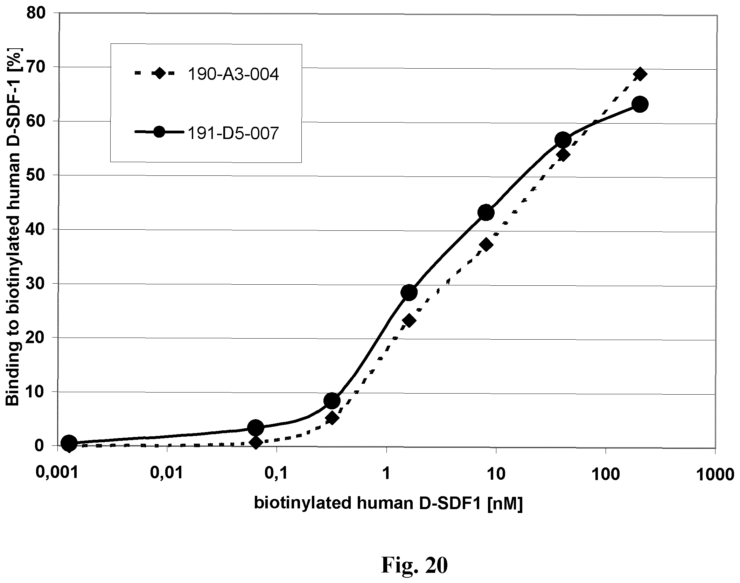

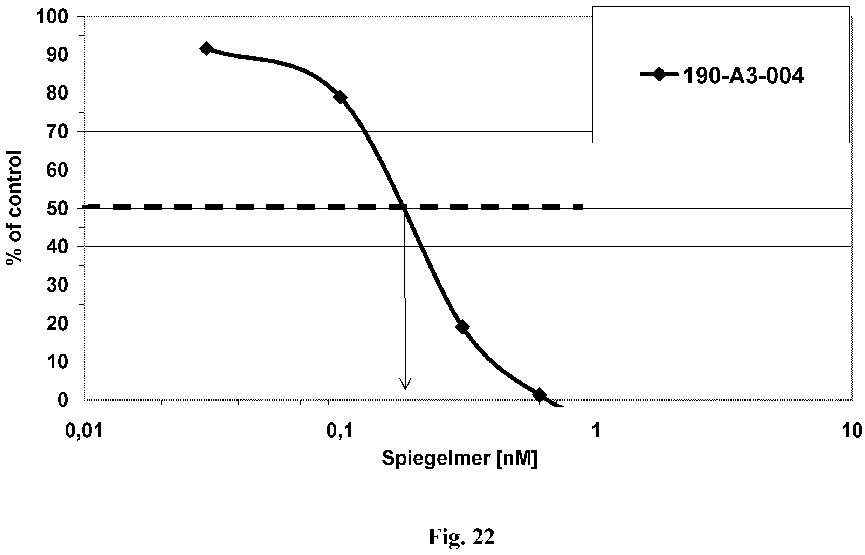

Type C SDF-1 binding nucleic acid 190-A3-001 (48 nt) comprises a 5'-terminal stretch of 17 nucleotides and a 3'-terminal stretch of 12 nucleotides whereby on the one hand the four nucleotides at the 5'-end of the 5'-terminal stretch and the four nucleotides at the 3'-end of the 3'-terminal stretch may hybridize to each other to form a terminal helix. Alternatively, the nucleotides **'UGAGA'** in the 5'-terminal stretch may hybridize to the nucleotides **'UCUCA'** in the 3'-terminal stretch to form a terminal helix. A reduction to eight nucleotides of the 5'-terminal stretch (**'GAGAUAGG'**) and to nine nucleotides of the 3'-terminal stretch (**'CUGAUUCUC'**) of molecule 190-A3-001 (whereby six out of the eight/nine nucleotides of the 5'- and 3'-terminal stretch can hybridize to each other) does not have an influence on the binding affinity to SDF-1 (190-A3-004; Fig. 6 and Fig. 19). The equilibrium binding constant K_{D} od 190-A3-004 was determined using the pull-down binding assay (K_{D} = 4.6 nM, Fig. 20) and by surface plasmon resonance measurement (K_{D} = 4.7 nM). The IC₅₀ (inhibitory concentration 50%) of 0.1 nM for 190-A3-004 was measured using a cell-culture *in vitro* chemotaxis assay (Fig. 22). However, the truncation to two nucleotides at the 5'-terminal stretch leads to a very strong reduction of binding affinity (190-A3-007; Fig. 6 and Fig. 19).

The Type C SDF-1 binding nucleic acids 191-D5-001, 197-B2 and 197-H1 (core nucleotide sequence: GGUUAGGGCUAGAAGUCGG), 197-H3/191-A5 (core nucleotide sequence: GGUUAGGGCUCGAAGUCGG) and 197-E3/197-B1 (core nucleotide sequence: GGUUAGGGCUUGAAGUCGG) share an almost identical core nucleotide sequence (Type C formula-4; nucleotide sequence: GGUUAGGGCUHGAAGUCGG). 191-D5-001, 197-B2 and 197-H1 do not share a similar 5'- and 3'-terminal stretch (197-H3 and 197-E3 have the identical 5'- and 3'-terminal stretch as 197-B2). However, the respective ten (197-B2, 197-E3, 197-H3) or nine out of the ten (191-D5-001, 197-H1) nucleotides of the 5'-terminal stretch may hybridize to the respective ten (197-B2, 197-E3, 197-H3) or nine out of the ten (191-D5-001, 197-H1) nucleotides of the 3'-terminal stretch (Fig. 5). Thus, the 5'-terminal stretch of Type C SDF-1 binding nucleic acids 197-B2, 191-D5-001, 197-H1, 197-E3 and 197-H3 as mentioned above plus 191-A5, 197-B1, 197-H2, 197-D1 and 197-D2 comprise a common generic nucleotide sequence of 'RKSBUSNVGR' (Type C Formula-5-5'). The 3'-terminal stretch of Type C SDF-1 binding nucleic acids 197-B2, 191-D5-001, 197-H1, 197-E3, and 197-H3 as mentioned above plus 191-A5, 197-B1, 197-H2, 197-D1 and 197-D2 comprise a common generic nucleotide sequence of 'YYNRCASSMY' (Type C Formula-5-3'), whereby the 5' and the 3'-terminal stretches of Type C SDF-1 binding nucleic acids 197-B2, 191-D5-001, 197-H1, 197-E3 and 197-H3 are preferred. These preferred 5'- and 3'-terminal stretches of Type C SDF-1 binding nucleic acids 197-B2, 191-D5-001, 197-H1, 197-E3 and 197-H3 can be summarized in the generic formula 'RKSBUGSVGR' (Type C Formula-6-5'; 5'-terminal stretch) and 'YCNRCASSMY' (Type C Formula-6-3'; 3'-terminal stretch).

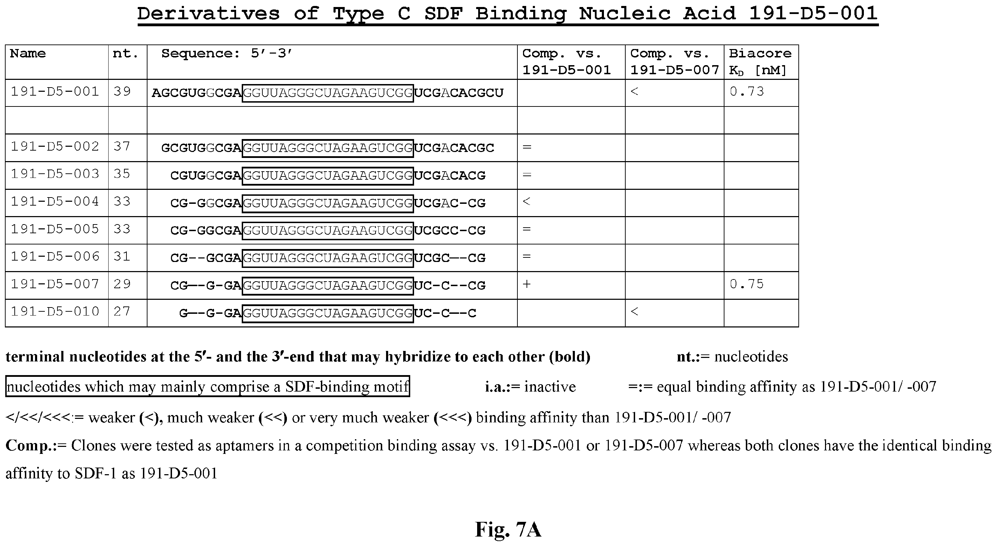

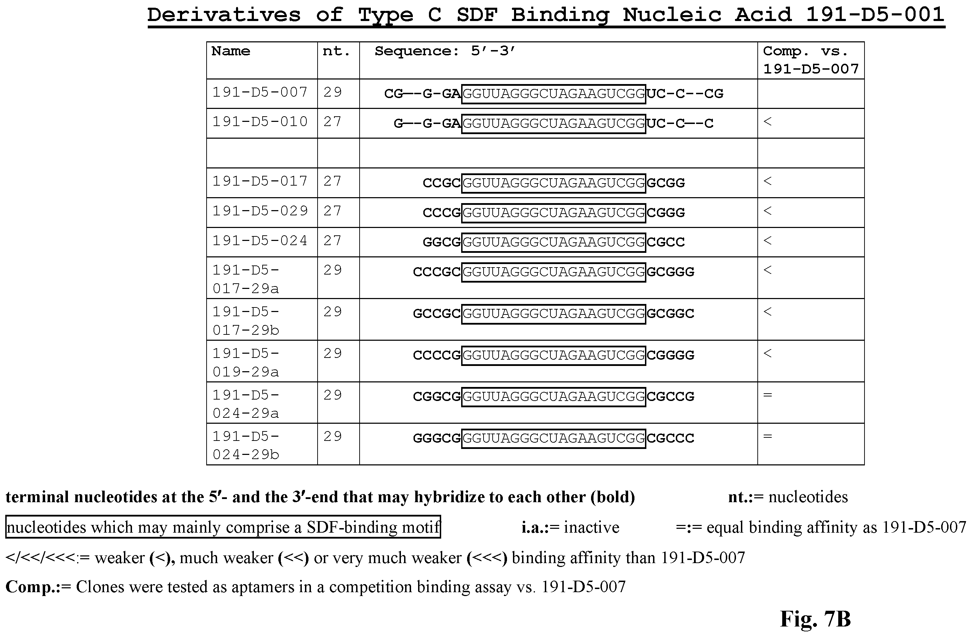

Truncated derivatives of Type C SDF-1 binding nucleic acid 191-D5-001 were constructed and tested in a competitive pull-down binding assay vs. the original molecule 191-D5-001 (Fig. 7A, Fig. 7B and Fig. 19). At first the length of the 5'- and 3'-terminal stretches were shortened from ten nucleotides (191-D5-001) each to seven nucleotides each (191-D5-004) as depicted in Fig. 7A whereby nine out of the ten (191-D5-001) or six out of the seven nucleotides (191-D5-004) of the 5'-terminal stretch and of the 3'-terminal stretch, respectively can hybridize to each other. The reduction to seven nucleotides of the 5'- and 3'- terminal stretch respectively (whereas six out of the seven nucleotides can hybridize to each other) led to reduced binding affinity to SDF-1 (191-D5-004). The terminal stretches of Type C SDF-1 binding nucleic acid 191-D5-004 were modified whereby the non-pairing nucleotide 'A' within the 3'-terminal stretch of 191-D5-004 was substituted by a 'C' (191-D5-005). This modification led to an improvement of binding. This derivative, Type C SDF-1 binding nucleic acid 191-D5-005, showed similar binding to SDF-1 as 191-D5-001. Further truncation of the 5'- and 3'-terminal stretch to five nucleotides respectively led to a molecule with a length of total 29 nucleotides (191-D5-007). Because of the similarities of 191-D5-001 and of the Type C SDF-1 binding nucleic acids 197-B2, 191-D5-001, 197-H1, 191-A5, 197-H3, 197-B1, 197-E3, 197-D1, 197-H2 and 197-D2 and because of the data shown for 191-D5-007 it may assume that the 5'-and 3'-terminal stretch can in principle be truncated down to five nucleotides whereby the nucleotide sequence 'CGGGA' for 5'-terminal stretch and 'UCCCG' for the 3'-terminal stretch was successfully tested (Type C SDF-1 binding nucleic acid 191-D5-007). Type C SDF-1 binding nucleic acid 191-D5-007 surprisingly binds somewhat better to SDF-1 than 191-D5-001 (determined on aptamer level using the competition binding assay). The equilibrium binding constant K_{D} of 191-D5-007 was determined using the pull-down binding assay (K_{D} = 2.2 nM, Fig. 20) and by surface plasmon resonance measurement (K_{D} = 0.8 nM, Fig. 21). The IC₅₀ (inhibitory concentration 50%) of 0.1 nM for 191-D5-007 was measured using a cell-culture *in vitro* chemotaxis assay. Further truncation of both terminal stretches to four nucleotides (191-D5-010, Fig.7A).

Further derivatives of Type C SDF-1 binding nucleic acid 191-D5-001 (191-D5-017/ -024/ -029) bearing 5'- and 3'-terminal stretches of respectively four nucleotides also showed reduced binding affinity to SDF-1 in the competition pull-down binding assay vs. 191-D5-007 (Fig. 7B). Alternative 5'- and 3'-terminal stretches with a length of respectively five nucleotides were additionally tested, too (191-D5-017-29a, 191-D5-017-29b, 191-D5-019-29a, 191-D5-024-29a, 191-D5-024-29b). The generic formula of these derivatives for the 5'-terminal stretch is 'X_{S}SSSV' (Type C Formula-7-5') and for the 3'-stretch is 'BSSSX_{S}' Type C Formula-7-3'), whereby X_{S} is absent or ,S'. Two out of the five tested variants showed identical binding affinity to SDF-1 as 191-D5-007 (191-D5-024-29a, 191-D5-024-29b; Fig. 7B). The sequences of the 5'-terminal and 3'-terminal stretches of 191-D5-001-derivatives that show the best binding affinity to SDF-1 and comprise a 5'-terminal and 3'-terminal stretch of five nucleotides respectively (191-D5-007, 191-D5-024-29a, 191-D5-024-29b) can be summarized in a generic formula (5'-terminal stretch: 'SGGSR', Type C Formula-8-5'; 3'-terminal stretch: , YSCCS', Type C Formula-8-3').

Truncated derivatives of Type C SDF-1 binding nucleic acid 197-B2 were analyzed in a competitive pull-down binding assay vs. the original molecule 197-B2 and 191-D5-007 (Fig. 8). Using the competitive pull-down binding assay vs. 191-D5-007 it was shown that 197-B2 has the same binding affinity to SDF-1 as 191-D5-007. The 5'- and 3'-terminal stretches were shortened without loss of binding affinity from ten nucleotides (197-B2) each to five nucleotides each (197-B2-005) whereby the nucleotides of the 5'-terminal stretch and of the 3'-terminal stretch can completely hybridize to each other. If the 5'-terminal (**'GCGGG'**) and 3'-terminal (**'CCUGC'**) stretch of 197-B2-005 was substituted by **'GCCGG'** (5'-terminal stretch) and by **'CCGGC'** (3'-terminal stretch) of 197-B2-006, the binding affinity to SDF-1 fully persisted. Because 197-B2 and 191-D5-001 (and their derivatives) share the identical core nucleotide sequence (GGUUAGGGCUAGAAGUCG) and several derivatives of 191-D5 with 5'- and 3'-terminal stretches with a length of respectively four nucleotides were tested, a further truncation of the 5'- and 3'-terminal stretch was omitted. Two further derivatives were designed that comprise six nucleotides at the 5'- and 3'-end (5'- and 3'-terminal stretches) respectively. The binding affinity to SDF-1 of both molecules (197-B2-006-31a and 197-B2-006-31b) is the same as shown for 191-D5-007 and 197-B2-006 (Fig. 8). The sequences of the 5'-terminal and 3'-terminal stretches of 197-B2 derivatives that show the best binding affinity to SDF-1 and comprise a 5'-terminal and 3'-terminal stretch of five nucleotides respectively can be summarized in a generic formula (5'-terminal stretch: 'GCSGG', Type C Formula-9-5'; 3'-terminal stretch: ,CCKGC', Type C Formula-9-3').

Combining the preferred 5'- and 3'-stretches of truncated derivatives of Type C SDF-1 binding nucleic acids 191-D5-001 (5'-terminal stretch: 'SGGSR', Type C Formula-8-5'; 3'-terminal stretch: 'YSCCS', Type C Formula-8-3') and 197-B2 (5'-terminal stretch: 'GCSGG', Type C Formula-9-5'; 3'-terminal stretch: ,CCKGC', Type C Formula-9-3') the common preferred generic formula for the 5'-terminal and the 3'-terminal stretch is 'SSSSR' (5'-terminal stretch, Type C Formula-10-5') and 'YSBSS' (3'-terminal stretch: Type C Formula-10-3').

### 1.4 Further SDF-1 binding nucleic acids

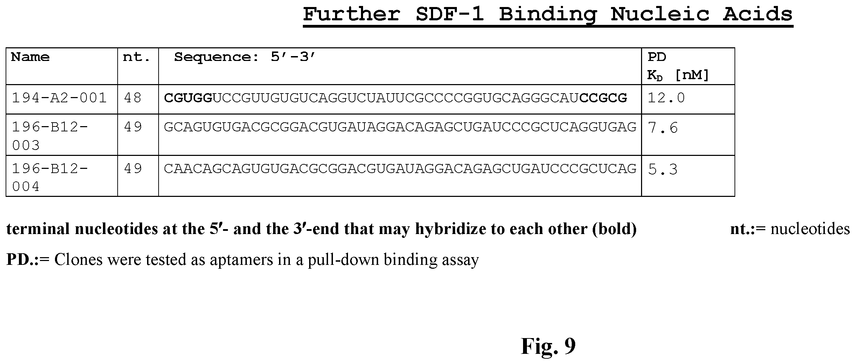

Additionally, further three SDF-1 binding nucleic acids that do not share the SDF-1 binding motifs of 'Type A', 'Type B' and 'Type C' were identified. There were analyzed as aptamers using the pull-down binding assay (Fig. 9).

It is to be understood that any of the sequences shown in Figs. 1 through 9 are nucleic acids according to the present invention, including those truncated forms thereof but also including those extended forms thereof under the proviso, however, that the thus truncated and extended, respectively, nucleic acid molecules are still capable of binding to the target.

The above SDF-1 binding nucleic acid molecules as well as Examples 2 to 8 are also disclosed in WO 2008/009437 A2.

### Example 2: 40kda-PEG and other Modification of SDF-binding Spiegelmers

In order to prolong the Spiegelmer's plasma residence time *in vivo*, the Spiegelmers 193-G2-012, 192-A10-008, 191-D5-007, 197-B2-006 and 197-B2-006-31b were covalently coupled to a 40 kDa polyethylene glycol (PEG) moiety at the 5'-end as described in chapter 3 (PEGylated-clones: 193-G2-012-5'-PEG, 192-A10-008-5'PEG, 191-D5-007-5'PEG, 197-B2-006-5'PEG and 197-B2-006-31b-5'PEG).

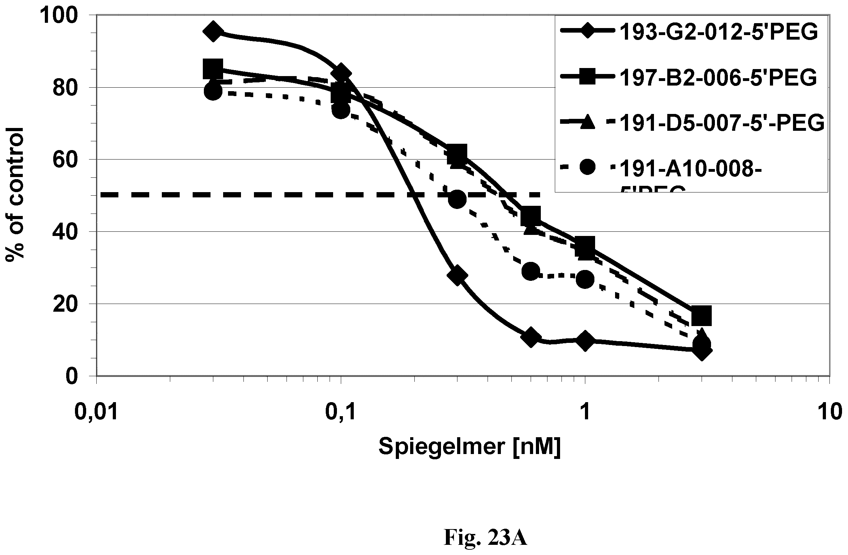

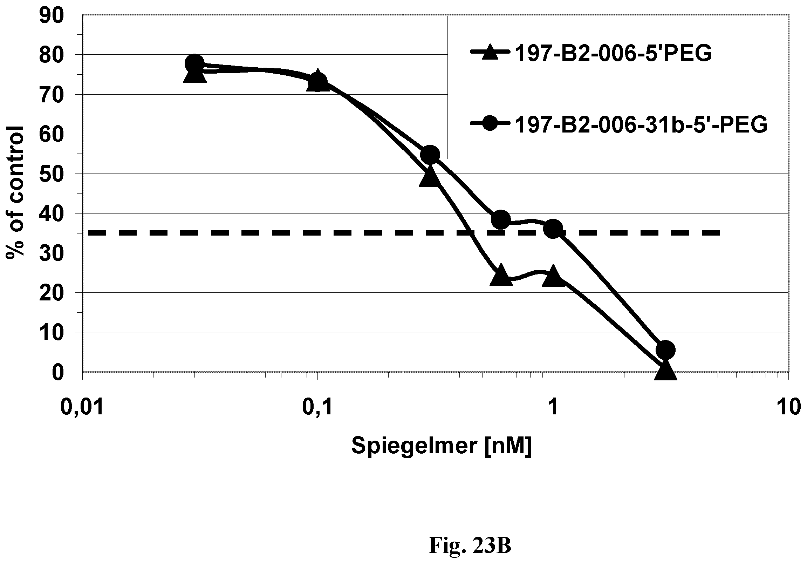

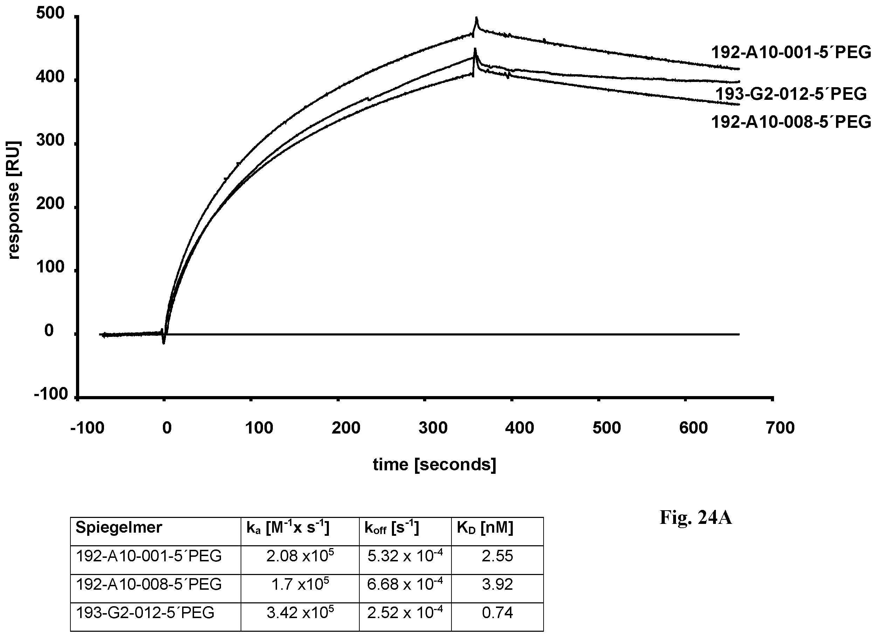

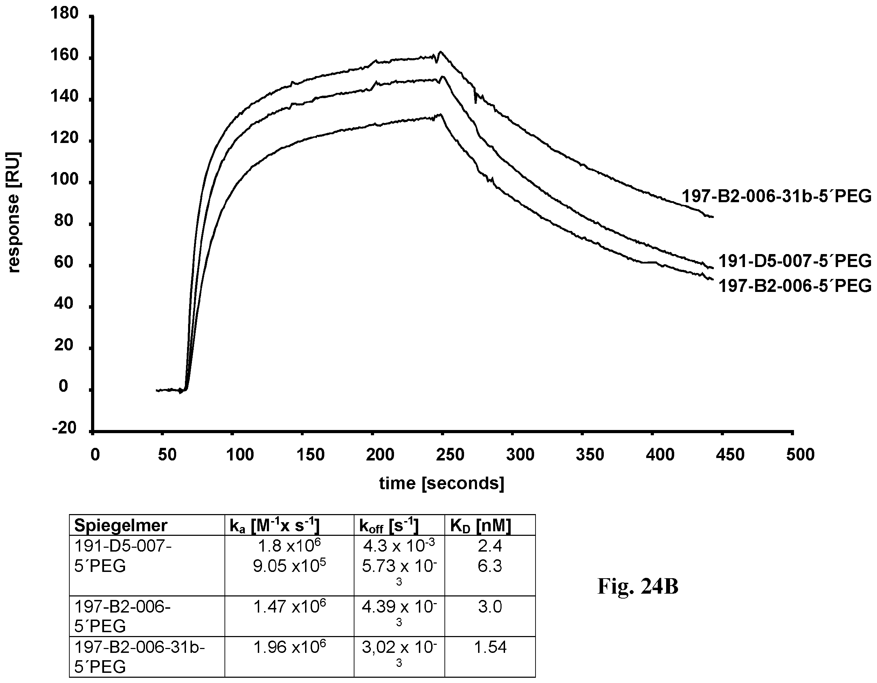

The PEGylated Spiegelmer molecules were analyzed in a cell culture *in vitro* TAX-assay (Chapter 5) and by plasmon resonance measurements using a Biacore (Chapter 6). All 40 kDa-PEG-modified Spiegelmers are still able to inhibit SDF-1 induced chemotaxis and to bind to SDF-1 in low nanomolar range (Fig. 23A, 23B, 24A and Fig. 24B).

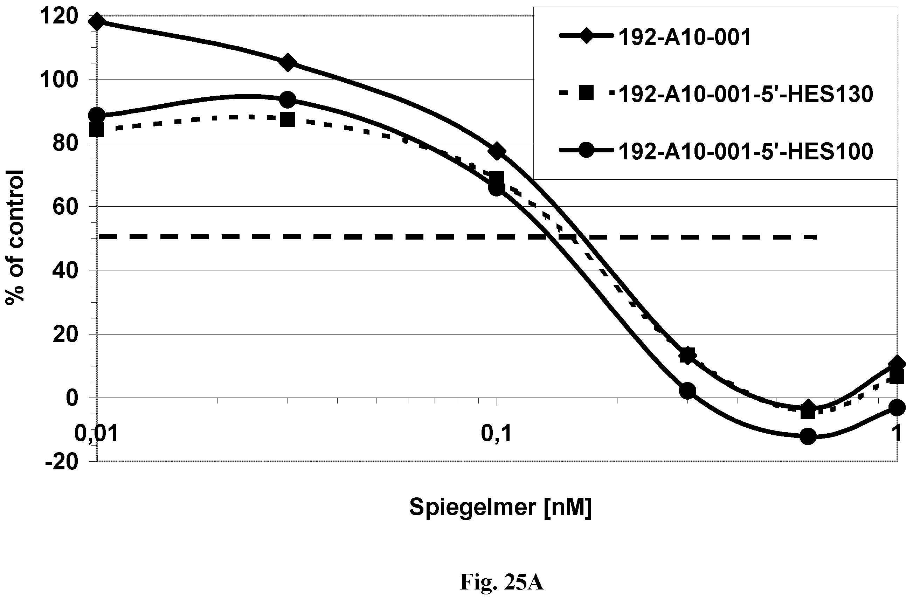

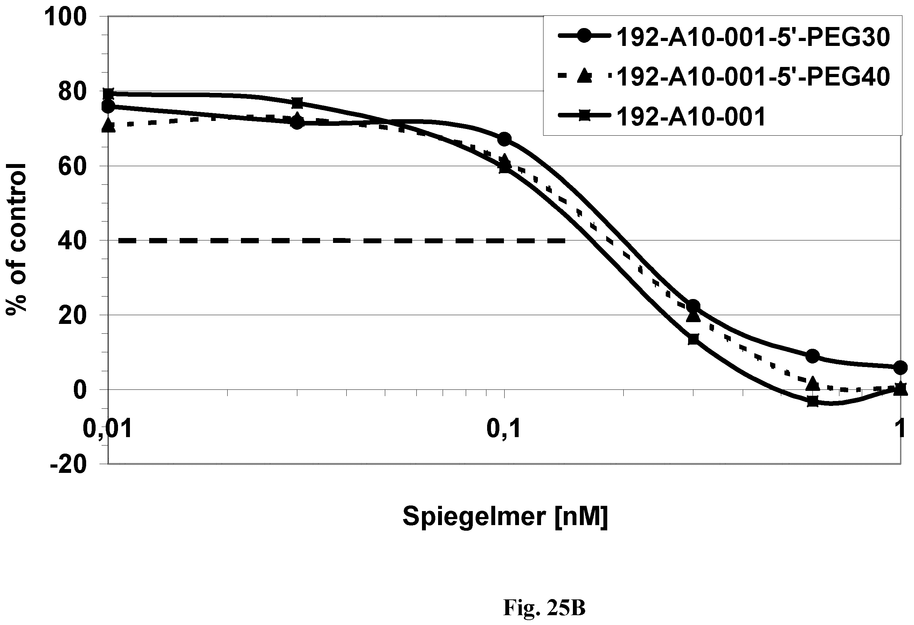

Additionally, SDF-binding Spiegelmer 192-A10-001 was modified with 40kDa-PEG, 30 kDa-PEG, 100kDa-HES or 130 kDa-HES (PEGylated-clones: 192-A10-001-5'PEG40, 192-A10-001-5'PEG30, 192-A10-001-5'HES100, 192-A10-001-5'HES130; coupling procedure in chapter 3). As depicted in Fig. 25A and Fig. 25B neither a PEG-moiety or a HES-moiety has an influence on Spiegelmers potency to inhibit SDF-1 induced chemotaxis.

### Example 3: Synthesis and derivatization of Aptamers and Spiegelmers

### 3.1 SMALL SCALE SYNTHESIS

Aptamers and Spiegelmers were produced by solid-phase synthesis with an ABI 394 synthesizer (Applied Biosystems, Foster City, CA, USA) using 2'TBDMS RNA phosphoramidite chemistry (Damha and Ogilvie, 1993). rA(N-Bz)-, rC(Ac)-, rG(N-ibu)-, and rU- phosphoramidites in the D- and L-configuration were purchased from ChemGenes, Wilmington, MA. Aptamers and Spiegelmers were purified by gel electrophoresis.

### 3.2 LARGE SCALE SYNTHESIS PLUS MODIFICATION

The Spiegelmers were produced by solid-phase synthesis with an AktaPilot100 synthesizer (Amersham Biosciences; General Electric Healthcare, Freiburg) using 2'TBDMS RNA phosphoramidite chemistry (Damha and Ogilvie 1993). L-rA(N-Bz)-, L-rC(Ac)-, L-rG(N-ibu)-, and L-rU- phosphoramidites were purchased from ChemGenes (Wilmington, MA, USA). The 5'-amino-modifier was purchased from American International Chemicals Inc. (Framingham, MA, USA). Synthesis of the Spiegelmers was started on L-riboG; L-riboC, L-riboA, L-riboU respectively modified CPG pore size 1000 Å (Link Technology, Glasgow, UK). For coupling (15 min per cycle), 0.3 M benzylthiotetrazole (American International Chemicals Inc., Framingham, MA, USA) in acetonitrile, and 3.5 equivalents of the respective 0.2 M phosphoramidite solution in acetonitrile was used. An oxidation-capping cycle was used. Further standard solvents and reagents for oligonucleotide synthesis were purchased from Biosolve (Valkenswaard, NL). The Spiegelmers were synthesized DMT-ON; after deprotection, it was purified via preparative RP-HPLC (Wincott, DiRenzo et al. 1995) using Source15RPC medium (Amersham). The 5'DMT-group was removed with 80% acetic acid (90 min at RT). Subsequently, aqueous 2 M NaOAc solution was added and the Spiegelmer was desalted by tangential-flow filtration using a 5 K regenerated cellulose membrane (Millipore, Bedford, MA).

### 3.3 PEGYLATION

In order to prolong the Spiegelmer's plasma residence time *in vivo*, the Spiegelmers were covalently coupled to a 40 kDa polyethylene glycol (PEG) moiety at the 5'-end.

For PEGylation (for technical details of the method for PEGylation see European patent application EP 1 306 382), the purified 5'-amino modified Spiegelmerd were dissolved in a mixture of H₂O (2.5 ml), DMF (5 ml), and buffer A (5 ml; prepared by mixing citric acid • H₂O [7 g], boric acid [3.54 g], phosphoric acid [2.26 ml], and 1 M NaOH [343 ml] and adding water to a final volume of 1 l; pH = 8.4 was adjusted with 1 M HCl).

The pH of the Spiegelmer solution was brought to 8.4 with 1 M NaOH. Then, 40 kDa PEG-NHS ester (Nektar Therapeutics, Huntsville, AL) was added at 37°C every 30 min in six portions of 0.25 equivalents until a maximal yield of 75 to 85% was reached. The pH of the reaction mixture was kept at 8 - 8.5 with 1 M NaOH during addition of the PEG-NHS ester.

The reaction mixture was blended with 4 ml urea solution (8 M), , and 4 ml buffer B (0.1 M triethylammonium acetate in H₂O) and heated to 95°C for 15 min. The PEGylated Spiegelmer was then purified by RP-HPLC with Source 15RPC medium (Amersham), using an acetonitrile gradient (buffer B; buffer C: 0.1 M triethylammonium acetate in acetonitrile). Excess PEG eluted at 5% buffer C, PEGylated Spiegelmer at 10 - 15% buffer C. Product fractions with a purity of >95% (as assessed by HPLC) were combined and mixed with 40 ml 3 M NaOAC. The PEGylated Spiegelmer was desalted by tangential-flow filtration (5 K regenerated cellulose membrane, Millipore, Bedford MA).

### 3.4 HESylation

In order to prolong the Spiegelmer's plasma residence time *in vivo*, the Spiegelmers were covalently coupled to Hydroxyl Ethyl Starch (HES) of various molecular weights of >130 kDa and substitution degree >0.5. The 5'-end of the Spiegelmer is the preferred site for conjugation.

For HESylation (for technical details of the method for Hesylation of nucleic acids see German Offenlegungsschrift DE 101 12 825 A1, and for D/L-nucleic acids PCT WO 02/080979 A2), the purified 5'-amino modified Spiegelmer was dissolved in sodium bicarbonate (0.3M, 1 ml) and the pH is adjusted to 8.5.

In respect to the Spiegelmer, a 5-fold excess of the free HES acid (3.3 mmol, Supramol, Rosbach, Germany) and di(N-succinimidyl) carbonate (3.3 mmol) were added to N,N-dimethylformamide (1 ml) to yield a solution of the activated N-hydroxysuccimide ester of HES. To dissolve all reactants the mixture was stirred briefly at 60°C, cooled to 25 °C and then stirred for 1.5 h at 25 °C. The solution of Spiegelmer was added to the solution of activated HES, and the resulting mixture was stirred at 25°C and pH 8.5. The reaction was monitored by analytical IEX-HPLC. Typically the conjugation proceeded to >75 % within 1hr.

For IEX-HPLC purification via Source 15Q medium (GE, Freiburg, Germany) the reaction mixture was blended with a 10fold-quantity of buffer A (1mM EDTA, 25mM Tris, 10 mM NaClO4 in water/acetonitrile 9:1, pH 4). Excess HES elutes at 5% buffer A (1mM EDTA, 25mM Tris, 500 mM NaClO4 in water/acetonitrile 9:1, pH 4), whereas the HES-Spiegelmer conjugate elutes at 20-30% buffer B. Product fractions with a purity of >95% (as assessed by HPLC) were combined and desalted by tangential-flow filtration (5 K regenerated cellulose membrane, Millipore, Bedford MA).

### Example 4: Determination of binding constants (Pull-down binding assay)

### 4.1 Direct pull-down binding assay

The affinity of aptamers to biotinlayted human D-SDF-1 was measured in a pull-down binding assay format at 37°C. Aptamers were 5'-phosphate labeled by T4 polynucleotide kinase (Invitrogen, Karlsruhe, Germany) using [γ-³²P]-labeled ATP (Hartmann Analytic, Braunschweig, Germany). The specific radioactivity of labeled aptamers was 200,000 - 800,000 cpm/pmol. Aptamers were incubated after de- and renaturation at 10, 20, 30 or 40 pM concentration at 37°C in selection buffer (20 mM Tris-HCl pH 7.4; 137 mM NaCl; 5 mM KCl; 1 mM MgCl₂; 1 mM CaCl₂; 0.1% [w/vol] Tween-20) together with varying amounts of biotinlayted human D-SDF-1 for 4 - 12 hours in order to reach equilibrium at low concentrations. Selection buffer was supplemented with 10 µg/ml human serum albumin (Sigma-Aldrich, Steinheim, Germany), and 10 µg/ml yeast RNA (Ambion, Austin, USA) in order to prevent adsorption of binding partners with surfaces of used plasticware or the immobilization matrix. The concentration range of biotinlayted human D-SDF-1 was set from 8 pM to 100 nM; total reaction volume was 1 ml. Peptide and peptide-aptamer complexes were immobilized on 1.5 µl Streptavidin Ultralink Plus particles (Pierce Biotechnology, Rockford, USA) which had been preequilibrated with selection buffer and resuspended in a total volume of 6 µl. Particles were kept in suspension for 30 min at the respective temperature in a thermomixer. Immobilized radioactivity was quantitated in a scintillation counter after detaching the supernatant and appropriate washing. The percentage of binding was plotted against the concentration of biotinlayted human D-SDF-1 and dissociation constants were obtained by using software algorithms (GRAFIT; Erithacus Software; Surrey U.K.) assuming a 1:1 stoichiometry.

### 4.2 Competitive pull-down binding assay

In order to compare different D-SDF-1 binding aptamers, a competitive ranking assay was performed. For this purpose, the most affine aptamer available was radioactively labeled (see above) and served as reference. After de- and renaturation it was incubated at 37°C with biotinlayted human D-SDF-1 in 1 ml selection buffer at conditions that resulted in around 5 - 10 % binding to the peptide after immobilization and washing on NeutrAvidin agarose or Streptavidin Ultralink Plus (both from Pierce) without competition. An excess of de- and renatured non-labeled D-RNA aptamer variants was added to different concentrations (e.g. 2, 10, and 50 nM) with the labeled reference aptamer to parallel binding reactions. The aptamers to be tested competed with the reference aptamer for target binding, thus decreasing the binding signal in dependence of their binding characteristics. The aptamer that was found most active in this assay could then serve as a new reference for comparative analysis of further aptamer variants.

### Example 5: Analysis of the inhibition of SDF-1-induced chemotaxis by SDF-1-binding Spiegelmers

Jurkat human T cell leukemia cells (obtained from DSMZ, Braunschweig) were cultivated at 37°C and 5% CO₂ in RPMI 1640 medium with Glutamax (Invitrogen, Karlsruhe, Germany) which contains 10% fetal bovine serum, 100 units/ml penicillin and 100 µg/ml streptomycin (Invitrogen, Karlsruhe, Germany). One day before the experiment, cells were seeded in a new flask with a density of 0.3 x 10⁶/ml (9 x 10⁶/30 ml) in standard medium (Invitrogen, Karlsruhe, Germany).

For the experiment, cells were centrifuged (5min at 300g), resuspended, counted and washed once with 15 ml HBH (Hanks balanced salt solution containing 1 mg/ml bovine serum albumin and 20 mM HEPES; Invitrogen, Karlsruhe, Germany). Then the cells were resuspended at 3 x 10⁶/ml or 1.33 x 10⁶/ml, depending on the type of filter plate used. Cells were then allowed to migrate through the porous membranes of the filter plates for several hours towards a solution containing SDF-1 and various amounts of Spiegelmer. Either Transwell plates and inserts with porous Polycarbonate membrane, 5 µm pore size (Corning; 3421) or MultiScreen MIC plates (Millipore, MAMIC5S10) were used.

### 5.1 Protocol for Transwell plates

The stimulation solutions (SDF-1 + various concentrations of Spiegelmer) were made up in 600 µl HBH in the lower compartments of the Transwell plates and incubated for 20 - 30 min. All conditions were made up at least twice. The inserts were transferred to the wells containing the stimulation solutions and 100 µl of a cell suspension with 3 x 10⁶/ml were added to the inserts (3 x 10⁵ cells/well). The cells were then allowed to migrate for 3 h at 37°C.

Thereafter, the inserts were removed and 60 µl resazurin (Sigma, Deisenhofen, Germany) working solution (440 µM in PBS; Biochrom, Berlin, Germany) were added to the wells (also to calibration wells). The plates were then incubated at 37°C for 2.5 to 3 h. After incubation, 200µl of each well were transferred to a black 96 well plate. Measurement of the fluorescence signals was done at 544 nm (excitation) and 590 nm (emission) in a Fluostar Optima multidetection plate reader (BMG, Offenburg, Germany).

### 5.2 Protocol for Millipore MultiScreen plates

The stimulation solutions (SDF-1 + various concentrations of Spiegelmer) were made up as 10X solutions in a 0.2 ml low profile 96-tube plate. 135 µl HBH were pipetted into the lower compartments of the *MultiScreen* plate and 15 µl of the stimulation solutions were added. All conditions were made up as triplicates. After 20 to 30 min the filter plate was inserted into the plate containing the stimulation solutions and 75 µl of a cell suspension with 1.33 x 10⁶/ml were added to the wells of the filter plate (1 x 10⁵ cells/well). The cells were then allowed to migrate for 3 h at 37°C.

Thereafter, the insert plate is removed and 20 µl resazurin working solution (440 µM in PBS) are added to the lower wells. The plates were then incubated at 37°C for 2.5 to 3 h.

After incubation, 100µl of each well were transferred to a black 96 well plate. Measurement of the fluorescence signals was performed as described above.

### 5.3 Evaluation

For evaluation, fluorescence values were corrected for background fluorescence (no cells in well). Then the difference between experimental conditions with and without SDF-1 was calculated. The value for the sample without Spiegelmer (SDF-1 only) was set 100% and the values for the samples with Spiegelmer were calculated as per cent of this. For a dose-response curve the per cent-values were plotted against Spiegelmer concentration and the IC₅₀-value (concentration of Spiegelmer at which 50% of the activity without Spiegelmer is present) was determined graphically from the resulting curve.

### 5.4 Results

### 5.4.1 Dose dependent stimulation of Jurkat cells by human SDF-1

Human SDF-1 was found to stimulate migration of Jurkat cells in a dose dependent manner, with half-maximal stimulation at about 0.3 nM (Fig. 11).

### 5.4.2 Dose dependent inhibition of human SDF-1 induced chemotaxis by SDF-1 binding Spiegelmers

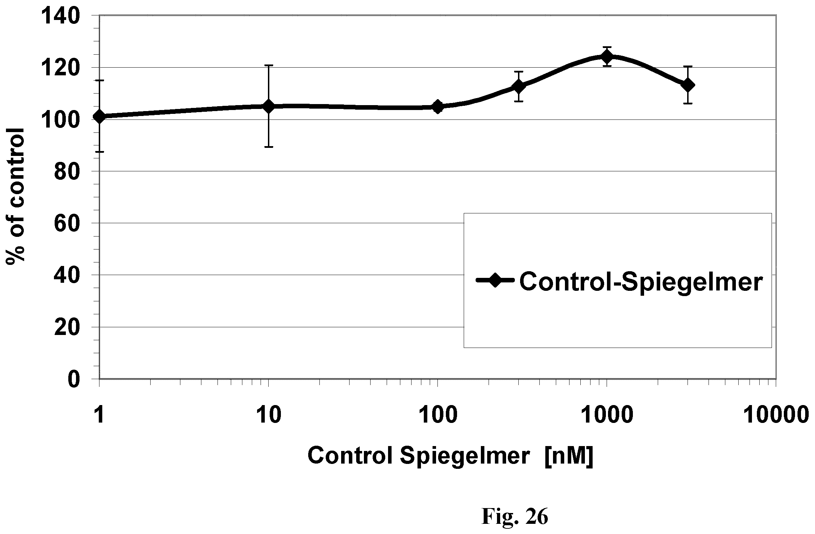

When cells were allowed to migrate towards a solution containing human SDF-1 plus increasing concentrations of SDF-1 binding Spiegelmers, dose-dependent inhibition was observed. The respective IC50s of the tested Spiegelmers are specified in Example 1. When an unspecific Control Spiegelmer was used instead of SDF-1 binding Spiegelmers, no inhibitory effect was observed up to 1 µM (Fig. 26).

### 5.4.3 Dose dependant inhibition of mouse SDF-1 induced chemotaxis by SDF-1 binding Spiegelmers

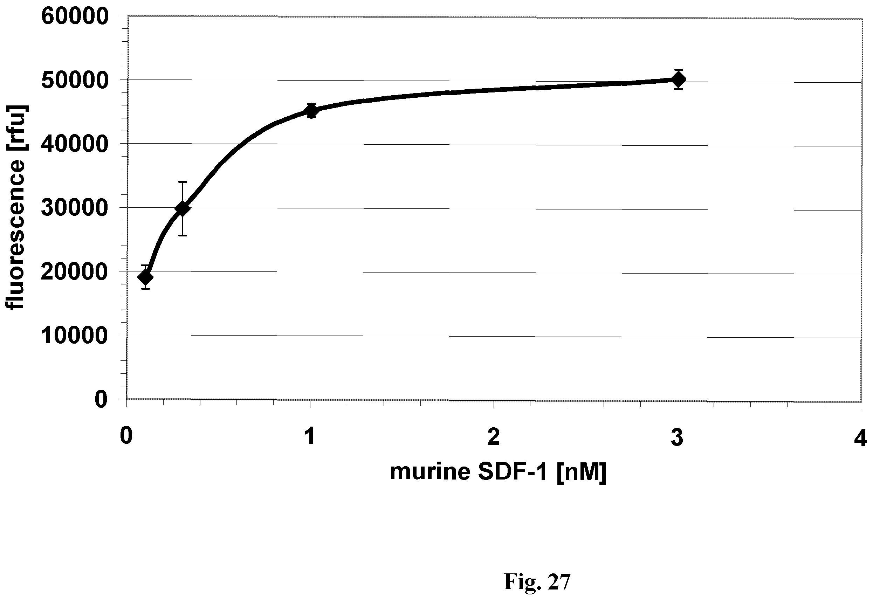

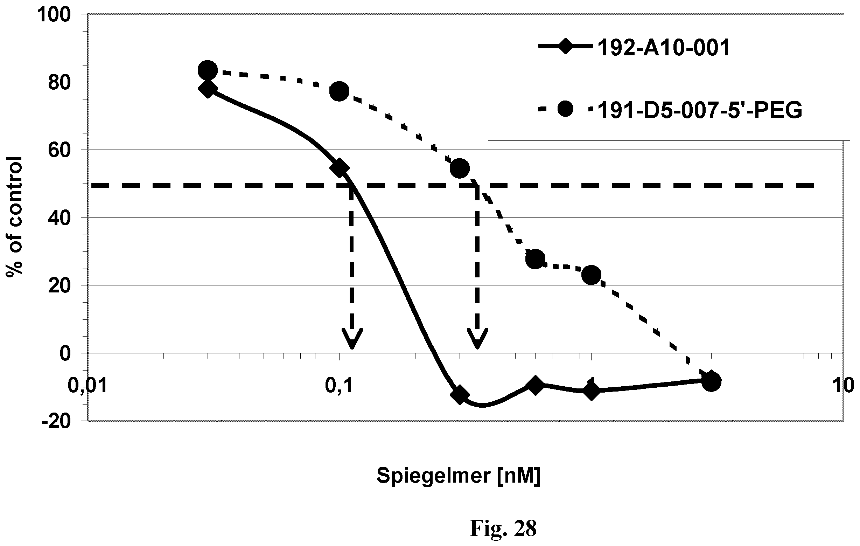

SDF-1 is well conserved across species: SDF-1 from mouse differs from human SDF-1a in one amino acid (isoleucin at position 18 instead of valine). Murine SDF-1 can stimulate chemotaxis of Jurkat cells (Fig. 27) and this action was found to be inhibited by Spiegelmers 192-A10-001 and 191-D5-007-5'-PEG with the same potency as in the case of human SDF-1 (Fig. 28).

### Example 6: Binding Analysis by Surface Plasmon Resonance Measurement

The Biacore 2000 instrument (Biacore AB, Uppsala, Sweden) was used to analyze binding of Spiegelmers to human SDF-1α. When coupling of SDF-1α was to be achieved via amine groups, SDF-1α was dialyzed against water for 1 - 2 h (Millipore VSWP mixed cellulose esters; pore size, 0.025 µM) to remove interfering amines. CM4 sensor chips (Biacore AB, Uppsala, Sweden) were activated before protein coupling by a 35-µl injection of a 1:1 dilution of 0.4 M NHS and 0.1 M EDC at a flow of 5 µl/min. Chemokine was then injected in concentrations of 0.1 - 1.5 µg/ml at a flow of 2 µl/min until the instrument's response was in the range of 1000 - 2000 RU (relative units). Unreacted NHS esters were deactivated by injection of 35 µl ethanolamine hydrochloride solution (pH 8.5) at a flow of 5 µl/min. The sensor chip was primed twice with binding buffer and equilibrated at 10 µl/min for 1 - 2 hours until the baseline appeared stable. For all proteins, kinetic parameters and dissociation constants were evaluated by a series of Spiegelmer injections at concentrations of 1000, 500, 250, 125, 62.5, 31.25, and 0 nM in selection buffer (Tris-HCl, 20 mM; NaCl, 137 mM; KCl, 5 mM; CaCl₂, 1 mM; MgCl₂, 1 mM; Tween20, 0.1% [w/v]; pH 7.4). In all experiments, the analysis was performed at 37°C using the Kinject command defining an association time of 180 and a dissociation time of 360 seconds at a flow of 10 µl/min. Data analysis and calculation of dissociation constants (K_{D}) was done with the BIAevaluation 3.0 software (BIACORE AB, Uppsala, Sweden) using the Langmuir 1:1 stochiometric fitting algorithm.

### Example 7: Inhibition of [¹²⁵J]-SDF-1-binding to CXCR4 expressing cells by SDF-1-binding Spiegelmers

### 7.1 Method

A cDNA clone coding for human CXCR4-receptor (NM_003467.2) was purchased from OriGene Technologies (Rockville, MD) and cloned into the pCR3.1-vector (Invitrogen, Karlsruhe, Germany). The resulting vector was transfected into CHO-K1 cells (DSMZ, Braunschweig, Germany) using Lipofectamin 2000 (Invitrogen) and stable expressing cell lines were selected by treatment with geneticin. Expression of receptors was verified by RT-PCR.

For binding assays CXCR4-expressing cells were seeded into polylysine-coated 24-well plates at a cell density of 1 x 10⁵ cells/well and cultivated overnight at 37°C and 5% CO₂ in CHO-Ultra medium (Cambrex, Verviers, Belgium) containing 50 units/ml penicillin, 50 µg/ml streptomycin and 0.5 mg/ml geneticin.

For the binding experiment, the medium was removed, and the cells were washed once with Hanks balanced salt solution, additionally containing 20 mM HEPES, 1 mg/ml bovine serum albumin, 0.1 mg/ml bacitracin (HBB). Then the cells were incubated in 0.2 ml HBB for 1 h at room temperature together with 50 pM [¹²⁵J]-SDF-1 (PerkinElmer, Rodgau, Germany) and varying concentrations of Spiegelmer.

Non-specific binding was determined by adding unlabeled human SDF-1 (R & D Systems, Wiesbaden, Germany) to a final concentration of 0.5µM to several wells.

After the incubation period the supernatant was removed, and the wells were washed 3 times with ice-cold HBB. Thereafter the cells were lysed with 0.1 ml 0.1 M NaOH. Lysates were transfered into szintillation vials and after addition of 4 ml Unisafe 1 Liquid Szintillation cocktail (Zinsser, Frankfurt, Germany) were counted in a Beckman LS6500 szintillation counter.

Since the values for non-specific binding (binding in the presence of high amont of unlabeled SDF-1) were somewhat higher than the values for total binding in the presence of high concentrations (500 pM) of Spiegelmer, the difference between maximal binding ("max") and binding in the presence of 500 pM Spiegelmer was used for calculation of IC₅₀-values.

### 7.2 Results

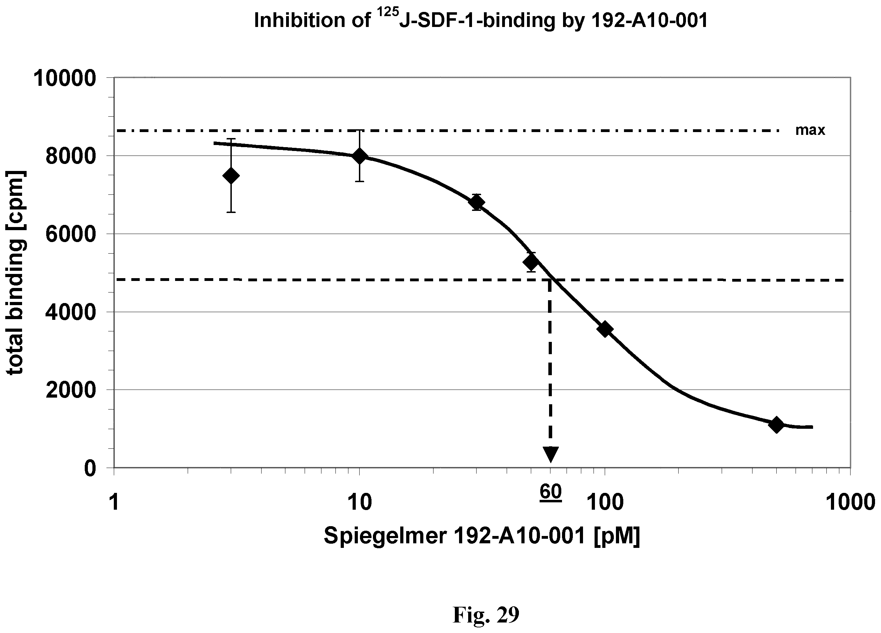

Plotting bound [¹²⁵J]-SDF-1 against Spiegelmer concentration revealed that binding of SDF-1 could be blocked by Spiegelmer 192-A10-001 with an IC₅₀ of about 60 pM (Fig. 29).

### Example 8: Inhibition of SDF-1-induced MAP-kinase activation by SDF-1-binding Spiegelmers

### 8.1 Method

CXCR4-expressing CHO cells were seeded in 6-well plates at a density of 0.5 x 10⁶ cells/well and cultivated for about three hours at 37°C and 5% CO₂ in CHO-Ultra medium (Cambrex, Verviers, Belgium) containing 50 units/ml penicillin, 50 µg/ml streptomycin and 0.5 mg/ml geneticin. After cell attachment the medium was removed and replaced by Ham's F12 medium containing 50 units/ml penicillin, 50 µg/ml streptomycin. Cells were then incubated overnight at 37°C and 5% CO₂. Three hours before stimulation the medium was replaced once more by fresh Ham's F12 medium. Cells were stimulated with human1 nM SDF-1 and various amounts of Spiegelmer for 5 or 10 minutes. Thereafter the medium was removed, and the cells were quickly washed once with 1 ml ice-cold phosphate buffered saline (PBS), followed by lysis with SDS-sample buffer (Tris/HCl, pH 6.8, 62.5 mM; glycerol, 10%; SDS, 2%; bromophenolblue, 0.01 %; beta-mercaptoethanol, 5%). 1 µl 0.5 u/µl Benzonase (Merck, Darmstadt, Germany) was added to each well and after incubation for 5 to 10 min at room temperature, lysates were transferred to Eppendorf tubes, incubated at 95°C for 5 min and stored at -20°C until further analysis.

25 µl of the lysates were separated on 10% denaturing SDS-polyacrylamide gels. Proteins were then transferred by electroblotting onto HybondECL nitrocellulose membranes (Amersham/GE Healthcare, Munich, Germany). After blotting, the membranes were stained with Ponceau-red (0.2% in 3% trichloroacetic acid) for control of protein loading and transfer and then blocked by incubation in TBS-T (Tris-buffered saline (20 mM Tris/HCl, pH 7.6, 137 mM NaCl) with 0.1% Tween 20) containing 10% nonfat dried milk at 2 - 8°C overnight.

The membrane was then incubated with a rabbit anti-Phospho-MAP-kinase antibody (1:1000 in 10% milk in TBS-T) for 2 h at room temperature. After washing three times for 5 min with TBS-T, the membrane was incubated with anti-rabbit-IgG-HRP-conjugate (1:2000 in 10% milk in TBS-T) for 1 h at room temperature. Then the membrane was again washed three times for 5 min with TBS-T, followed by incubation for 1 min in LumiGlo^{R} chemiluminescent reagent. Luminescence was detected by exposure to Hyperfilm^{™}ECL chemiluminescence films (Amersham/GE Healthcare) for 30 seconds to 2 minutes. The antibodies and the luminescence detection reagent were components of the PhosphoPlus p44/42 MAP Kinase (Thr202/Tyr204) Antibody kit from Cell Signaling Technology (New England Biolabs, Frankfurt a.M., Germany)

### 8.2 Results

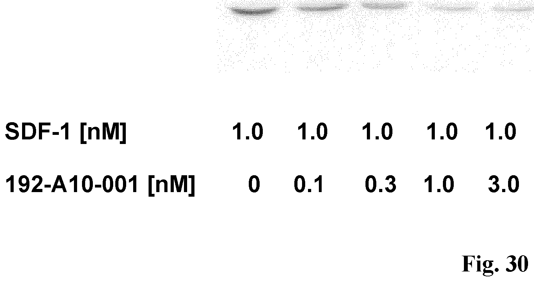

Stimulation of CXCR4-expressing cells with 1 nM human SDF-1 for 5 min led to a profound stimulation of MAP-kinase, indicated by an increase in intensity of the band reflecting activated MAP-kinase. This activation of MAP-kinase could be dose-dependently inhibited by Spiegelmer 191-A10-001 (Fig. 30).

### Example 9: Enhanced anti-tumor activity of TCR-T cells in SYO1-A2-Luc+ synovial sarcoma xenografts through combined treatment with CXCL12 or CXCR4 inhibition and IFNγ

### Materials and Methods

**Mouse xenograft model** - In order to assess the anti-tumor activity of TCR-T cells combined with CXCL12 or CXCR4 inhibition and IFNγ in a mouse model of synovial sarcoma, a xenograft experiment was conducted. A xenograft involves transplanting human tumor cells into immunocompromised mice, such as athymic nude mice, which lack a thymus and, consequently, a functional immune system. This allows human cells to grow as a tumor without being rejected by the mouse's immune system. This setup also enables the study of transferred T cells, as these will be the only T cells present in the model.

**SYO1-A2-Luc+ cell line generation** - The human synovial sarcoma tumor cell line SYO1 (Kawai, Naito et al. 2004) was chosen for the xenograft experiment due to its well-characterized genetic background and its relevance to synovial sarcoma research. Its ability to express specific antigens and respond to targeted therapies makes it an ideal model for studying the interactions between tumor cells and immune cells.

The cell line was transfected to express luciferase (Luc+), which allows for easy tracking and imaging of the cells in vivo: On Day 1, approximately 150,000 cells were seeded into each well of a 6-well tissue culture plate. Each well was supplemented with 2 mL of culture medium consisting of RPMI 1640, 10% fetal bovine serum (FBS), 1% penicillin-streptomycin (Pen/Strep), 1% sodium pyruvate, and 1% non-essential amino acids. On Day 2, 50 µL of firefly luciferase lentivirus (Luc-P2A-eGFP, BPS Bioscience #79980-P) was added to each well containing cells, along with 5 µg/mL polybrene to enhance transduction efficiency. The plate was gently swirled to ensure thorough mixing of the cells and virus. Subsequently, the plate was incubated at 37°C in a humidified atmosphere containing 5% CO₂ for a period of 48-72 hours. On Day 3, the transduction efficiency was assessed by measuring the percentage of GFP-positive cells using a Countess cell counter (Invitrogen).

Additionally, the cell line was engineered to express the HLA type HLA-A*02:01, a specific allele of the human leukocyte antigen (HLA) system as described in Pollack 2012 (Pollack, Jungbluth et al. 2012). The HLA system plays a crucial role in the immune system by presenting peptide antigens to T cells. This particular allele is known for its ability to present the NY-ESO-1₁₅₇₋₁₆₅ epitope, a cancer-testis antigen often expressed in various tumors, including synovial sarcoma. This setup is particularly useful for evaluating the efficacy of T cell receptor (TCR)-based therapies targeting the NY-ESO-1₁₅₇₋₁₆₅ epitope such as the 1G4 TCR used in this xenograft experiment (TCR-T cells).

**TCR-T cell generation** - 1x10⁶ /well human PBMCs (STEMCELL Technologies, Cat#70025) were added to 24 well plate with human T-Activator CD3/CD28 (STEMCELL, Cat #10971) in CTL media (500 mL RPMI + 10% FBS + 1% Penicillin & Streptomycin + 1% L-Glutamine + 1% Non-Essential Amino Acids + 1% Sodium Pyruvate + 50 mM β-mercaptoethanol) supplemented with 200 IU/mL recombinant human IL-2 (Cat# 200-02-1MG, Peprotech; amino acid sequence MAPTSSSTKK TQLQLEHLLL DLQMILNGIN NYKNPKLTRM LTFKFYMPKK ATELKHLQCL EEELKPLEEV LNLAQSKNFH LRPRDLISNI NVIVLELKGS ETTFMCEYAD ETATIVEFLN RWITFAQSII STLT (SEQ ID NO: 246 )). After 2 days, Lenti-NY-ESO-1 TCR (1G4-αLY)-VP virus (Creative Biolabs, VP-TCR-C048) was added to transfect activated T cells, spin the plate for 1 hr, room temperature, 800 x g, incubated at 37 °C in a 5% CO₂. 24 hours later, the medium was replaced and maintained with CTL media. Transduced T cells were examined for NY-ESO-1 TCR surface expression using HLA-A2 NY-ESO-1-APC tetramer (produced by Immune Monitoring Lab, Fred Hutchinson Cancer Research Center) by flow cytometry after 5 days. Cell concentration was adjusted to 0.5-1x10⁶ cells/mL with CTL media supplemented with 200 IU/mL rhIL2 until day 14.

***In vivo* experiment** - 3x10⁶ SYO1-A2-Luc+ human sarcoma cells were resuspended in 100 µl PBS and subcutaneously injected at the right flank to four- to six-week-old female athymic nude mice (NU/J Strain #002019, the Jackson laboratory, ME, USA) to induce a tumor. Once tumors reached a diameter of 3 mm as determined by caliper measurement, the mice were randomly grouped into two subgroups with and without TCR-T cell treatment as described in the following:
3x10⁶ human NY-ESO-1 specific TCR T cells were resuspended in 100µl DPBS without Ca²⁺ and Mg²⁺ and intravenously injected by tail-vein. After one week, human IFNγ (2x10⁵ unit, intraperitoneally in 100 µl DPBS twice per week, Cat# 300-02, Peprotech; Amino acid sequence MQDPYVKEAE NLKKYFNAGH SDVADNGTLF LGILKNWKEE SDRKIMQSQI VSFYFKLFKN FKDDQSIQKS VETIKEDMNV KFFNSNKKKR DDFEKLTNYS VTDLNVQRKA IHELIQVMAE LSPAAKTGKR KRSQMLFQGR RASQ (SEQ ID NO:245)), AMD3100 (5 mg/kg, intraperitoneally in 100 µl DPBS twice per week, Cat# S8030, Selleckchem), NOX-A12 (20 mg/kg, subcutaneously in 5% sterile glucose twice per week), or combinations as depicted in the table below, were applied. Tumor sizes were measured with calipers twice weekly. Tumor volume was calculated using the formula V=1 x w² /2 (with 1 being length and w being width). The mice were sacrificed by CO₂ asphyxiation.

Treatment groups and their respective schedules are summarized in the following table:

| **Treatment Group** | **Mouse #** | **Treatment Schedule** |
|---|---|---|
| Control | 5 | No treatment |
| AMD3100 | 5 | 5 mg/kg, intraperitoneally twice per week |
| NOX-A12 | 5 | 20 mg/kg, subcutaneously twice per week |
| IFNγ | 5 | 2x10^5 units, intraperitoneally twice per week |
| IFNγ + AMD3100 | 5 | IFNγ: 2x10^5 units, intraperitoneally twice per week; AMD3100: 5 mg/kg, intraperitoneally twice per week |
| IFNγ + NOX-A12 | 5 | IFNγ: 2x10^5 units, intraperitoneally twice per week; NOX-A12: 20 mg/kg, subcutaneously twice per week |
| TCR-T | 5 | 3x10^6 human NY-ESO-1 specific TCR T cells, intravenously via tail-vein |
| TCR-T + AMD3100 | 5 | TCR-T: 3x10^6 human NY-ESO-1 specific TCR T cells, intravenously via tail-vein; AMD3100: 5 mg/kg, intraperitoneally twice per week |
| TCR-T + NOX-A12 | 5 | TCR-T: 3x10^6 human NY-ESO-1 specific TCR T cells, intravenously via tail-vein; NOX-A12: 20 mg/kg, subcutaneously twice per week |
| TCR-T + IFNγ | 5 | TCR-T: 3x10^6 human NY-ESO-1 specific TCR T cells, intravenously via tail-vein; IFNγ: 2x10^5 units, intraperitoneally twice per week |
| TCR-T + IFNγ + AMD3100 | 5 | TCR-T: 3x10^6 human NY-ESO-1 specific TCR T cells, intravenously via tail-vein; IFNγ: 2x10^5 units, intraperitoneally twice per week; AMD3100: 5 mg/kg, intraperitoneally twice per week |
| TCR-T + IFNγ + NOX-A12 | 5 | TCR-T: 3x10^6 human NY-ESO-1 specific TCR T cells, intravenously via tail-vein; IFNγ: 2x10^5 units, intraperitoneally twice per week; NOX-A12: 20 mg/kg, subcutaneously twice per week |

**Bioluminescence in vivo imaging** - Animals were anesthetized using isoflurane throughout the imaging procedure to ensure minimal movement and stress. D-Luciferin (GoldBio, Catalog Number: LUCK-100) was dissolved in DPBS without Ca²⁺ and Mg²⁺. Each animal received an intraperitoneal injection of 3 mg of D-Luciferin in 200 µL of the prepared solution. Bioluminescence imaging was performed using the IVIS system (SII Lago, Spectral Instruments Imaging) featuring a highly sensitive, air-cooled CCD camera capable of detecting low levels of bioluminescence. The system includes a heated platform to maintain animal body temperature and ensure consistent enzyme kinetics. Animals were placed in the imaging chamber 10 minutes post D-Luciferin injection. Images were acquired and analyzed using the Aura Imaging Software.

### Results:

The primary aim of this study was to evaluate whether CXCL12 or CXCR4 inhibition combined with IFNγ could enhance the anti-tumor efficacy of transferred TCR-T cells in a synovial sarcoma xenograft using the SYO1-A2-Luc+ cell line.

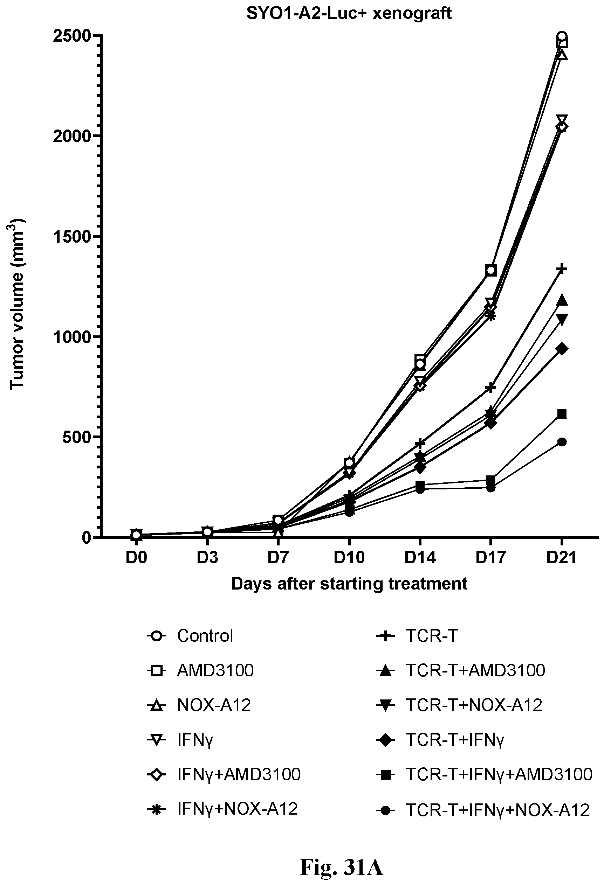

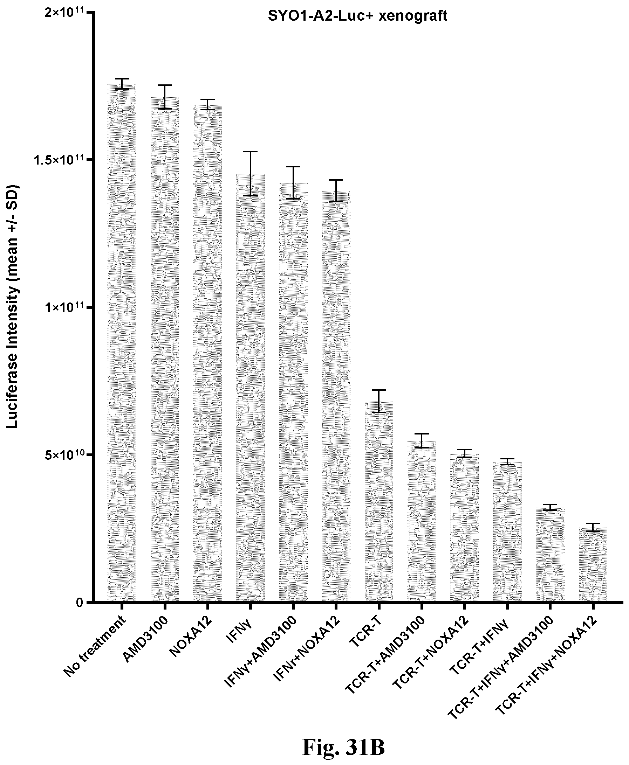

IFNγ as a single agent modestly restricted tumor growth, with noticeable effects by day 21. TCR-T cells alone reduced tumor growth by approximately 50% at day 21. When combined with TCR-T cells, IFNγ, AMD3100, or NOX-A12 each enhanced the anti-tumor activity of the TCR-T cells. Notably, the combination of IFNγ with either AMD3100 or NOX-A12 inhibited tumor growth more effectively than any of the agents alone. The most pronounced anti-tumor effect was observed with the combination of NOX-A12 and IFNγ with TCR-T cells, nearly eradicating tumor growth (Fig. 31 A). The results were confirmed by luciferase intensity measurements (Fig. 31 B).

### Example 10: Increased TCR-T cell infiltration in SYO1-A2-Luc+ synovial sarcoma xenografts through combined treatment with CXCL12 or CXCR4 inhibition and IFNγ

### Materials and Methods

**Tissue processing and analysis** - Freshly harvested tumors were sectioned into three pieces, ensuring each piece included the tumor periphery. The sections were then placed in formalin for a minimum of 24-48 hours before being processed into FFPE (formalin-fixed paraffin-embedded) blocks. The FFPE blocks were subsequently cut and stained at Northwestern's Mouse Histopathology Lab. Prior to embedding, the tissue was trimmed to fit the embedding molds appropriately. The tissue was fixed in formalin at a volume at least 20 times greater than the tissue volume, with a recommended fixation duration of 24-48 hours. Tissue sections were cut at a thickness of 4 µm, with one section per slide. IHC staining was performed using the anti-CD3 epsilon antibody (Clone SP7, abcam # ab16669) on a Roche Ventana Discovery XT IHC/ISH Slide Staining Instrument System according to the manufacturer's protocol. Briefly, paraffin-embedded tissue samples were deparaffinized. Cell conditioning was performed using Conditioner #1 with Mild CC1 protocol. The anti-CD3 epsilon antibody (Clone ID: SP7, Rabbit Monoclonal) was diluted 1:75 in Antibody Diluent (ab227819) and manually applied (100 µL) to the slides. The slides were incubated with the primary antibody for 32 minutes at 37°C. Following primary antibody incubation, OmniMap anti-Rb HRP (Multimer HRP) was applied and incubated for 8 minutes. Counterstaining was performed using hematoxylin II for 4 minutes, followed by a post-counterstain incubation with Bluing Reagent for 4 minutes. The stained slides were analyzed using the Universal Procedure Software provided with the Roche Ventana Discovery XT IHC/ISH Slide Staining Instrument System.

For flow cytometric assessment of splenocytes, mouse spleens were homogenized on a wetted 70 µm filter using the plunger base of a 3 mL syringe while pouring DMEM. Single splenocytes were collected in a 50 mL tube. Cell concentrations were adjusted to 1 million cells per 100 µL of FACS buffer (Invitrogen, # 00-4222-26), and 100 µL of the cell suspension was pipetted into 5 mL flow cytometry tubes for staining. An antibody cocktail (50 µL) was added, and the mixture was incubated at 4°C for 30 minutes. The following monoclonal antibodies were used: anti-human CD45 (clone 2D1), anti-human CD3 (clone HIT3a), and anti-mouse CD45 (clone 30-F11), all obtained from BioLegend. Subsequently, cells were washed by adding 2 mL of FACS buffer, then resuspended in 250 µL of FACS buffer containing 2% formaldehyde. Flow cytometric analysis was performed using a BD Biosciences Fortessa flow cytometer, and the resulting data were analyzed using FlowJo software.

### Results:

To enable TCR-T cells to effectively kill tumor cells, they must come into direct contact with the tumor cells. Efficient infiltration of TCR-T cells into the tumor is crucial for this process. Therefore, it is essential to analyze TCR-T cell infiltration and expansion within the tumor environment. This involves assessing how well the TCR-T cells penetrate the tumor tissue and their ability to proliferate once inside the body. Tumors from the xenograft experiment in Example 9 were harvested for this purpose. Immunohistochemistry (IHC) staining for specific markers, such as human CD3, was used to visualize and quantify TCR-T cell infiltration in the tumors. Additionally, the expansion of these transferred TCR-T cells was evaluated by analyzing spleen tissues from mice treated in Example 9 for the presence of human T cell markers such as human CD3 and CD45.

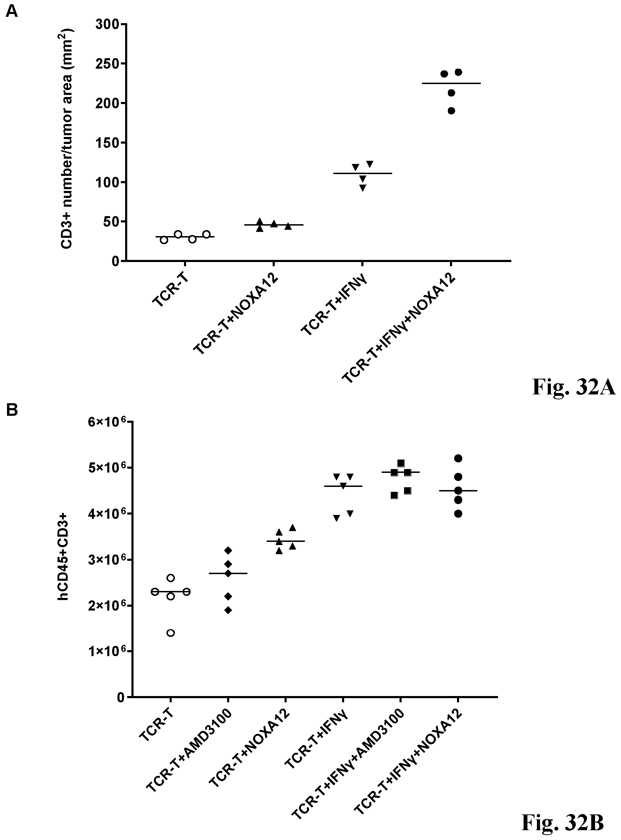

Infiltration of TCR-T cells, assessed by IHC staining for human CD3 in the tumors, was increased by IFNγ and even more so by the addition of NOX-A12. Tumors of mice treated with TCR-T cells + NOX-A12 showed increased infiltration versus mice receiving TCR-T cells alone. The combined addition of IFNγ increased T cell infiltration over AMD3100 alone and NOX-A12 alone (Fig. 32 A). To evaluate the expansion of transferred T cells in the xenograft model, spleens were digested at the time of sacrifice and evaluated for expression of human CD3 and CD45. Counts of TCR-T cells in the group treated with NOX-A12 were increased. The addition of IFNγ increased T cell expansion of both AMD3100 alone and NOX-A12 alone (Fig. 32 B).

### Example 11: Enhanced anti-tumor activity of TCR-T cells in Yamato-Luc+ synovial sarcoma xenografts through combined treatment with CXCL12 or CXCR4 inhibition and IFNγ

### Materials and Methods

**Mouse xenograft model** - In order to assess the anti-tumor activity of TCR-T cells combined with CXCL12 or CXCR4 inhibition and IFNγ in a mouse model of synovial sarcoma, a xenograft experiment was conducted as described in Example 9, but this time using the Yamato-Luc+ cell line.

**Yamato-Luc+ cell line generation** - The human synovial sarcoma tumor cell line Yamato (Naka, Takenaka et al. 2010) was selected for the xenograft experiment due to its well-characterized genetic background and relevance to synovial sarcoma research. Yamato has native expression of HLA-A*0206 (closely related to HLA-A*0201) and presents the same NY-ESO-1₁₅₇₋₁₆₅ (SLLMWITQV (SEQ ID NO: 247)) epitope recognized by the 1G4 TCR. Its ability to express specific antigens and respond to targeted therapies makes it an ideal model for studying the interactions between tumor cells and immune cells. The cell line was transfected to express luciferase (Luc+), which allows for easy tracking and imaging of the cells in vivo, as described in Example 9.

***In vivo* experiment** - 3x10⁶ Yamato-Luc+ human sarcoma cells were resuspended in 100 µl PBS and subcutaneously injected at the right flank to four- to six-week-old female athymic nude mice (NU/J Strain #002019, the Jackson laboratory, ME, USA) to induce a tumor. Once tumors reached a diameter of 3 mm as determined by caliper measurement, the mice were randomly grouped into two subgroups with and without TCR-T cell treatment as described in the following:
3x10⁶ human NY-ESO-1 specific TCR T cells (generated as described in Example 9) were resuspended in 100 µl DPBS without Ca²⁺ and Mg²⁺ and intravenously injected by tail vein. After one week, human IFNγ (2x10⁵ units, intraperitoneally in 100 µl DPBS twice per week, Cat# 300-02, Peprotech), AMD3100 (5 mg/kg, intraperitoneally in 100 µl DPBS twice per week, Cat# S8030, Selleckchem), NOX-A12 (20 mg/kg, subcutaneously in 5% sterile glucose twice per week), or combinations as depicted in the table below, were applied. Tumor sizes were measured with calipers twice weekly. Tumor volume was calculated using the formula V=l x w² /2 (with 1 being length and w being width). Bioluminescent imaging was performed at Day 21 as described in Example 9. The mice were sacrificed by CO₂ asphyxiation.

Treatment groups and their respective schedules are summarized in the following table:

| **Treatment Group** | **Mouse #** | **Treatment Schedule** |
|---|---|---|
| Control | 5 | No treatment |
| AMD3100 | 5 | 5 mg/kg, intraperitoneally twice per week |
| NOX-A12 | 5 | 20 mg/kg, subcutaneously twice per week |
| IFNγ | 5 | 2x10^5 units, intraperitoneally twice per week |
| IFNγ + AMD3100 | 5 | IFNγ: 2x10^5 units, intraperitoneally twice per week; AMD3100: 5 mg/kg, intraperitoneally twice per week |
| IFNγ + NOX-A12 | 5 | IFNγ: 2x10^5 units, intraperitoneally twice per week; NOX-A12: 20 mg/kg, subcutaneously twice per week |
| TCR-T | 5 | 3x10^6 human NY-ESO-1 specific TCR T cells, intravenously via tail-vein |
| TCR-T + AMD3100 | 5 | TCR-T: 3x10^6 human NY-ESO-1 specific TCR T cells, intravenously via tail-vein; AMD3100: 5 mg/kg, intraperitoneally twice per week |
| TCR-T + NOX-A12 | 5 | TCR-T: 3x10^6 human NY-ESO-1 specific TCR T cells, intravenously via tail-vein; NOX-A12: 20 mg/kg, subcutaneously twice per week |
| TCR-T + IFNγ | 5 | TCR-T: 3x10^6 human NY-ESO-1 specific TCR T cells, intravenously via tail-vein; IFNγ: 2x10^5 units, intraperitoneally twice per week |
| TCR-T + IFNγ AMD3100 + | 5 | TCR-T: 3x10^6 human NY-ESO-1 specific TCR T cells, intravenously via tail-vein; IFNγ: 2x10^5 units, intraperitoneally twice per week; AMD3100: 5 mg/kg, intraperitoneally twice per week |
| TCR-T + IFNγ + NOX-A12 | 5 | TCR-T: 3x10^6 human NY-ESO-1 specific TCR T cells, intravenously via tail-vein; IFNγ: 2x10^5 units, intraperitoneally twice per week; NOX-A12: 20 mg/kg, subcutaneously twice per week |

### Results:

The primary aim of this study was to evaluate whether CXCL12 or CXCR4 inhibition combined with IFNγ could enhance the anti-tumor efficacy of transferred TCR-T cells in a synovial sarcoma xenograft using the Yamato-Luc+ cell line.

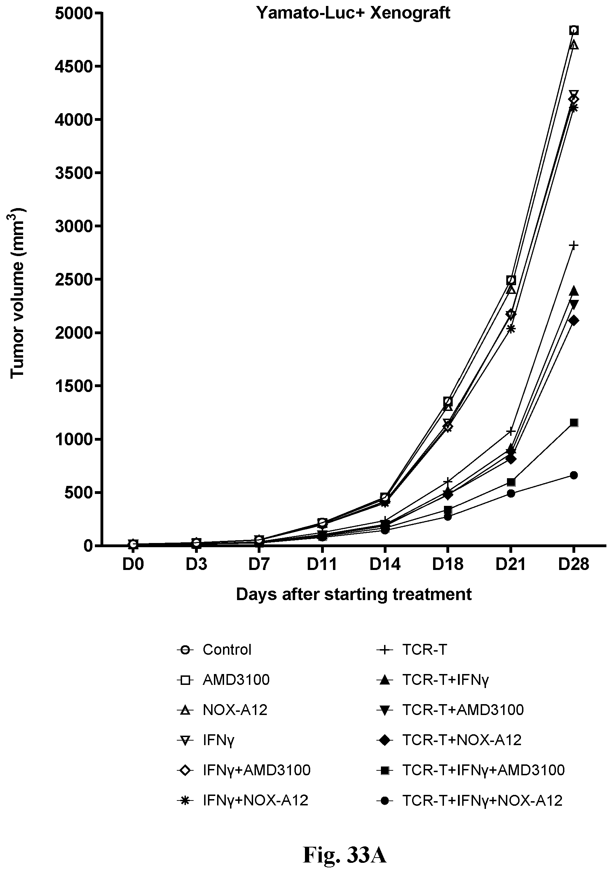

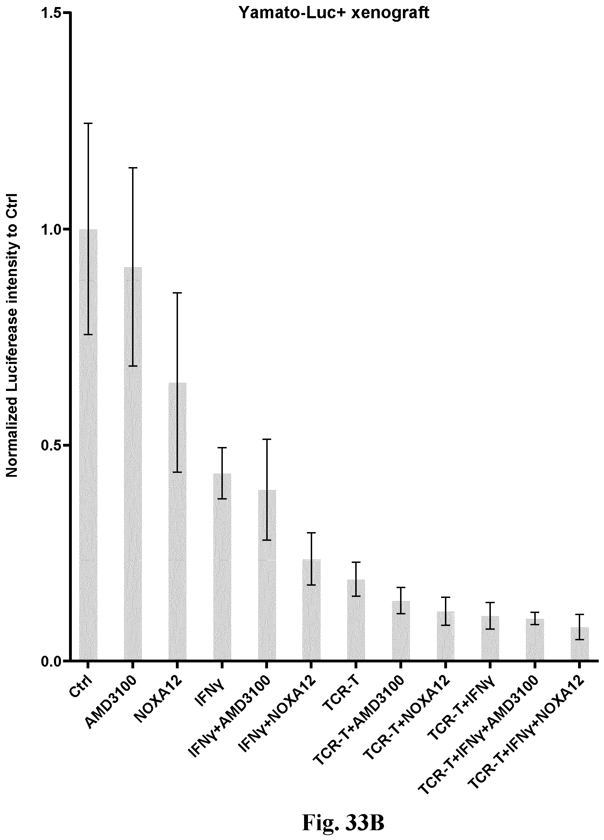

IFNγ as a single agent modestly restricted tumor growth, with noticeable effects by day 28. TCR-T cells alone reduced tumor growth by approximately 50% at day 28. When combined with TCR-T cells, IFNγ, AMD3100, or NOX-A12 each enhanced the anti-tumor activity of the TCR-T cells. Notably, the combination of IFNγ with either AMD3100 or NOX-A12 inhibited tumor growth more effectively than any of the agents alone. The most pronounced anti-tumor effect was observed with the combination of NOX-A12 and IFNγ with TCR-T cells, nearly eradicating tumor growth (Fig. 33 A). The results were confirmed by luciferase intensity measurements on Day 21 (Fig. 33 B).

### Example 12: Enhanced anti-tumor activity of TCR-T cells in Yamato-Luc+ synovial sarcoma xenografts through combined treatment with CXCL12 or CXCR4 inhibition and IFNα

**Mouse xenograft model** - In order to assess the anti-tumor activity of TCR-T cells combined with CXCL12 or CXCR4 inhibition and IFNα in a mouse model of synovial sarcoma, a xenograft experiment was conducted as described in Example 11, however instead of IFNγ, the type I interferon IFNα (2x10⁵ units, intraperitoneally twice per week, Cat# 300-02AA, Peprotech; Amino acid sequence MCDLPQTHSL GSRRTLMLLA QMRKISLFSC LKDRHDFGFP QEEFGNQFQK AETIPVLHEM IQQIFNLFST KDSSAAWDET LLDKFYTELY QQLNDLEACV IQGVGVTETP LMKEDSILAV RKYFQRITLY LKEKKYSPCA WEVVRAEIMR SFSLSTNLQE SLRSKE (SEQ ID NO: 243) ) was used.

Treatment groups and their respective schedules are summarized in the following table:

| **Treatment Group** | **Mouse #** | **Treatment Schedule** |
|---|---|---|
| Control | 5 | No treatment |
| NOX-A12 | 5 | 20 mg/kg, subcutaneously twice per week |
| IFNα | 5 | 2x10^5 units, intraperitoneally twice per week |
| IFNα + NOX-A12 | 5 | IFNα: 2x10^5 units, intraperitoneally twice per week; NOX-A12: 20 mg/kg, subcutaneously twice per week |
| TCR-T | 5 | 3x10^6 human NY-ESO-1 specific TCR T cells, intravenously via tail-vein |
| TCR-T + NOX-A12 | 5 | TCR-T: 3x10^6 human NY-ESO-1 specific TCR T cells, intravenously via tail-vein; NOX-A12: 20 mg/kg, subcutaneously twice per week |
| TCR-T + IFNα | 5 | TCR-T: 3x10^6 human NY-ESO-1 specific TCR T cells, intravenously via tail-vein; IFNα: 2x10^5 units, intraperitoneally twice per week |
| TCR-T + IFNα + NOX-A12 | 5 | TCR-T: 3x10^6 human NY-ESO-1 specific TCR T cells, intravenously via tail-vein; IFNα: 2x10^5 units, intraperitoneally twice per week; NOX-A12: 20 mg/kg, subcutaneously twice per week |

### Results:

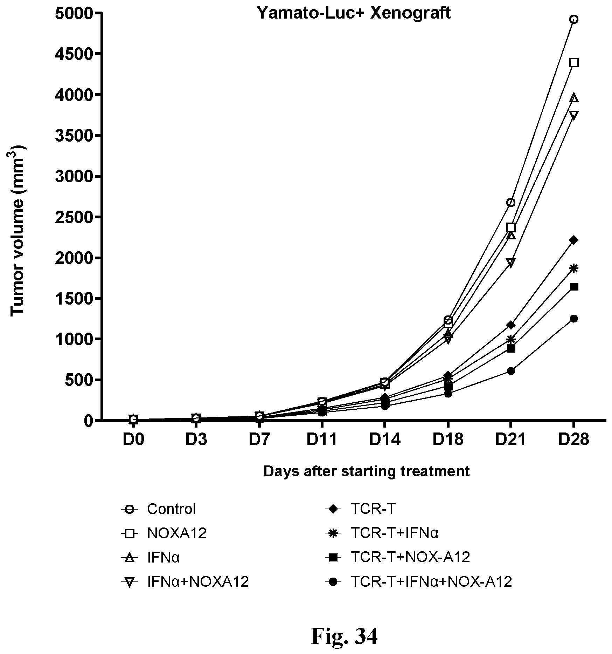

To test whether the synergistic effects observed with the combination of NOX-A12 and type II interferon IFNγ could also be achieved with type I interferon IFNα, an experiment was conducted using IFNα instead of IFNγ. The purpose of this experiment was to determine if the beneficial interaction between NOX-A12 and interferons is specific to IFNγ or if it can be generalized to other types of interferons, such as IFNα. In fact, NOX-A12 in combination with IFNα, plus adoptive therapy with TCR-T cells reduced Yamato synovial sarcoma xenograft growth to a similar extent as in combination with IFNγ (Fig. 34), broadening the therapeutic applications of NOX-A12 to combination with different interferons.

### Example 13: Enhanced T cell anti-tumor activity in an immunocompetent synovial sarcoma model through combination of NOX-A12 and IFNγ

***In vivo* experiment** - For this immune competent mouse model, tumors were induced by TATCre protein injection into the quadriceps muscle (10 µL injection of 50 µM TATCre, procured from Excellgen) in 8-day old mice homozygous for the *Rosa26-hSS2* allele, which undergoes Cre-mediated recombination to remove a floxed stop cassette that then enables expression of the human cDNA for SS18::SSX2, as well as enhanced green fluorescent protein on an internal ribosomal entry site (described in (Jones, Barrott et al. 2016)). The mice were then monitored with caliper measurements during predictable tumorigenesis at the site of injection over approximately 120 days. After tumors reached a calculated volume of approximately 0.75 cm³ on average, tumor-bearing mice from each litter were randomized to treatment groups (n = 5, each). Saline was used as a control with injection volumes of 10 µL/g body mass. IFNγ was dosed at 50 mg/kg via intraperitoneal injections on days 2 and 4, each week. NOX-A12 was dosed at 20 mg/kg body weight via subcutaneous injection on days 1, 3, and 5, each week.

Treatment groups and their respective schedules are summarized in the following table:

| **Treatment Group** | **Mouse #** | **Treatment Schedule** |
|---|---|---|
| Control | 5 | Saline; injection volumes of 10 µL/g body weight |
| NOX-A12 | 5 | 20 mg/kg via subcutaneous injection on days 1, 3, and 5, each week |
| IFNγ | 5 | 50 mg/kg via intraperitoneal injections on days 2 and 4, each week |
| IFNγ + NOX-A12 | 5 | NOX-A12 20 mg/kg via subcutaneous injection on days 1, 3, and 5, each week; IFNγ 50 mg/kg via intraperitoneal injections on days 2 and 4, each week |

### Results:

To investigate whether blocking the CXCL12/CXCR4 axis synergizes with IFNγ in an immunocompetent synovial sarcoma model, an experiment was conducted using mice that developed tumors following conditional SS18::SSX2 expression. The mice were treated with IFNγ, NOX-A12, or a combination of both. This model relied on the endogenous T cell response rather than the adoptive transfer of tumor-specific T cells.

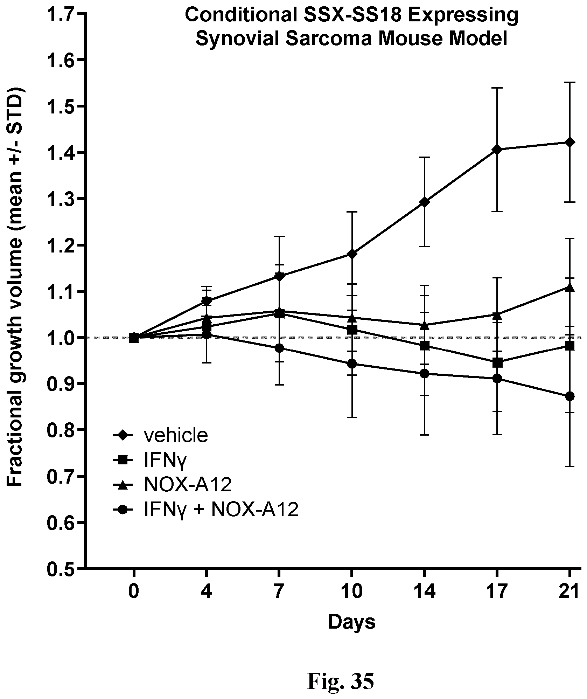

The purpose of this experiment was to determine if the combination of NOX-A12 and IFNγ would result in a greater reduction in tumor growth compared to either treatment alone. The findings showed that while both IFNγ and NOX-A12 individually reduced tumor growth compared to vehicle control-treated mice, the combination of NOX-A12 and IFNγ even led to a reduction in tumor volume (Fig. 35). This anti-tumor effect was attributed to the endogenous T cells, and the effectiveness of these T cells was enhanced by the combination therapy. This shows a synergistic effect between NOX-A12 and IFNγ, indicating that this is a very effective therapeutic strategy for treating synovial sarcoma.

### Example 14: Enhanced anti-tumor activity of CAR-T cells in a MG-63-Luc+ osteosarcoma xenograft model through combined treatment with NOX-A12 and IFNγ

**Generation of cell lines** - Human osteosarcoma tumor cell line MG-63 (ATCC, CRL-1427) was transfected to express Luciferase (Luc+) as described in Example 9.

For B7-H3 CAR-T cells, the B7-H3 (CD276) CAR construct was a gift from Michael C. Jensen, Seattle Children's Therapeutics (Vitanza, Wilson et al. 2023). The transduction method was adapted from Poncette 2019 (Poncette, Chen et al. 2019). Briefly, human peripheral blood mononuclear cells (PBMC) were isolated from the blood of a healthy donor using Ficoll (GE Life Sciences). To activate human T cells, the cells were cultivated in CTL medium supplemented with IL-2 and activated according to the supplier's specifications. Specifically, CTL medium (500 mL RPMI + 10% FBS + 1% penicillin & streptomycin + 1% L-glutamine + 1% non-essential amino acids + 1% sodium pyruvate + 50 mM β-mercaptoethanol) was supplemented with 200 IU/mL recombinant human IL-2 (Cat# 200-02-1MG, Peprotech; amino acid sequence MAPTSSSTKK TQLQLEHLLL DLQMILNGIN NYKNPKLTRM LTFKFYMPKK ATELKHLQCL EEELKPLEEV LNLAQSKNFH LRPRDLISNI NVIVLELKGS ETTFMCEYAD ETATIVEFLN RWITFAQSII STLT (SEQ ID NO: 246)), and T cells were activated using 20 µL/mL ImmunoCult^{™} Human CD3/CD28/CD2 T Cell Activator reagent at a cell density of 1 × 10⁶ cells/mL (StemCell Technologies, Vancouver, Canada). T cells were transduced twice to ensure high transduction efficiency. On day 3, CAR-encoding viral supernatant supplemented with 200 IU/mL rhIL-2 and 6 µg/mL protamine sulfate was added to the cell culture for the first transduction. For the second transduction, non-tissue culture-treated cell culture plates were coated with 10 µg/mL retronectin (Takara) at 4°C overnight. The next day, the plates were blocked with PBS containing 2% BSA, washed with PBS, and the viral supernatant was added. The plates were then spun at 2,000 × g for 2 hours. The viral supernatant was removed, and cells transduced with protamine sulfate were resuspended and added to the retronectin and virus-coated plates. Two days after the second transduction, the cells were transferred to a G-Rex 6M Well Plate (Wilson Wolf) for expansion over 10 days. Every 3-4 days, fresh rhIL-2 was added by replacing half the volume of CTL medium.

**In vivo experiment** - 3× 10⁶ human osteosarcoma MG63-Luc+ cells were resuspended in 100 µl PBS and were subcutaneously injected at the right flank to four- to six-week-old female athymic nude mice (NU/J Strain #002019, the Jackson laboratory, ME, USA). Once tumors reached a diameter of 3 mm, the mice were randomly grouped into two subgroups with and without T cells treatment. 3x10⁶ human B7-H3 CAR transduced T cells were intravenously injected by tail vein. After one week human IFNγ (2x10⁵ unit, intraperitoneally twice per week, Cat# 300-02, Peprotech), NOX-A12 (20 mg/kg, subcutaneously twice per week), or combinations as indicated, were applied. Tumor sizes were measured with calipers twice weekly. Tumor volume was calculated using the formula V=l x w² /2 (with 1 being length and w being width). Bioluminescent imaging was performed at Day 21 as described in Example 9. The mice were sacrificed by CO₂ asphyxiation.

Treatment groups and their respective schedules are summarized in the following table:

| **Treatment Group** | **Mouse #** | **Treatment Schedule** |
|---|---|---|
| Control | 5 | No treatment |
| NOX-A12 | 5 | 20 mg/kg, subcutaneously twice per week |
| IFNγ | 5 | 2x10^5 units, intraperitoneally twice per week |
| IFNγ + NOX-A12 | 5 | IFNγ: 2x10^5 units, intraperitoneally twice per week; NOX-A12: 20 mg/kg, subcutaneously twice per week |
| TCR-T | 5 | 3x10^6 human NY-ESO-1 specific TCR T cells, intravenously via tail-vein |
| TCR-T + NOX-A12 | 5 | TCR-T: 3x10^6 human NY-ESO-1 specific TCR T cells, intravenously via tail-vein; NOX-A12: 20 mg/kg, subcutaneously twice per week |
| TCR-T + IFNγ | 5 | TCR-T: 3x10^6 human NY-ESO-1 specific TCR T cells, intravenously via tail-vein; IFNγ: 2x10^5 units, intraperitoneally twice per week |
| TCR-T + IFNγ + NOX-A12 | 5 | TCR-T: 3x10^6 human NY-ESO-1 specific TCR T cells, intravenously via tail-vein; IFNγ: 2x10^5 units, intraperitoneally twice per week; NOX-A12: 20 mg/kg, subcutaneously twice per week |

### Results:

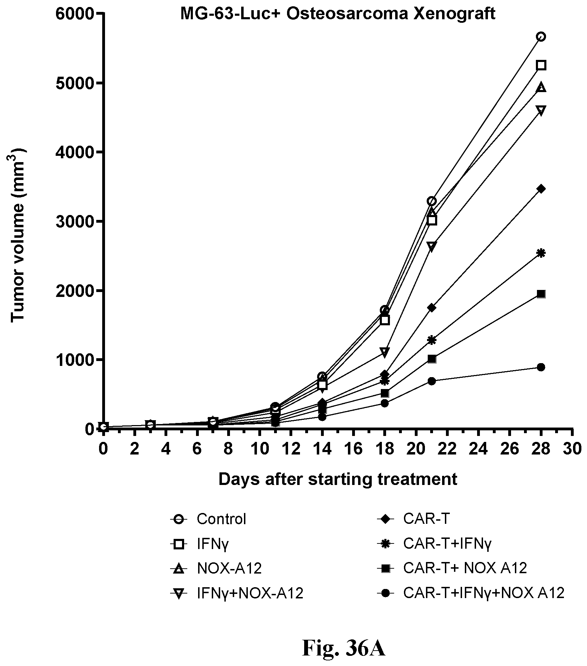

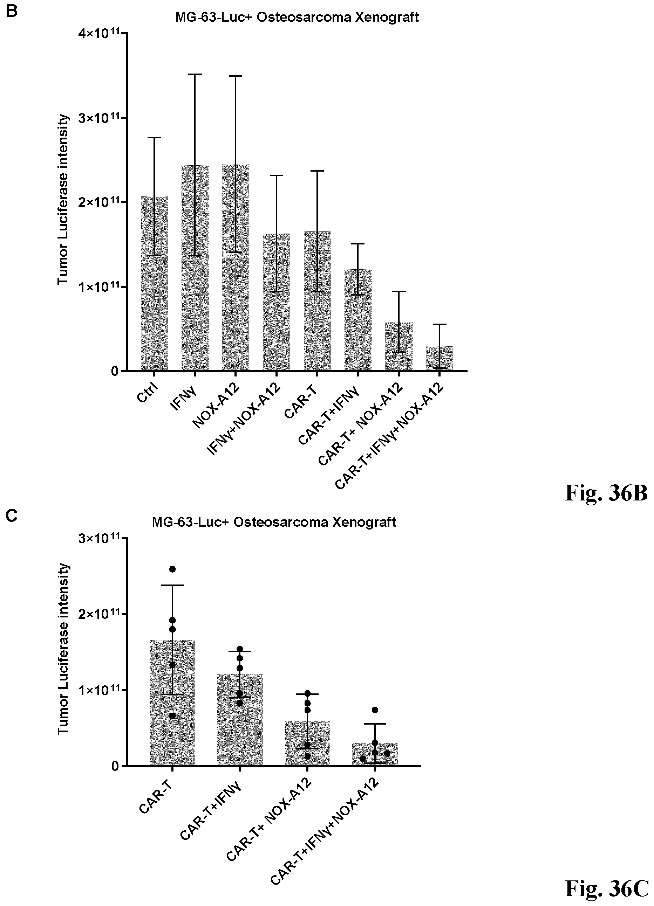

The primary aim of this study was to evaluate whether the combination of NOX-A12 and IFNγ could enhance the anti-tumor efficacy of CAR-T cells in an MG-63-Luc+ xenograft model. IFNγ alone modestly restricted tumor growth. CAR-T cells alone reduced tumor growth by day 28. When combined with CAR-T cells, NOX-A12 or IFNγ each enhanced the anti-tumor activity of the CAR-T cells. Notably, the combination of NOX-A12 and IFNγ with CAR-T cells inhibited tumor growth more effectively than any of the agents alone. The most pronounced anti-tumor effect was observed with the combination of NOX-A12 and IFNγ with CAR-T cells, demonstrating superior efficacy compared to CAR-T cells alone or CAR-T cells plus IFNγ (Fig. 36 A). These results were further confirmed by luciferase intensity measurements on Day 21 (Fig. 36 B + Fig. 36 C).

### Example 15: Enhanced anti-tumor activity of CAR-T cells in a PANC-1-Luc+ pancreatic cancer xenograft model through combined treatment with NOX-A12 and IFNγ

**Generation of cell lines** - Human tumor cell line PANC-1 (ATCC, CRL-1469) was transfected to express Luciferase (Luc+) as described in Example 9. B7-H3 CAR-T cells were generated as described in Example 14.

**In vivo experiment** - 3× 10⁶ human pancreatic cells PANC-1-Luc+ cells were resuspended in 100 µl PBS and were subcutaneously injected at the right flank to four- to six-week-old female athymic nude mice (NU/J Strain #002019, the Jackson laboratory, ME, USA). Once tumors reached a diameter of 3 mm, the mice were randomly grouped into two subgroups with and without T cells treatment. 3x10⁶ human B7H3 CAR transduced T cells were intravenously injected by tail-vein. After one week human IFNγ (2x10⁵ unit, intraperitoneally twice per week, Cat# 300-02, Peprotech), NOX-A12 (20 mg/kg, subcutaneously twice per week), or combinations as indicated, were applied. Tumor sizes were measured with calipers twice weekly. Tumor volume was calculated using the formula V=1 x w² /2 (with 1 being length and w being width). Bioluminescent imaging was performed at Day 28 as described in Example 9. The mice were sacrificed by CO₂ asphyxiation.

Treatment groups and their respective schedules are summarized in the following table:

| **Treatment Group** | **Mouse #** | **Treatment Schedule** |
|---|---|---|
| Control | 5 | No treatment |
| NOX-A12 | 5 | 20 mg/kg, subcutaneously twice per week |
| IFNγ | 5 | 2x10^5 units, intraperitoneally twice per week |
| IFNγ + NOX-A12 | 5 | IFNγ: 2x10^5 units, intraperitoneally twice per week; NOX-A12: 20 mg/kg, subcutaneously twice per week |
| TCR-T | 5 | 3x10^6 human NY-ESO-1 specific TCR T cells, intravenously via tail-vein |
| TCR-T + NOX-A12 | 5 | TCR-T: 3x10^6 human NY-ESO-1 specific TCR T cells, intravenously via tail-vein; NOX-A12: 20 mg/kg, subcutaneously twice per week |
| TCR-T + IFNγ | 5 | TCR-T: 3x10^6 human NY-ESO-1 specific TCR T cells, intravenously via tail-vein; IFNγ: 2x10^5 units, intraperitoneally twice per week |
| TCR-T + IFNγ + NOX-A12 | 5 | TCR-T: 3x10^6 human NY-ESO-1 specific TCR T cells, intravenously via tail-vein; IFNγ: 2x10^5 units, intraperitoneally twice per week; NOX-A12: 20 mg/kg, subcutaneously twice per week |

### Results:

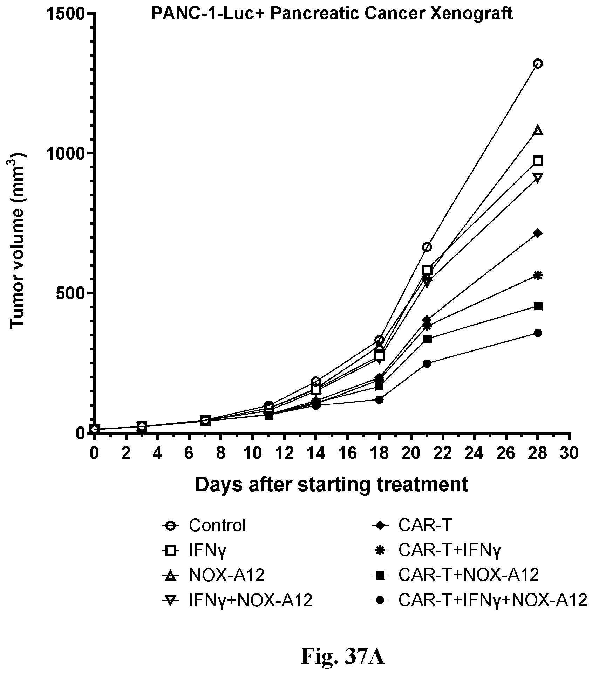

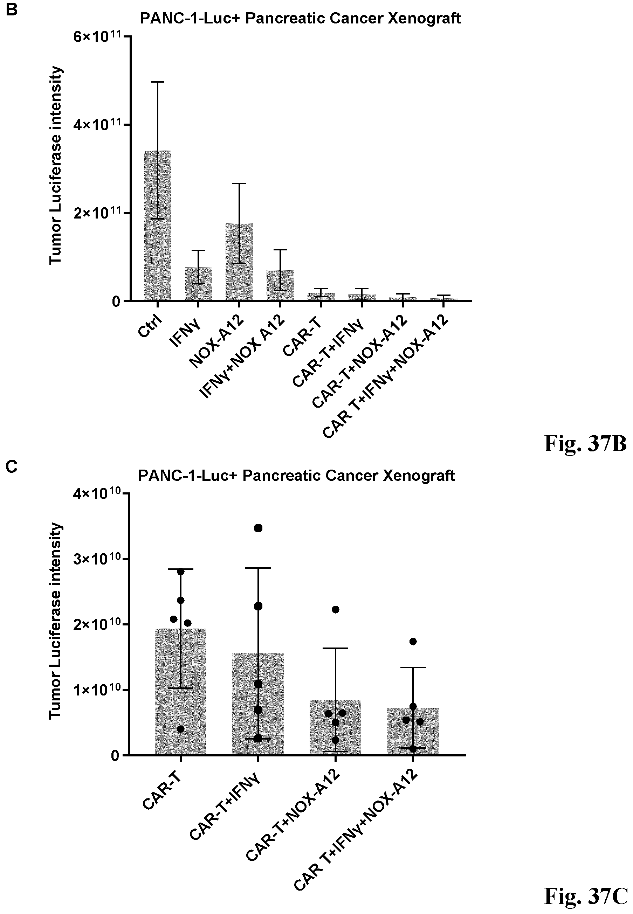

The primary aim of this study was to evaluate whether the combination of NOX-A12 and IFNγ could enhance the anti-tumor efficacy of CAR-T cells in an PANC-1-Luc+ xenograft model. IFNγ alone modestly restricted tumor growth. CAR-T cells alone reduced tumor growth by day 28. When combined with CAR-T cells, NOX-A12 or IFNγ each enhanced the anti-tumor activity of the CAR-T cells. Notably, the combination of NOX-A12 and IFNγ with CAR-T cells inhibited tumor growth more effectively than any of the agents alone. The most pronounced anti-tumor effect was observed with the combination of NOX-A12 and IFNγ with CAR-T cells, demonstrating superior efficacy compared to CAR-T cells alone or CAR-T cells plus IFNγ (Fig. 37 A). These results were further confirmed by luciferase intensity measurements on Day 21 (Fig. 37 B + Fig. 37C).

### References

The complete bibliographic data of the documents recited herein are, if not indicated to the contrary, as follows, whereby the disclosure of said references is incorporated herein by reference.
Albarran, V., M. San Roman, J. Pozas, J. Chamorro, D. I. Rosero, P. Guerrero, J. C. Calvo, C. Gonzalez, C. Garcia de Quevedo, P. Perez de Aguado, J. Moreno, A. Cortes and A. Soria (2024). "Adoptive T cell therapy for solid tumors: current landscape and future challenges." Front Immunol 15: 1352805.
Alberts, D. S., C. Marth, R. D. Alvarez, G. Johnson, M. Bidzinski, D. R. Kardatzke, W. Z. Bradford, J. Loutit, D. H. Kirn, M. C. Clouser, M. Markman and G. C. T. Consortium (2008). "Randomized phase 3 trial of interferon gamma-1b plus standard carboplatin/paclitaxel versus carboplatin/paclitaxel alone for first-line treatment of advanced ovarian and primary peritoneal carcinomas: results from a prospectively designed analysis of progression-free survival." Gynecol Oncol 109(2): 174-181.
Aljubran, A. H., A. Griffin, M. Pintilie and M. Blackstein (2009). "Osteosarcoma in adolescents and adults: survival analysis with and without lung metastases." Ann Oncol 20(6): 1136-1141.
Altschul, S. F., W. Gish, W. Miller, E. W. Myers and D. J. Lipman (1990). "Basic local alignment search tool." J Mol Biol 215(3): 403-410.
Altschul, S. F., T. L. Madden, A. A. Schaffer, J. Zhang, Z. Zhang, W. Miller and D. J. Lipman (1997). "Gapped BLAST and PSI-BLAST: a new generation of protein database search programs." Nucleic Acids Res 25(17): 3389-3402.
Amary, M. F., F. Berisha, C. Bernardi Fdel, A. Herbert, M. James, J. S. Reis-Filho, C. Fisher, A. G. Nicholson, R. Tirabosco, T. C. Diss and A. M. Flanagan (2007). "Detection of SS18-SSX fusion transcripts in formalin-fixed paraffin-embedded neoplasms: analysis of conventional RT-PCR, qRT-PCR and dual color FISH as diagnostic tools for synovial sarcoma." Mod Pathol 20(4): 482-496.
Amin, M. B., Edge, S. B. (2017). AJCC Cancer Staging Manual. Amsterdam, The Netherlands, Springer.
Anderson, A. C., N. Joller and V. K. Kuchroo (2016). "Lag-3, Tim-3, and TIGIT: Co-inhibitory Receptors with Specialized Functions in Immune Regulation." Immunity 44(5): 989-1004.
Andersson, A., L. Larsson, L. Stenbeck, F. Salmen, A. Ehinger, S. Z. Wu, G. Al-Eryani, D. Roden, A. Swarbrick, A. Borg, J. Frisen, C. Engblom and J. Lundeberg (2021). "Spatial deconvolution of HER2-positive breast cancer delineates tumor-associated cell type interactions." Nat Commun 12(1): 6012.
Anninga, J. K., H. Gelderblom, M. Fiocco, J. R. Kroep, A. H. Taminiau, P. C. Hogendoorn and R. M. Egeler (2011). "Chemotherapeutic adjuvant treatment for osteosarcoma: where do we stand?" Eur J Cancer 47(16): 2431-2445.
Ayala Ceja, M., M. Khericha, C. M. Harris, C. Puig-Saus and Y. Y. Chen (2024). "CAR-T cell manufacturing: Major process parameters and next-generation strategies." J Exp Med 221(2).
Ayers, M., J. Lunceford, M. Nebozhyn, E. Murphy, A. Loboda, D. R. Kaufman, A. Albright, J. D. Cheng, S. P. Kang, V. Shankaran, S. A. Piha-Paul, J. Yearley, T. Y. Seiwert, A. Ribas and T. K. McClanahan (2017). "IFN-gamma-related mRNA profile predicts clinical response to PD-1 blockade." J Clin Invest 127(8): 2930-2940.
Aytekin, M. N., R. Ozturk, K. Amer and A. Yapar (2020). "Epidemiology, incidence, and survival of synovial sarcoma subtypes: SEER database analysis." J Orthop Surg (Hong Kong) 28(2): 2309499020936009.
Baheti, A. D., S. H. Tirumani, R. Sewatkar, A. B. Shinagare, J. L. Hornick, N. H. Ramaiya and J. P. Jagannathan (2015). "Imaging features of primary and metastatic extremity synovial sarcoma: a single institute experience of 78 patients." Br J Radiol 88(1046): 20140608.
Bai, R., N. Chen, L. Li, N. Du, L. Bai, Z. Lv, H. Tian and J. Cui (2020). "Mechanisms of Cancer Resistance to Immunotherapy." Front Oncol 10: 1290.
Bakri, A., A. B. Shinagare, K. M. Krajewski, S. A. Howard, J. P. Jagannathan, J. L. Hornick and N. H. Ramaiya (2012). "Synovial sarcoma: imaging features of common and uncommon primary sites, metastatic patterns, and treatment response." AJR Am J Roentgenol 199(2): W208-215.
Balkwill, F. and A. Mantovani (2001). "Inflammation and cancer: back to Virchow?" Lancet 357(9255): 539-545.
Barber, D. L., E. J. Wherry, D. Masopust, B. Zhu, J. P. Allison, A. H. Sharpe, G. J. Freeman and R. Ahmed (2006). "Restoring function in exhausted CD8 T cells during chronic viral infection." Nature 439(7077): 682-687.
Baulu, E., C. Gardet, N. Chuvin and S. Depil (2023). "TCR-engineered T cell therapy in solid tumors: State of the art and perspectives." Sci Adv 9(7): eadf3700.
Behjati, S., P. S. Tarpey, K. Haase, H. Ye, M. D. Young, L. B. Alexandrov, S. J. Farndon, G. Collord, D. C. Wedge, I. Martincorena, S. L. Cooke, H. Davies, W. Mifsud, M. Lidgren, S. Martin, C. Latimer, M. Maddison, A. P. Butler, J. W. Teague, N. Pillay, A. Shlien, U. McDermott, P. A. Futreal, D. Baumhoer, O. Zaikova, B. Bjerkehagen, O. Myklebost, M. F. Amary, R. Tirabosco, P. Van Loo, M. R. Stratton, A. M. Flanagan and P. J. Campbell (2017). "Recurrent mutation of IGF signalling genes and distinct patterns of genomic rearrangement in osteosarcoma." Nat Commun 8: 15936.
Beird, H. C., S. S. Bielack, A. M. Flanagan, J. Gill, D. Heymann, K. A. Janeway, J. A. Livingston, R. D. Roberts, S. J. Strauss and R. Gorlick (2022). "Osteosarcoma." Nat Rev Dis Primers 8(1): 77.
Bertoni, F. and P. Bacchini (1998). "Classification of bone tumors." Eur J Radiol 27 Suppl 1: S74-76.
Bettegowda, C., M. Sausen, R. J. Leary, I. Kinde, Y. Wang, N. Agrawal, B. R. Bartlett, H. Wang, B. Luber, R. M. Alani, E. S. Antonarakis, N. S. Azad, A. Bardelli, H. Brem, J. L. Cameron, C. C. Lee, L. A. Fecher, G. L. Gallia, P. Gibbs, D. Le, R. L. Giuntoli, M. Goggins, M. D. Hogarty, M. Holdhoff, S. M. Hong, Y. Jiao, H. H. Juhl, J. J. Kim, G. Siravegna, D. A. Laheru, C. Lauricella, M. Lim, E. J. Lipson, S. K. Marie, G. J. Netto, K. S. Oliner, A. Olivi, L. Olsson, G. J. Riggins, A. Sartore-Bianchi, K. Schmidt, M. Shih 1, S. M. Oba-Shinjo, S. Siena, D. Theodorescu, J. Tie, T. T. Harkins, S. Veronese, T. L. Wang, J. D. Weingart, C. L. Wolfgang, L. D. Wood, D. Xing, R. H. Hruban, J. Wu, P. J. Allen, C. M. Schmidt, M. A. Choti, V. E. Velculescu, K. W. Kinzler, B. Vogelstein, N. Papadopoulos and L. A. Diaz, Jr. (2014). "Detection of circulating tumor DNA in early- and late-stage human malignancies." Sci Transl Med 6(224): 224ra224.
Biau, D. J., P. C. Ferguson, P. Chung, A. M. Griffin, C. N. Catton, B. O'Sullivan and J. S. Wunder (2012). "Local recurrence of localized soft tissue sarcoma: a new look at old predictors." Cancer 118(23): 5867-5877.
Bielack, S. S., B. Kempf-Bielack, G. Delling, G. U. Exner, S. Flege, K. Helmke, R. Kotz, M. Salzer-Kuntschik, M. Werner, W. Winkelmann, A. Zoubek, H. Jurgens and K. Winkler (2002). "Prognostic factors in high-grade osteosarcoma of the extremities or trunk: an analysis of 1,702 patients treated on neoadjuvant cooperative osteosarcoma study group protocols." J Clin Oncol 20(3): 776-790.
Bielack, S. S., N. Marina, S. Ferrari, L. J. Helman, S. Smeland, J. S. Whelan and G. H. Reaman (2008). "Osteosarcoma: the same old drugs or more?" J Clin Oncol 26(18): 3102-3103; author reply 3104-3105.
Bielack, S. S., S. Smeland, J. S. Whelan, N. Marina, G. Jovic, J. M. Hook, M. D. Krailo, M. Gebhardt, Z. Papai, J. Meyer, H. Nadel, R. L. Randall, C. Deffenbaugh, R. Nagarajan, B. Brennan, G. D. Letson, L. A. Teot, A. Goorin, D. Baumhoer, L. Kager, M. Werner, C. C. Lau, K. Sundby Hall, H. Gelderblom, P. Meyers, R. Gorlick, R. Windhager, K. Helmke, M. Eriksson, P. M. Hoogerbrugge, P. Schomberg, P. U. Tunn, T. Kuhne, H. Jurgens, H. van den Berg, T. Bohling, S. Picton, M. Renard, P. Reichardt, J. Gerss, T. Butterfass-Bahloul, C. Morris, P. C. Hogendoorn, B. Seddon, G. Calaminus, M. Michelagnoli, C. Dhooge, M. R. Sydes, M. Bernstein and E.-. investigators (2015). "Methotrexate, Doxorubicin, and Cisplatin (MAP) Plus Maintenance Pegylated Interferon Alfa-2b Versus MAP Alone in Patients With Resectable High-Grade Osteosarcoma and Good Histologic Response to Preoperative MAP: First Results of the EURAMOS-1 Good Response Randomized Controlled Trial." J Clin Oncol 33(20): 2279-2287.
Bilgeri, A., A. Klein, L. H. Lindner, S. Nachbichler, T. Knosel, C. Birkenmaier, V. Jansson, A. Baur-Melnyk and H. R. Durr (2020). "The Effect of Resection Margin on Local Recurrence and Survival in High Grade Soft Tissue Sarcoma of the Extremities: How Far Is Far Enough?" Cancers (Basel) 12(9).
Bixby, S. D., S. Hettmer, G. A. Taylor and S. D. Voss (2010). "Synovial sarcoma in children: imaging features and common benign mimics." AJR Am J Roentgenol 195(4): 1026-1032.
Blay, J. Y., M. von Mehren, R. L. Jones, J. Martin-Broto, S. Stacchiotti, S. Bauer, H. Gelderblom, D. Orbach, N. Hindi, A. Dei Tos and M. Nathenson (2023). "Synovial sarcoma: characteristics, challenges, and evolving therapeutic strategies." ESMO Open 8(5): 101618.
Bleul, C. C., M. Farzan, H. Choe, C. Parolin, I. Clark-Lewis, J. Sodroski and T. A. Springer (1996). "The lymphocyte chemoattractant SDF-1 is a ligand for LESTR/fusin and blocks HIV-1 entry." Nature 382(6594): 829-833.
Borden, E. C. (2019). "Interferons alpha and beta in cancer: therapeutic opportunities from new insights." Nat Rev Drug Discov 18(3): 219-234.
Borden, E. C., G. C. Sen, G. Uze, R. H. Silverman, R. M. Ransohoff, G. R. Foster and G. R. Stark (2007). "Interferons at age 50: past, current and future impact on biomedicine." Nat Rev Drug Discov 6(12): 975-990.
Bosetti, C., E. Lucenteforte, D. T. Silverman, G. Petersen, P. M. Bracci, B. T. Ji, E. Negri, D. Li, H. A. Risch, S. H. Olson, S. Gallinger, A. B. Miller, H. B. Bueno-de-Mesquita, R. Talamini, J. Polesel, P. Ghadirian, P. A. Baghurst, W. Zatonski, E. Fontham, W. R. Bamlet, E. A. Holly, P. Bertuccio, Y. T. Gao, M. Hassan, H. Yu, R. C. Kurtz, M. Cotterchio, J. Su, P. Maisonneuve, E. J. Duell, P. Boffetta and C. La Vecchia (2012). "Cigarette smoking and pancreatic cancer: an analysis from the International Pancreatic Cancer Case-Control Consortium (Panc4)." Ann Oncol 23(7): 1880-1888.
Bosetti, C., V. Rosato, D. Li, D. Silverman, G. M. Petersen, P. M. Bracci, R. E. Neale, J. Muscat, K. Anderson, S. Gallinger, S. H. Olson, A. B. Miller, H. Bas Bueno-de-Mesquita, G. Scelo, V. Janout, I. Holcatova, P. Lagiou, D. Serraino, E. Lucenteforte, E. Fabianova, P. A. Baghurst, W. Zatonski, L. Foretova, E. Fontham, W. R. Bamlet, E. A. Holly, E. Negri, M. Hassan, A. Prizment, M. Cotterchio, S. Cleary, R. C. Kurtz, P. Maisonneuve, D. Trichopoulos, J. Polesel, E. J. Duell, P. Boffetta, C. La Vecchia and P. Ghadirian (2014). "Diabetes, antidiabetic medications, and pancreatic cancer risk: an analysis from the International Pancreatic Cancer Case-Control Consortium." Ann Oncol 25(10): 2065-2072.
Cameron, J. L., T. S. Riall, J. Coleman and K. A. Belcher (2006). "One thousand consecutive pancreaticoduodenectomies." Ann Surg 244(1): 10-15.
Canter, R. J., L. X. Qin, R. G. Maki, M. F. Brennan, M. Ladanyi and S. Singer (2008). "A synovial sarcoma-specific preoperative nomogram supports a survival benefit to ifosfamide-based chemotherapy and improves risk stratification for patients." Clin Cancer Res 14(24): 8191-8197.
Caracciolo, J. T., E. Henderson-Jackson and O. Binitie (2019). "Synovial sarcoma of bone: Sarcoma typically of soft tissues presenting as a primary bone tumor." Radiol Case Rep 14(2): 204-207.
Chandrasekar, C. R., H. Wafa, R. J. Grimer, S. R. Carter, R. M. Tillman and A. Abudu (2008). "The effect of an unplanned excision of a soft-tissue sarcoma on prognosis." J Bone Joint Surg Br 90(2): 203-208.
Chang, D. K., S. M. Grimmond and A. V. Biankin (2014). "Pancreatic cancer genomics." Curr Opin Genet Dev 24: 74-81.
Charych, D. H., U. Hoch, J. L. Langowski, S. R. Lee, M. K. Addepalli, P. B. Kirk, D. Sheng, X. Liu, P. W. Sims, L. A. VanderVeen, C. F. Ali, T. K. Chang, M. Konakova, R. L. Pena, R. S. Kanhere, Y. M. Kirksey, C. Ji, Y. Wang, J. Huang, T. D. Sweeney, S. S. Kantak and S. K. Doberstein (2016). "NKTR-214, an Engineered Cytokine with Biased IL2 Receptor Binding, Increased Tumor Exposure, and Marked Efficacy in Mouse Tumor Models." Clin Cancer Res 22(3): 680-690.
Chawla, S. P., B. A. Van Tine, S. M. Pollack, K. N. Ganjoo, A. D. Elias, R. F. Riedel, S. Attia, E. Choy, S. H. Okuno, M. Agulnik, M. von Mehren, M. B. Livingston, V. L. Keedy, C. F. Verschraegen, T. Philip, G. C. Bohac, S. Yurasov, A. Yakovich, H. Lu, M. Chen and R. G. Maki (2022). "Phase II Randomized Study of CMB305 and Atezolizumab Compared With Atezolizumab Alone in Soft-Tissue Sarcomas Expressing NY-ESO-1." J Clin Oncol 40(12): 1291-1300.
Chen, C., R. Borker, J. Ewing, W. Y. Tseng, M. D. Hackshaw, S. Saravanan, R. Dhanda and E. Nadler (2014). "Epidemiology, treatment patterns, and outcomes of metastatic soft tissue sarcoma in a community-based oncology network." Sarcoma 2014: 145764.
Chen, X., A. Bahrami, A. Pappo, J. Easton, J. Dalton, E. Hedlund, D. Ellison, S. Shurtleff, G. Wu, L. Wei, M. Parker, M. Rusch, P. Nagahawatte, J. Wu, S. Mao, K. Boggs, H. Mulder, D. Yergeau, C. Lu, L. Ding, M. Edmonson, C. Qu, J. Wang, Y. Li, F. Navid, N. C. Daw, E. R. Mardis, R. K. Wilson, J. R. Downing, J. Zhang, M. A. Dyer and P. St. Jude Children's Research Hospital-Washington University Pediatric Cancer Genome (2014). "Recurrent somatic structural variations contribute to tumorigenesis in pediatric osteosarcoma." Cell Rep 7(1): 104-112.
Chotel, F., A. Unnithan, C. R. Chandrasekar, R. Parot, L. Jeys and R. J. Grimer (2008). "Variability in the presentation of synovial sarcoma in children: a plea for greater awareness." J Bone Joint Surg Br 90(8): 1090-1096.
Chou, A. J., E. S. Kleinerman, M. D. Krailo, Z. Chen, D. L. Betcher, J. H. Healey, E. U. Conrad, 3rd, M. L. Nieder, M. A. Weiner, R. J. Wells, R. B. Womer, P. A. Meyers and G. Children's Oncology (2009). "Addition of muramyl tripeptide to chemotherapy for patients with newly diagnosed metastatic osteosarcoma: a report from the Children's Oncology Group." Cancer 115(22): 5339-5348.
Ciernik, I. F., A. Niemierko, D. C. Harmon, W. Kobayashi, Y. L. Chen, T. I. Yock, D. H. Ebb, E. Choy, K. A. Raskin, N. Liebsch, F. J. Hornicek and T. F. Delaney (2011). "Proton-based radiotherapy for unresectable or incompletely resected osteosarcoma." Cancer 117(19): 4522-4530.
Clark, C. E., S. R. Hingorani, R. Mick, C. Combs, D. A. Tuveson and R. H. Vonderheide (2007). "Dynamics of the immune reaction to pancreatic cancer from inception to invasion." Cancer Res 67(19): 9518-9527.
Cole, S., D. M. Gianferante, B. Zhu and L. Mirabello (2022). "Osteosarcoma: A Surveillance, Epidemiology, and End Results program-based analysis from 1975 to 2017." Cancer 128(11): 2107-2118.
Collins, F. S. and H. Varmus (2015). "A new initiative on precision medicine." N Engl J Med 372(9): 793-795.
Constantinidou, A., S. Pollack, E. Loggers, E. Rodler and R. L. Jones (2013). "The evolution of systemic therapy in sarcoma." Expert Rev Anticancer Ther 13(2): 211-223.
Cortes-Ciriano, I., J. J. Lee, R. Xi, D. Jain, Y. L. Jung, L. Yang, D. Gordenin, L. J. Klimczak, C. Z. Zhang, D. S. Pellman, P. S. V. W. Group, P. J. Park and P. Consortium (2020). "Comprehensive analysis of chromothripsis in 2,658 human cancers using whole-genome sequencing." Nat Genet 52(3): 331-341.
Damha, M. J. and K. K. Ogilvie (1993). "Oligoribonucleotide synthesis. The silyl-phosphoramidite method." Methods Mol Biol 20: 81-114.
Davis, A. M., B. O'Sullivan, R. Turcotte, R. Bell, C. Catton, P. Chabot, J. Wunder, A. Hammond, V. Benk, R. Kandel, K. Goddard, C. Freeman, A. Sadura, B. Zee, A. Day, D. Tu, J. Pater, G. Canadian Sarcoma and N. C. I. C. C. T. G. R. Trial (2005). "Late radiation morbidity following randomization to preoperative versus postoperative radiotherapy in extremity soft tissue sarcoma." Radiother Oncol 75(1): 48-53.
De Silva, M. V., A. Barrett and R. Reid (2003). "Premonitory Pain Preceding Swelling: A Distinctive Clinical Presentation of Synovial Sarcoma which may Prompt Early Detection." Sarcoma 7(3-4): 131-135.
Deshmukh, R., H. J. Mankin and S. Singer (2004). "Synovial sarcoma: the importance of size and location for survival." Clin Orthop Relat Res(419): 155-161.
Donnadieu, E., L. Dupre, L. G. Pinho and V. Cotta-de-Almeida (2020). "Surmounting the obstacles that impede effective CAR T cell trafficking to solid tumors." J Leukoc Biol 108(4): 1067-1079.
   dos Santos, N. R., D. R. de Bruijn and A. G. van Kessel (2001). "Molecular mechanisms underlying human synovial sarcoma development." Genes Chromosomes Cancer 30(1): 1-14.
Duan, J., L. Cui, X. Zhao, H. Bai, S. Cai, G. Wang, Z. Zhao, J. Zhao, S. Chen, J. Song, C. Qi, Q. Wang, M. Huang, Y. Zhang, D. Huang, Y. Bai, F. Sun, J. J. Lee, Z. Wang and J. Wang (2020). "Use of Immunotherapy With Programmed Cell Death 1 vs Programmed Cell Death Ligand 1 Inhibitors in Patients With Cancer: A Systematic Review and Meta-analysis." JAMA Oncol 6(3): 375-384.
Duncan, C. J. A., R. E. Randall and S. Hambleton (2021). "Genetic Lesions of Type I Interferon Signalling in Human Antiviral Immunity." Trends Genet 37(1): 46-58.
Dunn, G. P., A. T. Bruce, K. C. Sheehan, V. Shankaran, R. Uppaluri, J. D. Bui, M. S. Diamond, C. M. Koebel, C. Arthur, J. M. White and R. D. Schreiber (2005). "A critical function for type I interferons in cancer immunoediting." Nat Immunol 6(7): 722-729.
Eilber, F. C., M. F. Brennan, F. R. Eilber, J. J. Eckardt, S. R. Grobmyer, E. Riedel, C. Forscher, R. G. Maki and S. Singer (2007). "Chemotherapy is associated with improved survival in adult patients with primary extremity synovial sarcoma." Ann Surg 246(1): 105-113.
Enneking, W. F. (1988). "A system of staging musculoskeletal neoplasms." Instr Course Lect 37: 3-10. FDA (2021). "Product Information INTRON A." FDA label.
FDA (2023). "Product information PEGASYS." FDA label.
FDA (2024). "Product Information TECELRA." FDA label.
Fearon, D. T. (2014). "The carcinoma-associated fibroblast expressing fibroblast activation protein and escape from immune surveillance." Cancer Immunol Res 2(3): 187-193.
Feig, C., J. O. Jones, M. Kraman, R. J. Wells, A. Deonarine, D. S. Chan, C. M. Connell, E. W. Roberts, Q. Zhao, O. L. Caballero, S. A. Teichmann, T. Janowitz, D. I. Jodrell, D. A. Tuveson and D. T. Fearon (2013). "Targeting CXCL12 from FAP-expressing carcinoma-associated fibroblasts synergizes with anti-PD-L1 immunotherapy in pancreatic cancer." Proc Natl Acad Sci U S A 110(50): 20212-20217.
Ferguson, P. C., A. M. Griffin, B. O'Sullivan, C. N. Catton, A. M. Davis, A. Murji, R. S. Bell and J. S. Wunder (2006). "Bone invasion in extremity soft-tissue sarcoma: impact on disease outcomes." Cancer 106(12): 2692-2700.
Ferrari, A., G. Bisogno, R. Alaggio, G. Cecchetto, P. Collini, A. Rosolen, C. Meazza, P. Indolfi, A. Garaventa, L. De Sio, P. D'Angelo, P. Tamaro, M. Casanova and M. Carli (2008). "Synovial sarcoma of children and adolescents: the prognostic role of axial sites." Eur J Cancer 44(9): 1202-1209.
Ferrari, A., Y. Y. Chi, G. L. De Salvo, D. Orbach, B. Brennan, R. L. Randall, M. B. McCarville, J. O. Black, R. Alaggio, D. S. Hawkins, G. Bisogno and S. L. Spunt (2017). "Surgery alone is sufficient therapy for children and adolescents with low-risk synovial sarcoma: A joint analysis from the European paediatric soft tissue sarcoma Study Group and the Children's Oncology Group." Eur J Cancer 78: 1-6.
Ferrari, A., G. L. De Salvo, B. Brennan, M. M. van Noesel, A. De Paoli, M. Casanova, N. Francotte, A. Kelsey, R. Alaggio, O. Oberlin, M. Carli, M. Ben-Arush, C. Bergeron, J. H. Merks, M. Jenney, M. C. Stevens, G. Bisogno and D. Orbach (2015). "Synovial sarcoma in children and adolescents: the European Pediatric Soft Tissue Sarcoma Study Group prospective trial (EpSSG NRSTS 2005)." Ann Oncol 26(3): 567-572.
Ferrari, A., A. Gronchi, M. Casanova, C. Meazza, L. Gandola, P. Collini, L. Lozza, R. Bertulli, P. Olmi and P. G. Casali (2004). "Synovial sarcoma: a retrospective analysis of 271 patients of all ages treated at a single institution." Cancer 101(3): 627-634.
Ferrari, A., I. Sultan, T. T. Huang, C. Rodriguez-Galindo, A. Shehadeh, C. Meazza, K. K. Ness, M. Casanova and S. L. Spunt (2011). "Soft tissue sarcoma across the age spectrum: a population-based study from the Surveillance Epidemiology and End Results database." Pediatr Blood Cancer 57(6): 943-949.
Fiore, M., A. Sambri, P. Spinnato, R. Zucchini, C. Giannini, E. Caldari, M. G. Pirini and M. De Paolis (2021). "The Biology of Synovial Sarcoma: State-of-the-Art and Future Perspectives." Curr Treat Options Oncol 22(12): 109.
Foy, S. P., K. Jacoby, D. A. Bota, T. Hunter, Z. Pan, E. Stawiski, Y. Ma, W. Lu, S. Peng, C. L. Wang, B. Yuen, O. Dalmas, K. Heeringa, B. Sennino, A. Conroy, M. T. Bethune, I. Mende, W. White, M. Kukreja, S. Gunturu, E. Humphrey, A. Hussaini, D. An, A. J. Litterman, B. B. Quach, A. H. C. Ng, Y. Lu, C. Smith, K. M. Campbell, D. Anaya, L. Skrdlant, E. Y. Huang, V. Mendoza, J. Mathur, L. Dengler, B. Purandare, R. Moot, M. C. Yi, R. Funke, A. Sibley, T. Stallings-Schmitt, D. Y. Oh, B. Chmielowski, M. Abedi, Y. Yuan, J. A. Sosman, S. M. Lee, A. J. Schoenfeld, D. Baltimore, J. R. Heath, A. Franzusoff, A. Ribas, A. V. Rao and S. J. Mandl (2023). "Non-viral precision T cell receptor replacement for personalized cell therapy." Nature 615(7953): 687-696.
Francisco, L. M., P. T. Sage and A. H. Sharpe (2010). "The PD-1 pathway in tolerance and autoimmunity." Immunol Rev 236: 219-242.
Galassi, C., T. A. Chan, I. Vitale and L. Galluzzi (2024). "The hallmarks of cancer immune evasion." Cancer Cell 42(11): 1825-1863.
Gallucci, S., S. Meka and A. M. Gamero (2021). "Abnormalities of the type I interferon signaling pathway in lupus autoimmunity." Cytokine 146: 155633.
Galon, J. and D. Bruni (2019). "Approaches to treat immune hot, altered and cold tumours with combination immunotherapies." Nat Rev Drug Discov 18(3): 197-218.
Gao, J., L. Z. Shi, H. Zhao, J. Chen, L. Xiong, Q. He, T. Chen, J. Roszik, C. Bernatchez, S. E. Woodman, P. L. Chen, P. Hwu, J. P. Allison, A. Futreal, J. A. Wargo and P. Sharma (2016). "Loss of IFN-gamma Pathway Genes in Tumor Cells as a Mechanism of Resistance to Anti-CTLA-4 Therapy." Cell 167(2): 397-404 e399.
Garraway, L. A., J. Verweij and K. V. Ballman (2013). "Precision oncology: an overview." J Clin Oncol 31(15): 1803-1805.
Gazendam, A. M., S. Popovic, S. Munir, N. Parasu, D. Wilson and M. Ghert (2021). "Synovial Sarcoma: A Clinical Review." Curr Oncol 28(3): 1909-1920.
Genkinger, J. M., C. M. Kitahara, L. Bernstein, A. Berrington de Gonzalez, M. Brotzman, J. W. Elena, G. G. Giles, P. Hartge, P. N. Singh, R. Z. Stolzenberg-Solomon, E. Weiderpass, H. O. Adami, K. E. Anderson, L. E. Beane-Freeman, J. E. Buring, G. E. Fraser, C. S. Fuchs, S. M. Gapstur, J. M. Gaziano, K. J. Helzlsouer, J. V. Lacey, Jr., M. S. Linet, J. J. Liu, Y. Park, U. Peters, M. P. Purdue, K. Robien, C. Schairer, H. D. Sesso, K. Visvanathan, E. White, A. Wolk, B. M. Wolpin, A. Zeleniuch-Jacquotte and E. J. Jacobs (2015). "Central adiposity, obesity during early adulthood, and pancreatic cancer mortality in a pooled analysis of cohort studies." Ann Oncol 26(11): 2257-2266.
Ghert, M. A., A. Abudu, N. Driver, A. M. Davis, A. M. Griffin, D. Pearce, L. White, B. O'Sullivan, C. N. Catton, R. S. Bell and J. S. Wunder (2005). "The indications for and the prognostic significance of amputation as the primary surgical procedure for localized soft tissue sarcoma of the extremity." Ann Surg Oncol 12(1): 10-17.
Giannopoulos, A., C. Constantinides, E. Fokaeas, C. Stravodimos, M. Giannopoulou, A. Kyroudi and A. Gounaris (2003). "The immunomodulating effect of interferon-gamma intravesical instillations in preventing bladder cancer recurrence." Clin Cancer Res 9(15): 5550-5558.
Gill, J. and R. Gorlick (2021). "Advancing therapy for osteosarcoma." Nat Rev Clin Oncol 18(10): 609-624.
Gingrich, A. A., A. S. Marrufo, Y. Liu, C. S. Li, M. A. Darrow, A. M. Monjazeb, S. W. Thorpe and R. J. Canter (2020). "Radiotherapy is Associated With Improved Survival in Patients With Synovial Sarcoma Undergoing Surgery: A National Cancer Database Analysis." J Surg Res 255: 378-387.
Gore, A. J., S. L. Deitz, L. R. Palam, K. E. Craven and M. Korc (2014). "Pancreatic cancer-associated retinoblastoma 1 dysfunction enables TGF-beta to promote proliferation." J Clin Invest 124(1): 338-352.
Gorlick, R., K. Janeway, S. Lessnick, R. L. Randall, N. Marina and C. O. G. B. T. Committee (2013). "Children's Oncology Group's 2013 blueprint for research: bone tumors." Pediatr Blood Cancer 60(6): 1009-1015.
Gril, B., D. Palmieri, Y. Qian, D. Smart, L. Ileva, D. J. Liewehr, S. M. Steinberg and P. S. Steeg (2011). "Pazopanib reveals a role for tumor cell B-Raf in the prevention of HER2+ breast cancer brain metastasis." Clin Cancer Res 17(1): 142-153.
Grimer, R. J., S. Bielack, S. Flege, S. R. Cannon, G. Foleras, I. Andreeff, T. Sokolov, A. Taminiau, M. Dominkus, M. San-Julian, Y. Kollender, G. Gosheger and S. European Musculo Skeletal Oncology (2005). "Periosteal osteosarcoma--a European review of outcome." Eur J Cancer 41(18): 2806-2811.
Grimer, R. J., S. R. Cannon, A. M. Taminiau, S. Bielack, B. Kempf-Bielack, R. Windhager, M. Dominkus, G. Saeter, H. Bauer, I. Meller, M. Szendroi, G. Folleras, M. San-Julian and J. van der Eijken (2003). "Osteosarcoma over the age of forty." Eur J Cancer 39(2): 157-163.
Guadagnolo, B. A., G. K. Zagars, M. T. Ballo, S. R. Patel, V. O. Lewis, P. W. Pisters, R. S. Benjamin and R. E. Pollock (2007). "Long-term outcomes for synovial sarcoma treated with conservation surgery and radiotherapy." Int J Radiat Oncol Biol Phys 69(4): 1173-1180.
Gumber, D. and L. D. Wang (2022). "Improving CAR-T immunotherapy: Overcoming the challenges of T cell exhaustion." EBioMedicine 77: 103941.
Gundle, K. R., S. Gupta, L. Kafchinski, A. M. Griffin, R. A. Kandel, B. C. Dickson, P. W. Chung, C. N. Catton, B. O'Sullivan, P. C. Ferguson and J. S. Wunder (2017). "An Analysis of Tumor- and Surgery-Related Factors that Contribute to Inadvertent Positive Margins Following Soft Tissue Sarcoma Resection." Ann Surg Oncol 24(8): 2137-2144.
Guo, F., Y. Wang, J. Liu, S. C. Mok, F. Xue and W. Zhang (2016). "CXCL12/CXCR4: a symbiotic bridge linking cancer cells and their stromal neighbors in oncogenic communication networks." Oncogene 35(7): 816-826.
Guyon, A. (2014). "CXCL12 chemokine and its receptors as major players in the interactions between immune and nervous systems." Front Cell Neurosci 8: 65.
Harjunpaa, H. and C. Guillerey (2020). "TIGIT as an emerging immune checkpoint." Clin Exp Immunol 200(2): 108-119.
Harlin, H., Y. Meng, A. C. Peterson, Y. Zha, M. Tretiakova, C. Slingluff, M. McKee and T. F. Gajewski (2009). "Chemokine expression in melanoma metastases associated with CD8+ T-cell recruitment." Cancer Res 69(7): 3077-3085.
Hartupee, C., B. M. Nagalo, C. Y. Chabu, M. Z. Tesfay, J. Coleman-Barnett, J. T. West and O. Moaven (2024). "Pancreatic cancer tumor microenvironment is a major therapeutic barrier and target." Front Immunol 15: 1287459.
He, J., N. Ahuja, M. A. Makary, J. L. Cameron, F. E. Eckhauser, M. A. Choti, R. H. Hruban, T. M. Pawlik and C. L. Wolfgang (2014). "2564 resected periampullary adenocarcinomas at a single institution: trends over three decades." HPB (Oxford) 16(1): 83-90.
Hebert, J. S., M. Rehani and R. Stiegelmar (2017). "Osseointegration for Lower-Limb Amputation: A Systematic Review of Clinical Outcomes." JBJS Rev 5(10): e10.
Hegde, P. S. and D. S. Chen (2020). "Top 10 Challenges in Cancer Immunotherapy." Immunity 52(1): 17-35.
Hezel, A. F., A. C. Kimmelman, B. Z. Stanger, N. Bardeesy and R. A. Depinho (2006). "Genetics and biology of pancreatic ductal adenocarcinoma." Genes Dev 20(10): 1218-1249.
Huang, R., X. Li, Y. He, W. Zhu, L. Gao, Y. Liu, L. Gao, Q. Wen, J. F. Zhong, C. Zhang and X. Zhang (2020). "Recent advances in CAR-T cell engineering." J Hematol Oncol 13(1): 86.
Hurwitz, H. I., A. Dowlati, S. Saini, S. Savage, A. B. Suttle, D. M. Gibson, J. P. Hodge, E. M. Merkle and L. Pandite (2009). "Phase I trial of pazopanib in patients with advanced cancer." Clin Cancer Res 15(12): 4220-4227.
Ikeda, H., L. J. Old and R. D. Schreiber (2002). "The roles of IFN gamma in protection against tumor development and cancer immunoediting." Cytokine Growth Factor Rev 13(2): 95-109.
Isakoff, M. S., S. S. Bielack, P. Meltzer and R. Gorlick (2015). "Osteosarcoma: Current Treatment and a Collaborative Pathway to Success." J Clin Oncol 33(27): 3029-3035.
Italiano, A., N. Penel, Y. M. Robin, B. Bui, A. Le Cesne, S. Piperno-Neumann, M. Tubiana-Hulin, E. Bompas, C. Chevreau, N. Isambert, S. Leyvraz, P. P. du Chatelard, A. Thyss, J. M. Coindre and J. Y. Blay (2009). "Neo/adjuvant chemotherapy does not improve outcome in resected primary synovial sarcoma: a study of the French Sarcoma Group." Ann Oncol 20(3): 425-430.
Ivashkiv, L. B. and L. T. Donlin (2014). "Regulation of type I interferon responses." Nat Rev Immunol 14(1): 36-49.
Jacobs, A. J., C. D. Morris and A. S. Levin (2018). "Synovial Sarcoma Is Not Associated With a Higher Risk of Lymph Node Metastasis Compared With Other Soft Tissue Sarcomas." Clin Orthop Relat Res 476(3): 589-598.
Jaffe, N., A. Puri and H. Gelderblom (2013). "Osteosarcoma: evolution of treatment paradigms." Sarcoma 2013: 203531.
Janas, M. L., G. Varano, K. Gudmundsson, M. Noda, T. Nagasawa and M. Turner (2010). "Thymic development beyond beta-selection requires phosphatidylinositol 3-kinase activation by CXCR4." J Exp Med 207(1): 247-261.
Janiaud, P., S. Serghiou and J. P. A. Ioannidis (2019). "New clinical trial designs in the era of precision medicine: An overview of definitions, strengths, weaknesses, and current use in oncology." Cancer Treat Rev 73: 20-30.
Jo, V. Y. and C. D. Fletcher (2014). "WHO classification of soft tissue tumours: an update based on the 2013 (4th) edition." Pathology 46(2): 95-104.
Jones, K. B. (2011). "Osteosarcomagenesis: modeling cancer initiation in the mouse." Sarcoma 2011: 694136.
Jones, K. B., J. J. Barrott, M. Xie, M. Haldar, H. Jin, J. F. Zhu, M. J. Monument, T. L. Mosbruger, E. M. Langer, R. L. Randall, R. K. Wilson, B. R. Cairns, L. Ding and M. R. Capecchi (2016). "The impact of chromosomal translocation locus and fusion oncogene coding sequence in synovial sarcomagenesis." Oncogene 35(38): 5021-5032.
Judson, I., J. Verweij, H. Gelderblom, J. T. Hartmann, P. Schoffski, J. Y. Blay, J. M. Kerst, J. Sufliarsky, J. Whelan, P. Hohenberger, A. Krarup-Hansen, T. Alcindor, S. Marreaud, S. Litiere, C. Hermans, C. Fisher, P. C. Hogendoorn, A. P. dei Tos, W. T. van der Graaf, O. European, T. Treatment of Cancer Soft and G. Bone Sarcoma (2014). "Doxorubicin alone versus intensified doxorubicin plus ifosfamide for first-line treatment of advanced or metastatic soft-tissue sarcoma: a randomised controlled phase 3 trial." Lancet Oncol 15(4): 415-423.
Kager, L., A. Zoubek, U. Potschger, U. Kastner, S. Flege, B. Kempf-Bielack, D. Branscheid, R. Kotz, M. Salzer-Kuntschik, W. Winkelmann, G. Jundt, H. Kabisch, P. Reichardt, H. Jurgens, H. Gadner, S. S. Bielack and G. Cooperative German-Austrian-Swiss Osteosarcoma Study (2003). "Primary metastatic osteosarcoma: presentation and outcome of patients treated on neoadjuvant Cooperative Osteosarcoma Study Group protocols." J Clin Oncol 21(10): 2011-2018.
Kampe, C. E., G. Rosen, F. Eilber, J. Eckardt, S. Lowenbraun, J. Foster, C. Forscher and M. Selch (1993). "Synovial sarcoma. A study of intensive chemotherapy in 14 patients with localized disease." Cancer 72(7): 2161-2169.
Kao, S. C. (2019). "Overview of the clinical and imaging features of the most common nonrhabdomyosarcoma soft-tissue sarcomas." Pediatr Radiol 49(11): 1524-1533.
Kareva, I. (2017). "A Combination of Immune Checkpoint Inhibition with Metronomic Chemotherapy as a Way of Targeting Therapy-Resistant Cancer Cells." Int J Mol Sci 18(10).
Kasraeian, S., D. C. Allison, E. R. Ahlmann, A. N. Fedenko and L. R. Menendez (2010). "A comparison of fine-needle aspiration, core biopsy, and surgical biopsy in the diagnosis of extremity soft tissue masses." Clin Orthop Relat Res 468(11): 2992-3002.
Kawaguchi, N., A. R. Ahmed, S. Matsumoto, J. Manabe and Y. Matsushita (2004). "The concept of curative margin in surgery for bone and soft tissue sarcoma." Clin Orthop Relat Res(419): 165-172.
Kawai, A., N. Araki, H. Sugiura, T. Ueda, T. Yonemoto, M. Takahashi, H. Morioka, H. Hiraga, T. Hiruma, T. Kunisada, A. Matsumine, T. Tanase, T. Hasegawa and S. Takahashi (2015). "Trabectedin monotherapy after standard chemotherapy versus best supportive care in patients with advanced, translocation-related sarcoma: a randomised, open-label, phase 2 study." Lancet Oncol 16(4): 406-416.
Kawai, A., N. Naito, A. Yoshida, Y. Morimoto, M. Ouchida, K. Shimizu and Y. Beppu (2004). "Establishment and characterization of a biphasic synovial sarcoma cell line, SYO-1." Cancer Lett 204(1): 105-113.
Keam, S. J. (2024). "Afamitresgene Autoleucel: First Approval." Mol Diagn Ther 28(6): 861-866.
Kempf-Bielack, B., S. S. Bielack, H. Jurgens, D. Branscheid, W. E. Berdel, G. U. Exner, U. Gobel, K. Helmke, G. Jundt, H. Kabisch, M. Kevric, T. Klingebiel, R. Kotz, R. Maas, R. Schwarz, M. Semik, J. Treuner, A. Zoubek and K. Winkler (2005). "Osteosarcoma relapse after combined modality therapy: an analysis of unselected patients in the Cooperative Osteosarcoma Study Group (COSS)." J Clin Oncol 23(3): 559-568.
Kioi, M., H. Vogel, G. Schultz, R. M. Hoffman, G. R. Harsh and J. M. Brown (2010). "Inhibition of vasculogenesis, but not angiogenesis, prevents the recurrence of glioblastoma after irradiation in mice." J Clin Invest 120(3): 694-705.
Kleeff, J., M. Korc, M. Apte, C. La Vecchia, C. D. Johnson, A. V. Biankin, R. E. Neale, M. Tempero, D. A. Tuveson, R. H. Hruban and J. P. Neoptolemos (2016). "Pancreatic cancer." Nat Rev Dis Primers 2: 16022.
Klein, M. J. and G. P. Siegal (2006). "Osteosarcoma: anatomic and histologic variants." Am J Clin Pathol 125(4): 555-581.
Kohli, K., L. Yao, T. S. Nowicki, S. Zhang, R. G. Black, B. A. Schroeder, E. A. Farrar, J. Cao, H. Sloan, D. Stief, L. D. Cranmer, M. J. Wagner, D. S. Hawkins, V. G. Pillarisetty, A. Ribas, J. Campbell, R. H. Pierce, E. Y. Kim, R. L. Jones, S. R. Riddell, C. Yee and S. M. Pollack (2021). "IL-15 mediated expansion of rare durable memory T cells following adoptive cellular therapy." J Immunother Cancer 9(5).
Kotecha, K., K. Tree, W. A. Ziaziaris, S. C. McKay, H. Wand, J. Samra and A. Mittal (2024). "Centralization of Pancreaticoduodenectomy: A Systematic Review and Spline Regression Analysis to Recommend Minimum Volume for a Specialist Pancreas Service." Ann Surg 279(6): 953-960.
Koyama, S., E. A. Akbay, Y. Y. Li, G. S. Herter-Sprie, K. A. Buczkowski, W. G. Richards, L. Gandhi, A. J. Redig, S. J. Rodig, H. Asahina, R. E. Jones, M. M. Kulkarni, M. Kuraguchi, S. Palakurthi, P. E. Fecci, B. E. Johnson, P. A. Janne, J. A. Engelman, S. P. Gangadharan, D. B. Costa, G. J. Freeman, R. Bueno, F. S. Hodi, G. Dranoff, K. K. Wong and P. S. Hammerman (2016). "Adaptive resistance to therapeutic PD-1 blockade is associated with upregulation of alternative immune checkpoints." Nat Commun 7: 10501.
Kozin, S. V., W. S. Kamoun, Y. Huang, M. R. Dawson, R. K. Jain and D. G. Duda (2010). "Recruitment of myeloid but not endothelial precursor cells facilitates tumor regrowth after local irradiation." Cancer Res 70(14): 5679-5685.
Krieg, A. H., F. Hefti, B. M. Speth, G. Jundt, L. Guillou, U. G. Exner, A. R. von Hochstetter, M. D. Cserhati, B. Fuchs, E. Mouhsine, A. Kaelin, F. M. Klenke and K. A. Siebenrock (2011). "Synovial sarcomas usually metastasize after >5 years: a multicenter retrospective analysis with minimum follow-up of 10 years for survivors." Ann Oncol 22(2): 458-467.
Kusser, W. (2000). "Chemically modified nucleic acid aptamers for in vitro selections: evolving evolution." J Biotechnol 74(1): 27-38.
Ladanyi, M., C. R. Antonescu, D. H. Leung, J. M. Woodruff, A. Kawai, J. H. Healey, M. F. Brennan, J. A. Bridge, J. R. Neff, F. G. Barr, J. D. Goldsmith, J. S. Brooks, J. R. Goldblum, S. Z. Ali, J. Shipley, C. S. Cooper, C. Fisher, B. Skytting and O. Larsson (2002). "Impact of SYT-SSX fusion type on the clinical behavior of synovial sarcoma: a multi-institutional retrospective study of 243 patients." Cancer Res 62(1): 135-140.
Lai, J. P., P. F. Robbins, M. Raffeld, P. P. Aung, M. Tsokos, S. A. Rosenberg, M. M. Miettinen and C. C. Lee (2012). "NY-ESO-1 expression in synovial sarcoma and other mesenchymal tumors: significance for NY-ESO-1-based targeted therapy and differential diagnosis." Mod Pathol 25(6): 854-858.
Lawlor, R. T., N. Veronese, A. Pea, A. Nottegar, L. Smith, C. Pilati, J. Demurtas, M. Fassan, L. Cheng and C. Luchini (2019). "Alternative lengthening of telomeres (ALT) influences survival in soft tissue sarcomas: a systematic review with meta-analysis." BMC Cancer 19(1): 232.
Lee, E. S. and J. M. Lee (2014). "Imaging diagnosis of pancreatic cancer: a state-of-the-art review." World J Gastroenterol 20(24): 7864-7877.
Lee, Y. G., I. Marks, M. Srinivasarao, A. K. Kanduluru, S. M. Mahalingam, X. Liu, H. Chu and P. S. Low (2019). "Use of a Single CAR T Cell and Several Bispecific Adapters Facilitates Eradication of Multiple Antigenically Different Solid Tumors." Cancer Res 79(2): 387-396.
Lewis, D. R., N. L. Seibel, A. W. Smith and M. R. Stedman (2014). "Adolescent and young adult cancer survival." J Natl Cancer Inst Monogr 2014(49): 228-235.
Lewis, J. J., C. R. Antonescu, D. H. Leung, D. Blumberg, J. H. Healey, J. M. Woodruff and M. F. Brennan (2000). "Synovial sarcoma: a multivariate analysis of prognostic factors in 112 patients with primary localized tumors of the extremity." J Clin Oncol 18(10): 2087-2094.
Liang, C., H. Mao, J. Tan, Y. Ji, F. Sun, W. Dou, H. Wang, H. Wang and J. Gao (2015). "Synovial sarcoma: Magnetic resonance and computed tomography imaging features and differential diagnostic considerations." Oncol Lett 9(2): 661-666.
Lin, H., J. Cheng, W. Mu, J. Zhou and L. Zhu (2021). "Advances in Universal CAR-T Cell Therapy." Front Immunol 12: 744823.
Lin, Y. H., B. E. Jewell, J. Gingold, L. Lu, R. Zhao, L. L. Wang and D. F. Lee (2017). "Osteosarcoma: Molecular Pathogenesis and iPSC Modeling." Trends Mol Med 23(8): 737-755.
Liu, S. C., R. Alomran, S. B. Chernikova, F. Lartey, J. Stafford, T. Jang, M. Merchant, D. Zboralski, S. Zollner, A. Kruschinski, S. Klussmann, L. Recht and J. M. Brown (2014). "Blockade of SDF-1 after irradiation inhibits tumor recurrences of autochthonous brain tumors in rats." Neuro Oncol 16(1): 21-28.
Liu, Y., Y. Hu, J. Xue, J. Li, J. Yi, J. Bu, Z. Zhang, P. Qiu and X. Gu (2023). "Advances in immunotherapy for triple-negative breast cancer." Mol Cancer 22(1): 145.
Lopez de Padilla, C. M. and T. B. Niewold (2016). "The type I interferons: Basic concepts and clinical relevance in immune-mediated inflammatory diseases." Gene 576(1 Pt 1): 14-21.
Losic, B., A. J. Craig, C. Villacorta-Martin, S. N. Martins-Filho, N. Akers, X. Chen, M. E. Ahsen, J. von Felden, I. Labgaa, D. D'Avola, K. Allette, S. A. Lira, G. C. Furtado, T. Garcia-Lezana, P. Restrepo, A. Stueck, S. C. Ward, M. I. Fiel, S. P. Hiotis, G. Gunasekaran, D. Sia, E. E. Schadt, R. Sebra, M. Schwartz, J. M. Llovet, S. Thung, G. Stolovitzky and A. Villanueva (2020). "Intratumoral heterogeneity and clonal evolution in liver cancer." Nat Commun 11(1): 291.
Lugano, R., M. Ramachandran and A. Dimberg (2020). "Tumor angiogenesis: causes, consequences, challenges and opportunities." Cell Mol Life Sci 77(9): 1745-1770.
Luk, S. J., I. J. ME, A. Somarakis, I. Acem, I. B. de Bruijn, K. Szuhai, J. Bovee, N. de Miranda, J. H. F. Falkenburg and M. H. M. Heemskerk (2024). "Immunological differences between monophasic and biphasic synovial sarcoma with implications for immunotherapy." Cancer Immunol Immunother 74(1): 31.
Ma, S., X. Li, X. Wang, L. Cheng, Z. Li, C. Zhang, Z. Ye and Q. Qian (2019). "Current Progress in CAR-T Cell Therapy for Solid Tumors." Int J Biol Sci 15(12): 2548-2560.
Ma, T., B. W. Renz, M. Ilmer, D. Koch, Y. Yang, J. Werner and A. V. Bazhin (2022). "Myeloid-Derived Suppressor Cells in Solid Tumors." Cells 11(2).
Machelon, V., F. Gaudin, S. Camilleri-Broet, S. Nasreddine, L. Bouchet-Delbos, E. Pujade-Lauraine, J. Alexandre, L. Gladieff, F. Arenzana-Seisdedos, D. Emilie, S. Prevot, P. Broet and K. Balabanian (2011). "CXCL12 expression by healthy and malignant ovarian epithelial cells." BMC Cancer 11: 97.
Maduekwe, U. N., F. J. Hornicek, D. S. Springfield, K. A. Raskin, D. C. Harmon, E. Choy, A. E. Rosenberg, G. P. Nielsen, T. F. DeLaney, Y. L. Chen, M. J. Ott and S. S. Yoon (2009). "Role of sentinel lymph node biopsy in the staging of synovial, epithelioid, and clear cell sarcomas." Ann Surg Oncol 16(5): 1356-1363.
Manguso, R. T., H. W. Pope, M. D. Zimmer, F. D. Brown, K. B. Yates, B. C. Miller, N. B. Collins, K. Bi, M. W. LaFleur, V. R. Juneja, S. A. Weiss, J. Lo, D. E. Fisher, D. Miao, E. Van Allen, D. E. Root, A. H. Sharpe, J. G. Doench and W. N. Haining (2017). "In vivo CRISPR screening identifies Ptpn2 as a cancer immunotherapy target." Nature 547(7664): 413-418.
Mankin, H. J., C. J. Mankin and M. A. Simon (1996). "The hazards of the biopsy, revisited. Members of the Musculoskeletal Tumor Society." J Bone Joint Surg Am 78(5): 656-663.
Mariathasan, S., S. J. Turley, D. Nickles, A. Castiglioni, K. Yuen, Y. Wang, E. E. Kadel, III, H. Koeppen, J. L. Astarita, R. Cubas, S. Jhunjhunwala, R. Banchereau, Y. Yang, Y. Guan, C. Chalouni, J. Ziai, Y. Senbabaoglu, S. Santoro, D. Sheinson, J. Hung, J. M. Giltnane, A. A. Pierce, K. Mesh, S. Lianoglou, J. Riegler, R. A. D. Carano, P. Eriksson, M. Hoglund, L. Somarriba, D. L. Halligan, M. S. van der Heijden, Y. Loriot, J. E. Rosenberg, L. Fong, I. Mellman, D. S. Chen, M. Green, C. Derleth, G. D. Fine, P. S. Hegde, R. Bourgon and T. Powles (2018). "TGFbeta attenuates tumour response to PD-L1 blockade by contributing to exclusion of T cells." Nature 554(7693): 544-548.
Marina, N. M., S. Smeland, S. S. Bielack, M. Bernstein, G. Jovic, M. D. Krailo, J. M. Hook, C. Arndt, H. van den Berg, B. Brennan, B. Brichard, K. L. B. Brown, T. Butterfass-Bahloul, G. Calaminus, H. E. Daldrup-Link, M. Eriksson, M. C. Gebhardt, H. Gelderblom, J. Gerss, R. Goldsby, A. Goorin, R. Gorlick, H. E. Grier, J. P. Hale, K. S. Hall, J. Hardes, D. S. Hawkins, K. Helmke, P. C. W. Hogendoorn, M. S. Isakoff, K. A. Janeway, H. Jurgens, L. Kager, T. Kuhne, C. C. Lau, P. J. Leavey, S. L. Lessnick, L. Mascarenhas, P. A. Meyers, H. Mottl, M. Nathrath, Z. Papai, R. L. Randall, P. Reichardt, M. Renard, A. A. Safwat, C. L. Schwartz, M. C. G. Stevens, S. J. Strauss, L. Teot, M. Werner, M. R. Sydes and J. S. Whelan (2016). "Comparison of MAPIE versus MAP in patients with a poor response to preoperative chemotherapy for newly diagnosed high-grade osteosarcoma (EURAMOS-1): an open-label, international, randomised controlled trial." Lancet Oncol 17(10): 1396-1408.
Mariuzza, R. A., S. Shahid and S. S. Karade (2024). "The immune checkpoint receptor LAG3: Structure, function, and target for cancer immunotherapy." J Biol Chem 300(5): 107241.
Marofi, F., R. Motavalli, V. A. Safonov, L. Thangavelu, A. V. Yumashev, M. Alexander, N. Shomali, M. S. Chartrand, Y. Pathak, M. Jarahian, S. Izadi, A. Hassanzadeh, N. Shirafkan, S. Tahmasebi and F. M. Khiavi (2021). "CAR T cells in solid tumors: challenges and opportunities." Stem Cell Res Ther 12(1): 81.
Mastrangelo, G., J. M. Coindre, F. Ducimetiere, A. P. Dei Tos, E. Fadda, J. Y. Blay, A. Buja, U. Fedeli, L. Cegolon, A. Frasson, D. Ranchere-Vince, C. Montesco, I. Ray-Coquard and C. R. Rossi (2012). "Incidence of soft tissue sarcoma and beyond: a population-based prospective study in 3 European regions." Cancer 118(21): 5339-5348.
Matsunobu, A., R. Imai, T. Kamada, T. Imaizumi, H. Tsuji, H. Tsujii, Y. Shioyama, H. Honda, S. Tatezaki, B. Working Group for and S. Soft Tissue (2012). "Impact of carbon ion radiotherapy for unresectable osteosarcoma of the trunk." Cancer 118(18): 4555-4563.
Mayer-Barber, K. D. and B. Yan (2017). "Clash of the Cytokine Titans: counter-regulation of interleukin-1 and type I interferon-mediated inflammatory responses." Cell Mol Immunol 14(1): 22-35.
McGinnis, S. and T. L. Madden (2004). "BLAST: at the core of a powerful and diverse set of sequence analysis tools." Nucleic Acids Res 32(Web Server issue): W20-25.
McGrory, J. E., D. J. Pritchard, C. A. Arndt, A. G. Nascimento, E. D. Remstein and C. M. Rowland (2000). "Nonrhabdomyosarcoma soft tissue sarcomas in children. The Mayo Clinic experience." Clin Orthop Relat Res(374): 247-258.
McNab, F., K. Mayer-Barber, A. Sher, A. Wack and A. O'Garra (2015). "Type I interferons in infectious disease." Nat Rev Immunol 15(2): 87-103.
Meltzer, P. S. and L. J. Helman (2021). "New Horizons in the Treatment of Osteosarcoma." N Engl J Med 385(22): 2066-2076.
Meyer, J. S., H. R. Nadel, N. Marina, R. B. Womer, K. L. Brown, J. F. Eary, R. Gorlick, H. E. Grier, R. L. Randall, E. R. Lawlor, S. L. Lessnick, P. J. Schomberg and M. D. Kailo (2008). "Imaging guidelines for children with Ewing sarcoma and osteosarcoma: a report from the Children's Oncology Group Bone Tumor Committee." Pediatr Blood Cancer 51(2): 163-170.
Meyer, O. (2009). "Interferons and autoimmune disorders." Joint Bone Spine 76(5): 464-473.
Meyers, P. A., C. L. Schwartz, M. D. Krailo, J. H. Healey, M. L. Bernstein, D. Betcher, W. S. Ferguson, M. C. Gebhardt, A. M. Goorin, M. Harris, E. Kleinerman, M. P. Link, H. Nadel, M. Nieder, G. P. Siegal, M. A. Weiner, R. J. Wells, R. B. Womer, H. E. Grier and G. Children's Oncology (2008). "Osteosarcoma: the addition of muramyl tripeptide to chemotherapy improves overall survival--a report from the Children's Oncology Group." J Clin Oncol 26(4): 633-638.
Michal, M., J. C. Fanburg-Smith, J. Lasota, J. F. Fetsch, J. Lichy and M. Miettinen (2006). "Minute synovial sarcomas of the hands and feet: a clinicopathologic study of 21 tumors less than 1 cm." Am J Surg Pathol 30(6): 721-726.
Miller, K. D., L. Nogueira, A. B. Mariotto, J. H. Rowland, K. R. Yabroff, C. M. Alfano, A. Jemal, J. L. Kramer and R. L. Siegel (2019). "Cancer treatment and survivorship statistics, 2019." CA Cancer J Clin 69(5): 363-385.
Mirabello, L., R. J. Troisi and S. A. Savage (2009). "International osteosarcoma incidence patterns in children and adolescents, middle ages and elderly persons." Int J Cancer 125(1): 229-234.
Mirabello, L., R. J. Troisi and S. A. Savage (2009). "Osteosarcoma incidence and survival rates from 1973 to 2004: data from the Surveillance, Epidemiology, and End Results Program." Cancer 115(7): 1531-1543.
Mirabello, L., B. Zhu, R. Koster, E. Karlins, M. Dean, M. Yeager, M. Gianferante, L. G. Spector, L. M. Morton, D. Karyadi, L. L. Robison, G. T. Armstrong, S. Bhatia, L. Song, N. Pankratz, M. Pinheiro, J. M. Gastier-Foster, R. Gorlick, S. R. C. de Toledo, A. S. Petrilli, A. Patino-Garcia, F. Lecanda, M. Gutierrez-Jimeno, M. Serra, C. Hattinger, P. Picci, K. Scotlandi, A. M. Flanagan, R. Tirabosco, M. F. Amary, N. Kurucu, I. E. Ilhan, M. L. Ballinger, D. M. Thomas, D. A. Barkauskas, G. Mejia-Baltodano, P. Valverde, B. D. Hicks, B. Zhu, M. Wang, A. A. Hutchinson, M. Tucker, J. Sampson, M. T. Landi, N. D. Freedman, S. Gapstur, B. Carter, R. N. Hoover, S. J. Chanock and S. A. Savage (2020). "Frequency of Pathogenic Germline Variants in Cancer-Susceptibility Genes in Patients With Osteosarcoma." JAMA Oncol 6(5): 724-734.
Mirnezami, R., J. Nicholson and A. Darzi (2012). "Preparing for precision medicine." N Engl J Med 366(6): 489-491.
Mitchell, G., S. M. Pollack and M. J. Wagner (2021). "Targeting cancer testis antigens in synovial sarcoma." J Immunother Cancer 9(6).
Morotti, M., A. Albukhari, A. Alsaadi, M. Artibani, J. D. Brenton, S. M. Curbishley, T. Dong, M. L. Dustin, Z. Hu, N. McGranahan, M. L. Miller, L. Santana-Gonzalez, L. W. Seymour, T. Shi, P. Van Loo, C. Yau, H. White, N. Wietek, D. N. Church, D. C. Wedge and A. A. Ahmed (2021). "Promises and challenges of adoptive T-cell therapies for solid tumours." Br J Cancer 124(11): 1759-1776.
Motosugi, U., T. Ichikawa, H. Morisaka, H. Sou, A. Muhi, K. Kimura, K. Sano and T. Araki (2011). "Detection of pancreatic carcinoma and liver metastases with gadoxetic acid-enhanced MR imaging: comparison with contrast-enhanced multi-detector row CT." Radiology 260(2): 446-453.
Mutsaers, A. J. and C. R. Walkley (2014). "Cells of origin in osteosarcoma: mesenchymal stem cells or osteoblast committed cells?" Bone 62: 56-63.
Naing, K. W., A. M. Monjazeb, C. S. Li, L. Y. Lee, A. Yang, D. Borys and R. J. Canter (2015). "Perioperative radiotherapy is associated with improved survival among patients with synovial sarcoma: A SEER analysis." J Surg Oncol 111(2): 158-164.
Naka, N., S. Takenaka, N. Araki, T. Miwa, N. Hashimoto, K. Yoshioka, S. Joyama, K. Hamada, Y. Tsukamoto, Y. Tomita, T. Ueda, H. Yoshikawa and K. Itoh (2010). "Synovial sarcoma is a stem cell malignancy." Stem Cells 28(7): 1119-1131.
Narvani, A. A., E. Tsiridis, A. Saifuddin, T. Briggs and S. Cannon (2009). "Does image guidance improve accuracy of core needle biopsy in diagnosis of soft tissue tumours?" Acta Orthop Belg 75(2): 239-244.
Neoptolemos, J. P., D. D. Stocken, C. Bassi, P. Ghaneh, D. Cunningham, D. Goldstein, R. Padbury, M. J. Moore, S. Gallinger, C. Mariette, M. N. Wente, J. R. Izbicki, H. Friess, M. M. Lerch, C. Dervenis, A. Olah, G. Butturini, R. Doi, P. A. Lind, D. Smith, J. W. Valle, D. H. Palmer, J. A. Buckels, J. Thompson, C. J. McKay, C. L. Rawcliffe, M. W. Buchler and C. European Study Group for Pancreatic (2010). "Adjuvant chemotherapy with fluorouracil plus folinic acid vs gemcitabine following pancreatic cancer resection: a randomized controlled trial." JAMA 304(10): 1073-1081.
Newick, K., S. O'Brien, E. Moon and S. M. Albelda (2017). "CAR T Cell Therapy for Solid Tumors." Annu Rev Med 68: 139-152.
O'Donnell, P. W., A. M. Griffin, W. C. Eward, A. Stemheim, C. N. Catton, P. W. Chung, B. O'Sullivan, P. C. Ferguson and J. S. Wunder (2014). "The effect of the setting of a positive surgical margin in soft tissue sarcoma." Cancer 120(18): 2866-2875.
O'Rourke, D. M., M. P. Nasrallah, A. Desai, J. J. Melenhorst, K. Mansfield, J. J. D. Morrissette, M. Martinez-Lage, S. Brem, E. Maloney, A. Shen, R. Isaacs, S. Mohan, G. Plesa, S. F. Lacey, J. M. Navenot, Z. Zheng, B. L. Levine, H. Okada, C. H. June, J. L. Brogdon and M. V. Maus (2017). "A single dose of peripherally infused EGFRvIII-directed CAR T cells mediates antigen loss and induces adaptive resistance in patients with recurrent glioblastoma." Sci Transl Med 9(399).
O'Sullivan, B., A. M. Griffin, C. I. Dickie, M. B. Sharpe, P. W. Chung, C. N. Catton, P. C. Ferguson, J. S. Wunder, B. M. Deheshi, L. M. White, R. A. Kandel, D. A. Jaffray and R. S. Bell (2013). "Phase 2 study of preoperative image-guided intensity-modulated radiation therapy to reduce wound and combined modality morbidities in lower extremity soft tissue sarcoma." Cancer 119(10): 1878-1884.
O'Sullivan, P. J., A. C. Harris and P. L. Munk (2008). "Radiological features of synovial cell sarcoma." Br J Radiol 81(964): 346-356.
Oettle, H., P. Neuhaus, A. Hochhaus, J. T. Hartmann, K. Gellert, K. Ridwelski, M. Niedergethmann, C. Zulke, J. Fahlke, M. B. Arning, M. Sinn, A. Hinke and H. Riess (2013). "Adjuvant chemotherapy with gemcitabine and long-term outcomes among patients with resected pancreatic cancer: the CONKO-001 randomized trial." JAMA 310(14): 1473-1481.
Ogiya, R., N. Niikura, N. Kumaki, G. Bianchini, S. Kitano, T. Iwamoto, N. Hayashi, K. Yokoyama, R. Oshitanai, M. Terao, T. Morioka, B. Tsuda, T. Okamura, Y. Saito, Y. Suzuki and Y. Tokuda (2016). "Comparison of tumor-infiltrating lymphocytes between primary and metastatic tumors in breast cancer patients." Cancer Sci 107(12): 1730-1735.
Ohue, Y. and H. Nishikawa (2019). "Regulatory T (Treg) cells in cancer: Can Treg cells be a new therapeutic target?" Cancer Sci 110(7): 2080-2089.
Overholtzer, M., P. H. Rao, R. Favis, X. Y. Lu, M. B. Elowitz, F. Barany, M. Ladanyi, R. Gorlick and A. J. Levine (2003). "The presence of p53 mutations in human osteosarcomas correlates with high levels of genomic instability." Proc Natl Acad Sci U S A 100(20): 11547-11552.
Pagliano, O., R. M. Morrison, J. M. Chauvin, H. Banerjee, D. Davar, Q. Ding, T. Tanegashima, W. Gao, S. R. Chakka, R. DeBlasio, A. Lowin, K. Kara, M. Ka, B. Zidi, R. Amin, I. Raphael, S. Zhang, S. C. Watkins, C. Sander, J. M. Kirkwood, M. Bosenberg, A. C. Anderson, V. K. Kuchroo, L. P. Kane, A.
J. Korman, A. Rajpal, S. M. West, M. Han, C. Bee, X. Deng, X. M. Schebye, P. Strop and H. M. Zarour (2022). "Tim-3 mediates T cell trogocytosis to limit antitumor immunity." J Clin Invest 132(9).
Papagelopoulos, P. J., E. C. Galanis, C. Vlastou, P. A. Nikiforidis, J. A. Vlamis, P. J. Boscainos, E. G. Fragiadakis, K. G. Stamos, T. Pantazopoulos and F. H. Sim (2000). "Current concepts in the evaluation and treatment of osteosarcoma." Orthopedics 23(8): 858-867; quiz 868-859.
Parikh, U., C. Marcus, R. Sarangi, M. Taghipour and R. M. Subramaniam (2015). "FDG PET/CT in Pancreatic and Hepatobiliary Carcinomas: Value to Patient Management and Patient Outcomes." PET Clin 10(3): 327-343.
Pasca di Magliano, M., A. V. Biankin, P. W. Heiser, D. A. Cano, P. J. Gutierrez, T. Deramaudt, D. Segara, A. C. Dawson, J. G. Kench, S. M. Henshall, R. L. Sutherland, A. Dlugosz, A. K. Rustgi and M. Hebrok (2007). "Common activation of canonical Wnt signaling in pancreatic adenocarcinoma." PLoS One 2(11): e1155.
Pasrija, R. and M. Naime (2021). "The deregulated immune reaction and cytokines release storm (CRS) in COVID-19 disease." Int Immunopharmacol 90: 107225.
Patel, S. J., N. E. Sanjana, R. J. Kishton, A. Eidizadeh, S. K. Vodnala, M. Cam, J. J. Gartner, L. Jia, S. M. Steinberg, T. N. Yamamoto, A. S. Merchant, G. U. Mehta, A. Chichura, O. Shalem, E. Tran, R. Eil, M. Sukumar, E. P. Guijarro, C. P. Day, P. Robbins, S. Feldman, G. Merlino, F. Zhang and N. P. Restifo (2017). "Identification of essential genes for cancer immunotherapy." Nature 548(7669): 537-542.
Pazzaglia, L., S. Pollino, M. Vitale, E. Bientinesi, S. Benini, C. Ferrari, E. Palmerini, M. Gambarotti, P. Picci and M. S. Benassi (2019). "miR-494.3p expression in synovial sarcoma: Role of CXCR4 as a potential target gene." Int J Oncol 54(1): 361-369.
Peng, H., X. He and Q. Wang (2022). "Targeted drug delivery system for ovarian cancer microenvironment: Improving the effects of immunotherapy." Front Immunol 13: 1035997.
Perry, J. A., A. Kiezun, P. Tonzi, E. M. Van Allen, S. L. Carter, S. C. Baca, G. S. Cowley, A. S. Bhatt, E. Rheinbay, C. S. Pedamallu, E. Helman, A. Taylor-Weiner, A. McKenna, D. S. DeLuca, M. S. Lawrence, L. Ambrogio, C. Sougnez, A. Sivachenko, L. D. Walensky, N. Wagle, J. Mora, C. de Torres, C. Lavarino, S. Dos Santos Aguiar, J. A. Yunes, S. R. Brandalise, G. E. Mercado-Celis, J. Melendez-Zajgla, R. Cardenas-Cardos, L. Velasco-Hidalgo, C. W. Roberts, L. A. Garraway, C. Rodriguez-Galindo, S. B. Gabriel, E. S. Lander, T. R. Golub, S. H. Orkin, G. Getz and K. A. Janeway (2014). "Complementary genomic approaches highlight the PI3K/mTOR pathway as a common vulnerability in osteosarcoma." Proc Natl Acad Sci U S A 111(51): E5564-5573.
Petty, A. J., D. H. Owen, Y. Yang and X. Huang (2021). "Targeting Tumor-Associated Macrophages in Cancer Immunotherapy." Cancers (Basel) 13(21).
Picci, P., L. Sangiorgi, B. T. Rougraff, J. R. Neff, R. Casadei and M. Campanacci (1994). "Relationship of chemotherapy-induced necrosis and surgical margins to local recurrence in osteosarcoma." J Clin Oncol 12(12): 2699-2705.
Piperdi, S., L. Austin-Page, D. Geller, M. Ahluwalia, S. Gorlick, J. Gill, A. Park, W. Zhang, N. Li, S. H. Chung and R. Gorlick (2012). "beta-Catenin Does Not Confer Tumorigenicity When Introduced into Partially Transformed Human Mesenchymal Stem Cells." Sarcoma 2012: 164803.
Pollack, S. M., Q. He, J. H. Yearley, R. Emerson, M. Vignali, Y. Zhang, M. W. Redman, K. K. Baker, S. Cooper, B. Donahue, E. T. Loggers, L. D. Cranmer, M. B. Spraker, Y. D. Seo, V. G. Pillarisetty, R. W. Ricciotti, B. L. Hoch, T. K. McClanahan, E. Murphy, W. M. Blumenschein, S. M. Townson, S. Benzeno, S. R. Riddell and R. L. Jones (2017). "T-cell infiltration and clonality correlate with programmed cell death protein 1 and programmed death-ligand 1 expression in patients with soft tissue sarcomas." Cancer 123(17): 3291-3304.
Pollack, S. M., A. A. Jungbluth, B. L. Hoch, E. A. Farrar, M. Bleakley, D. J. Schneider, E. T. Loggers, E. Rodler, J. F. Eary, E. U. Conrad, 3rd, R. L. Jones and C. Yee (2012). "NY-ESO-1 is a ubiquitous immunotherapeutic target antigen for patients with myxoid/round cell liposarcoma." Cancer 118(18): 4564-4570.
Pollack, S. M., N. Somaiah, D. M. Araujo, M. Druta, B. A. Van Tine, M. A. Burgess, S. P. Chawla, M. Seetharam, S. H. Okuno, C. Bohac, M. Chen, S. Yurasov and S. Attia (2020). "Clinical outcomes of patients with advanced synovial sarcoma or myxoid/round cell liposarcoma treated at major cancer centers in the United States." Cancer Med 9(13): 4593-4602.
Poncette, L., X. Chen, F. K. Lorenz and T. Blankenstein (2019). "Effective NY-ESO-1-specific MHC II-restricted T cell receptors from antigen-negative hosts enhance tumor regression." J Clin Invest 129(1): 324-335.
Poruk, K. E., D. Z. Gay, K. Brown, J. D. Mulvihill, K. M. Boucher, C. L. Scaife, M. A. Firpo and S. J. Mulvihill (2013). "The clinical utility of CA 19-9 in pancreatic adenocarcinoma: diagnostic and prognostic updates." Curr Mol Med 13(3): 340-351.
Potter, B. K., S. C. Adams, J. D. Pitcher, Jr. and H. T. Temple (2008). "Local recurrence of disease after unplanned excisions of high-grade soft tissue sarcomas." Clin Orthop Relat Res 466(12): 3093-3100.
Preis, M. and M. Korc (2011). "Signaling pathways in pancreatic cancer." Crit Rev Eukaryot Gene Expr 21(2): 115-129.
Pretell-Mazzini, J., M. D. Barton, Jr., S. A. Conway and H. T. Temple (2015). "Unplanned excision of soft-tissue sarcomas: current concepts for management and prognosis." J Bone Joint Surg Am 97(7): 597-603.
Propper, D. J., D. Chao, J. P. Braybrooke, P. Bahl, P. Thavasu, F. Balkwill, H. Turley, N. Dobbs, K. Gatter, D. C. Talbot, A. L. Harris and T. S. Ganesan (2003). "Low-dose IFN-gamma induces tumor MHC expression in metastatic malignant melanoma." Clin Cancer Res 9(1): 84-92.
Ramu, E. M., M. T. Houdek, C. E. Isaac, C. I. Dickie, P. C. Ferguson and J. S. Wunder (2017). "Management of soft-tissue sarcomas; treatment strategies, staging, and outcomes." SICOT J 3: 20.
Regine, W. F., K. A. Winter, R. Abrams, H. Safran, J. P. Hoffman, A. Konski, A. B. Benson, J. S. Macdonald, T. A. Rich and C. G. Willett (2011). "Fluorouracil-based chemoradiation with either gemcitabine or fluorouracil chemotherapy after resection of pancreatic adenocarcinoma: 5-year analysis of the U.S. Intergroup/RTOG 9704 phase III trial." Ann Surg Oncol 18(5): 1319-1326.
Rubin, M. A. (2015). "Health: Make precision medicine work for cancer care." Nature 520(7547): 290-291.
Ruggieri, P., A. F. Mavrogenis, E. Pala, M. Romantini, M. Manfrini and M. Mercuri (2013). "Outcome of expandable prostheses in children." J Pediatr Orthop 33(3): 244-253.
Rytlewski, J., M. M. Milhem and V. Monga (2021). "Turning 'Cold' tumors 'Hot': immunotherapies in sarcoma." Ann Transl Med 9(12): 1039.
Sambri, A., E. Caldari, M. Fiore, R. Zucchini, C. Giannini, M. G. Pirini, P. Spinnato, A. Cappelli, D. M. Donati and M. De Paolis (2021). "Margin Assessment in Soft Tissue Sarcomas: Review of the Literature." Cancers (Basel) 13(7).
Samstein, R. M., C. H. Lee, A. N. Shoushtari, M. D. Hellmann, R. Shen, Y. Y. Janjigian, D. A. Barron, A. Zehir, E. J. Jordan, A. Omuro, T. J. Kaley, S. M. Kendall, R. J. Motzer, A. A. Hakimi, M. H. Voss, P. Russo, J. Rosenberg, G. Iyer, B. H. Bochner, D. F. Bajorin, H. A. Al-Ahmadie, J. E. Chaft, C. M. Rudin, G. J. Riely, S. Baxi, A. L. Ho, R. J. Wong, D. G. Pfister, J. D. Wolchok, C. A. Barker, P. H. Gutin, C. W. Brennan, V. Tabar, I. K. Mellinghoff, L. M. DeAngelis, C. E. Ariyan, N. Lee, W. D. Tap, M. M. Gounder, S. P. D'Angelo, L. Saltz, Z. K. Stadler, H. I. Scher, J. Baselga, P. Razavi, C. A. Klebanoff, R. Yaeger, N. H. Segal, G. Y. Ku, R. P. DeMatteo, M. Ladanyi, N. A. Rizvi, M. F. Berger, N. Riaz, D. B. Solit, T. A. Chan and L. G. T. Morris (2019). "Tumor mutational load predicts survival after immunotherapy across multiple cancer types." Nat Genet 51(2): 202-206.
Sayles, L. C., M. R. Breese, A. L. Koehne, S. G. Leung, A. G. Lee, H. Y. Liu, A. Spillinger, A. T. Shah, B. Tanasa, K. Straessler, F. K. Hazard, S. L. Spunt, N. Marina, G. E. Kim, S. J. Cho, R. S. Avedian, D. G. Mohler, M. O. Kim, S. G. DuBois, D. S. Hawkins and E. A. Sweet-Cordero (2019). "Genome-Informed Targeted Therapy for Osteosarcoma." Cancer Discov 9(1): 46-63.
Sbaraglia, M., E. Bellan and A. P. Dei Tos (2021). "The 2020 WHO Classification of Soft Tissue Tumours: news and perspectives." Pathologica 113(2): 70-84.
Schildberg, F. A., S. R. Klein, G. J. Freeman and A. H. Sharpe (2016). "Coinhibitory Pathways in the B7-CD28 Ligand-Receptor Family." Immunity 44(5): 955-972.
Schreiber, G. (2020). "The Role of Type I Interferons in the Pathogenesis and Treatment of COVID-19." Front Immunol 11: 595739.
Seidensaal, K., M. Mattke, S. Haufe, H. Rathke, U. Haberkorn, N. Bougatf, A. Kudak, C. Blattmann, S. Oertel, M. Kirchner, C. Buesch, M. Kieser, K. Herfarth, A. Kulozik, J. Debus, M. Uhl and S. B. Harrabi (2021). "The role of combined ion-beam radiotherapy (CIBRT) with protons and carbon ions in a multimodal treatment strategy of inoperable osteosarcoma." Radiother Oncol 159: 8-16.
Sermer, D. and R. Brentjens (2019). "CAR T-cell therapy: Full speed ahead." Hematol Oncol 37 Suppl 1: 95-100.
Seymour, A. B., R. H. Hruban, M. Redston, C. Caldas, S. M. Powell, K. W. Kinzler, C. J. Yeo and S. E. Kern (1994). "Allelotype of pancreatic adenocarcinoma." Cancer Res 54(10): 2761-2764.
Shank, B. R., B. Do, A. Sevin, S. E. Chen, S. S. Neelapu and S. B. Horowitz (2017). "Chimeric Antigen Receptor T Cells in Hematologic Malignancies." Pharmacotherapy 37(3): 334-345.
Shankaran, V., H. Ikeda, A. T. Bruce, J. M. White, P. E. Swanson, L. J. Old and R. D. Schreiber (2001). "IFNgamma and lymphocytes prevent primary tumour development and shape tumour immunogenicity." Nature 410(6832): 1107-1111.
Sharma, M., H. Khong, F. Fa'ak, S. E. Bentebibel, L. M. E. Janssen, B. C. Chesson, C. A. Creasy, M. A. Forget, L. M. S. Kahn, B. Pazdrak, B. Karki, Y. Hailemichael, M. Singh, C. Vianden, S. Vennam, U. Bharadwaj, D. J. Tweardy, C. Haymaker, C. Bernatchez, S. Huang, K. Rajapakshe, C. Coarfa, M. E. Hurwitz, M. Sznol, P. Hwu, U. Hoch, M. Addepalli, D. H. Charych, J. Zalevsky, A. Diab and W. W. Overwijk (2020). "Bempegaldesleukin selectively depletes intratumoral Tregs and potentiates T cell-mediated cancer therapy." Nat Commun 11(1): 661.
Sharma, P. and J. P. Allison (2015). "The future of immune checkpoint therapy." Science 348(6230): 56-61.
Shin, E. J., M. Topazian, M. G. Goggins, S. Syngal, J. R. Saltzman, J. H. Lee, J. J. Farrell and M. I. Canto (2015). "Linear-array EUS improves detection of pancreatic lesions in high-risk individuals: a randomized tandem study." Gastrointest Endosc 82(5): 812-818.
Siegel, R. L., K. D. Miller, N. S. Wagle and A. Jemal (2023). "Cancer statistics, 2023." CA Cancer J Clin 73(1): 17-48.
Singer, S., E. H. Baldini, G. D. Demetri, J. A. Fletcher and J. M. Corson (1996). "Synovial sarcoma: prognostic significance of tumor size, margin of resection, and mitotic activity for survival." J Clin Oncol 14(4): 1201-1208.
Sleijfer, S., M. Ouali, M. van Glabbeke, A. Krarup-Hansen, S. Rodenhuis, A. Le Cesne, P. C. Hogendoorn, J. Verweij and J. Y. Blay (2010). "Prognostic and predictive factors for outcome to first-line ifosfamide-containing chemotherapy for adult patients with advanced soft tissue sarcomas: an exploratory, retrospective analysis on large series from the European Organization for Research and Treatment of Cancer-Soft Tissue and Bone Sarcoma Group (EORTC-STBSG)." Eur J Cancer 46(1): 72-83.
Smeland, S., S. S. Bielack, J. Whelan, M. Bernstein, P. Hogendoorn, M. D. Krailo, R. Gorlick, K. A. Janeway, F. C. Ingleby, J. Anninga, I. Antal, C. Arndt, K. L. B. Brown, T. Butterfass-Bahloul, G. Calaminus, M. Capra, C. Dhooge, M. Eriksson, A. M. Flanagan, G. Friedel, M. C. Gebhardt, H. Gelderblom, R. Goldsby, H. E. Grier, R. Grimer, D. S. Hawkins, S. Hecker-Nolting, K. Sundby Hall, M. S. Isakoff, G. Jovic, T. Kuhne, L. Kager, T. von Kalle, E. Kabickova, S. Lang, C. C. Lau, P. J. Leavey, S. L. Lessnick, L. Mascarenhas, R. Mayer-Steinacker, P. A. Meyers, R. Nagarajan, R. L. Randall, P. Reichardt, M. Renard, C. Rechnitzer, C. L. Schwartz, S. Strauss, L. Teot, B. Timmermann, M. R. Sydes and N. Marina (2019). "Survival and prognosis with osteosarcoma: outcomes in more than 2000 patients in the EURAMOS-1 (European and American Osteosarcoma Study) cohort." Eur J Cancer 109: 36-50.
Smith, M. A., N. L. Seibel, S. F. Altekruse, L. A. Ries, D. L. Melbert, M. O'Leary, F. O. Smith and G. H. Reaman (2010). "Outcomes for children and adolescents with cancer: challenges for the twenty-first century." J Clin Oncol 28(15): 2625-2634.
Smolle, M. A., M. Parry, L. Jeys, S. Abudu and R. Grimer (2019). "Synovial sarcoma: Do children do better?" Eur J Surg Oncol 45(2): 254-260.
Song, S., J. Park, H. J. Kim, I. H. Kim, I. Han, H. S. Kim and S. Kim (2017). "Effects of Adjuvant Radiotherapy in Patients With Synovial Sarcoma." Am J Clin Oncol 40(3): 306-311.
Spurrell, E. L., C. Fisher, J. M. Thomas and I. R. Judson (2005). "Prognostic factors in advanced synovial sarcoma: an analysis of 104 patients treated at the Royal Marsden Hospital." Ann Oncol 16(3): 437-444.
Stacchiotti, S. and B. A. Van Tine (2018). "Synovial Sarcoma: Current Concepts and Future Perspectives." J Clin Oncol 36(2): 180-187.
Stegmaier, S., I. Leuschner, C. Poremba, R. Ladenstein, B. Kazanowska, G. Ljungman, M. Scheer, B. Blank, S. Bielack, T. Klingebiel and E. Koscielniak (2017). "The prognostic impact of SYT-SSX fusion type and histological grade in pediatric patients with synovial sarcoma treated according to the CWS (Cooperative Weichteilsarkom Studie) trials." Pediatr Blood Cancer 64(1): 89-95.
Strauss, S. J., A. M. Frezza, N. Abecassis, J. Bajpai, S. Bauer, R. Biagini, S. Bielack, J. Y. Blay, S. Bolle, S. Bonvalot, I. Boukovinas, J. Bovee, K. Boye, B. Brennan, T. Brodowicz, A. Buonadonna, E. de Alava, A. P. Dei Tos, X. Garcia Del Muro, A. Dufresne, M. Eriksson, F. Fagioli, A. Fedenko, V. Ferraresi, A. Ferrari, N. Gaspar, S. Gasperoni, H. Gelderblom, F. Gouin, G. Grignani, A. Gronchi, R. Haas, A. B. Hassan, S. Hecker-Nolting, N. Hindi, P. Hohenberger, H. Joensuu, R. L. Jones, C. Jungels, P. Jutte, L. Kager, B. Kasper, A. Kawai, K. Kopeckova, D. A. Krakorova, A. Le Cesne, F. Le Grange, E. Legius, A. Leithner, A. Lopez Pousa, J. Martin-Broto, O. Merimsky, C. Messiou, A. B. Miah, O. Mir, M. Montemurro, B. Morland, C. Morosi, E. Palmerini, M. A. Pantaleo, R. Piana, S. Piperno-Neumann, P. Reichardt, P. Rutkowski, A. A. Safwat, C. Sangalli, M. Sbaraglia, S. Scheipl, P. Schoffski, S. Sleijfer, D. Strauss, K. Sundby Hall, A. Trama, M. Unk, M. A. J. van de Sande, W. T. A. van der Graaf, W. J. van Houdt, T. Frebourg, R. Ladenstein, P. G. Casali, S. Stacchiotti, E. G. Esmo Guidelines Committee and E. R. N. P. E. a. clinicalguidelines@esmo.org (2021). "Bone sarcomas: ESMO-EURACAN-GENTURIS-ERN PaedCan Clinical Practice Guideline for diagnosis, treatment and follow-up." Ann Oncol 32(12): 1520-1536.
Stultz, J. and L. Fong (2021). "How to turn up the heat on the cold immune microenvironment of metastatic prostate cancer." Prostate Cancer Prostatic Dis 24(3): 697-717.
Su, L., H. Cheng, A. V. Sampaio, T. O. Nielsen and T. M. Underhill (2010). "EGR1 reactivation by histone deacetylase inhibitors promotes synovial sarcoma cell death through the PTEN tumor suppressor." Oncogene 29(30): 4352-4361.
Sultan, I., C. Rodriguez-Galindo, R. Saab, S. Yasir, M. Casanova and A. Ferrari (2009). "Comparing children and adults with synovial sarcoma in the Surveillance, Epidemiology, and End Results program, 1983 to 2005: an analysis of 1268 patients." Cancer 115(15): 3537-3547.
Sung, H., J. Ferlay, R. L. Siegel, M. Laversanne, I. Soerjomataram, A. Jemal and F. Bray (2021). "Global Cancer Statistics 2020: GLOBOCAN Estimates of Incidence and Mortality Worldwide for 36 Cancers in 185 Countries." CA Cancer J Clin 71(3): 209-249.
Tan, E., N. Gakhar and K. Kirtane (2021). "TCR gene-engineered cell therapy for solid tumors." Best Pract Res Clin Haematol 34(3): 101285.
Tanizaki, J., I. Okamoto, K. Sakai and K. Nakagawa (2011). "Differential roles of trans-phosphorylated EGFR, HER2, HER3, and RET as heterodimerisation partners of MET in lung cancer with MET amplification." Br J Cancer 105(6): 807-813.
Tateishi, U., T. Hasegawa, Y. Beppu, M. Satake and N. Moriyama (2004). "Synovial sarcoma of the soft tissues: prognostic significance of imaging features." J Comput Assist Tomogr 28(1): 140-148.
Tempero, M. A., M. P. Malafa, M. Al-Hawary, H. Asbun, A. Bain, S. W. Behrman, A. B. Benson, 3rd, E. Binder, D. B. Cardin, C. Cha, E. G. Chiorean, V. Chung, B. Czito, M. Dillhoff, E. Dotan, C. R. Ferrone, J. Hardacre, W. G. Hawkins, J. Herman, A. H. Ko, S. Komanduri, A. Koong, N. LoConte, A. M. Lowy, C. Moravek, E. K. Nakakura, E. M. O'Reilly, J. Obando, S. Reddy, C. Scaife, S. Thayer, C. D. Weekes, R. A. Wolff, B. M. Wolpin, J. Burns and S. Darlow (2017). "Pancreatic Adenocarcinoma, Version 2.2017, NCCN Clinical Practice Guidelines in Oncology." J Natl Compr Canc Netw 15(8): 1028-1061.
Toro, J. R., L. B. Travis, H. J. Wu, K. Zhu, C. D. Fletcher and S. S. Devesa (2006). "Incidence patterns of soft tissue sarcomas, regardless of primary site, in the surveillance, epidemiology and end results program, 1978-2001: An analysis of 26,758 cases." Int J Cancer 119(12): 2922-2930.
Tramacere, I., L. Scotti, M. Jenab, V. Bagnardi, R. Bellocco, M. Rota, G. Corrao, F. Bravi, P. Boffetta and C. La Vecchia (2010). "Alcohol drinking and pancreatic cancer risk: a meta-analysis of the dose-risk relation." Int J Cancer 126(6): 1474-1486.
Trampont, P. C., A. C. Tosello-Trampont, Y. Shen, A. K. Duley, A. E. Sutherland, T. P. Bender, D. R. Littman and K. S. Ravichandran (2010). "CXCR4 acts as a costimulator during thymic beta-selection." Nat Immunol 11(2): 162-170.
Trassard, M., V. Le Doussal, K. Hacene, P. Terrier, D. Ranchere, L. Guillou, M. Fiche, F. Collin, M. O. Vilain, G. Bertrand, J. Jacquemier, X. Sastre-Garau, N. B. Bui, F. Bonichon and J. M. Coindre (2001). "Prognostic factors in localized primary synovial sarcoma: a multicenter study of 128 adult patients." J Clin Oncol 19(2): 525-534.
Traverso, L. W. (2006). "Pancreatic cancer: surgery alone is not sufficient." Surg Endosc 20 Suppl 2: S446-449.
Tsimberidou, A. M., E. Fountzilas, M. Nikanjam and R. Kurzrock (2020). "Review of precision cancer medicine: Evolution of the treatment paradigm." Cancer Treat Rev 86: 102019.
Venkatesan, N., S. J. Kim and B. H. Kim (2003). "Novel phosphoramidite building blocks in synthesis and applications toward modified oligonucleotides." Curr Med Chem 10(19): 1973-1991.
Vitanza, N. A., A. L. Wilson, W. Huang, K. Seidel, C. Brown, J. A. Gustafson, J. K. Yokoyama, A. J. Johnson, B. A. Baxter, R. W. Koning, A. N. Reid, M. Meechan, M. C. Biery, C. Myers, S. D. Rawlings-Rhea, C. M. Albert, S. R. Browd, J. S. Hauptman, A. Lee, J. G. Ojemann, M. E. Berens, M. D. Dun, J. B. Foster, E. E. Crotty, S. E. S. Leary, B. L. Cole, F. A. Perez, J. N. Wright, R. J. Orentas, T. Chour, E. W. Newell, J. R. Whiteaker, L. Zhao, A. G. Paulovich, N. Pinto, J. Gust, R. A. Gardner, M. C. Jensen and J. R. Park (2023). "Intraventricular B7-H3 CAR T Cells for Diffuse Intrinsic Pontine Glioma: Preliminary First-in-Human Bioactivity and Safety." Cancer Discov 13(1): 114-131.
Vlenterie, M., V. K. Ho, S. E. Kaal, R. Vlenterie, R. Haas and W. T. van der Graaf (2015). "Age as an independent prognostic factor for survival of localised synovial sarcoma patients." Br J Cancer 113(11): 1602-1606.
von Mehren, M., R. L. Randall, R. S. Benjamin, S. Boles, M. M. Bui, E. U. Conrad, 3rd, K. N. Ganjoo, S. George, R. J. Gonzalez, M. J. Heslin, J. M. Kane, 3rd, H. Koon, J. Mayerson, M. McCarter, S. V. McGarry, C. Meyer, R. J. O'Donnell, A. S. Pappo, I. B. Paz, I. A. Petersen, J. D. Pfeifer, R. F. Riedel, S. Schuetze, K. D. Schupak, H. S. Schwartz, W. D. Tap, J. D. Wayne, M. A. Bergman and J. Scavone (2016). "Soft Tissue Sarcoma, Version 2.2016, NCCN Clinical Practice Guidelines in Oncology." J Natl Compr Canc Netw 14(6): 758-786.
Waddell, N., M. Pajic, A. M. Patch, D. K. Chang, K. S. Kassahn, P. Bailey, A. L. Johns, D. Miller, K. Nones, K. Quek, M. C. Quinn, A. J. Robertson, M. Z. Fadlullah, T. J. Bruxner, A. N. Christ, I. Harliwong, S. Idrisoglu, S. Manning, C. Nourse, E. Nourbakhsh, S. Wani, P. J. Wilson, E. Markham, N. Cloonan, M. J. Anderson, J. L. Fink, O. Holmes, S. H. Kazakoff, C. Leonard, F. Newell, B. Poudel, S. Song, D. Taylor, N. Waddell, S. Wood, Q. Xu, J. Wu, M. Pinese, M. J. Cowley, H. C. Lee, M. D. Jones, A. M. Nagrial, J. Humphris, L. A. Chantrill, V. Chin, A. M. Steinmann, A. Mawson, E. S. Humphrey, E. K. Colvin, A. Chou, C. J. Scarlett, A. V. Pinho, M. Giry-Laterriere, I. Rooman, J. S. Samra, J. G. Kench, J. A. Pettitt, N. D. Merrett, C. Toon, K. Epari, N. Q. Nguyen, A. Barbour, N. Zeps, N. B. Jamieson, J. S. Graham, S. P. Niclou, R. Bjerkvig, R. Grutzmann, D. Aust, R. H. Hruban, A. Maitra, C. A. Iacobuzio-Donahue, C. L. Wolfgang, R. A. Morgan, R. T. Lawlor, V. Corbo, C. Bassi, M. Falconi, G. Zamboni, G. Tortora, M. A. Tempero, I. Australian Pancreatic Cancer Genome, A. J. Gill, J. R. Eshleman, C. Pilarsky, A. Scarpa, E. A. Musgrove, J. V. Pearson, A. V. Biankin and S. M. Grimmond (2015). "Whole genomes redefine the mutational landscape of pancreatic cancer." Nature 518(7540): 495-501.
Wang, J., Y. Song, X. Liu, J. Jin, W. Wang, Z. Yu, Y. Liu, N. Li, H. Fang, H. Ren, Y. Tang, Y. Tang, B. Chen, N. Lu, S. Qi, S. Wang and Y. Li (2019). "Comparison of outcome and toxicity of postoperative intensity-modulated radiation therapy with two-dimensional radiotherapy in patients with soft tissue sarcoma of extremities and trunk." Cancer Med 8(3): 902-909.
Wang, J., J. Yang, A. Narang, J. He, C. Wolfgang, K. Li and L. Zheng (2024). "Consensus, debate, and prospective on pancreatic cancer treatments." J Hematol Oncol 17(1): 92.
Wang, L., H. Geng, Y. Liu, L. Liu, Y. Chen, F. Wu, Z. Liu, S. Ling, Y. Wang and L. Zhou (2023). "Hot and cold tumors: Immunological features and the therapeutic strategies." MedComm (2020) 4(5): e343.
Wang, V., M. Gauthier, V. Decot, L. Reppel and D. Bensoussan (2023). "Systematic Review on CAR-T Cell Clinical Trials Up to 2022: Academic Center Input." Cancers (Basel) 15(4).
WHO (2020). Classification of Tumours Editorial. Soft Tissue and Bone Tumours, IARC Press: Lyon, France.
Wilson, D. A. J., A. Gazendam, J. Visgauss, D. Perrin, A. M. Griffin, P. W. Chung, C. N. Catton, D. Shultz, P. C. Ferguson and J. S. Wunder (2020). "Designing a Rational Follow-Up Schedule for Patients with Extremity Soft Tissue Sarcoma." Ann Surg Oncol 27(6): 2033-2041.
Wincott, F., A. DiRenzo, C. Shaffer, S. Grimm, D. Tracz, C. Workman, D. Sweedler, C. Gonzalez, S. Scaringe and N. Usman (1995). "Synthesis, deprotection, analysis and purification of RNA and ribozymes." Nucleic Acids Res 23(14): 2677-2684.
Windbichler, G. H., H. Hausmaninger, W. Stummvoll, A. H. Graf, C. Kainz, J. Lahodny, U. Denison, E. Muller-Holzner and C. Marth (2000). "Interferon-gamma in the first-line therapy of ovarian cancer: a randomized phase III trial." Br J Cancer 82(6): 1138-1144.
Wolf, R. E. and W. F. Enneking (1996). "The staging and surgery of musculoskeletal neoplasms." Orthop Clin North Am 27(3): 473-481.
Wolfgang, C. L., J. M. Herman, D. A. Laheru, A. P. Klein, M. A. Erdek, E. K. Fishman and R. H. Hruban (2013). "Recent progress in pancreatic cancer." CA Cancer J Clin 63(5): 318-348.
Wu, C. C., H. C. Beird, J. Andrew Livingston, S. Advani, A. Mitra, S. Cao, A. Reuben, D. Ingram, W. L. Wang, Z. Ju, C. Hong Leung, H. Lin, Y. Zheng, J. Roszik, W. Wang, S. Patel, R. S. Benjamin, N. Somaiah, A. P. Conley, G. B. Mills, P. Hwu, R. Gorlick, A. Lazar, N. C. Daw, V. Lewis and P. A. Futreal (2020). "Immuno-genomic landscape of osteosarcoma." Nat Commun 11(1): 1008.
Yang, J., A. Kumar, A. E. Vilgelm, S. C. Chen, G. D. Ayers, S. V. Novitskiy, S. Joyce and A. Richmond (2018). "Loss of CXCR4 in Myeloid Cells Enhances Antitumor Immunity and Reduces Melanoma Growth through NK Cell and FASL Mechanisms." Cancer Immunol Res 6(10): 1186-1198.
Yang, Y. J. and T. A. Damron (2004). "Comparison of needle core biopsy and fine-needle aspiration for diagnostic accuracy in musculoskeletal lesions." Arch Pathol Lab Med 128(7): 759-764.
Yu, S. and X. Yao (2024). "Advances on immunotherapy for osteosarcoma." Mol Cancer 23(1): 192.
Yu, S., M. Yi, S. Qin and K. Wu (2019). "Next generation chimeric antigen receptor T cells: safety strategies to overcome toxicity." Mol Cancer 18(1): 125.
Yusuf, N., S. R. Allie and B. E. Strauss (2023). "Editorial: Interferons: key modulators of the immune system in cancer." Front Immunol 14: 1327311.
Yuti, P., N. Sawasdee, K. Natungnuy, P. Rujirachaivej, P. Luangwattananun, J. Sujjitjoon and P. T. Yenchitsomanus (2023). "Enhanced antitumor efficacy, proliferative capacity, and alleviation of T cell exhaustion by fifth-generation chimeric antigen receptor T cells targeting B cell maturation antigen in multiple myeloma." Biomed Pharmacother 168: 115691.
Zboralski, D., K. Hoehlig, D. Eulberg, A. Fromming and A. Vater (2017). "Increasing Tumor-Infiltrating T Cells through Inhibition of CXCL12 with NOX-A12 Synergizes with PD-1 Blockade." Cancer Immunol Res 5(11): 950-956.
Zhang, C., J. Liu, J. F. Zhong and X. Zhang (2017). "Engineering CAR-T cells." Biomark Res 5: 22.
Zhang, S., K. Kohli, R. G. Black, L. Yao, S. M. Spadinger, Q. He, V. G. Pillarisetty, L. D. Cranmer, B. A. Van Tine, C. Yee, R. H. Pierce, S. R. Riddell, R. L. Jones and S. M. Pollack (2019). "Systemic Interferon-gamma Increases MHC Class I Expression and T-cell Infiltration in Cold Tumors: Results of a Phase 0 Clinical Trial." Cancer Immunol Res 7(8): 1237-1243.
Zhang, Y., Z. Liu, W. Wei and Y. Li (2022). "TCR engineered T cells for solid tumor immunotherapy." Exp Hematol Oncol 11(1): 38.
Zhong, C., J. Wang, B. Li, H. Xiang, M. Ultsch, M. Coons, T. Wong, N. Y. Chiang, S. Clark, R. Clark, L. Quintana, P. Gribling, E. Suto, K. Barck, R. Corpuz, J. Yao, R. Takkar, W. P. Lee, L. A. Damico-Beyer, R. D. Carano, C. Adams, R. F. Kelley, W. Wang and N. Ferrara (2013). "Development and preclinical characterization of a humanized antibody targeting CXCL12." Clin Cancer Res 19(16): 4433-4445.
Zhou, Y, Q. Lu, J. Xu, R. Yan, J. Zhu, J. Xu, X. Jiang, J. Li and F. Wu (2017). "The effect of pathological fractures on the prognosis of patients with osteosarcoma: a meta-analysis of 14 studies." Oncotarget 8(42): 73037-73049.

## Claims

1. A CXCL12 antagonist for use in a method for treating a tumor in a subject, wherein the method comprises administering to the subject the CXCL12 antagonist and an interferon, wherein the tumor is an immunologically cold tumor.

2. An interferon for use in a method for treating a tumor in a subject, wherein the method comprises administering to the subject the interferon and a CXCL12 antagonist, wherein the tumor is an immunologically cold tumor.

3. A CXCL12 antagonist for use in a method for converting an immunologically cold tumor in a subject into an immunologically hot tumor in the subject, wherein the method comprises administering to the subject the CXCL12 antagonist and an interferon, wherein upon administration of the CXCL12 antagonist and the interferon the immunologically cold tumor is or will be converted into an immunologically hot tumor.

4. An interferon for use in a method for converting an immunologically cold tumor in a subject into an immunologically hot tumor in the subject, wherein the method comprises administering to the subject the interferon and a CXCL12 antagonist, wherein upon administration of the interferon and the CXCL12 antagonist the immunologically cold tumor is or will be converted into an immunologically hot tumor.

5. The CXCL12 antagonist for use of any one of claims 1 and 3 and the interferon for use of any one of claims 2 and 4, wherein the immunologically cold tumor is refractory to an immunotherapy.

6. A CXCL12 antagonist for use in a method for treating a tumor in a subject, wherein the method comprises administering to the subject the CXCL12 antagonist and an interferon, wherein the tumor is refractory to immunotherapy.

7. An interferon for use in a method for treating a tumor in a subject, wherein the method comprises administering to the subject the interferon and a CXCL12 antagonist, wherein the tumor is refractory to immunotherapy.

8. A CXCL12 antagonist for use in a method for converting an immunotherapy refractory tumor in a subject into an immunotherapy non-refractory tumor in the subject, wherein the method comprises administering to the subject the CXCL12 antagonist and an interferon, wherein upon administration of the CXCL12 antagonist and the interferon the immunotherapy refractory tumor is or will be converted into an immunotherapy non-refractory tumor.

9. An interferon for use in a method for converting an immunotherapy refractory tumor in a subject into an immunotherapy non-refractory tumor in the subject, wherein the method comprises administering to the subject the interferon and a CXCL12 antagonist, wherein upon administration of the interferon and the CXCL12 antagonist the immunotherapy refractory tumor is or will be converted into an immunotherapy non-refractory tumor.

10. The CXCL12 antagonist for use of any one of claims 5, 6, and 8 and the interferon for use of any one of claims 5, 7 and 9, wherein the tumor refractory to an immunotherapy is an immunologically cold tumor.

11. The CXCL12 antagonist for use of any one of claims 5, 6, 8 and 10 and the interferon for use of any one of claims 5, 7, 9 and 10, wherein the immunotherapy is selected from the group consisting of checkpoint inhibition, adoptive cell therapy and tumor vaccines.

12. The CXCL12 antagonist for use of any one of claims 1, 3, 5, 6, 8, 10 and 11 and the interferon for use of any one of claims 2, 4, 5, 7, 9, 10 and 11, wherein the cold tumor and the tumor refractory to an immunotherapy is **characterized by** an immunosuppressive tumor microenvironment.

13. The CXCL12 antagonist for use of any one of claims 1, 3, 5, 6, 8, 10, 11 and 12 and the interferon for use of any one of claims 2, 4, 5, 7, 9, 10, 11 and 12, wherein the cold tumor and the tumor refractory to immunotherapy have at least one characteristic selected from the group comprising reduced or lacking expression of PD-L1, reduced number of tumor-infiltrating lymphocytes, genomic stability and reduced or lacking preexisting antitumor immune response.

14. The CXCL12 antagonist for use of claim 8 and the interferon for use of claim 9, wherein the tumor non-refractory to an immunotherapy is a tumor which is sensitive to an immunotherapy, preferably the immunotherapy is selected from the group consisting of checkpoint inhibition, adoptive cell therapy and tumor vaccines.

15. The CXCL12 antagonist for use of any one of claims 8 and 14 and the interferon for use of any one of claims 9 and 14, wherein the tumor non-refractory to an immunotherapy is a tumor which is a hot tumor, preferably the tumor non-refractory to an immunotherapy and the immunologically hot tumor are **characterized by** a non-immunosuppressive tumor microenvironment and/or one characteristic selected from the group comprising expression of PD-L1, non-reduced number of tumor-infiltrating lymphocytes, genomic instability and non-reduced or preexisting antitumor immune response.

16. The CXCL12 antagonist for use of any one of claims 1, 3, 5, 6, 8 and 10 to 15 and 12 and the interferon for use of any one of claims 2, 4, 5, 7, 9 and 10 to 15, wherein the tumor is a solid tumor, preferably, the tumor is selected from the group comprising pancreatic cancer, triple-negative breast cancer, prostate cancer, ovarian cancer, soft tissue sarcomas and osteosarcoma, more preferably the tumor is selected from the group consisting of synovial sarcoma, pancreatic cancer and osteosarcoma.

17. The CXCL12 antagonist for use of any one of claims 1, 3, 5, 6, 8 and 10 to 16 and the interferon for use of any one of claims 2, 4, 5, 7, 9 and 10 to 16, wherein the method comprises administering to the subject an adoptive cell therapy, preferably the adoptive cell therapy comprises administering a CAR T cell and/or a TCR-T cell to the subject.

18. The CXCL12 antagonist for use of any one of claims 1, 3, 5, 6, 8 and 10 to 17 and the interferon for use of any one of claims 2, 4, 5, 7, 9 and 10 to 17, wherein the CXCL12 antagonist and the interferon are administered concomitantly to the subject or the CXCL12 antagonist is administered to the subject after the administration of the interferon to the subject.

19. The CXCL12 antagonist for use of any one of claims 1, 3, 5, 6, 8 and 10 to 18 and the interferon for use of any one of claims 2, 4, 5, 7, 9 and 10 to 18, wherein the interferon is a type I interferon, preferably the interferon is interferon alpha, more preferably the interferon is interferon alpha comprising an amino acid sequence of SEQ ID NO: 242 or comprising an amino acid sequence of SEQ ID NO: 243.

20. The CXCL12 antagonist for use of any one of claims 1, 3, 5, 6, 8 and 10 to 18 and the interferon for use of any one of claims 2, 4, 5, 7, 9 and 10 to 18, wherein the interferon is a type II interferon, preferably the interferon is interferon gamma, more preferably the interferon is interferon gamma comprising an amino acid sequence of SEQ ID NO: 245.

21. The CXCL12 antagonist for use of any one of claims 1, 3, 5, 6, 8 and 10 to 20 and the interferon for use of any one of claims 2, 4, 5, 7, 9 and 10 to 20, wherein the CXCL12 antagonist is a C-X-C motif chemokine 12 (CXCL12) antagonist, wherein the CXCL12 antagonist inhibits the binding of CXCL12 to CXCL12 receptor C-X-C chemokine receptor type 4 (CXCR4) and/or to CXCL12 receptor C-X-C chemokine receptor type 7 (CXCR7), preferably the CXCL12 antagonist is binding to CXCL12.

22. The CXCL12 antagonist for use of any one of claims 1, 3, 5, 6, 8 and 10 to 21 and the interferon for use of any one of claims 2, 4, 5, 7, 9 and 10 to 21, wherein the CXCL12 antagonist is a nucleic acid molecule binding to CXCL12, wherein the nucleic acid molecule binding to CXCL12 comprises a central stretch of nucleotides, whereby the central stretch of nucleotides comprises the following nucleotide sequence:
5' GUGUGAUCUAGAUGUADWGGCUGWUCCUAGUYAGG 3' (SEQ ID NO: 52).

23. The CXCL12 antagonist for use of any one of claims 1, 3, 5, 6, 8 and 10 to 22, preferably of claim 22 and the interferon for use of claim 2, 4, 5, 7, 9 and 10 to 22, preferably of 22, wherein the CXCL12 antagonist is a nucleic acid molecule binding to CXCL12, wherein the nucleic acid molecule binding to CXCL12 comprises a nucleotide sequence according to any one of SEQ ID NO: 22 to SEQ ID NO: 28 and SEQ ID NO: 5 to SEQ ID NO: 20, or a nucleotide sequence at least 80 % identical thereto,
preferably a nucleotide sequence according to any one of SEQ ID NO: 22, SEQ ID NO: 28, SEQ ID NO: 5, SEQ ID NO: 6, SEQ ID NO: 7 and SEQ ID NO: 16, or a nucleotide sequence at least 80 % identical thereto,
more preferably a nucleotide sequence according to any one of SEQ ID NO: 22 and SEQ ID NO: 28, or a nucleotide sequence at least 80 % identical thereto.

24. The CXCL12 antagonist for use of any one of claims 1, 3, 5, 6, 8 and 10 to 23, preferably of claim 23 and the interferon for use of any one of 2, 4, 5, 7, 9 and 10 to 23, preferably of claim 23, wherein the nucleic acid molecule binding to CXCL12 is an L-ribonucleic acid molecule comprising a nucleotide sequence of
GCGUGGUGUGAUCUAGAUGUAUUGGCUGAUCCUAGUCAGGUACGC (SEQ ID NO: 22), preferably the L-ribonucleic acid molecule comprises a 40 kDa polyethylene glycol moiety, wherein the 40 kDa polyethylene glycol moiety is attached to the 5' end of the nucleotide sequence, preferably the CXCL12 antagonist is NOX-A12.
